# EUROPEAN PATENT SPECIFICATION

(11) **EP 1 768 978 B1**
(45) Date of publication and mention of the grant of the patent: **09.07.2008**
(21) Application number: 05758844.4
(22) Date of filing: 07.07.2005
(51) Int. Cl.: C07D 413/04, C07D 413/14, A61K 31/5377, A61P 35/00

(54) **2,4,6-TRISUBSTITUTED PYRIMIDINES AS PHOSPHOTIDYLINOSITOL (PI) 3-KINASE INHIBITORS AND THEIR USE IN THE TREATMENT OF CANCER**
2,4,6-TRISUBSTITUIERTE PYRIMIDINE ALS INHIBITOREN VON PHOSPHATIDYLINOSIT-(PI-)3-KINASE UND DEREN VERWENDUNG BEI DER BEHANDLUNG VON KREBS
PYRIMIDINES A SUBSTITUTION TRIPLE AUX POSITIONS 2, 4, 6 UTILISES EN TANT QU'INHIBITEURS DE PHOSPHOTIDYLINOSITOL (PI) 3-KINASE ET UTILISATIONS DE CELLES-CI POUR TRAITER UN CANCER

(30) Priority: 09.07.2004 GB 0415365
(43) Date of publication of application: 04.04.2007
(73) Proprietor: AstraZeneca AB, 151 85 Södertälje (SE)
(72) Inventor: BAILEY, John, Peter, Macclesfield Cheshire SK10 4TG (GB); GILES, Michael, Brian, Macclesfield Cheshire SK10 4TG (GB); PASS, Martin, Macclesfield Cheshire SK10 4TG (GB)
(86) International application number: PCT/GB2005/002664
(87) International publication number: WO 2006/005914

(56) References cited:
- EP-A- 1 277 738
- "Inhibition of lysophosphatidic acid acyltransferase-beta (LPAAT-b) by CT-32228 inhibits activation of RAS-RAF-Erk and PI3K/AKT/m-TOR pathways and selectively induces tumor cell apoptosis" EUROPEAN JOURNAL OF CANCER, PERGAMON PRESS, OXFORD, GB, vol. 38, November 2002 (2002-11), page S90, XP004403737 ISSN: 0959-8049
- STEIN, R. C., WATERFIELD, M.D.: "PI3-kinase inhibition: a target for drug development?" MOLECULAR MEDECINE TODAY, vol. 6, no. 9, 1 September 2000 (2000-09-01), pages 347-357, XP002351307

## Description

The invention concerns certain novel pyrimidine derivatives, or pharmaceutically-acceptable salts, solvates or pro-drugs thereof, which possess anti-tumour activity and are accordingly useful in methods of treatment of the human or animal body. The invention also concerns processes for the manufacture of said pyrimidine derivatives, to pharmaceutical compositions containing them and to their use in therapeutic methods, for example in the manufacture of medicaments for use in production of an anti-proliferative effect in a warm-blooded animal such as man.

Many of the current treatment regimes for cell proliferation diseases such as cancer and psoriasis utilise compounds which inhibit DNA synthesis. Such compounds are toxic to cells generally but their toxic effect on rapidly dividing cells such as tumour cells can be beneficial. Alternative approaches to anti-tumour agents which act by mechanisms other than the inhibition of DNA synthesis have the potential to display enhanced selectivity of action.

In recent years it has been discovered that a cell may become cancerous by virtue of the transformation of a portion of its DNA into an oncogene, that is a gene which, on activation, leads to the formation of malignant tumour cells (Bradshaw, Mutagenesis, 1986, 1, 91). Several such oncogenes give rise to the production of peptides which are receptors for growth factors. Activation of the growth factor receptor complex subsequently leads to an increase in cell proliferation. It is known, for example, that several oncogenes encode tyrosine kinase enzymes and that certain growth factor receptors are also tyrosine kinase enzymes (Yarden et al., Ann. Rev. Biochem., 1988, 57, 443; Larsen et al., Ann. Reports in Med. Chem., 1989, Chpt. 13). The first group of tyrosine kinases to be identified arose from such viral oncogenes, for example pp60^{v-Src} tyrosine kinase (otherwise known as v-Src), and the corresponding tyrosine kinases in normal cells, for example pp60^{c-Src} tyrosine kinase (otherwise known as c-Src).

Receptor tyrosine kinases are important in the transmission of biochemical signals which initiate cell replication. They are large enzymes which span the cell membrane and possess an extracellular binding domain for growth factors such as epidermal growth factor (EGF) and an intracellular portion which functions as a kinase to phosphorylate tyrosine amino acids in proteins and hence to influence cell proliferation. Various classes of receptor tyrosine kinases are known (Wilks, Advances in Cancer Research, 1993, 60, 43-73) based on families of growth factors which bind to different receptor tyrosine kinases. The classification includes Class I receptor tyrosine kinases comprising the EGF family of receptor tyrosine kinases such as the EGF, TGFα, Neu and erbB receptors.

It is also known that certain tyrosine kinases belong to the class of non-receptor tyrosine kinases which are located intracellularly and are involved in the transmission of biochemical signals such as those that influence tumour cell motility, dissemination and invasiveness and subsequently metastatic tumour growth. Various classes of non-receptor tyrosine kinases are known including the Src family such as the Src, Lyn, Fyn and Yes tyrosine kinases.

It is also known that certain kinases belong to the class of serine/threonine kinases which are located intracellularly and downstream of tyrosine kinase activation and are involved in the transmission of biochemical signals such as those that influence tumour cell growth. Such serine/threonine signalling pathways include the Raf-MEK-ERK cascade and those downstream of PI3K such as PDK-1, AKT and mTOR (Blume-Jensen and Hunter, Nature, 2001, 411, 355).

It is also known that certain other kinases belong to the class of lipid kinases which are located intracellularly and are also involved in the transmission of biochemical signals such as those that influence tumour cell growth and invasiveness. Various classes of lipid kinases are known including the phosphoinositide 3-kinase (abbreviated hereinafter to PI3K) family that is alternatively known as the phosphatidylinositol-3-kinase family.

It is now well understood that deregulation of oncogenes and tumour-suppressor genes contributes to the formation of malignant tumours, for example by way of increased cell proliferation or increased cell survival. It is also now known that signalling pathways mediated by the PI3K family have a central role in a number of cell processes including proliferation and survival, and deregulation of these pathways is a causative factor a wide spectrum of human cancers and other diseases (Katso et al., Annual Rev. Cell Dev. Biol., 2001, 17: 615-617 and Foster et al., J. Cell Science, 2003, 116: 3037-3040).

The PI3K family of lipid kinases is a group of enzymes that phosphorylate the 3-position of the inositol ring of phosphatidylinositol (abbreviated hereinafter to PI). Three major groups of PI3K enzymes are known which are classified according to their physiological substrate specificity (Vanhaesebroeck et al., Trends in Biol. Sci., 1997, 22, 267). Class III PI3K enzymes phosphorylate PI alone. In contrast, Class II PI3K enzymes phosphorylate both PI and PI 4-phosphate [abbreviated hereinafter to PI(4)P]. Class I PI3K enzymes phosphorylate PI, PI(4)P and PI 4,5-bisphosphate [abbreviated hereinafter to PI(4,5)P2], although only PI(4,5)P2 is believed to be the physiological cellular substrate. Phosphorylation of PI(4,5)P2 produces the lipid second messenger PI 3,4,5-triphosphate [abbreviated hereinafter to PI(3,4,5)P3]. More distantly related members of this superfamily are Class IV kinases such as mTOR and DNA-dependent kinase that phosphorylate serine/threonine residues within protein substrates. The most studied and understood of these lipid kinases are the Class I PI3K enzymes.

Class I PI3K is a heterodimer consisting of a p110 catalytic subunit and a regulatory subunit, and the family is further divided into Class Ia and Class Ib enzymes on the basis of regulatory partners and mechanism of regulation. Class Ia enzymes consist of three distinct catalytic subunits (p110α, p110β and p110δ) that dimerise with five distinct regulatory subunits (p85a, p55α, p50α, p85β and p55γ), with all catalytic subunits being able to interact with all regulatory subunits to form a variety of heterodimers. Class Ia PI3K are generally activated in response to growth factor-stimulation of receptor tyrosine kinases, *via* interaction of the regulatory subunit SH2 domains with specific phospho-tyrosine residues of the activated receptor or adaptor proteins such as IRS-1. Both p110α and p110β are constitutively expressed in all cell types, whereas p110δ expression is more restricted to leukocyte populations and some epithelial cells. In contrast, the single Class Ib enzyme consists of a p110γ catalytic subunit that interacts with a p101 regulatory subunit. Furthermore, the Class Ib enzyme is activated in response to G-protein coupled receptor (GPCR) systems and its expression appears to be limited to leucoccytes.

There is now considerable evidence indicating that Class Ia PI3K enzymes contribute to tumourigenesis in a wide variety of human cancers, either directly or indirectly (Vivanco and Sawyers, Nature Reviews Cancer, 2002, 2*,* 489-501). For example, the p110α subunit is amplified in some tumours such as those of the ovary (Shayesteh *et al.,* Nature Genetics. 1999, 21: 99-102) and cervix (Ma *et al.,* Oncogene, 2000, 19: 2739-2744). More recently, activating mutations within the catalytic site of p110α have been associated with various other tumours such as those of the colorectal region and of the breast and lung (Samuels *et al.,* Science. 2004, 304, 554). Tumour-related mutations in p85α have also been identified in cancers such as those of the ovary and colon (Philp *et al.,* Cancer Research, 2001, 61, 7426-7429). In addition to direct effects, it is believed that activation of Class Ia PI3K contributes to tumourigenic events that occur upstream in signalling pathways, for example by way of ligand-dependent or ligand-independent activation of receptor tyrosine kinases, GPCR systems or integrins (Vara et al., Cancer Treatment Reviews, 2004, 30, 193-204). Examples of such upstream signalling pathways include over-expression of the receptor tyrosine kinase Erb2 in a variety of tumours leading to activation of PI3K-mediated pathways (Harari et al., Oncogene, 2000, 19, 6102-6114) and over-expression of the oncogene Ras (Kauffmann-Zeh et al., Nature, 1997, 385, 544-548). In addition, Class Ia PI3Ks may contribute indirectly to tumourigenesis caused by various downstream signalling events. For example, loss of the effect of the PTEN tumour-suppressor phosphatase that catalyses conversion of PI(3,4,5)P3 back to PI(4,5)P2 is associated with a very broad range of tumours via deregulation of PI3K-mediated production of PI(3,4,5)P3 (Simpson and Parsons, Exp. Cell Res., 2001, 264, 29-41). Furthermore, augmentation of the effects of other PI3K-mediated signalling events is believed to contribute to a variety of cancers, for example by activation of Akt (Nicholson and Anderson, Cellular Signalling, 2002, 14, 381-395).

In addition to a role in mediating proliferative and survival signalling in tumour cells, there is also good evidence that Class Ia PI3K enzymes will also contribute to tumourigenesis via its function in tumour-associated stromal cells. For example, PI3K signalling is known to play an important role in mediating angiogenic events in endothelial cells in response to pro-angiogenic factors such as VEGF (Abid et al., Arterioscler. Thromb. Vasc. Biol., 2004, 24, 294-300). As Class I PI3K enzymes are also involved in motility and migration (Sawyer, Expert Opinion Investig. Drugs, 2004, 13, 1-19), PI3K inhibitors should provide therapeutic benefit via inhibition of tumour cell invasion and metastasis.

In addition, Class I PI3K enzymes play an important role in the regulation of immune cells with PI3K activity contributing to pro-tumourigenic effects of inflammatory cells (Coussens and Werb, Nature, 2002, 420, 860-867).

These findings suggest that pharmacological inhibitors of Class I PI3K enzymes should be of therapeutic value for treatment of the various forms of the disease of cancer comprising solid tumours such as carcinomas and sarcomas and the leukaemias and lymphoid malignancies. In particular, inhibitors of Class I PI3K enzymes should be of therapeutic value for treatment of, for example, cancer of the breast, colorectum, lung (including small cell lung cancer, non-small cell lung cancer and bronchioalveolar cancer) and prostate, and of cancer of the bile duct, bone, bladder, head and neck, kidney, liver, gastrointestinal tissue, oesophagus, ovary, pancreas, skin, testes, thyroid, uterus, cervix and vulva, and of leukaemias (including ALL and CML), multiple myeloma and lymphomas.

Generally, investigators have explored the physiological and pathological roles of the PI3K enzyme family using the PI3K inhibitors LY294002 and wortmannin. Although use of those compounds may suggest a role for PI3K in a cellular event, they are not sufficiently selective within the PI3K family to allow dissection of the individual roles of the family members. For this reason, more potent and selective pharmaceutical PI3K inhibitors would be useful to allow a more complete understanding of PI3K function and to provide useful therapeutic agents.

In addition to tumourigenesis, there is evidence that Class I PI3K enzymes play a role in other diseases (Wymann et al., Trends in Pharmacological Science, 2003, 24, 366-376). Both Class Ia PI3K enzymes and the single Class Ib enzyme have important roles in cells of the immune system (Koyasu, Nature Immunology, 2003, 4, 313-319) and thus they are therapeutic targets for inflammatory and allergic indications. Inhibition of PI3K is also useful to treat cardiovascular disease *via* anti-inflammatory effects or directly by affecting cardiac myocytes (Prasad et al., Trends in Cardiovascular Medicine, 2003, 13, 206-212). Thus inhibitors of Class I PI3K enzymes are expected to be of value in the prevention and treatment of a wide variety of diseases in addition to cancer.

It is disclosed in International Patent Application WO 2004/048365 that certain pyrimidine derivatives possess PI3K enzyme inhibitory activity and are useful in the treatment of cancer. The disclosure focuses on arylamino- and heteroarylamino-substituted pyrimidines. The scope of disclosure does not embrace 2-aryl substituted pyrimidines.

It is disclosed in European Patent Application 1 277 738 that a variety of structures possess PI3K enzyme inhibitory activity and are useful in the treatment of cancer. The disclosure includes mention of 4-morpholino-substituted bicyclic heteroaryl compounds such as quinazoline and pyrido[3,2-*d*]pyrimidine derivatives and 4-morpholino-substituted tricyclic heteroaryl compounds such as compounds described as pyrido[3',2':4,5]furo[3,2-*d*]pyrimidine derivatives. The scope of disclosure does not embrace monocyclic pyrimidine derivatives.

We have now found that surprisingly certain pyrimidine derivatives possess potent anti-tumour activity, being useful in inhibiting the uncontrolled cellular proliferation which arises from malignant disease. Without wishing to imply that the compounds disclosed in the present invention possess pharmacological activity only by virtue of an effect on a single biological process, it is believed that the compounds provide an anti-tumour effect by way of inhibition of Class I PI3K enzymes, particularly by way of inhibition of the Class Ia PI3K enzymes and/or the Class Ib PI3K enzyme, more particularly by way of inhibition of the Class Ia PI3K enzymes.

The compounds of the present invention are also useful in inhibiting the uncontrolled cellular proliferation which arises from various non-malignant diseases such as inflammatory diseases (for example rheumatoid arthritis and inflammatory bowel disease), fibrotic diseases (for example hepatic cirrhosis and lung fibrosis), glomerulonephritis, multiple sclerosis, psoriasis, benign prostatic hypertrophy (BPH), hypersensitivity reactions of the skin, blood vessel diseases (for example atherosclerosis and restenosis), allergic asthma, insulin-dependent diabetes, diabetic retinopathy and diabetic nephropathy.

Generally, the compounds of the present invention possess potent inhibitory activity against Class I PI3K enzymes, particularly against Class Ia PI3K enzymes, whilst possessing less potent inhibitory activity against tyrosine kinase enzymes such as the receptor tyrosine kinases, for example EGF receptor tyrosine kinase and/or VEGF receptor tyrosine kinase, or against non-receptor tyrosine kinases such as Src. Furthermore, certain compounds of the present invention, possess substantially better potency against Class I PI3K enzymes, particularly against Class Ia PI3K enzymes, than against EGF receptor tyrosine kinase or VEGF receptor tyrosine kinase or Src non-receptor tyrosine kinase. Such compounds possess sufficient potency against Class I PI3K enzymes that they may be used in an amount sufficient to inhibit Class I PI3K enzymes, particularly to inhibit Class Ia PI3K enzymes, whilst demonstrating little activity against EGF receptor tyrosine kinase or VEGF receptor tyrosine kinase or Src non-receptor tyrosine kinase.

According to one aspect of the invention there is provided a pyrimidine derivative of the Formula I 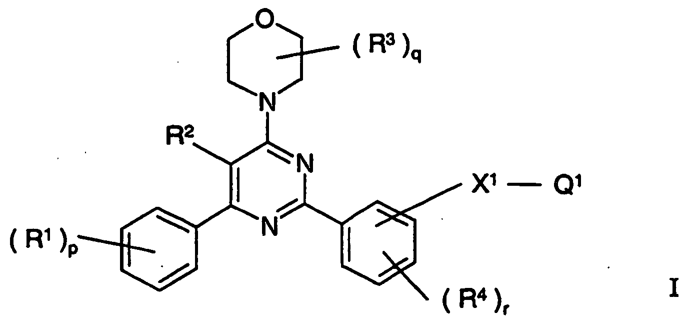 wherein **p** is 1, 2 or 3;
each **R¹ group**, which may be the same or different, is selected from halogeno, trifluoromethyl, cyano, isocyano, nitro, hydroxy, mercapto, amino, formyl, carboxy, carbamoyl, ureido, (1-8C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, *N-*(1-6C)alkylcarbamoyl, *N,N-*di-[(1-6C)alkyl]carbamoyl, (2-6C)alkanoyl, (2-6C)alkanoyloxy, (2-6C)alkanoylamino, *N*-(1-6C)alkyl-(2-6C)alkanoylamino, (3-6C)alkenoylamino, *N*-(1-6C)alkyl-(3-6C)alkenoylamino, (3-6C)alkynoylamino, *N*-(1-6C)alkyl-(3-6C)alkynoylamino, *N*'-(1-6C)alkylureido, *N*',*N*'-di-[(1-6C)alkyl]ureido, *N*-(1-6C)alkylureido, *N,N*'-di-[(1-6C)alkyl]ureido, *N,N',N*'-tri-[(1-6C)alkyl]ureido, *N*-(1-6C)alkylsulphamoyl, *N,N*-di-[(1-6C)alkyl]sulphamoyl, (1-6C)alkanesulphonylamino and *N*-(1-6C)alkyl-(1-6C)alkanesulphonylamino, or from a group of the formula:

Q²-X²-

wherein X² is a direct bond or is selected from O, S, SO, SO₂, N(R⁵), CO, CH(OR⁵), CON(R⁵), N(R⁵)CO, N(R⁵)CON(R⁵), SO₂N(R⁵), N(R⁵)SO₂, OC(R⁵)₂, SC(R⁵)₂ and N(R⁵)C(R⁵)₂, wherein R⁵ is hydrogen or (1-8C)alkyl, and Q² is aryl, aryl-(1-6C)alkyl, (3-8C)cycloallcyl, (3-8C)cycloalkyl-(1-6C)alkyl, (3-8C)cycloalkenyl, (3-8C)cycloalkenyl-(1-6P)alkyl, heteroaryl, heteroaryl-(1-6C)alkyl, heterocyclyl or heterocyclyl-(1-6C)alkyl, or (R¹)ₚ is (1-3C)alkylenedioxy,
and wherein any CH, CH₂ or CH₃ group within a R¹ substituent optionally bears on each said CH, CH₂ or CH₃ group one or more halogeno or (1-8C)alkyl substituents and/or a substituent selected from hydroxy, mercapto, amino, cyano, carboxy, carbamoyl, ureido, (1-6C)alkoxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, *N*-(1-6C)alkylcarbamoyl, *N,N*-di-[(1-6C)alkyl]carbamoyl, (2-6C)alkanoyl, (2-6C)alkanoyloxy, (2-6C)allcanoylamino, *N*-(1-6C)alkyl-(2-6C)alkanoylamino, *N*-(1-6C)alkylureido, *N*'-(1-6C)alkylureido, *N',N'*-di-[(1-6C)alkyl]ureido, *N,N*'-di-[(1-6C)alkyl]ureido, *N,N',N*'-tri-[(1-6C)alkyl]ureido, *N*-(1-6C)alkylsulphamoyl, *N,N*-di-[(1-6C)alkyl]sulphamoyl, (1-6C)alkanesulphonylamino and *N*-(1-6C)alkyl-(1-6C)alkanesulphonylamino, or from a group of the formula:

-X³-Q³

wherein X³ is a direct bond or is selected from O, S, SO, SO₂, N(R⁶), CO, CH(OR⁶), CON(R⁶), N(R⁶)CO, N(R⁶)CON(R⁶), SO₂N(R⁶), N(R⁶)SO₂, C(R⁶)₂O, C(R⁶)₂S and C(R⁶)₂N(R⁶), wherein R⁶ is hydrogen or (1-8C)alkyl, and Q³ is aryl, aryl-(1-6C)allcyl, (3-8C)cycloallcyl, (3-8C)cycloallcyl-(1-6C)alkyl, (3-8C)cycloalkenyl, (3-8C)cycloalkenyl-(1-6C)alkyl, heteroaryl, heteroaryl-(1-6C)alkyl, heterocyclyl or heterocyclyl-(1-6C)alkyl,
and wherein any aryl, (3-8C)cycloalkyl, (3-8C)cycloalkenyl, heteroaryl or heterocyclyl group within a substituent on R¹ optionally bears 1, 2 or 3 substituents, which may be the same or different, selected from halogeno, trifluoromethyl, cyano, nitro, hydroxy, amino, carboxy, carbamoyl, ureido, (1-8C)alkyl, (2-8C)allcenyl, (2-8C)alkynyl, (1-6C)alkoxy, (2-6C)alkenyloxy, (2-6C)alkynyloxy, (1-6C)alkylthio, (1-6C)alkylsulphinyl, (1-6C)allcylsulphonyl, (1-6C)alkylamino, di-[(1-6C)alkyl]amino, (1-6C)alkoxycarbonyl, (2-6C)alkanoyl, (2-6C)alkanoyloxy, *N-*(1-6C)alkylcarbamoyl, *N,N-*di-[(1-6C)alkyl]carbamoyl, (2-6C)alkanoylamino, *N-*(1-6C)alkyl-(2-6C)alkanoylamino, *N*-(1-6C)alkylureido, *N*'-(1-6C)alkylureido, *N'*,*N'*-di-[(1-6C)alkyl]ureido, *N*,*N*'-di-[(1-6C)alkyl]ureido, *N*,*N'*,*N*'-tri-[(1-6C)alkyl]ureido, *N-*(1-6C)alkylsulphamoyl, *N,N* di-[(1-6C)alkyl]sulphamoyl, (1-6C)alkanesulphonylamino and *N*-(1-6C)alkyl-(1-6C)alkanesulphonylamino, or from a group of the formula:

-X⁴-R⁷

wherein X⁴ is a direct bond or is selected from O and N(R⁸), wherein R⁸ is hydrogen or (1-8C)alkyl, and R⁷ is halogeno-(1-6C)alkyl, hydroxy-(1-6C)alkyl, mercapto-(1-6C)alkyl, (1-6C)alkoxy-(1-6C)alkyl, (1-6C)alkylthio-(1-6C)alkyl, cyano-(1-6C)alkyl, amino-(1-6C)alkyl, (1-6C)alkylamino-(1-6C)alkyl, di-[(1-6C)alkyl]amino-(1-6C)alkyl, (2-6C)alkamoylamino-(1-6C)alkyl, (1-6C)alkoxycarbonylamino-(1-6C)alkyl, *N*-(1-6C)alkylureido-(1-6C)alkyl, *N*'-(1-6C)alkylureido-(1-6C)alkyl, *N',N'*-di-[(1-6C)alkyl]ureido-(1-6C)alkyl, *N,N'*-di-[(1-6C)alkyl]ureido-(1-6C)alkyl or *N,N'*,*N'*-tri-[(1-6C)alkyl]ureido-(1-6C)alkyl, or from a group of the formula:

- X⁵-Q⁴

wherein X⁵ is a direct bond or is selected from O, CO and N(R⁹), wherein R⁹ is hydrogen or (1-8C)alkyl, and Q⁴ is aryl, aryl-(1-6C)alkyl, heteroaryl, heteroaryl-(1-6C)alkyl, heterocyclyl or heterocyclyl-(1-6C)alkyl which optionally bears 1 or 2 substituents, which may be the same or different, selected from halogeno, hydroxy, (1-8C)alkyl and (1-6C)alkoxy,
and wherein any heterocyclyl group within a substituent on R¹ optionally bears 1 or 2 oxo or thioxo substituents,
and wherein adjacent carbon atoms in any (2-6C)alkylene chain within a R¹ substituent are optionally separated by the insertion into the chain of a group selected from O, S, SO, SO₂, N(R¹⁰), CO, CH(OR¹⁰), CON(R¹⁰), N(R¹⁰)CO, N(R¹⁰CON(R¹⁰), SO₂N(R¹⁰), N(R¹⁰)SO₂, CH=CH and C≡C wherein R¹⁰ is hydrogen or (1-8C)alkyl;
**R²** is hydrogen or (1-8C)alkyl;
**q** is 0,1,2,3 or 4;
**each R³ group**, which may be the same or different, is (1-8C)alkyl or a group of the formula:

-X⁶-R¹¹

wherein X⁶ is a direct bond or is selected from O and N(R¹²), wherein R¹² is hydrogen or (1-8C)alkyl, and R¹¹ is halogeno-(1-6C)alkyl, hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl, di-[(1-6C)alkyl]amino-(1-6C)alkyl or (2-6C)alkanoylamino-(1-6C)alkyl;
**r** is 0, 1 or 2;
**each R⁴ group,** which may be the same or different, is selected from halogeno, trifluoromethyl, cyano, nitro, hydroxy, mercapto, amino, carboxy, carbamoyl, ureido, (1-8C)alkyl, (2-8C)alkenyl, (2-8C)alkynyl, (1-6C)alkoxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, *N*-(1-6C)alkylcarbamoyl, *N,N*-di-[(1-6C)alkyl]carbamoyl, (2-6C)alkanoyl, (2-6C)alkanoyloxy, (2-6C)alkanoylamino, *N*-(1-6C)alkyl-(2-6C)alkanoylamino, *N*'-(1-6C)alkylureido, *N',N*'-di-[(1-6C)alkyl]ureido, *N*-(1-6C)alkylureido, *N,N'*-di-[(1-6C)alkyl]ureido, *N,N',N*'-tri-[(1-6C)alkyl]ureido, *N*-(1-6C)alkylsulphamoyl, *N,N*-di-[(1-6C)alkyl]sulphamoyl, (1-6C)alkanesulphonylamino and *N*-(1-6C)alkyl-(1-6C)alkanesulphonylamino;
**X¹** is selected from CO, N(R¹³)CO, CON(R¹³), N(R¹³)CON(R¹³), N(R¹³)COC(R¹³)₂O, N(R¹³)COC(R¹³)₂S , N(R¹³)COC(R¹³)₂N(R¹³) and N(R¹³)COC(R¹³)₂N(R¹³)CO, wherein R¹³ is hydrogen or (1-8C)alkyl; and
**Q¹** is (1-8C)alkyl, (2-8C)allcenyl, (2-8C)alkynyl, halogeno-(1-6C)allryl, hydroxy-(1-6C)alkyl, mercapto-(1-6C)alkyl, (1-6C)alkoxy-(1-6C)alkyl, cyano-(1-6C)alkyl, amino-(1-6C)alkyl, (1-6C)alkylamino-(1-6C)alkyl, di-[(1-6C)alkyl]amino-(1-6C)alkyl, (1-6C)alkylthio-(1-6C)alkyl, (1-6C)alkylsulphinyl-(1-6C)alkyl, (1-6C)alkylsulphonyl-(1-6C)alkyl, (2-6C)alkanoylamino-(1-6C)alkyl, *N*-(1-6C)alkyl-(2-6C)alkanoylamino-(1-6C)alkyl, (1-6C)alkoxycarbonylamino-(1-6C)alkyl, *N*-(1-6C)alkylureido-(1-6C)alkyl, *N*'-(1-6C)alkylureido-(1-6C)alkyl, *N',N'*-di-[(1-6C)alkyl]ureido-(1-6C)alkyl, *N*,*N'*-di-[(1-6C)alkyl]ureido-(1-6C)alkyl, *N,N'*,*N*-tri-[(1-6C)alkyl]ureido-(1-6C)alkyl, (1-6C)alkanesulphonylamino-(1-6C)alkyl or *N*-(1-6C)alkyl-(1-6C)alkanesulphonylamino-(1-6C)alkyl, or **Q¹** is aryl, aryl-(1-6C)alkyl, (3-8C)cycloalkyl, (3-8C)cycloalkyl-(1-6C)alkyl, (3-8C)cycloalkenyl, (3-8C)cycloalkenyl-(1-6C)alkyl, heteroaryl, heteroaryl-(1-6C)alkyl, heterocyclyl or heterocyclyl-(1-6C)alkyl,
and wherein any CH, CH₂ or CH₃ group within the Q¹ group optionally bears on each said CH, CH₂ or CH₃ group one or more halogeno or (1-8C)alkyl substituents and/or a substituent selected from hydroxy, mercapto, amino, cyano, carboxy, carbamoyl, ureido, (1-6C)alkoxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, *N*-(1-6C)alkylcarbamoyl, *N,N*-di-[(1-6C)alkyl]carbamoyl, (2-6C)alkanoyl, (2-6C)alkanoyloxy, (2-6C)alkanoylamino, *N*-(1-6C)alkyl-(2-6C)alkanoylamino, *N'*-(1-6C)alkylureido, *N'*,*N'*-di-[(1-6C)alkyl]ureido, *N*-(1-6C)alkylureido, *N,N'*-di-[(1-6C)alkyl]ureido, *N*,*N'*,*N'*-tri-[(1-6C)alkyl]ureido, *N*-(1-6C)alkylsulphamoyl, *N*-(1-6C)alkylsulphamoyl, *N,N*-di-[(1-6C)alkyl]sulphamoyl, (1-6C)alkanesulphonylamino and *N*-(1-6C)alkyl-(1-6C)alkanesulphonylamino,
and wherein any aryl, (3-8C)cycloalkyl, (3-8C)cycloalkenyl, heteroaryl or heterocyclyl group within the Q¹ group optionally bears 1, 2 or 3 substituents, which may be the same or different, selected from halogeno, trifluoromethyl, cyano, nitro, hydroxy, amino, carboxy, carbamoyl, ureido, (1-8C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, . (2-6C)alkanoyl, (2-6C)alkanoyloxy, *N*-(1-6C)alkylcarbamoyl, *N*,*N*-di-[(1-6C)alkyl]carbamoyl, (2-6C)alkanoylamino, *N*-(1-6C)alkyl-(2-6C)alkanoylamino, *N*'-(1-6C)alkylureido, *N*',*N*'-di-[(1-6C)alkyl]ureido, *N*-(1-6C)alkylureido, *N*,*N*'-di-[(1-6C)alkyl]ureido, *N*,*N*',*N*'-tri-[(1-6C)alkyl]ureido, *N*-(1-6C)alkylsulphamoyl, *N*,*N*-di-[(1-6C)alkyl]sulphamoyl, (1-6C)alkanesulphonylamino and *N*-(1-6C)alkyl-(1-6C)alkanesulphonylamino, or from a group of the formula:

-X⁷-R¹⁴

wherein X⁷ is a direct bond or is selected from O and N(R¹⁵), wherein R¹⁵ is hydrogen or (1-8C)alkyl, and R¹⁴ is halogeno-(1-6C)alkyl, hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl or di-[(1-6C)alkyl]amino-(1-6C)alkyl, or from a group of the formula:

-X⁸-Q⁵

wherein X⁸ is a direct bond or is selected from O, CO and N(R¹⁷), wherein R¹⁷ is hydrogen or (1-8C)alkyl, and Q⁵ is aryl, aryl-(1-6C)alkyl, heteroaryl, heteroaryl-(1-6C)alkyl, heterocyclyl or heterocyclyl-(1-6C)alkyl which optionally bears 1 or 2 substituents, which may be the same or different, selected from halogeno, hydroxy, (1-8C)alkyl and (1-6C)alkoxy,
and wherein any heterocyclyl group within the Q¹ group optionally bears 1 or 2 oxo or thioxo substituents,
and wherein adjacent carbon atoms in any (2-6C)alkylene chain within the Q¹ group are optionally separated by the insertion into the chain of a group selected from O, S, SO, SO₂ N(R¹⁶), N(R¹⁶)CO, CON(R¹⁶), N(R¹⁶)CON(R¹⁶), CO, CH(OR¹⁶), N(R¹⁶SO₂, SO₂N(R¹⁶), CH=CH and C≡C wherein R¹⁶ is hydrogen or (1-8C)alkyl; or a pharmaceutically-acceptable salt, solvate or pro-drug thereof.

In this specification the generic term "(1-8C)alkyl" includes both straight-chain and branched-chain alkyl groups such as propyl, isopropyl and tert-butyl, and also (3-8C)cycloalkyl groups such as cyclopropyl, cyclobutyl, cyclopentyl, cyclohexyl and cycloheptyl, and also (3-6C)cycloalkyl-(1-2C)alkyl groups such as cyclopropylmethyl, 2-cyclopropylethyl, cyclobutylmethyl, 2-cyclobutylethyl, cyclopentylmethyl, 2-cyclopentylethyl, cyclohexylmethyl and 2-cyclohexylethyl. However references to individual alkyl groups such as "propyl" are specific for the straight-chain version only, references to individual branched-chain alkyl groups such as "isopropyl" are specific for the branched-chain version only and references to individual cycloalkyl groups such as "cyclopentyl" are specific for that 5-membered ring only. An analogous convention applies to other generic terms, for example (1-6C)alkoxy includes (3-6C)cycloalkyloxy groups and (3-SC)cycloalkyl-(1-2C)alkoxy groups, for example methoxy, ethoxy, propoxy, isopropoxy, cyclopropyloxy, cyclobutyloxy, cyclopentyloxy, cyclohexyloxy, cyclopropylmethoxy, 2-cyclopropylethoxy, cyclobutylmethoxy, 2-cyclobutylethoxy and cyclopentylmethoxy; (1-6C)alkylamino includes (3-6C)cycloallcylamino groups and (3-5C)cycloalkyl-(1-2C)alkylamino groups, for example methylamino, ethylamino, propylamino, cyclopropylamino, cyclobutylamino, cyclohexylamino, cyclopropylmethylamino, 2-cyclopropylethylamino, cyclobutylmethylamino, 2-cyclobutylethylamino and cyclopentylmethylamino; and di-[(1-6Calkyl]amino includes di-[(3-6C)cycloalkyl]amino groups and di-[(3-5C)cycloalkyl-(1-2C)alkyl]amino groups, for example dimethylamino, diethylamino, dipropylamino, *N*-cyclopropyl-*N*-methylamino, *N*-cyclobutyl-*N*-methylamino, *N*-cyclohexyl-*N*-ethylamino, *N*-cyclopropylmethyl-*N*-methylamino, *N*-(2-cyclopropylethyl)-*N*-methylamino and *N*-cyclopentylmethyl-*N-*methylamino.

It is to be understood that, insofar as certain of the compounds of Formula I defined above may exist in optically active or racemic forms by virtue of one or more asymmetric carbon atoms, the invention includes in its definition any such optically active or racemic form which possesses the above-mentioned activity. The synthesis of optically active forms may be carried out by standard techniques of organic chemistry well known in the art, for example by synthesis from optically active starting materials or by resolution of a racemic form. Similarly, the above-mentioned activity may be evaluated using the standard laboratory techniques referred to hereinafter.

It is to be understood that certain compounds of Formula I defined above may exhibit the phenomenon of tautomerism. In particular, tautomerism may affect heterocyclic groups within the R¹ and Q¹ groups that bear 1 or 2 oxo or thioxo substituents. It is to be understood that the present invention includes in its definition any such tautomeric form, or a mixture thereof, which possesses the above-mentioned activity and is not to be limited merely to any one tautomeric form utilised within the formulae drawings or named in the Examples.

It is to be understood that the -X¹-Q¹ group may be located at any available position on the phenyl group that is located at the 2-position on the pyrimidine ring. Conveniently, the -X¹-Q¹ group is located at the 3- or 4-position on said phenyl group. More conveniently, the -X¹-Q¹ group is located at the 3-position on said phenyl group.

It is further to be understood that any R¹ group that is present on the phenyl group that is located at the 4-position on the pyrimidine ring may be located at any available position on said phenyl group. When multiple R¹ groups are present, the R¹ groups may be the same or different. Conveniently, there is a single R¹ group. More conveniently, the single R¹ group is located at the 3-position on said phenyl group.

It is further to be understood that any R³ group that may be present on the morpholinyl group that is located at the 6-position on the pyrimidine ring may be located at any available position on said morpholinyl group. Conveniently, there is a single R³ group. More conveniently, no R³ group is present (q=0).

It is further to be understood that any R⁴ group that may be present on the phenyl group that is located at the 2-position on the pyrimidine ring may be located at any available position on said phenyl group. Conveniently, there is a single R⁴ group. More conveniently, no R⁴ group is present (r=0).

Suitable values for the generic radicals referred to above include those set out below.

A suitable value for any one of the 'Q' groups (Q¹ to Q⁵) when it is aryl or for the aryl group within a 'Q' group is, for example, phenyl or naphthyl, preferably phenyl.

A suitable value for any one of the 'Q' groups (Q¹ to Q³) when it is (3-8C)cycloalkyl or for the (3-8C)cycloalkyl group within a 'Q' group is, for example, cyclopropyl, cyclobutyl, cyclopentyl, cyclohexyl, cycloheptyl, bicyclo[2.2.1]heptyl or cyclooctyl and a suitable value for any one of the 'Q' groups (Q¹ to Q³) when it is (3-8C)cycloalkenyl or for the (3-8C)cycloalkenyl group within a 'Q' group is, for example, cyclobutenyl, cyclopentenyl, cyclohexenyl, cycloheptenyl or cyclooctenyl.

A suitable value for any one of the 'Q' groups (Q¹ to Q⁵) when it is heteroaryl or for the heteroaryl group within a 'Q' group is, for example, an aromatic 5- or 6-membered monocyclic ring or a 9- or 10-membered bicyclic ring with up to five ring heteroatoms selected from oxygen, nitrogen and sulphur, for example furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazenyl, benzofuranyl, indolyl, benzothienyl, benzoxazolyl, benzimidazolyl, benzothiazolyl, indazolyl, benzofurazanyl, quinolyl, isoquinolyl, quinazolinyl, quinoxalinyl, cinnolinyl or naphthyridinyl.

A suitable value for any one of the 'Q' groups (Q¹ to Q⁵) when it is heterocyclyl or for the heterocyclyl group within a 'Q' group is, for example, a non-aromatic saturated or partially saturated 3 to 10 membered monocyclic or bicyclic ring with up to five heteroatoms selected from oxygen, nitrogen and sulphur, for example oxiranyl, oxetanyl, tetrahydrofuranyl, tetrahydropyranyl, oxepanyl, tetrahydrothienyl, 1,1-dioxotetrahydrothienyl, tetrahydrothiopyranyl, 1,1-dioxotetrahydrothiopyranyl, azetidinyl, pyrrolinyl, pyrrolidinyl, imidazolinyl, imidazolidinyl, pyrazolinyl, pyrazolidinyl, morpholinyl, tetrahydro-1,4-thiazinyl, 1,1-dioxotetrahydro-1,4-thiazinyl, piperidinyl, homopiperidinyl, piperazinyl, homopiperazinyl, 2-azabicyclo[2.2.1]heptyl, quinuclidinyl, chromanyl, isochromanyl, indolinyl, isoindolinyl, dihydropyridinyl, tetrahydropyridinyl, dihydropyrimidinyl or tetrahydropyrimidinyl, preferably tetrahydrofuranyl, tetrahydropyranyl, tetrahydrothiopyranyl, pyrrolinyl, pyrrolidinyl, morpholinyl, piperidinyl, piperazinyl, indolinyl or isoindolinyl. A suitable value for such a group which bears 1 or 2 oxo or thioxo substituents is, for example, 2-oxopyrrolidinyl, 2-thioxopyrrolidinyl, 2-oxoimidazolidinyl, 2-thioxoimidazolidinyl, 2-oxopiperidinyl, 4-oxo-1,4-dihydnopyridinyl, 2,5-dioxopyaolidinyl, 2,5-dioxoimidazolidinyl or 2,6-dioxopiperidinyl.

A suitable value for a 'Q' group when it is heteroaryl-(1-6C)alkyl is, for example, heteroarylmethyl, 2-heteroarylethyl and 3-heteroarylpropyl. The invention comprises corresponding suitable values for 'Q' groups when, for example, rather than a heteroaryl-(1-6C)alkyl group, an aryl-(1-6C)alkyl, (3-8C)cycloalkyl-(1-6C)alkyl, (3-8C)cycloalkenyl-(1-6C)alkyl or heterocyclyl-(1-6C)alkyl group is present.

Suitable values for any of the 'R' groups (R¹ to R¹⁷), or for various groups within an R¹, R³ or R⁴ substituent, or for Q¹, or for various groups within Q¹ include:-
- for halogeno: fluoro, chloro, bromo and iodo;
- for (1-8C)alkyl:: methyl, ethyl, propyl, isopropyl, tert-butyl, cyclobutyl, cyclohexyl, cyclohexylmethyl and 2-cyclopropylethyl;
- for (2-8C)alkenyl:: vinyl, isopropenyl, allyl and but-2-enyl;
- for (2-8C)alkynyl:: ethynyl, 2-propynyl and but-2-ynyl;
- for (1-6C)alkoxy:: methoxy, ethoxy, propoxy, isopropoxy and butoxy;
- for (2-6C)alkenyloxy:: vinyloxy and allyloxy;
- for (2-6C)alkynyloxy:: ethynyloxy and 2-propynyloxy;
- for (1-6C)alkylthio:: methylthio, ethylthio and propylthio;
- for (1-6C)alkylsulphinyl:: methylsulphinyl and ethylsulphinyl;
- for (1-6C)alkylsulphonyl:: methylsulphonyl and ethylsulphonyl;
- for (1-6C)alkylamino:: methylamino, ethylamino, propyl amino, isopropylamino and butylamino;
- for di-[(1-6C)alkyl]amino:: dimethylamino, diethylamino, *N*-ethyl-*N*-methylamino and diisopropylamino;
- for (1-6C)alkoxycarbonyl:: methoxycarbonyl, ethoxycarbonyl, propoxycarbonyl and *tert*-butoxycarbonyl;
- for *N-*(1-6C)alkylcarbamoyl:: *N*-methylcarbamoyl, *N*-ethylcarbamoyl and *N*-propylcarbamoyl;
- for *N*,*N*-di-[(1-6C)alkyl]carbamoyl:: *N*,*N*-dimethylcarbamoyl, *N*-ethyl-*N*-methylcarbamoyl and *N*,*N*-diethylcarbamoyl;
- for (2-6C)alkanoyl:: acetyl, propionyl and isobutyryl;
- for (2-6C)alkanoyloxy:: acetoxy and propionyloxy;
- for (2-6C)alkanoylamino:: acetamido and propionamido;
- for *N*-(1-6C)alkyl-(2-6C)alkanoylamino:: *N*-methylacetamido and *N*-methylpropionamido;
- for (3-6C)alkenoylamino:: acrylamido, methacrylamido and crotonamido;
- for *N* (1-6C)alkyl-(3-6C)alkenoylamino:: *N*-methylacrylamido and *N*-methylcrotonamido;
- for (3-6C)alkynoylamino:: propiolamido;
- for *N*-(1-6C)alkyl-(3-6C)alkynoylamino:: *N*-methylpropiolamido;
- for *N*'-(1-6C)alkylureido:: *N*'-methylureido and *N*'-ethylureido;
- for *N*',*N*'-di-[(1-6C)alkyl]ureido:: *N*',*N*'-dimethylureido and *N*'-methyl-*N*'-ethylureido;
- for *N*-(1-6C)alkylureido:: *N*-methylureido and *N*-ethylureido;
- for *N*,*N'*-di-[(1-6C)alkyl]ureido:: *N,N'*-dimethylureido, *N*-methyl-*N'*-ethylureido and *N*-ethyl-*N'*-methylureido;
- for *N,N',N'*-di-[(1-6C)alkyl]ureido:: *N,N',N'*-trimethylureido, *N*-ethyl-*N*',*N*'-dimethylureido and *N*-methyl-*N*',*N*'-diethylureido;
- for *N-*(1-6C)alkylsulphamoyl:: *N*-methylsulphamoyl and *N*-ethylsulphamoyl;
- for *N*,*N*-di-[(1-6C)alkyl]sulphamoyl:: *N*,*N*-dimethylsulphamoyl;
- for (1-6C)alkanesulphonylamino:: methanesulphonylamino and ethanesulphonylamino;
- for *N*-(1-6C)alkyl-(1-6C)alkanesulphonylamino:: *N*-methylmethanesulphonylamino and *N*-methylethanesulphonylamino;
- for halogeno-(1-6C)alkyl:: chloromethyl, 2-fluoroethyl, 2-chloroethyl, 1-chloroethyl, 2,2-difluoroethyl, 2,2,2-trifluoroethyl, 3-fluoropropyl, 3-chloropropyl, 3,3-difluoropropyl and 3,3,3-trifluoropropyl;
- for hydroxy-(1-6C)alkyl:: hydroxymethyl, 2-hydroxyethyl, 1-hydroxyethyl and 3-hydroxypropyl;
- for mercapto-(1-6C)alkyl:: mercaptomethyl, 2-mercaptoethyl, 1-mercaptoethyl and 3-mercaptopropyl;
- for (1-6C)alkoxy-(1-6C)alkyl:: methoxymethyl, ethoxymethyl, 1-methoxyethyl, 2-methoxyethyl, 2-ethoxyethyl and 3-methoxypropyl;
- for (1-6C)alkylthio-(1-6C)alkyl:: methylthiomethyl, ethylthiomethyl, 2-methylthioethyl, 1-methylthioethyl and 3-methylthiopropyl;
- for (1-6C)alkylsulphinyl-(1-6C)alkyl:: methylsulphinylmethyl, ethylsulphinylmethyl, 2-methylsulphinylethyl, 1-methylsulphinylethyl and 3-methylsulphinylpropyl;
- for (1-6C)alkylsulphonyl-(1-6C)alkyl:: methylsulphonylmethyl, ethylsulphonylmethyl, 2-methylsulphonylethyl, 1-methylsulphonylethyl and 3-methylsulphonylpropyl;
- for cyano-(1-6C)alkyl:: cyanomethyl, 2-cyanoethyl, 1-cyanoethyl and 3-cyanopropyl;
- for amino-(1-6C)alkyl:: aminomethyl, 2-aminoethyl, 1-aminoethyl, 3-aminopropyl, 1-aminopropyl and 5-aminopropyl;
- for (1-6C)alkylamino-(1-6C)alkyl:: methylaminomethyl, ethylaminomethyl, 1-methylaminoethyl, 2-methylaminoethyl, 2-ethylaminoethyl and 3-methylaminopropyl;
- for di-[(1-6C)alkyl]amino-(1-6C)alkyl:: dimethylaminomethyl, diethylaminomethyl, 1-dimethylaminoethyl, 2-dimethylaminoethyl and 3-dimethylaminopropyl;
- for (2-6C)alkanoylamino-(1-6C)alkyl:: acetamidomethyl, propionamidomethyl, 2-acetamidoethyl and 1-acetamidoethyl;
- for *N*-(1-6C)alkyl-(2-6C)alkanoylamino-(1-6C)alkyl:: *N*-methylacetamidomethyl, *N*-methylpropionamidomethyl, 2-(*N*-methylacetamido)ethyl and 1-(*N*-methylacetamido)ethyl;
- for (1-6C)alicoxycarbonylamino-(1-6C)alkyl:: methoxycarbonylaminomethyl, ethoxycarbonylaminomethyl, *tert*-butoxycarbonylaminomethyl and 2-methoxycarbonylaminoethyl;
- for *N*'-(1-6C)alkylureido-(1-6C)alkyl:: *N*'-methylureidomethyl, 2-(*N*'-methylureido)ethyl and 1-(*N'*-methylureido)ethyl;
- for *N*',*N*'-di-[(1-6C)alkyl]ureido-(1-6C)alkyl:: *N',N'*-dimethylureidomethyl, 2-(*N',N'*-dimethylureido)ethyl and 1-(*N*',*N*'-dimethylureido)ethyl;
- for *N*-(1-6C)alkylureido-(1-6C)alkyl:: *N*-methylureidomethyl, 2-(*N*-methylureido)ethyl and 1-(*N*-methylureido)ethyl;
- for *N,N'*-di-[(1-6C)alkyl]ureido-(1-6C)alkyl:: *N,N'*-dimethylureidomethyl, 2-(*N,N'*-dimethylureido)ethyl and 1-(*N,N'*-dimethylureido)ethyl;
- for *N,N',N'-*di-[(1-6C)alkyl]ureido-(1-6C)alkyl:: *N,N',N'*-trimethylureidomethyl, *2-*(*N,N',N'-*trimethylureido)ethyl and 1*-(N,N',N'*-trimethylureido)ethyl;
- for (1-6C)alkanesulphonylamino-(1-6C)alkyl:: methanesulphonylaminomethyl, 2-(methanesulphonylamino)ethyl and 1-(methanesulphonylamino)ethyl; and
- for *N*-(1-6C)alkyl-(1-6C)alkanesulphonylamino-(1-6C)alkyl:: *N*-methylmethanesulphonylaminomethyl, 2-(*N*-methylmethanesulphonylamino)ethyl and 1-(*N*-methylmethanesulphonylamino)ethyl.

A suitable value for (R¹)ₚ when it is a (1-3C)alkylenedioxy group is, for example, methylenedioxy, ethylidenedioxy, isopropylidenedioxy or ethylenedioxy and the oxygen atoms thereof occupy adjacent ring positions.

When, as defined hereinbefore, an R¹ group forms a group of the formula Q²-X²- and, for example, X² is a OC(R⁵)₂ linking group, it is the carbon atom, not the oxygen atom, of the OC(R⁵)₂ linking group which is attached to the phenyl ring and the oxygen atom is attached to the Q² group. Similarly, when, for example a CH₃ group within a R¹ substituent bears a group of the formula -X³-Q³ and, for example, X³ is a C(R⁶)₂O linking group, it is the carbon atom, not the oxygen atom, of the C(R⁶)₂O linking group which is attached to the CH₃ group and the oxygen atom is linked to the Q³ group.

As defined hereinbefore, adjacent carbon atoms in any (2-6C)alkylene chain within a R¹ substituent may be optionally separated by the insertion into the chain of a group such as O, CON(R¹⁰) or C≡C. For example, insertion of an O atom into the alkylene chain within a 4-methoxybutoxy group gives rise to, for example, a 2-(2-methoxyethoxy)ethoxy group, for example, insertion of a C≡C group into the ethylene chain within a 2-hydroxyethoxy group gives rise to a 4-hydroxybut-2-ynyloxy group and, for example, insertion of a CONH group into the ethylene chain within a 3-methoxypropoxy group gives rise to, for example, a 2-(2-methoxyacetamido)ethoxy group.

When, as defined hereinbefore, any CH, CH₂ or CH₃ group within a R¹ substituent optionally bears on each said CH, CH₂ or CH₃ group one or more halogeno or (1-8C)alkyl substituents, there is suitably 1 halogeno or (1-8C)alkyl substituent present on each said CH group, there are suitably 1 or 2 such substituents present on each said CH₂ group and there are suitably 1, 2 or 3 such substituents present on each said CH₃ group.

When, as defined hereinbefore, any CH, CH₂ or CH₃ group within a R¹ substituent optionally bears on each said CH, CH₂ or CH₃ group a substituent as defined hereinbefore, suitable R¹ substituents so formed include, for example, hydroxy-substituted (1-8C)alkyl groups such as hydroxymethyl, 1-hydroxyethyl and 2-hydroxyethyl, hydroxy-substituted (1-6C)alkoxy groups such as 2-hydroxypropoxy and 3-hydroxypropoxy, (1-6C)alkoxy-substituted (1-6C)alkoxy groups such as 2-methoxyethoxy and 3-ethoxypropoxy, hydroxy-substituted amino-(2-6C)alkoxy groups such as 3-amino-2-hydroxypropoxy, hydroxy-substituted (1-6C)alkylamino-(2-6C)alkoxy groups such as 2-hydroxy-3-methylaminopmpoxy, hydroxy-substituted di-[(1-6C)alkyl]amino-(2-6C)alkoxy groups such as 3-dimethylamino-2-hydroxypropoxy, hydroxy-substituted amino-(2-6C)alkylamino groups such as 3-amino-2-hydroxypropylamino, hydroxy-substituted (1-6C)alkylamino-(2-6C)alkylamino groups such as 2-hydroxy-3-methylaminopropylamino and hydroxy-substituted di-[(1-6C)alkyl]amino-(2-6C)alkylamino groups such as 3-dimethylamino-2-hydroxypropylamino.

It is further to be understood that when, as defined hereinbefore, any CH, CH₂ or CH₃ group within a R¹ substituent optionally bears on each said CH, CH₂ or CH₃ group a substituent as defined hereinbefore, such an optional substituent may be present on a CH, CH₂ or CH₃ group within the hereinbefore defined substituents that may be present on an aryl, heteroaryl or heterocyclyl group within a R¹ substituent. For example, if R¹ includes an aryl or heteroaryl group that is substituted by a (1-8C)alkyl group, the (1-8C)alkyl group may be optionally substituted on a CH, CH₂ or CH₃ group therein by one of the hereinbefore defined substituents therefor. For example, if R¹ includes a heteroaryl group that is substituted by, for example, a (1-6C)alkylamino-(1-6C)alkyl group, the terminal CH₃ group of the (1-6C)alkylamino group may be further substituted by, for example, a (1-6C)alkylsulphonyl group or a (2-6C)alkanoyl group. For example, the R¹ group may be a heteroaryl group such as a thienyl group that is substituted by a *N*-(2-methylsulphonylethyl)aminomethyl group such that R¹ is, for example, a 5-[*N*-(2-methylsulphonylethyl)aminomethyl]thien-2-yl group. Further, for example, if R¹ includes a heterocyclyl group such as a piperidinyl or piperazinyl group that is substituted on a nitrogen atom thereof by, for example, a (2-6C)alkanoyl group, the terminal CH₃ group of the (2-6C)alkmoyl group may be further substituted by, for example, a di-[(1-6C)alkyl]amino group. For example, the R¹ group may be a *N*-(2-dimethylaminoacetyl)piperidin-4-yl group or a 4-(2-dimethylaminoacetyl)piperazin-1-yl group.

Similar considerations apply to the attachments and substitutions within the -X¹-Q¹ group. For example, when, as defined hereinbefore, any CH, CH₂ or CH₃ group within a Q¹ group optionally bears on each said CH, CH₂ or CH₃ group a substituent as defined hereinbefore, suitable Q¹ groups so formed include, for example, hydroxy-substituted amino-(1-6C)alkyl groups such as 1-amino-2-hydroxyethyl or 1-amino-2-hydroxypropyl, an (1-6C)alkoxy-substituted amino-(1-6C)alkyl groups such as 1-amino-2-methoxyethyl, a (1-6C)alkylamino-(1-6C)alkyl-substituted heteroaryl group such as a 5*-*[*N-*(2-methylsulphonylethyl)aminomethyl]thien-2-yl group, and a (2-6C)alkanoyl-substituted heterocyclic group such as a *N*-(2-dimethylaminoacetyl)piperidin-4-yl group or a 4-(2-dimethylaminoacetyl)piperazin-1-yl group.

A suitable pharmaceutically-acceptable salt of a compound of the Formula I is, for example, an acid-addition salt of a compound of the Formula I, for example an acid-addition salt with an inorganic or organic acid such as hydrochloric, hydrobromic, sulphuric, trifluoroacetic, citric or maleic acid; or, for example, a salt of a compound of the Formula I which is sufficiently acidic, for example an alkali or alkaline earth metal salt such as a calcium or magnesium salt, or an ammonium salt, or a salt with an organic base such as methylamine, dimethylamine, trimethylamine, piperidine, morpholine or tris-(2-hydroxyethyl)amine. A further suitable pharmaceutically-acceptable salt of a compound of the Formula I is, for example, a salt formed within the human or animal body after administration of a compound of the Formula I.

A suitable pharmaceutically-acceptable solvate of a compound of the Formula I is, for example, a hydrate such as a hemi-hydrate, a mono-hydrate, a di-hydrate or a tri-hydrate or an alternative quantity thereof.

The compounds of the invention may be administered in the form of a pro-drug, that is a compound that is broken down in the human or animal body to release a compound of the invention. A pro-drug may be used to alter the physical properties and/or the pharmacokinetic properties of a compound of the invention. A pro-drug can be formed when the compound of the invention contains a suitable group or substituent to which a property-modifying group can be attached. Examples of pro-drugs include *in vivo* cleavable ester derivatives that may be formed at a carboxy group or a hydroxy group in a compound of the Formula I and *in vivo* cleavable amide derivatives that may be formed at a carboxy group or an amino group in a compound of the Formula I.

Accordingly, the present invention includes those compounds of the Formula I as defined hereinbefore when made available by organic synthesis and when made available within the human or animal body by way of cleavage of a pro-drug thereof. Accordingly, the present invention includes those compounds of the Formula I that are produced by organic synthetic means and also such compounds that are produced in the human or animal body by way of metabolism of a precursor compound, that is a compound of the Formula I may be a synthetically-produced compound or a metabolically-produced compound.

A suitable pharmaceutically-acceptable pro-drug of a compound of the Formula I is one that is based on reasonable medical judgement as being suitable for administration to the human or animal body without undesirable pharmacological activities and without undue toxicity.

Various forms of pro-drug have been described, for example in the following documents :-
a) Methods in Enzymology, Vol. 42, p. 309-396, edited by K. Widder, et al. (Academic Press, 1985);
b) Design of Pro-drugs, edited by H. Bundgaard, (Elsevier, 1985);
c) A Textbook of Drug Design and Development, edited by Krogsgaard-Larsen and H. Bundgaard, Chapter 5 "Design and Application of Pro-drugs", by H. Bundgaard p. 113-191 (1991);
d) H. Bundgaard, Advanced Drug Delivery Reviews, 8, 1-38 (1992);
e) H. Bundgaard, et al., Journal of Pharmaceutical Sciences, 77, 285 (1988);
f) N. Kakeya, et al., Chem. Pharm. Bull., 32, 692 (1984);
g) T. Higuchi and V. Stella, "Pro-Drugs as Novel Delivery Systems", A.C.S. Symposium Series, Volume 14; and
h) E. Roche (editor), "Bioreversible Carriers in Drug Design", Pergamon Press, 1987.

A suitable pharmaceutically-acceptable pro-drug of a compound of the Formula I that possesses a carboxy group is, for example, an *in vivo* cleavable ester thereof. An *in vivo* cleavable ester of a compound of the Formula I containing a carboxy group is, for example, a pharmaceutically-acceptable ester which is cleaved in the human or animal body to produce the parent acid. Suitable pharmaceutically-acceptable esters for carboxy include (1-6C)alkyl esters such as methyl, ethyl and tert-butyl, (1-6C)alkoxymethyl esters such as methoxymethyl esters, (1-6C)alkanoyloxymethyl esters such as pivaloyloxymethyl esters, 3-phthalidyl esters, (3-8C)cycloalkylcarbonyloxy-(1-6C)alkyl esters such as cyclopentylcarbonyloxymethyl and 1-cyclohexylcarbonyloxyethyl esters, 2-oxo-1,3-dioxolenylmethyl esters such as 5-methyl-2-oxo-1,3-dioxolen-4-ylmethyl esters and (1-6C)alkoxycarbonyloxy-(1-6C)alkyl esters such as methoxycarbonyloxymethyl and 1-methoxycarbonyloxyethyl esters.

A suitable pharmaceutically-acceptable pro-drug of a compound of the Formula I that possesses a hydroxy group is, for example, an *in vivo* cleavable ester or ether thereof. An *in vivo* cleavable ester or ether of a compound of the Formula I containing a hydroxy group is, for example, a pharmaceutically-acceptable ester or ether which is cleaved in the human or animal body to produce the parent hydroxy compound. Suitable pharmaceutically-acceptable ester forming groups for a hydroxy group include inorganic esters such as phosphate esters (including phosphoramidic cyclic esters). Further suitable pharmaceutically-acceptable ester forming groups for a hydroxy group include (1-10C)alkanoyl groups such as acetyl, benzoyl, phenylacetyl and substituted benzoyl and phenylacetyl groups, (1-10C)alkoxycarbonyl groups such as ethoxycarbonyl, *N,N-*[di-(1-4C)alkyl]carbamoyl, 2-dialkylaminoacetyl and 2-carboxyacetyl groups. Examples of ring substituents on the phenylacetyl and benzoyl groups include aminomethyl, *N*-alkylaminomethyl, *N,N-*dialkylaminomethyl, morpholinomethyl, piperazin-1-ylmethyl and 4-(1-4C)alkylpiperazin-1-ylmethyl. Suitable pharmaceutically-acceptable ether forming groups for a hydroxy group include α-acyloxyalkyl groups such as acetoxymethyl and pivaloyloxymethyl groups.

A suitable pharmaceutically-acceptable pro-drug of a compound of the Formula I that possesses a carboxy group is, for example, an *in vivo* cleavable amide thereof, for example an amide formed with an amine such as ammonia, a (1-4C)alkylamine such as methylamine, a di-(1-4C)alkylamine such as dimethylamine, *N-*ethyl-*N-*methylamine or diethylamine, a (1-4C)alkoxy-(2-4C)alkylamine such as 2-methoxyethylamine, a phenyl-(1-4C)alkylamine such as benzylamine and amino acids such as glycine or an ester thereof.

A suitable pharmaceutically-acceptable pro-drug of a compound of the Formula I that possesses an amino group is, for example, an *in vivo* cleavable amide derivative thereof. Suitable pharmaceutically-acceptable amides from an amino group include, for example an amide formed with (1-10C)alkanoyl groups such as an acetyl, benzoyl, phenylacetyl and substituted benzoyl and phenylacetyl groups. Examples of ring substituents on the phenylacetyl and benzoyl groups include aminomethyl, *N*-alkylaminomethyl, *N*,*N*-dialkylaminomethyl, morpholinomethyl, piperazin-1-ylmethyl and 4-(1-4C)allcylpiperazin-1-ylmethyl.

The *in vivo* effects of a compound of the Formula I may be exerted in part by one or more metabolites that are formed within the human or animal body after administration of a compound of the Formula I. As stated hereinbefore, the *in vivo* effects of a compound of the Formula I may also be exerted by way of metabolism of a precursor compound (a pro-drug).

According to a further aspect of the invention there is provided a pyrimidine derivative of the Formula I as defined hereinbefore
wherein **p** is 1, 2 or 3;
**each R¹ group,** which may be the same or different, is selected from halogeno, trifluoromethyl, cyano, isocyano, nitro, hydroxy, mercapto, amino, formyl, carboxy, carbamoyl, ureido, (1-8C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, *N*-(1-6C)alkylcarbamoyl, *N,N-*di-[(1-6C)alkyl]carbamoyl, (2-6C)alkanoyl, (2-6C)alkanoyloxy, (2-6C)alkanoylamino, *N*-(1-6C)allcyl-(2-6C)alkanoylamino, (3-6C)alkenoylamino, *N*-(1-6C)alkyl-(3-6C)alkenoylamino, (3-6C)alkynoylamino, *N*-(1-6C)alkyl-(3-6C)alkynoylamino, *N*'-(1-6C)alkylureido, *N',N'*-di-[(1-6C)alkyl]ureido, *N*-(1-6C)alkylureido, *N,N*'-di-[(1-6C)alkyl]ureido, *N,N',N*'-tri-[(1-6C)alkyl]ureido, *N*-(1-6C)alkylsulphamoyl, *N,N*-di-[(1-6C)alkyl]sulphamoyl, (1-6C)alkanesulphonylamino and *N*-(1-6C)alkyl-(1-6C)alkanesulphonylamino, or from a group of the formula:

Q²-X²-

wherein X² is a direct bond or is selected from O, S, SO, SO₂, N(R⁵), CO, CH(OR⁵), CON(R⁵). N(R⁵)CO, N(R⁵)CON(R⁵), SO₂N(R⁵), N(R⁵)SO₂, OC(R⁵)₂, SC(R⁵)₂ and N(R⁵)C(R⁵)₂, wherein R⁵ is hydrogen or (1-8C)alkyl, and Q² is aryl, aryl-(1-6C)alkyl, (3-8C)cycloalkyl, (3-8C)cycloalkyl-(1-6C)alkyl, (3-8C)cycloalkenyl, (3-8C)cycloalkenyl-(1-6C)alkyl, heteroaryl, heteroaryl-(1-6C)alkyl, heterocyclyl or heterocyclyl-(1-6C)alkyl, or (R¹)ₚ is (1-3C)alkylenedioxy,
and wherein any CH, CH₂ or CH₃ group within a R¹ substituent optionally bears on each said CH, CH₂ or CH₃ group one or more halogeno or (1-8C)alkyl substituents and/or a substituent selected from hydroxy, mercapto, amino, cyano, carboxy, carbamoyl, ureido, (1-6C)alkoxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, *N*-(1-6C)alkylcarbamoyl, *N,N* di-[(1-6C)allcyl]carbamoyl, (2-6C)alkanoyl, (2-6C)alkanoyloxy, (2-6C)allcanoylamino, *N-*(1-6C)alkyl-(2-6C)alkanoylamino, *N-*(1-6C)alkylureido, *N*'-(1-6C)alkylureido, *N',N'*-di-[(1-6C)alkyl]ureido, *N,N'*-di-[(1-6C)alkyl]ureido, *N*,*N*',*N*'-tri-[(1-6C)alkyl]ureido, *N*-(1-6C)alkylsulphamoyl, *N,N-*di-[(1-6C)alkyl]sulphamoyl, (1-6C)alkanesulphonylamino and *N*-(1-6C)alkyl-(1-6C)alkanesulphonylamino, or from a group of the formula:

- X³ - Q³

wherein X³ is a direct bond or is selected from O, S, SO, SO₂, N(R⁶), CO, CH(OR⁶), CON(R⁶), N(R⁶)CO, N(R⁶)CON(R⁶), SO₂N(R⁶), N(R⁶)SO₂, C(R⁶)₂O, C(R⁶)₂S and C(R⁶)₂N(R⁶), wherein R⁶ is hydrogen or (1-8C)alkyl, and Q³ is aryl, aryl-(1-6C)alkyl, (3-8C)cycloalkyl, (3-8C)cycloalkyl-(1-6C)alkyl, (3-8C)cycloalkenyl, (3-8C)cycloalkenyl-(1-6C)alkyl, heteroaryl, heteroaryl-(1-6C)alkyl, heterocyclyl or heterocyclyl-(1-6C)alkyl,
and wherein any aryl, (3-8C)cycloalkyl, (3-8C)cycloalkenyl, heteroaryl or heterocyclyl group within a substituent on R¹ optionally bears 1, 2 or 3 substituents, which may be the same or different, selected from halogeno, trifluoromethyl, cyano, nitro, hydroxy, amino, carboxy, carbamoyl, ureido, (1-8C)alkyl, (2-8C)alkenyl, (2-8C)alkynyl, (1-6C)alkoxy. (2-6C)alkenyloxy, (2-6C)alkynyloxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, (2-6C)alkanoyl, (2-6C)alkanoyloxy, *N*-(1-6C)alkylcarbamoyl, *N,N*-di-[(1-6C)alkyl]carbamoyl, (2-6C)alkanoylamino, *N-*(1-6C)alkyl-(2-6C)alkanoylamino, *N*-(1-6C)alkylureido, *N*'-(1-6C)alkylureido, *N',N*'-di-[(1-6C)alkyl]ureido, *N*,*N*'-di-[(1-6C)alkyl]ureido, *N*,*N*',*N*'-tri-[(1-6C)alkyl]ureido, *N*-(1-6C)alkylsulphamoyl, *N,N-*di-[(1-6C)alkyl]sulphamoyl, (1-6C)alkanesulphonylamino and *N*-(1-6C)alkyl-(1-6C)alkanesulphonylamino, or from a group of the formula :

-X⁴-R⁷

wherein X⁴ is a direct bond or is selected from O and N(R⁸), wherein R⁸ is hydrogen or (1-8C)alkyl, and R⁷ is halogeno-(1-6C)alkyl, hydroxy-(1-6C)alkyl, mercapto-(1-6C)alkyl, (1-6C)alkoxy-(1-6C)alkyl, (1-6C)alkylthio-(1-6C)alkyl, cyano-(1-6C)alkyl, amino-(1-6C)alkyl, (1-6C)alkylamino-(1-6C)alkyl, di-[(1-6C)alkyl]amino-(1-6C)alkyl, (2-6C)alkanoylamino-(1-6C)alkyl, (1-6C)alkoxycarbonylamino-(1-6C)alkyl. *N*-(1-6C)alkylureido-(1-6C)alkyl*, N'*-(1-6C)alkylureido-(1-6C)alkyl, *N',N'*-di-[(1-6C)alkyl]ureido-(1-6C)alkyl, *N,N'*-di-[(1-6C)alkyl]ureido-(1-6C)alkyl or *N*,*N'*,*N'*-tri-[(1-6C)alkyl]ureido-(1-6C)alkyl, or from a group of the formula:

- X⁵-Q⁴

wherein X⁵ is a direct bond or is selected from O, CO and N(R⁹), wherein R⁹ is hydrogen or (1-8C)alkyl, and Q⁴ is aryl, aryl-(1-6C)alkyl, heteroaryl, heteroaryl-(1-6C)alkyl, heterocyclyl or heterocyclyl-(1-6C)alkyl which optionally bears 1 or 2 substituents, which may be the same or different, selected from halogeno, hydroxy, (1-8C)alkyl and (1-6C)alkoxy,
and wherein any heterocyclyl group within a substituent on R¹ optionally bears 1 or 2 oxo or thioxo substituents,
and wherein adjacent carbon atoms in any (2-6C)alkylene chain within a R¹ substituent are optionally separated by the insertion into the chain of a group selected from O, S, SO, SO₂, N(R¹⁰), CO, CH(OR¹⁰), CON(R¹⁰), N(R¹⁰)CO, N(R¹⁰)CON(R¹⁰), SO₂N(R¹⁰), N(R¹⁰)SO₂, CH=CH and C≡C wherein R¹⁰ is hydrogen or (1-8C)alkyl;
**R²** is hydrogen or (1-8C)alkyl;
**q** is 0, 1, 2,3 or 4;
**each R³ group**, which may be the same or different, is (1-8C)alkyl or a group of the formula:

-X⁶-R¹¹

wherein X⁶ is a direct bond or is selected from O and N(R¹²), wherein R¹² is hydrogen or (1-8C)alkyl, and R¹¹ is halogeno-(1-6C)alkyl, hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl, di-[(1-6C)alkyl]amino-(1-6C)alkyl or (2-6C)alkanoylamino-(1-6C)alkyl;
**r** is 0,1 or 2;
**each R⁴ group,** which may be the same or different, is selected from halogeno, trifluoromethyl, cyano, nitro, hydroxy, mercapto, amino, carboxy, carbamoyl, ureido, (1-8C)alkyl, (2-8C)alkenyl, (2-8C)alkynyl, (1-6C)alkoxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, *N*-(1-6C)alkylcarbamoyl, *N*,*N*-di-[(1-6C)alkyl]carbamoyl, (2-6C)alkanoyl, (2-6C)alkanoyloxy, (2-6C)alkanoylamino, *N*-(1-6C)alkyl-(2-6C)alkanoylamino, *N'*-(1-6C)alkylureido, *N'*,*N'*-di-[(1-6C)alkyl]ureido, *N*-(1-6C)alkylureido, *N*,*N*'-di-[(1-6C)alkyl]ureido, *N*,*N'*,*N'*-tri-[(1-6C)alkyl]ureido, *N*-(1-6C)alkylsulphamoyl, *N*,*N*-di-[(1-6C)alkyl]sulphamoyl, (1-6C)alkanesulphonylamino and *N*-(1-6C)alkyl-(1-6C)alkanesulphonylamino;
**X¹** is selected from N(R¹³)CO, CON(R¹³), N(R¹³)CON(R¹³), N(R¹³)COC(R¹³)₂₀, N(R¹³)COC(R¹³)₂S, N(R¹³)COC(R¹³)₂N(R¹³) and N(R¹³)COC(R¹³)₂N(R¹³)CO, wherein R¹³ is hydrogen or (1-8C)alkyl; and
**Q¹** is (1-8C)alkyl, (2-8C)alkenyl, (2-8C)alkynyl, halogeno-(1-6C)alkyl, hydroxy-(1-6C)alkyl, mercapto-(1-6C)alkyl, (1-6C)alkoxy-(1-6C)alkyl, cyano-(1-6C)alkyl, amino-(1-6C)alkyl, (1-6C)alkylamino-(1-6C)alkyl, di-[(1-6C)alkyl]amino-(1-6C)alkyl, (1-6C)alkylthio-(1-6C)alkyl, (1-6C)alkylsulphinyl-(1-6C)alkyl, (1-6C)alkylsulphonyl-(1-6C)alkyl, (2-6C)alkanoylamino-(1-6C)alkyl, *N*-(1-6C)alkyl-(2-6C)alkanoylamino-(1-6C)alkyl, (1-6C)alkoxycarbonylamino-(1-6C)alkyl, *N*-(1-6d)alkylureido-(1-6C)alkyl, *N*'-(1-6C)alkylureido-(1-6C)alkyl, *N',N'*-di-[(1-6C)alkyl]ureido-(1-6C)alkyl, *N,N'*-di-[(1-6C)alkyl]ureido-(1-6C)alkyl, *N,N',N'*-tri-[(1-6C)alkyl]ureido-(1-6C)alkyl, (1-6C)alkanesulphonylamino-(1-6C)alkyl or *N*-(1-6C)alkyl-(1-6C)alkanesulphonylamino-(1-6C)alkyl, or **Q¹** is aryl, aryl-(1-6C)alkyl, (3-8C)cycloalkyl, (3-8C)cycloalkyl-(1-6C)alkyl, (3-8C)cycloalkenyl, (3-8C)cycloalkenyl-(1-6C)alkyl, heteroaryl, heteroaryl-(1-6C)alkyl, heterocyclyl or heterocyclyl-(1-6C)alkyl,
and wherein any CH, CH₂ or CH₃ group within the Q¹ group optionally bears on each said CH, CH₂ or CH₃ group one or more halogeno or (1-8C)alkyl substituents and/or a substituent selected from hydroxy, mercapto, amino, cyano, carboxy, carbamoyl, ureido, (1-6C)alkoxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, *N*-(1-6C)alkylcarbamoyl, *N,N*-di-[(1-6C)alkyl]carbamoyl, (2-6C)alkanoyl, (2-6C)alkanoyloxy, (2-6C)alkanoylamino, *N*-(1-6C)alkyl-(2-6C)alkanoylamino, *N'*-(1-6C)alkylureido, *N'*,*N'*-di-[(1-6C)alkyl]ureido, *N*-(1-6C)alkylureido, *N*,*N*'-di-[(1-6C)alkyl]ureido, *N*,*N'*,*N'-*tri-[(1-6C)alkyl]ureido, *N*-(1-6C)alkylsulphamoyl, *N*-(1-6C)alkylsulphamoyl, *N*,*N*-di-[(1-6C)alkyl]sulphamoyl, (1-6C)alkanesulphonylamino and *N*-(1-6C)alkyl-(1-6C)alkanesulphonylamino,
and wherein any aryl, (3-8C)cycloalkyl, (3-8C)cycloalkenyl, heteroaryl or heterocyclyl group within the Q¹ group optionally bears 1, 2 or 3 substituents, which may be the same or different, selected from halogeno, trifluoromethyl, cyano, nitro, hydroxy, amino, carboxy, carbamoyl, ureido, (1-8C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, (2-6C)alkanoyl, (2-6C)alkanoyloxy, *N*-(1-6C)alkylcarbamoyl, *N,N*-di-[(1-6C)alkyl]carbamoyl, (2-6C)alkanoylamino, *N*-(1-6C)alkyl-(2-6C)alkanoylamino, *N'*-(1-6C)alkylureido, *N',N*'-di-[(1-6C)alkyl]ureido, *N*-(1-6C)alkylureido, *N,N*'-di-[(1-6C)alkyl]ureido, *N,N',N'*-tri-[(1-6C)alkyl]ureido, *N*-(1-6C)alkylsulphamoyl, *N,N*-di-[(1-6C)alkyl]sulphamoyl, (1-6C)alkanesulphonylamino and *N*-(1-6C)alkyl-(1-6C)alkanesulphonylamino, or from a group of the formula:

-X⁷-R¹⁴

wherein X⁷ is a direct bond or is selected from O and N(R¹⁵), wherein R¹⁵ is hydrogen or (1-8C)alkyl, and R¹⁴ is halogeno-(1-6C)alkyl, hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl or di-[(1-6C)alkyl]amino-(1-6C)alkyl,
and wherein any heterocyclyl group within the Q¹ group optionally bears 1 or 2 oxo or thioxo substituents,
and wherein adjacent carbon atoms in any (2-6C)alkylene chain within the Q¹ group are optionally separated by the insertion into the chain of a group selected from O, S, SO, SO₂ N(R¹⁶), N(R¹⁶)CO, CON(R¹⁶), N(R¹⁶)CON(R¹⁶), CO, CH(OR¹⁶), N(R¹⁶)SO₂, SO₂N(R¹⁶), CH=CH and C≡C wherein R¹⁶ is hydrogen or (1-8C)alkyl; or a pharmaceutically-acceptable salt, solvate or pro-drug thereof.

Particular novel compounds of the invention include, for example, pyrimidine derivatives of the Formula I, or pharmaceutically-acceptable salts, solvates or pro-drugs thereof, wherein, unless otherwise stated, each of p, R¹, R², q, R³, r, R⁴, X¹ and Q¹ has any of the meanings defined hereinbefore or in paragraphs (a) to (rr) hereinafter :-
(a) p is 1, 2 or 3, and each R¹ group, which may be the same or different, is selected from halogeno, trifluoromethyl, cyano, hydroxy, mercapto, amino, carboxy, carbamoyl, ureido, (1-8C)alkyl, (2-8C)alkenyl, (2-8C)alkynyl, (1-6C)alkoxy, (2-6C)alkenyloxy, (2-6C)alkynyloxy, (1-6C)alkylamino, di-[(1-6C)alkyl]amino, (1-6C)alkoxycarbonyl, *N*-(1-6C)alkylcarbamoyl, *N,N-*di-[(1-6C)alkyl]carbamoyl, (2-6C)alkanoyloxy, (2-6C)alkanoylamino, *N*-(1-6C)alkyl-(2-6C)alkanoylamino, (3-6C)alkenoylamino, *N*-(1-6C)alkyl-(3-6C)alkenoylamino, (3-6C)alkynoylamino, *N*-(1-6C)alkyl-(3-6C)alkynoylamino, *N*-(1-6C)alkylsulphamoyl, *N,N*-di-[(1-6C)alkyl]sulphamoyl, (1-6C)alkanesulphonylamino and *N*-(1-6C)alkyl-(1-6C)alkanesulphonylamino, or from a group of the formula:

   Q²-X²-

   wherein X² is a direct bond or is selected from O, S, N(R⁵), CO, wherein R⁵ is hydrogen or (1-8C)alkyl, and Q² is aryl, aryl-(1-6C)allcyl, (3-8C)cycloalkyl, (3-8C)cycloalkyl-(1-6C)alkyl, heteroaryl, heteroaryl-(1-6C)alkyl, heterocyclyl or heterocyclyl-(1-6C)alkyl, or (R¹)ₚ is (1-3C)alkylenedioxy,
   and wherein any CH, CH₂ or CH₃ group within a R¹ substituent optionally bears on each said CH, CH₂ or CH₃ group one or more halogeno or (1-8C)alkyl substituents and/or a substituent selected from hydroxy, mercapto, amino, cyano, carboxy, carbamoyl, ureido, (1-6C)alkoxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, *N*-(1-6C)alkylcarbamoyl, *N*,*N*-di-[(1-6C)alkyl]carbamoyl, (2-6C)alkanoyloxy, (2-6C)alkanoylamino, *N-*(1-6C)alkyl-(2-6C)alkanoylamino, *N-*(1-6C)alkylsulphamoyl, *N,N-*di-[(1-6C)alkyl]sulphamoyl, (1-6C)alkanesulphonylamino and *N*-(1-6C)alkyl-(1-6C)alkanesulphonylamino,
   and wherein any aryl, (3-8C)cycloalkyl, heteroaryl or heterocyclyl group within a substituent on R¹ optionally bears 1, 2 or 3 substituents, which may be the same or different, selected from halogeno, trifluoromethyl, cyano, hydroxy, amino, (1-8C)alkyl, (2-8C)alkenyl, (2-8C)alkynyl, (1-6C)alkoxy, (1-6C)alkylamino and di-[(1-6C)alkyl]amino, and wherein any heterocyclyl group within a substituent on R¹ optionally bears 1 or 2 oxo or thioxo substituents;
(b) p is 1 or 2, and each R¹ group, which may be the same or different, is selected from halogeno, trifluoromethyl, cyano, hydroxy, amino, carboxy, carbamoyl, ureido, (1-8C)alkyl, (2-8C)alkenyl, (2-8C)alkynyl, (1-6C)alkoxy, (1-6C)alkylamino, di-[(1-6C)alkyl]amino, (1-6C)alkoxycarbonyl, (2-6C)alkanoylamino and *N*-(1-6C)alkyl-(2-6C)alkanoylamino,
   and wherein any CH, CH₂ or CH₃ group within a R¹ substituent optionally bears on each said CH, CH₂ or CH₃ group 1, 2 or 3 halogeno or (1-8C)alkyl substituents and/or a substituent selected from hydroxy, amino, cyano, carboxy, carbamoyl, ureido, (1-6C)alkoxy, (1-6C)alkylamino, di-[(1-6C)alkyl]amino, (1-6C)alkoxycarbonyl, *N*-(1-6C)alkylcarbamoyl, *N,N-*di-[(1-6C)alkyl]carbamoyl, (2-6C)alkanoylamino and *N*-(1-6C)alkyl-(2-6C)alkanoylamino;
(c) p is 1 or 2, and each R¹ group, which may be the same or different, is selected from fluoro, chloro, trifluoromethyl, cyano, hydroxy, amino, carboxy, carbamoyl, ureido, methyl, ethyl, propyl, vinyl, allyl, ethynyl, 2-propynyl, methoxy, ethoxy, propoxy, isopropoxy, methylamino, ethylamino, propylamino, dimethylamino, diethylamino, methoxycarbonyl, ethoxycarbonyl, acetamido, propionamido, *N*-methylacetamido, *N*-methylpropionamido, hydroxymethyl, 1-hydroxyethyl, 1-hydroxy-1-methylethyl, 2-hydroxyethyl, 2-hydroxy-1-methylethyl, 2-hydroxypropyl, 1,1-dimethyl-2-hydroxyethyl, 2-hydroxy-2-methylpmpyl, aminomethyl, 1-aminoethyl, 1-amino-1-methylethyl, 2-aminoethyl, 2-amino-1-methylethyl, 2-aminopropyl, 2-amino-1,1-dimethylethyl, 2-amino-2-methylpropyl, methylaminomethyl, 1-methylaminoethyl, 1-methylamino-1-methylethyl, 2-methylaminoethyl, 2-methylamino-1-methylethyl, 2-methylaminopropyl, 2-methylamino-1,1-dimethylethyl, 2-methylamino-2-methylpropyl, acetamidomethyl, 1-acetamidoethyl, 1-acetamido-1-methylethyl, 2-acetamidoethyl, 2-acetamido-1-methylethyl, 2-acetamidopropyl, 2-acetamido-1,1-dimethylethyl and 2-acetamido-2-methylpropyl;
(d) p is 1 or 2, and a first R¹ group is selected from hydroxy, amino, carboxy, carbamoyl, methylamino, ethylamino, dimethylamino, diethylamino, methoxycarbonyl, ethoxycarbonyl, acetamido, propionamido, *N*-methylacetamido, *N*-methylpropionamido, hydroxymethyl, 1-hydroxyethyl, 1-hydroxy-1-methylethyl, aminomethyl, 1-aminoethyl, 1-amino-1-methylethyl, methylaminomethyl, 1-methylaminoethyl, 1-methylamino-1-methylethyl, acetamidomethyl, 1-acetamidoethyl and 1-acetamido-1-methylethyl, and the optional second R¹ group is selected from fluoro, chloro, trifluoromethyl, cyano, hydroxy, methyl, ethyl, propyl, vinyl, allyl, ethynyl, 2-propynyl, methoxy, ethoxy, propoxy and isopropoxy;
(e) p is 1 or 2, and a first R¹ group is selected from hydroxy, carbamoyl, acetamido, propionamido, *N*-methylacetamido, *N*-methylpropionamido, hydroxymethyl, 1-hydroxyethyl and 1-hydroxy-1-methylethyl, and the optional second R¹ group is selected from fluoro, chloro, trifluoromethyl, cyano, hydroxy, methyl, ethyl, methoxy and ethoxy;
(f) p is 1 and the R¹ group is located at the 3- or 4-position on the phenyl group and is selected from hydroxy, carbamoyl, acetamido, hydroxymethyl, 1-hydroxyethyl and 1-hydroxy-1-methylethyl;
(g) p is 1 and the R¹ group is located at the 3-position on the phenyl group and is selected from hydroxy and hydroxymethyl;
(h) R² is hydrogen, methyl, ethyl or propyl;
(i) R² is hydrogen or methyl;
(j) R² is hydrogen;
(k) q is 0;
(l) q is 1, 2 or 3 and each R³ group, which may be the same or different, is methyl, ethyl or propyl;
(m) q is 1 and the R³ group is methyl;
(n) r is 0;
(o) r is 1 or 2 and each R⁴ group, which may be the same or different, is selected from halogeno, trifluoromethyl, cyano, hydroxy, amino, (1-8C)alkyl, (2-8C)alkenyl, (2-8C)alkynyl, (1-6C)alkoxy, (1-6C)alkylamino, di-[(1-6C)alkyl]amino, (2-6C)alkanoylamino and *N*-(1-6C)alkyl-(2-6C)alkanoylamino;
(p) r is 0 or r is 1 or 2 and each R⁴ group, which may be the same or different, is selected from fluoro, chloro, trifluoromethyl, cyano, hydroxy, amino, methyl, methoxy, methylamino and dimethylamino;
(q) r is 0 or r is 1 and the R⁴ group is selected from fluoro, chloro, trifluoromethyl, hydroxy, amino, methyl, methoxy, methylamino and dimethylamino;
(r) r is 0 or r is 1 and the R⁴ group is selected from fluoro, chloro and methyl;
(s) the X¹-Q¹ group is located at the 3- or 4-position;
(t) the X¹-Q¹ group is located at the 3-position;
(u) the X¹-Q¹ group is located at the 4-position;
(v) X¹ is selected from N(R¹³)CO, CON(R¹³), N(R¹³)CON(R¹³), N(R¹³)COC(R¹³)₂O, N(R¹³)COC(R¹³)₂N(R¹³) and N(R¹³)COC(R¹³)₂N(R¹³)CO, wherein R¹³ is hydrogen or (1-8C)alkyl;
(w) X¹ is selected from CO, N(R¹³)CO, CON(R¹³), N(R¹³)CON(R¹³), N(R¹³)COC(R¹³)₂O, N(R¹³)COC(R¹³)₂N(R¹³) and N(R¹³)COC(R¹³)₂N(R¹³)CO, wherein R¹³ is hydrogen or (1-8C)alkyl;
(x) X¹ is selected from NHCO, NHCONH. NHCOCH₂O, NHCOCH₂NH and NHCOCH₂NHCO;
(y) X¹ is selected from CO, NHCO, N(Me)CO, CONH, CON(Me), NHCONH, NHCOCH₂O, NHCOCH₂NH and NHCOCH₂NHCO;
(z) X¹ is selected from NHCO, NHCONH and NHCOCH₂O;
   (aa) X¹ is selected from NHCO, N(Me)CO, CONH, CON(Me), NHCONH and NHCOCH₂O;
   (bb) X¹ is NHCO;
   (cc) X¹ is NHCO or N(Me)CO;
   (dd) X¹ is CONH;
   (ee) X¹ is CONH or CON(Me);
   (ff) X¹ is CO;
   (gg) Q¹ is (1-8C)alkyl, (2-8C)alkenyl, (2-8C)alkynyl, halogeno-(1-6C)alkyl, hydroxy-(1-6C)alkyl, mercapto-(1-6C)alkyl, (1-6C)alkoxy-(1-6C)alkyl, cyano-(1-6C)alkyl, amino-(1-6C)alkyl, (1-6C)alkylamino-(1-6C)alkyl, di-[(1-6C)alkyl]amino-(1-6C)alkyl, (1-6C)alkylthio-(1-6C)alkyl, (1-6C)alkylsulphinyl-(1-6C)alkyl, (1-6C)alkylsulphonyl-(1-6C)alkyl or (2-6C)alkanoylamino-(1-6C)alkyl, or Q¹ is aryl, aryl-(1-6C)alkyl, (3-8C)cycloalkyl, (3-8C)cycloalkyl-(1-6C)alkyl, heteroaryl, heteroaryl-(1-6C)alkyl, heterocyclyl or heterocyclyl-(1-6C)alkyl,
      and wherein any CH, CH₂ or CH₃ group within the Q¹ group optionally bears on each said CH, CH₂ or CH₃ group one or more halogeno or (1-8C)alkyl substituents and/or a substituent selected from hydroxy, amino, cyano, carboxy, carbamoyl, ureido, (1-6C)alkoxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, *N*-(1-6C)alkylcarbamoyl, *N,N-*di-[(1-6C)alkyl]carbamoyl, (2-6C)alkanoylamino and *N*-(1-6C)alkyl-(2-6C)alkanoylamino,
      and wherein any aryl; (3-8C)cycloalkyl, heteroaryl or heterocyclyl group within the Q¹ group optionally bears 1, 2 or 3 substituents, which may be the same or different, selected from halogeno, trifluoromethyl, cyano, hydroxy, amino, (1-8C)alkyl, (2-8C)alkenyl, (2-8C)alkynyl, (1-6C)alkoxy, (1-6C)alkylamino and di-[(1-6C)alkyl]amino, or from a group of the formula:

      -X⁷-R¹⁴

      wherein X⁷ is a direct bond or is selected from O and N(R¹⁵), wherein R¹⁵ is hydrogen or (1-8C)alkyl, and R¹⁴ is hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl or di-[(1-6C)alkyl]amino-(1-6C)alkyl,
      and wherein any heterocyclyl group within the Q¹ group optionally bears 1 or 2 oxo or thioxo substituents;
   (hh) Q¹ is (1-8C)alkyl, (2-8C)alkenyl, (2-8C)alkynyl, halogeno-(1-6C)alkyl, hydroxy-(1-6C)alkyl, mercapto-(1-6C)alkyl, (1-6C)alkoxy-(1-6C)alkyl, cyano-(1-6C)alkyl, amino-(1-6C)alkyl, (1-6C)alkylamino-(1-6C)alkyl, di-[(1-6C)alkyl]amino-(1-6C)alkyl, (1-6C)alkylthio-(1-6C)alkyl, (1-6C)alkylsulphinyl-(1-6C)alkyl, (1-6C)alkylsulphonyl-(1-6C)alkyl or (2-6C)alkanoylamino-(1-6C)alkyl, or Q¹ is aryl, aryl-(1-6C)alkyl, (3-8C)cycloalkyl, (3-8C)cycloalkyl-(1-6C)alkyl, heteroaryl, heteroaryl-(1-6C)alkyl, heterocyclyl or heterocyclyl-(1-6C)alkyl,
      and wherein any CH, CH₂ or CH₃ group within the Q¹ group optionally bears on each said CH, CH₂ or CH₃ group one or more halogeno or (1-8C)alkyl substituents and/or a substituent selected from hydroxy, amino, cyano, carboxy, carbamoyl, ureido, (1-6C)alkoxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, *N*-(1-6C)alkylcarbamoyl, *N,N*-di-[(1-6C)alkyl]carbamoyl, (2-6C)alkanoylamino and *N*-(1-6C)alkyl-(2-6C)alkanoylamino,
      and wherein any aryl, (3-8C)cycloalkyl, heteroaryl or heterocyclyl group within the Q¹ group optionally bears 1, 2 or 3 substituents, which may be the same or different, selected from halogeno, trifluoromethyl, cyano, hydroxy, amino, carbamoyl, (1-8C)alkyl, (2-8C)alkenyl, (2-8C)alkynyl, (1-6C)alkoxy, (1-6C)alkylamino and di-[(1-6C)alkyl]amino, or from a group of the formula:

      -X⁷-R¹⁴

      wherein X⁷ is a direct bond or is selected from O and N(R¹⁵), wherein R¹⁵ is hydrogen or (1-8C)alkyl, and R¹⁴ is hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl or di-[(1-6C)alkyl]amino-(1-6C)alkyl, or from a group of the formula:

      -X⁸-Q⁵

      wherein X⁸ is a direct bond or is selected from O, CO and N(R¹⁷), wherein R¹⁷ is hydrogen or (1-8C)alkyl, and Q⁵ is heterocyclyl or heterocyclyl-(1-6C)alkyl which optionally bears 1 or 2 substituents, which may be the same or different, selected from halogeno, hydroxy, (1-8C)alkyl and (1-6C)alkoxy,
      and wherein any heterocyclyl group within the Q¹ group optionally bears 1 or 2 oxo or thioxo substituents;
   (ii) Q¹ is (1-8C)alkyl, hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl, di-[(1-6C)alkyl]amino-(1-6C)alkyl, (1-6C)alkylthio-(1-6C)alkyl or (2-6C)alkanoylamino-(1-6C)alkyl, or Q¹ is aryl, aryl-(1-6C)alkyl, (3-8C)cycloalkyl, (3-8C)cycloalkyl-(1-6C)alkyl, heteroaryl, heteroaryl-(1-6C)alkyl, heterocyclyl or heterocyclyl-(1-6C)alkyl,
      and wherein any CH, CH₂ or CH₃ group within the Q¹ group optionally bears on each said CH, CH₂ or CH₃ group 1, 2 or 3 halogeno or (1-8C)alkyl substituents and/or a substituent selected from hydroxy, amino, cyano, carboxy, carbamoyl, ureido, (1-6C)alkoxy, (1-6C)alkylthio, (1-6C)alkylamino, di-[(1-6C)alkyl]amino, (1-6C)alkoxycarbonyl, *N*-(1-6C)alkylcarbamoyl, *N*,*N*-di-[(1-6C)alkyl]carbamoyl, (2-6C)alkanoylamino and *N*-(1-6C)alkyl-(2-6C)alkanoylamino,
      and wherein any aryl, (3-8C)cycloalkyl, heteroaryl or heterocyclyl group within the Q¹ group optionally bears 1 or 2 substituents, which may be the same or different, selected from halogeno, trifluoromethyl, cyano, hydroxy, amino, (1-8C)alkyl, (1-6C)alkoxy, (1-6C)alkylamino and di-[(1-6C)alkyl]amino, or from a group of the formula :

      -X⁷-R¹⁴

      wherein X⁷ is a direct bond and R¹⁴ is hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl or di-[(1-6C)alkyl]amino-(1-6C)alkyl;
   (jj) Q¹ is (1-8C)alkyl, hydroxy-(1-6C)alkyl, (1-6C)alkoxy-(1-6C)alkyl, amino-(1-6C)alkyl, (1-6C)alkylamino-(1-6C)alkyl, di-[(1-6C)alkyl]amino-(1-6C)alkyl or (1-6C)alkylthio-(1-6C)alkyl, or Q¹ is aryl, aryl-(1-6C)alkyl, (3-8C)cycloalkyl, (3-8C)cycloalkyl-(1-6C)alkyl, heteroaryl, heteroaryl-(1-6C)alkyl, heterocyclyl or heterocyclyl-(1-6C)alkyl,
      and wherein any aryl, (3-8C)cycloalkyl, heteroaryl or heterocyclyl group within the Q¹ group optionally bears 1 or 2 substituents, which may be the same or different, selected from halogeno, trifluoromethyl, hydroxy, amino, (1-8C)alkyl, (1-6C)alkoxy, (1-6C)alkylamino, di-[(1-6C)alkyl]amino, hydroxy-(1-6C)alkyl, amino-(1-6C)alkyl, (1-6C)alkylamino-(1-6C)alkyl and di-[(1-6C)alkyl]amino-(1-6C)alkyl;
   (kk) Q¹ is (1-8C)alkyl, hydroxy (1-6C)alkyl, (1-6C)alkoxy-(1-6C)alkyl, cyano-(1-6C)alkyl, amino-(1-6C)alkyl, (1-6C)alkylamino-(1-6C)alkyl, di-[(1-6C)allcyl]amino-(1-6C)alkyl, (1-6C)alkylsulphonyl-(1-6C)alkyl or (2-6C)alkanoylamino-(1-6C)alkyl, or Q¹ is aryl, aryl-(1-6C)alkyl, (3-8C)cycloalkyl, (3-8C)cycloalkyl-(1-6C)alkyl, heteroaryl, heteroaryl-(1-6C)alkyl, heterocyclyl or heterocyclyl-(1-6C)alkyl,
      and wherein any CH, CH₂ or CH₃ group within the Q¹ group optionally bears on each said CH, CH₂ or CH₃ group a substituent selected from hydroxy, amino, cyano, carbamoyl, (1-6C)alkoxy, (1-6C)alkylsulphonyl, (1-6C)alkylamino, di-[(1-6C)alkyl]amino, (1-6C)alkoxycarbonyl, *N-*(1-6C)alkylcarbamoyl, *N,N* di-[(1-6C)alkyl]carbamoyl, (2-6C)allcanoyl, (2-6C)alkanoylamino and *N-*(1-6C)alkyl-(2-6C)alkanoylamino,
      and wherein any aryl, (3-8C)cycloalkyl, heteroaryl or heterocyclyl group within the Q¹ group optionally bears 1 or 2 substituents, which may be the same or different, selected from halogeno, trifluoromethyl, hydroxy, amino, carbamoyl, (1-8C)alkyl, (1-6C)alkoxy, (1-6C)alkylamino, di-[(1-6C)alkyl]amino, hydroxy-(1-6C)alkyl, cyano-(1-6C)alkyl, amino-(1-6C)alkyl, (1-6C)alkylamino-(1-6C)aUcyl, di-[(1-6C)alkyl]amino-(1-6C)alkyl and heterocyclyl-(1-6C)alkyl;
   (II) Q¹ is methyl, ethyl, propyl, butyl, pentyl, aminomethyl, 2-aminoethyl, 3-aminopropyl, 4-aminobutyl, 5-aminopentyl, methylaminomethyl, 2-methylaminoethyl, 3-methylaminopropyl, 4-methylaminobutyl, 5-methylaminopentyl, ethylaminomethyl, 2-ethylaminoethyl, 3-ethylaminopropyl, 4-ethylaminobutyl, 5-ethylaminopentyl, dimethylaminomethyl, 2-dimethylaminoethyl, 3-dimethylaminopropyl, 4-dimethylaminobutyl, 5-dimethylaminopentyl, diethylaminomethyl, 2-diethylaminoethyl, 3-diethylaminopropyl, 4-diethylaminobutyl or 5-diethylaminopentyl, or Q¹ is phenyl, benzyl, 2-phenylethyl, cyclopentyl, cyclohexyl, cycloheptyl, cyclopentylmethyl, cyclohexylmethyl, cycloheptylmethyl, furyl, thienyl, oxazolyl, imidazolyl, thiazolyl, oxadiazolyl, thiadiazolyl, pyridyl, pyrimidinyl, furylmethyl, thienylmethyl, oxazolylmethyl, imidazolylmethyl, thiazolylmethyl, oxadiazolylmethyl, thiadiazolylmethyl, pyridylmethyl, pyrimidinylmethyl, tetrahydrofuranyl, tetrahydropyranyl, tetrahydrothiopyranyl, pyrrolinyl, pyrrolidinyl, imidazolidinyl, pyrazolidinyl, morpholinyl, tetrahydro-1,4-thiazinyl, piperidinyl, homopiperidinyl, piperazinyl, homopiperazinyl, 2-azabicyclo[2.2.1]heptyl, indolinyl, isoindolinyl, dihydropyridinyl, tetrahydrofuranylmethyl, tetrahydropyranylmethyl, tetrahydrothiopyranylmethyl, pyrrolinylmethyl, pyrrolidinylmethyl, imidazolidinylmethyl, pyrazolidinylmethyl, morpholinylmethyl, 2-(morpholinyl)ethyl, tetrahydro-1,4-thiazinylmethyl, 2-(tetrahydro-1,4-thiazinyl)ethyl, piperidinylmethyl, 2-(piperidinyl)ethyl, homopiperidinylmethyl, piperazinylmethyl, 2-(piperazinyl)ethyl, homopiperazinylmethyl or 2-azabicyclo[2.2.1]heptylmethyl,
      and wherein any aryl, (3-8C)cycloalkyl, heteroaryl or heterocyclyl group within the Q¹ group optionally bears 1 or 2 substituents, which may be the same or different, selected from fluoro, chloro, trifluoromethyl, hydroxy, amino, methyl, ethyl, methoxy, ethoxy, methylamino, dimethylamino, hydroxymethyl, 2-hydroxyethyl, aminomethyl, 2-aminoethyl, methylaminomethyl, 2-methylaminoethyl, dimethylaminomethyl and 2-dimethylaminoethyl;
   (mm) Q¹ is methyl, ethyl, propyl, isopropyl, butyl, pentyl, allyl, 2-hydroxyethyl, 3-hydroxypropyl, 2-methoxyethyl, 3-methoxypropyl, 2-ethoxyethyl, 3-ethoxypropyl, cyanomethyl, 2-cyanoethyl, 3-cyanopropyl, 1-cyano-1-methylethyl, 4-cyanobutyl, 5-cyanopentyl, aminomethyl, 2-aminoethyl, 3-aminopropyl, 4-aminobutyl, 5-aminopentyl, methylaminomethyl, 2-methylaminoethyl, 3-methylaminopropyl, 4-methylaminobutyl, 5-methylaminopentyl, ethylaminomethyl, 2-ethylaminoethyl, 3-ethylaminopropyl, 4-ethylaminobutyl, 5-ethylaminopentyl, 1-isopropyl-1-methylaminomethyl, dimethylaminomethyl, 2-dimethylaminoethyl, 3-dimethylaminopropyl, 4-dimethylaminobutyl, 5-dimethylaminopentyl, diethylaminomethyl, 2-diethylaminoethyl, 3-diethylaminopropyl, 4-diethylaminobutyl, 5-diethylaminopentyl, 2-methylsulphonylethyl, 3-methylsulphonylpropyl, acetamidomethyl or 1-acetamidoethyl, or Q¹ is phenyl, benzyl, 2-phenylethyl, cyclopropyl, cyclobutyl, cyclopentyl, cyclohexyl, cycloheptyl, cyclopropylmethyl, cyclobutylmethyl, cyclopentylmethyl, cyclohexylmethyl, cycloheptylmethyl, furyl, thienyl, oxazolyl, isoxazolyl, imidazolyl, pyrazolyl, thiazolyl, triazolyl, oxadiazolyl, thiadiazolyl, tetrazolyl, pyridyl, pyrazinyl, pyridazinyl, pyrimidinyl, furylmethyl, 2-furylethyl, thienylmethyl, 2-thienylethyl, oxazolylmethyl, 2-oxazolylethyl, isoxazolylmethyl, 2-isoxazolylethyl, imidazolylmethyl, 2-imidazolylethyl, pyrazolylmethyl, 2-pyrazolylethyl, thiazolylmethyl, 2-thiazolylethyl, triazolylmethyl, 2-triazolylethyl, oxadiazolylmethyl, 2-oxadiazolylethyl, thiadiazolylmethyl, 2-thiadiazolylethyl, tetrazolylmethyl, 2-tetrazolylethyl, pyridylmethyl, 2-pyridylethyl, pyrazinylmethyl, 2-pyrazinylethyl, pyridazinylmethyl, 2-pyridazinylethyl, pyrimidinylmethyl, 2-pyrimidinylethyl, tetrahydrofuranyl, tetrahydropyranyl, tetrahydrothiopyranyl, azetidinyl, pyrrolinyl, pyrrolidinyl, imidazolidinyl, pyrazolidinyl, morpholinyl, tetrahydro-1,4-thiazinyl, piperidinyl, homopiperidinyl, piperazinyl, homopiperazinyl, 2-azabicyclo[2.2.1]heptyl, indolinyl, isoindolinyl, dihydropyridinyl, tetrahydrofuranylmethyl, tetrahydropyranylmethyl, tetrahydrothiopyranylmethyl, 1,3-dioxolanylmethyl, 1,4-dioxanylmethyl, pyrrolinylmethyl, pyrrolidinylmethyl, imidazolidinylmethyl, pyrazolidinylmethyl, morpholinylmethyl, 2-(morpholinyl)ethyl, tetrahydro-1,4-thiazinylmethyl, 2-(tetrahydro-1,4-thiazinyl)ethyl, piperidinylmethyl, 2-(piperidinyl)ethyl, homopiperidinylmethyl, piperazinylmethyl, 2-(piperazinyl)ethyl, homopiperazinylmethyl or 2-azabicyclo[2.2.1]heptylmethyl,
      and wherein any CH, CH₂ or CH₃ group within the Q¹ group optionally bears on each said CH, CH₂ or CH₃ group a substituent selected from hydroxy, amino, cyano, carbamoyl, methoxy, ethoxy, methylsulphonyl, methylamino, ethylamino, dimethylamino, diethylamino, methoxycarbonyl, ethoxycarbonyl, *N*-methylcarbamoyl, *N*-ethylcarbamoyl, *N*-isopropylcarbamoyl, *N,N*-dimethylcarbamoyl, *N,N*-diethylcarbamoyl, acetyl, propionyl, butyryl, pivaloyl, acetamido, propionamido and *N*-methylacetamido,
      and wherein any aryl, (3-8C)cycloalkyl, heteroaryl or heterocyclyl group within the Q¹ group optionally bears 1 or 2 substituents, which may be the same or different, selected from fluoro, chloro, trifluoromethyl, hydroxy, amino, carbamoyl, methyl, ethyl, methoxy, ethoxy, methylamino, dimethylamino, hydroxymethyl, 2-hydroxyethyl, methoxymethyl, 2-methoxyethyl, cyanomethyl, 2-cyanoethyl, aminomethyl, 2-aminoethyl, methylaminomethyl, 2-methylaminoethyl, dimethylaminomethyl, 2-dimethylaminoethyl, pyrrolidinylmethyl, morpholinylmethyl, piperidinylmethyl, homopiperidinylmethyl, piperazinylmethyl and homopiperazinylmethyl;
   (nn) Q¹ is methyl, ethyl, propyl, butyl, pentyl, aminomethyl, 2-aminoethyl, 3-aminopropyl, 4-aminobutyl, 5-aminopentyl, methylaminomethyl, 2-methylaminoethyl, 3-methylaminopropyl, 4-methylaminobutyl, 5-methylaminopentyl, dimethylaminomethyl, 2-dimethylaminoethyl, 3-dimethylaminopropyl, 4-dimethylaminobutyl or 5-dimethylaminopentyl, or Q¹ is phenyl, benzyl, 2-phenylethyl, cyclopentyl, cyclohexyl, cyclopentylmethyl, cyclohexylmethyl, thienyl, imidazolyl, thiazolyl, thiadiazolyl, thienylmethyl, imidazolylmethyl, thiazolylmethyl, thiadiazolylmethyl, tetrahydrofuranyl, tetrahydropyranyl, tetrahydrothiopyranyl, pyrrolinyl, pyrrolidinyl, morpholinyl, tetrahydro-1,4-thiazinyl, piperidinyl, homopiperidinyl, piperazinyl, homopiperazinyl, indolinyl, isoindolinyl, pyrrolidinylmethyl, morpholinylmethyl, 2-(morpholinyl)ethyl, piperidinylmethyl, 2-(piperidinyl)ethyl, homopiperidinylmethyl, piperazinylmethyl, 2-(piperazinyl)ethyl, homopiperazinylmethyl or 2-azabicyclo[2.2.1]heptylmethyl,
      and wherein any aryl, (3-8C)cycloalkyl, heteroaryl or heterocyclyl group within the Q¹ group optionally bears a substituent selected from fluoro, chloro, trifluoromethyl, hydroxy, amino, methyl, methoxy, methylamino and dimethylamino and any such aryl, (3-8C)cycloalkyl, heteroaryl or heterocyclyl group within the Q¹ group optionally bears a further substituent selected from aminomethyl, methylaminomethyl and dimethylaminomethyl;
   (oo) Q¹ is methyl, ethyl, propyl, isopropyl, butyl, pentyl, allyl, 2-methoxyethyl, 3-methoxypropyl, 2-ethoxyethyl, 3-ethoxypropyl, cyanomethyl, 2-cyanoethyl, 3-cyanopropyl, 1-cyano-1-methylethyl, 4-cyanobutyl, 5-cyanopentyl, aminomethyl, 2-aminoethyl, 3-aminopropyl, 4-aminobutyl, 5-aminopentyl, methylaminomethyl, 2-methylaminoethyl, 3-methylaminopropyl, 4-methylaminobutyl, 5-methylaminopentyl, ethylaminomethyl, 2-ethylaminoethyl, 3-ethylaminopropyl, 4-ethylaminobutyl, 5-ethylaminopentyl, dimethylaminomethyl, 2-dimethylaminoethyl, 3-dimethylaminopropyl, 4-dimethylaminobutyl, 5-dimethylaminopentyl, diethylaminomethyl, 2-diethylaminoethyl, 3-diethylaminopropyl, 4-diethylaminobutyl, 5-diethylaminopentyl, 2-methylsulphonylethyl or acetamidomethyl, or Q¹ is phenyl, benzyl, 2-phenylethyl, cyclopropyl, cyclobutyl, cyclopentyl, cyclohexyl, cyclopropylmethyl, cyclobutylmethyl, cyclopentylmethyl, cyclohexylmethyl, furyl, thienyl, oxazolyl, isoxazolyl, imidazolyl, pyrazolyl, thiazolyl, triazolyl, oxadiazolyl, thiadiazolyl, tetrazolyl, pyridyl, pyrazinyl, pyridazinyl, pyrimidinyl, furylmethyl, thienylmethyl, oxazolylmethyl, isoxazolylmethyl, imidazolylmethyl, 2-imidazolylethyl, pyrazolylmethyl, thiazolylmethyl, triazolylmethyl, oxadiazolylmethyl, thiadiazolylmethyl, tetrazolylmethyl, pyridylmethyl, 2-pyridylethyl, pyrazinylmethyl, 2-pyrazinylethyl, pyridazinylmethyl, 2-pyridazinylethyl, pyrimidinylmethyl, 2-pyrimidinylethyl, tetrahydrofuranyl, tetrahydropyranyl, tetrahydrothiopyranyl, azetidinyl, pyrrolinyl, pyrrolidinyl, morpholinyl, tetrahydro-1,4-thiazinyl, piperidinyl, homopiperidinyl, piperazinyl, homopiperazinyl, indolinyl, isoindolinyl, tetrahydrofuranylmethyl, tetrahydropyranylmethyl, 1,3-dioxolanylmethyl, 1,4-dioxanylmethyl, pyrrolidinylmethyl, morpholinylmethyl, 2-(morpholinyl)ethyl, piperidinylmethyl, 2-(piperidinyl)ethyl, homopiperidinylmethyl, piperazinylmethyl, 2-(piperazinyl)ethyl or homopiperazinylmethyl,
      and wherein any CH, CH₂ or CH₃ group within the Q¹ group optionally bears on each said CH, CH₂ or CH₃ group a substituent selected from hydroxy, amino, cyano, carbamoyl, methoxy, ethoxy, methylsulphonyl, methylamino, dimethylamino, methoxycarbonyl, ethoxycarbonyl, *N*-methylcarbamoyl, *N*-ethylcarbamoyl, *N*-isopropylcarbamoyl, *N,N*-dimethylcarbamoyl, acetyl, propionyl, pivaloyl, acetamido and *N*-methylacetamido,
      and wherein any aryl, (3-8C)cycloalkyl, heteroaryl or heterocyclyl group within the Q¹ group optionally bears 1 or 2 substituents, which may be the same or different, selected from fluoro, chloro, trifluoromethyl, hydroxy, amino, carbamoyl, methyl, methoxy, methylamino and dimethylamino and any such aryl, (3-8C)cycloalkyl, heteroaryl or heterocyclyl group within the Q¹ group optionally bears a substituent selected from hydroxymethyl, methoxymethyl, cyanomethyl, aminomethyl, methylaminomethyl, dimethylaminomethyl, pyrrolidinylmethyl, morpholinylmethyl, piperidinylmethyl and piperazinylmethyl;
   (pp) the X¹-Q¹ group is an α-amino carboxamido group;
   (qq) the X¹-Q¹ group is a naturally-occurring α-amino carboxamido group; and
   (rr) the X¹-Q¹ group is selected from glycylamino, sarcosylamino, (*N*,*N*-dimethylglycyl)amino, glycylglycylamino, L-alanylamino, 2-methylalanylamino, (*N*-methylalanyl)amino, (2S)-2-aminobutanoylamino, L-valylamino, (*N*-methyl-L-valyl)amino, 2-aminopent-4-ynoylamino, 2-aminopentanoylamino, L-isoleucylamino, L-leucylamino, 2-methyl-L-leucylamino, (*N*-methyl-L-leucyl)amino, serylamino, (*O*-methyl-L-seryl)amino, (*N*-methyl-L-seryl)amino, (*O*-methyl-L-homoseryl)amino, L-threonylamino, (*S*-methyl-L-cysteinyl)amino, (*S*-methyl-L-homocysteinyl)amino, L-methionylamino, (*N*-methyl-L-lysyl)amino, (*N*-methyl-L-ornithyl)amino, D-asparaginylamino, D-glutaminylamino, L-tyrosylamino, prolylamino and histidylamino.

A particular compound of the invention is a pyrimidine derivative of the Formula I wherein :-
p is 1 or 2, and a first R¹ group is selected from hydroxy, carbamoyl, acetamido, propionamido, *N*-methylacetamido, *N-*methylpropionamido, hydroxymethyl, 1-hydroxyethyl and 1-hydroxy-1-methylethyl, and the optional second R¹ group is selected from fluoro, chloro, trifluoromethyl, cyano, hydroxy, methyl, ethyl, methoxy and ethoxy;
R² is hydrogen or methyl;
q is 0 or q is 1 and the R³ group is methyl;
r is 0 or r is 1 and the R⁴ group is selected from fluoro, chloro, trifluoromethyl, hydroxy, amino, methyl, methoxy, methylamino and dimethylamino;
the X¹-Q¹ group is located at the 3-position;
X¹ is selected from NHCO, NHCONH, NHCOCH₂O, NHCOCH₂NH and NHCOCH₂NHCO; and
Q¹ is methyl, ethyl, propyl, butyl, pentyl, aminomethyl, 2-aminoethyl, 2-amino-2-methylpropyl, 3-aminopropyl, 4-aminobutyl, 5-aminopentyl, methylaminomethyl, 2-methylaminoethyl, 3-methylaminopropyl, 4-methylaminobutyl, 5-methylaminopentyl, dimethylaminomethyl, 2-dimethylaminoethyl, 3-dimethylaminopropyl, 4-dimethylaminobutyl or 5-dimethylaminopentyl, or Q¹ is phenyl, benzyl, 2-phenylethyl, cyclopentyl, cyclohexyl, cyclopentylmethyl, cyclohexylmethyl, thienyl, imidazolyl, thiazolyl, thiadiazolyl, thienylmethyl, imidazolylmethyl, thiazolylmethyl, thiadiazolylmethyl, tetrahydrofuranyl, tetrahydropyranyl, tetrahydrothiopyranyl, pyrrolinyl, pyrrolidinyl, morpholinyl, tetrahydro-1,4-thiazinyl, piperidinyl, homopiperidinyl, piperazinyl, homopiperazinyl, indolinyl, isoindolinyl, pyrrolidinylmethyl, morpholinylmethyl, 2-(morpholinyl)ethyl, piperidinylmethyl, 2-(piperidinyl)ethyl, piperidinyloxymethyl, homopiperidinylmethyl, piperazinylmethyl, 2-(piperazinyl)ethyl, homopiperazinylmethyl or 2-azabicyclo[2.2.1]heptylmethyl,
and wherein any aryl, (3-8C)cycloalkyl, heteroaryl or heterocyclyl group within the Q¹ group optionally bears a substituent selected from fluoro, chloro, trifluoromethyl, hydroxy, amino, methyl, methoxy, methylamino and dimethylamine and any such aryl, (3-8C)cycloalkyl, heteroaryl or heterocyclyl group within the Q¹ group optionally bears a further substituent selected from aminomethyl, methylaminomethyl and dimethylaminomethyl; or a pharmaceutically-acceptable salt, solvate or pro-drug thereof.

A further particular compound of the invention is a pyrimidine derivative of the Formula I wherein :-
p is 1 or 2, and a first R¹ group is selected from hydroxy, carbamoyl, acetamido, propionamido, *N*-methylacetamido, *N*-methylpropionamido, hydroxymethyl, 1-hydroxyethyl and 1-hydroxy-1-methylethyl, and the optional second R¹ group is selected from fluoro, chloro, trifluoromethyl, cyano, hydroxy, methyl, ethyl, methoxy and ethoxy;
R² is hydrogen or methyl;
q is 0 or q is 1 and the R³ group is methyl;
r is 0 or r is 1 and the R⁴ group is selected from fluoro, chloro and methyl;
the X¹-Q¹ group is located at the 3- or 4-position;
X¹ is selected from CO, NHCO, N(Me)CO, CONH, CON(Me), NHCONH, NHCOCH₂O, NHCOCH₂NH and NHCOCH₂NHCO; and
Q¹ is methyl, ethyl, propyl, isopropyl, butyl, pentyl, allyl, 2-methoxyethyl, 3-methoxypropyl, 2-ethoxyethyl, 3-ethoxypropyl, cyanomethyl, 2-cyanoethyl, 3-cyanopropyl, 1-cyano-1-methylethyl, 4-cyanobutyl, 5-cyanopentyl, aminomethyl, 2-aminoethyl, 3-aminopropyl, 4-aminobutyl, 5-aminopentyl, methylaminomethyl, 2-methylaminoethyl, 3-methylaminopropyl, 4-methylaminobutyl, 5-methylaminopentyl, ethylaminomethyl, 2-ethylaminoethyl, 3-ethylaminopropyl, 4-ethylaminobutyl, 5-ethylaminopentyl, dimethylaminomethyl, 2-dimethylaminoethyl, 3-dimethylaminopropyl, 4-dimethylaminobutyl, 5-dimethylaminopentyl, diethylaminomethyl, 2-diethylaminoethyl, 3-diethylaminopropyl, 4-diethylaminobutyl, 5-diethylaminopentyl, 2-methylsulphonylethyl or acetamidomethyl, or Q¹ is phenyl, benzyl, 2-phenylethyl, cyclopropyl, cyclobutyl, cyclopentyl, cyclohexyl, cyclopropylmethyl, cyclobutylmethyl, cyclopentylmethyl, cyclohexylmethyl, furyl, thienyl, oxazolyl, isoxazolyl, imidazolyl, pyrazolyl, thiazolyl, triazolyl, oxadiazolyl, thiadiazolyl, tetrazolyl, pyridyl, pyrazinyl, pyridazinyl, pyrimidinyl, furylmethyl, thienylmethyl, oxazolylmethyl, isoxazolylmethyl, imidazolylmethyl, 2-imidazolylethyl, pyrazolylmethyl, thiazolylmethyl, triazolylmethyl, oxadiazolylmethyl, thiadiazolylmethyl, tetrazolylmethyl, pyridylmethyl, 2-pyridylethyl, pyrazinylmethyl, 2-pyrazinylethyl, pyridazinylmethyl, 2-pyridazinylethyl, pyrimidinylmethyl, 2-pyrimidinylethyl, tetrahydrofuranyl, tetrahydropyranyl, tetrahydrothiopyranyl, azetidinyl, pyrrolinyl, pyrrolidinyl, morpholinyl, tetrahydro-1,4-thiazinyl, piperidinyl, homopiperidinyl, piperazinyl, homopiperazinyl, indolinyl, isoindolinyl, tetrahydrofuranylmethyl, tetrahydropyranylmethyl, 1,3-dioxolanylmethyl, 1,4-dioxanylmethyl, pyrrolidinylmethyl, morpholinylmethyl, 2-(morpholinyl)ethyl, piperidinylmethyl, 2-(pipendinyl)ethyl, homopiperidinylmethyl, piperazinylmethyl, 2-(piperazinyl)ethyl or homopiperazinylmethyl,
and wherein any CH, CH₂ or CH₃ group within the Q¹ group optionally bears on each said CH, CH₂ or CH₃ group a substituent selected from hydroxy, amino, cyano, carbamoyl, methoxy, ethoxy, methylsulphonyl, methylamino, dimethylamino, methoxycarbonyl, ethoxycarbonyl, *N*-methylcarbamoyl, *N*-ethylcarbamoyl, *N*-isopropylcarbamoyl, *N,N*-dimethylcarbamoyl, acetyl, propionyl, pivaloyl, acetamido and *N*-methylacetamido,
and wherein any aryl, (3-8C)cycloalkyl, heteroaryl or heterocyclyl group within the Q¹ group optionally bears 1 or 2 substituents, which may be the same or different, selected from fluoro, chloro, trifluoromethyl, hydroxy, amino, carbamoyl, methyl, methoxy, methylamino and dimethylamino and any such aryl, (3-8C)cycloalkyl, heteroaryl or heterocyclyl group within the Q¹ group optionally bears a substituent selected from hydroxymethyl, methoxymethyl, cyanomethyl, aminomethyl, methylaminomethyl, dimethylaminomethyl, pyrrolidinylmethyl, morpholinylmethyl, piperidinylmethyl and piperazinylmethyl; or a pharmaceutically-acceptable salt, solvate or pro-drug thereof.

A further particular compound of the invention is a pyrimidine derivative of the Formula I wherein :-
p is 1 or 2, and a first R¹ group is selected from hydroxy, carbamoyl, acetamido, propionamido, *N*-methylacetamido, *N-*methylpropionamido, hydroxymethyl, 1-hydroxyethyl and 1-hydroxy-1-methylethyl, and the optional second R¹ group is selected from fluoro, chloro, trifluoromethyl, cyano, hydroxy, methyl, ethyl, methoxy and ethoxy;
R² is hydrogen or methyl;
q is 0 or q is 1 and the R³ group is methyl;
r is 0 or r is 1 and the R⁴ group is selected from fluoro, chloro, trifluoromethyl, hydroxy, amino, methyl, methoxy, methylamino and dimethylamino; and
the X¹-Q¹ group is located at the 3-position and is selected from glycylamino, sarcosylamino, (*N,N-*dimethylglycyl)amino, glycylglycylamino, L-alanylamino, 2-methylalanylamino, (*N*-methylalanyl)amino, (2S)-2-aminobutanoylamino, L-valylamino, (*N*-methyl-L-valyl)amino, 2-aminopent-4-ynoylamino, 2-aminopentanoylamino, L-isoleucylamino, L-leucylamino, 2-methyl-leucylamino, (*N-*methyl-L-leucyl)amino, serylamino, (*O*-methyl-L-seryl)amino, (*N*-methyl-L-seryl)amino, (*O*-methyl-L-homoseryl)amino, L-threonylamino, (*S*-methyl-L-cysteinyl)amino, (*S*-methyl-L-homocysteinyl)amino, L-methionylamino, (*N*-methyl-L-lysyl)amino, (*N*-methyl-L-ornithyl)amino, D-asparaginylamino, D-glutaminylamino, L-tyrosylamino, prolylamino and histidylamino;
or a pharmaceutically-acceptable salt, solvate or pro-drug thereof.

A further particular compound of the invention is a pyrimidine derivative of the Formula I wherein :-
p is 1 and the R¹ group is located at the 3- or 4-position and is selected from hydroxy, carbamoyl, acetamido, hydroxymethyl, 1-hydroxyethyl and 1-hydroxy-1-methylethyl;
R² is hydrogen;
q is 0;
r is 0;
the X¹-Q¹ group is located at the 3-position;
X¹ is NHCO; and
Q¹ is methyl, aminomethyl, 2-aminopropyl, 2-amino-2-methylpropyl, 4-aminobutyl, 5-aminopentyl, methylaminomethyl, dimethylaminomethyl or 5-dimethylaminopentyl, or Q¹ is phenyl, benzyl, 2-phenylethyl, cyclopentyl, cyclohexyl, cyclohexylmethyl, thiazol-5-yl, thien-3-ylmethyl, imidazol-1-ylmethyl, 1,2,4-thiadiazol-3-ylmethyl, tetrahydropyran-4-yl, tetrahydrothiopyran-4-yl, 3-pyrrolin-2-yl, pymolidin-2-yl, pyrrolidin-3-yl, morpholin-2-yl, piperidin-2-yl, piperidin-3-yl, piperidin-4-yl, piperazin-1-yl, isoindolin-1-yl, pyrolidin-2-ylmethyl, piperidin-4-ylmethyl, 2-(piperidin-4-yl)ethyl; piperidin-4-yloxymethyl, piperazin-1-ylmethyl or 2-azabicyclo[2.2.1]hept-2-ylmethyl,
and wherein any aryl, (3-8C)cycloalkyl, heteroaryl or heterocyclyl group within the Q¹ group optionally bears a substituent selected from amino, methyl, methylamino and aminomethyl;
or a pharmaceutically-acceptable salt, solvate or pro-drug thereof.

A further particular compound of the invention is a pyrimidine derivative of the Formula I wherein :-
p is 1 and the R¹ group is located at the 3- or 4-position and is selected from hydroxy, acetamido, hydroxymethyl, 1-hydroxyethyl and 1-hydroxy-1-methylethyl;
R² is hydrogen;
q is 0;
r is 0 or r is 1 and the R⁴ group is selected from fluoro, chloro and methyl;
the X¹-Q¹ group is located at the 3- or 4-position;
X¹ is NHCO, N(Me)CO, CONH or CON(Me); and
Q¹ is methyl, ethyl; propyl, isopropyl, 2-ethoxyethyl, 3-ethoxypropyl, cyanomethyl, 2-cyanoethyl, aminomethyl, 2-aminoethyl, methylaminomethyl, 2-methylaminoethyl, ethylaminomethyl, 2-ethylaminoethyl, dimethylaminomethyl, 2-dimethylaminoethyl, 4-dimethylaminobutyl, 2-methylsulphonylethyl or acetamidomethyl, or Q¹ is phenyl, benzyl, cyclopropyl, cyclobutyl, cyclopentyl, cyclohexyl, cyclopropylmethyl, cyclobutylmethyl, cyclopentylmethyl, cyclohexylmethyl, oxazol-5-yl, isoxazol-3-yl, isoxazol-4-yl, imidazol-2-yl, imidazol-4-yl, pyrazol-3-yl, thiazol-5-yl, 1,2,3-triazol-5-yl, tetrazol-5-yl, pyridin-2-yl, pyridin-3-yl, pyridin-4-yl, pyrazin-2-yl, pyridazin-4-yl, pyrimidin-2-yl, pyrimidin-4-yl, thien-3-ylmethyl, oxazol-4-ylmethyl, isoxazol-3-ylmethyl, isoxazol-4-ylmethyl, imidazol-1-ylmethyl, imidazol-2-ylmethyl, 2-imidazol-1-ylethyl, 2-imidazol-2-ylethyl, 2-imidazol-4-ylethyl, pyrazol-1-ylmethyl, pyrazol-3-ylmethyl, 1,2,3-triazol-1-ylmethyl, 1,2,3-triazol-4-ylmethyl, 1,2,4-oxadiazol-3-ylmethyl, 1,2,3-thiadiazol-3-ylmethyl, tetrazol-1-ylmethyl, tetrazol-5-ylmethyl, pyridin-2-ylmethyl, pyridin-3-ylmethyl, pyridin-4-ylmethyl, 2-pyridin-2-ylethyl, 2-pyridin-3-ylethyl, 2-pyridin-4-ylethyl, pyrazin-2-ylmethyl, 2-pyrazin-2-ylethyl, pyridazin-4-ylmethyl, 2-pyridazin-4-ylethyl, pyrimidin-2-ylmethyl, pyrimidin-4-ylmethyl, 2-pyrimidin-2-ylethyl, 2-pyrimidin-4-ylethyl, tetrahydrofuran-2-yl, tetrahydropyran-4-yl, tetrahydrothiopyran-4-yl, azetidin-2-yl, 3-pyrrolin-2-yl, pyrrolidin-1-yl, pyrrolidin-2-yl, pyrrolidin-3-yl, morpholino, morpholin-2-yl, piperidino, piperidin-2-yl, piperidin-3-yl, piperidin-4-yl, piperazin-1-yl, isoindolin-1-yl, tetrahydrofuran-2-ylmethyl, tetrahydropyran-4-ylmethyl, 1,3-dioxolan-2-ylmethyl, 1,4-dioxan-2-ylmethyl, pyrrolidin-2-ylmethyl, piperidin-2-ylmethyl, piperidin-3-ylmethyl, piperidin-4-ylmethyl, 2-(piperidin-4-yl)ethyl, piperidin-4-yloxymethyl, piperazin-1-ylmethyl or 2-(piperazin-1-yl)ethyl,
and wherein any CH, CH₂ or CH₃ group within the Q¹ group optionally bears on each said CH, CH₂ or CH₃ group a substituent selected from hydroxy, carbamoyl, methoxycarbonyl, ethoxycarbonyl, *N*-methylcarbamoyl, *N*-ethylcarbamoyl, *N*-isopropylcarbamoyl, *N,N*-dimethylcarbamoyl, acetyl, propionyl, pivaloyl, acetamido and *N*-methylacetamido,
and wherein any aryl, (3-8C)cycloalkyl, heteroaryl or heterocyclyl group within the Q¹ group optionally bears 1 or 2 substituents, which may be the same or different, selected from fluoro, chloro, hydroxy, amino, carbamoyl, methyl, methylamino, dimethylamino, hydroxymethyl, methoxymethyl, cyanomethyl, aminomethyl, methylaminomethyl, dimethylaminomethyl and 1-methylpiperidin-4-ylmethyl;
or a pharmaceutically-acceptable salt, solvate or pro-drug thereof.

A further particular compound of the invention is a pyrimidine derivative of the Formula I wherein :-
p is 1 and R¹ is a hydroxy or hydroxymethyl group that is located at the 3-position;
R² is hydrogen;
q is 0;
r is 0; and
the X¹-Q¹ group is located at the 3-position and is selected from glycylamino, glycylglycylamino, L-alanylamino, (2S)-2-aminobutanoylamino, L-isoleucylamino, L-leucylamino, 2-methyl-L-leucylamino and (*N*-methyl-L-leucyl)amino; or a pharmaceutically-acceptable salt, solvate or pro-drug thereof.

A further particular compound of the invention is a pyrimidine derivative of the Formula I wherein :-
p is 1 and R¹ is a hydroxy or hydroxymethyl group that is located at the 3-position;
R² is hydrogen;
q is 0;
r is 0;
the X¹-Q¹ group is located at the 3-position;
X¹ is NHCO; and
Q¹ is aminomethyl, 2-aminopropyl, 2-amino-2-methylpropyl, 4-aminobutyl, 5-aminopentyl, 3-aminomethylphenyl, 4-aminomethylphenyl, 2-aminocyclopent-1-yl, 4-aminocyclohex-1-yl, 3-aminocyclohex-1-ylmethyl, 4-aminomethylcyclohex-1-yl, imidazol-1-ylmethyl, 5-amino-1,2,4-thiadiazol-3-ylmethyl, pyrrolidin-3-yl, *N*-methylpyrrolidin-2-yl, piperidin-2-yl, piperidin-3-yl, piperidin-4-yl, *N*-methylpiperidin-4-yl, pyrrolidin-2-ylmethyl, piperidin-4-ylmethyl, 2-(piperidin-4-yl)ethyl, piperidin-4-yloxymethyl or 4-methylpiperazin-1-ylmethyl,
or a pharmaceutically-acceptable salt, solvate or pro-drug thereof.

A further particular compound of the invention is a pyrimidine derivative of the Formula I wherein :-
p is 1 and R¹ is a hydroxy or hydroxymethyl group that is located at the 3-position;
R² is hydrogen;
q is 0;
r is 0 or r is 1 and the R⁴ group is selected from fluoro and methyl;
the X¹-Q¹ group is located at the 3- or 4-position;
X¹ is NHCO or N(Me)CO; and
Q¹ is aminomethyl, methylaminomethyl, ethylaminomethyl, dimethylaminomethyl, acetamidomethyl, 3-aminomethylphenyl, 4-aminomethylphenyl, 5-methylisoxazol-3-yl, 1-methylpyrazol-3-yl, 1*H*-1,2,3-triazol-5-yl, pyridin-4-yl, pyrazin-2-yl, 2-imidazol-1-ylethyl, 2-imidazol-2-ylethyl, 3,5-dimethyl-1*H* pyrazol-1-ylmethyl, 1*H*-tetrazol-5-ylmethyl, 2-pyridin-3-ylethyl, 2-pyridazin-4-ylethyl, azetidin-2-yl, 3-pyrrolin-2-yl, *N*-methylpymolidin-2-yl, 4-hydroxypyrrolidin-2-yl, piperidin-3-yl, piperidin-4-yl, *N*-methylpiperidin-4-yl, piperazin-1-yl, piperidin-3-ylmethyl, piperidin-4-yloxymethyl or piperazin-1-ylmethyl;
or a pharmaceutically-acceptable salt, solvate or pro-drug thereof.

A further particular compound of the invention is a pyrimidine derivative of the Formula I wherein:-
p is 1 and R¹ is a hydroxy or hydroxymethyl group that is located at the 3-position;
R² is hydrogen;
q is 0;
r is 0 or r is 1 and the R⁴ group is selected from fluoro and methyl;
the X¹-Q¹ group is located at the 3- or 4-position;
X¹ is CONH or CON(Me); and
Q¹ is methyl, ethyl, propyl, isopropyl, 2-ethoxyethyl, 3-ethoxypropyl, cyanomethyl, 1-cyano-1-methylethyl, 2-cyanoethyl, 5-cyanopentyl, 2-aminoethyl, 2-methylaminoethyl, 2-dimethylaminoethyl, 4-dimethylaminobutyl, 2-methylsulphonylethyl, 3-methoxycarbonylpropyl, carbamoylmethyl, 1-carbamoylethyl, 2-carbamoylethyl, *N*-methylcarbamoylmethyl, *N*-isopropylcarbamoylmethyl, *N*,*N*-dimethylcarbamoylmethyl, pivaloylmethyl, 4-aminomethylphenyl; 4-aminobenzyl, cyclopropyl, cyclobutyl, cyclopentyl, cyclopropylmethyl, cyclobutylmethyl, cyclopentylmethyl, thien-3-ylmethyl, oxazol-4-ylmethyl, 5-methylisoxazol-3-ylmethyl, isoxazol-4-ylmethyl, 1*H*-imidazol-1-ylmethyl, 1*H*-imidazol-2-ylmethyl, 2-(1*H*-imidazol-1-yl)ethyl, 2-(1*H*-imidazol-2-yl)ethyl, 2-(1*H*-imidazol-4-yl)ethyl, pyridin-2-ylmethyl, pyridin-3-ylmethyl, pyridin-4-ylmethyl, 2-pyridin-2-ylethyl, 2-pyridin-3-ylethyl, 2-pyridin-4-ylethyl, pyrazin-2-ylmethyl, 5-methylpyrazin-2-ylmethyl, tetrahydropyran-4-yl, tetrahydrothiopyran-4-yl, tetrahydrofuran-2-ylmethyl, tetrahydropyran-4-ylmethyl, 1,3-dioxolan-2-ylmethyl or 1,4-dioxan-2-ylmethyl;
or a pharmaceutically-acceptable salt, solvate or pro-drug thereof.

A further particular compound of the invention is a pyrimidine derivative of the Formula I wherein :-
p is 1 and R¹ is a hydroxy or hydroxymethyl group that is located at the 3-position;
R² is hydrogen;
q is 0;
r is 0 or r is 1 and the R⁴ group is selected from fluoro and methyl;
the X¹-Q¹ group is located at the 3- or 4-position;
X¹ is CO; and
Q¹ is 2-carbamoylpyrrolidin-1-yl, 2-methoxymethylpyrrolidin-1-yl, 4-aminopiperidin-1-yl, 4-aminomethylpiperidin-1-yl, 3-cyanomethylpiperidin-1-yl, 3-oxopiperazin-1-yl, 4-(1-methylpiperidin-4-ylmethyl)piperazin-1-yl or 5-oxo-1,4-diazepan-1-yl;
or a pharmaceutically-acceptable salt, solvate or pro-drug thereof.

A further particular compound of the invention is a pyrimidine derivative of the Formula I wherein :-
p is 1 and R¹ is a hydroxy or hydroxymethyl group that is located at the 3-position;
R² is hydrogen;
q is 0;
r is 0;
the X¹-Q¹ group is located at the 3-position;
X¹ is NHCO; and
Q¹ is 2-amino-2-methylpropyl, 5-aminopentyl, 3-aminomethylphenyl, 4-aminomethylphenyl, 2-aminocyclopent-1-yl, 4-aminocyclohex-1-yl, 3-aminocyclohex-1-ylmethyl, piperidin-3-yl, piperidin-4-yl, piperidin-4-ylmethyl or pigeridin-4-yloxymethyl;
or a pharmaceutically-acceptable salt, solvate or pro-drug thereof.

Particular compounds of the invention are, for example, the pyrimidine derivatives of the Formula I that are disclosed within Examples 4(3), 6(20); 6(24), 6(27), 6(29), 6(32), 6(33), 6(37), 6(41), 6(44), 6(48), 6(49), 8(2), 11 and 15 that are set out hereinafter.

A particular compound of the invention is, for example, a pyrimidine derivative of the Formula I selected from :-
4-(3-hydroxyphenyl)-6-morpholino-2-(3-piperidin-4-ylcarbonylaminophenyl)pyrimidine, 2-[3-(6-dimethylaminohexanoylamino)phenyl]-4-(3-hydroxymethylphenyl)-6-morpholinopyrimidine,
2-(3-{2-[(1R,3S)-3-aminocyclohex-1-yl]acetamido]phenyl}-4-(3-hydroxymethylphenyl)-6-morpholinopyrimidine,
4-(3-hydroxymethylphenyl)-6-morpholino-2-[3-(2-piperidin-4-yloxyacetamido)phenyl]-pyrimidine,
2-[3-(3-aminomethylbenzamido)phenyl]-4-(3-hydroxymethylphenyl)-6-morpholinopyrimidine, 4-(3-hydroxymethylphenyl)-6-morpholino-2-(3-piperidin-4-ylcarbonylaminophenyl)pyrimidine and 4-(3-hydroxymethylphenyl)-6-morpholino-2-(3-piperazin-1-ylcarbonylaminophenyl)-pyrimidine;
   or a pharmaceutically-acceptable salt, solvate or pro-drug thereof.

A pyrimidine derivative of the Formula I, or a pharmaceutically-acceptable salt, solvate or pro-drug thereof, may be prepared by any process known to be applicable to the preparation of chemically-related compounds. Such processes, when used to prepare a pyrimidine derivative of the Formula I are provided as a further feature of the invention and are illustrated by the following representative process variants in which, unless otherwise stated, p, R¹, R², q, R³, r, R⁴, X¹ and Q¹ have any of the meanings defined hereinbefore. Necessary starting materials may be obtained by standard procedures of organic chemistry. The preparation of such starting materials is described in conjunction with the following representative process variants and within the accompanying Examples. Alternatively necessary starting materials are obtainable by analogous procedures to those illustrated which are within the ordinary skill of an organic chemist.
(a) The reaction, conveniently in the presence of a suitable catalyst, of a pyrimidine of the Formula II 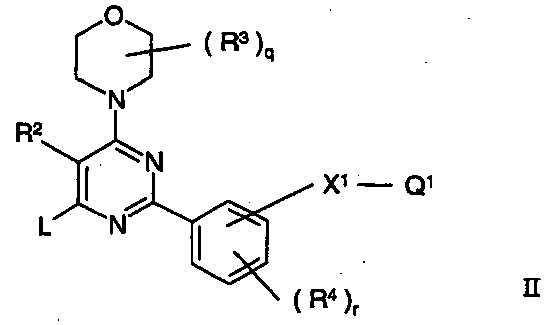 wherein L is a displaceable group and R², q, R³, r, R⁴, X¹ and Q¹ have any of the meanings defined hereinbefore except that any functional group is protected if necessary, with an organoboron reagent of the Formula III 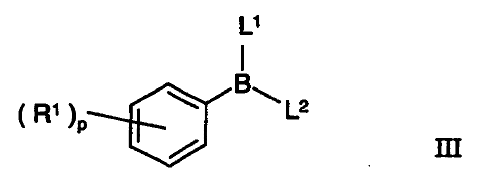 wherein each of L¹ and L², which may be the same or different, is a suitable ligand and p and R¹ have any of the meanings defined hereinbefore except that any functional group is protected if necessary, whereafter any protecting group that is present is removed by conventional means.
   A suitable displaceable group L is, for example, a halogeno, alkoxy, aryloxy or sulphonyloxy group, for example a chloro, bromo, methoxy, phenoxy, pentafluorophenoxy, methanesulphonyloxy or toluene-4-sulphonyloxy group.
   A suitable value for the ligands L¹ and L² which are present on the boron atom of the aryl-boron reagent include, for example, a hydroxy, (1-4C)alkoxy or (1-6C)alkyl ligand, for example a hydroxy, methoxy, ethoxy, propoxy, isopropoxy, butoxy, methyl, ethyl, propyl, isopropyl or butyl ligand. Alternatively the ligands L¹ and L² may be linked such that, together with the boron atom to which they are attached, they form a ring. For example, L¹ and L² together may define an oxy-(2-4C)alkylene-oxy group, for example an oxyethyleneoxy or oxytrimethyleneoxy group such that, together with the boron atom to which they are attached, they form a cyclic boronic acid ester group. Particularly suitable organoboron reagents include, for example, compounds wherein each of L¹ and L² is a hydroxy, a isopropoxy or an ethyl group.
   A suitable catalyst for the reaction includes, for example, a metallic catalyst such as a palladium(0), palladium(II), nickel(0) or nickel(II) catalyst, for example tetrakis(triphenylphosphine)palladium(0), palladium(II) chloride, palladium(II) bromide, bis(triphenylphosphine)palladium(II) chloride, tetrakis(triphenylphosphine)nickel(0), nickel(II) chloride, nickel(II) bromide or bis(triphenylphosphine)nickel(II) chloride or [1,1'-bis(diphenylphosphino)ferrocene]dichloropalladium(II). In addition, a free radical initiator may conveniently be added, for example an azo compound such as azo(bisisobutyronitrile). Conveniently, the reaction may be carried out in the presence of a suitable base such as an alkali or alkaline earth metal carbonate or hydroxide, for example sodium bicarbonate, sodium carbonate, potassium bicarbonate, potassium carbonate, calcium carbonate, caesium carbonate, sodium hydroxide or potassium hydroxide, or, for example, an alkali metal alkoxide, for example sodium *tert*-butoxide, or, for example, an alkali metal amide, for example sodium hexamethyldisilazane, or, for example, an alkali metal hydride, for example sodium hydride.
   The reaction is conveniently carried out in the presence of a suitable inert solvent or diluent, for example an ether such as tetrahydrofuran, 1,4-dioxan or 1,2-dimethoxyethane, an aromatic solvent such as benzene, toluene or xylene, or an alcohol such as methanol or ethanol, and the reaction is conveniently carried out at a temperature in the range, for example 10 to 250°C, preferably in the range 40 to 120°C.
   Aryl-boron reagents of the Formula III may be obtained by standard procedures of organic chemistry which are within the ordinary skill of an organic chemist, for example by the reaction of an aryl-metal reagent where the metal is, for example, lithium or the magnesium halide portion of a Grignard reagent, with an organoboron compound of the formula L-B(L¹)(L²) wherein L is a displaceable group as defined hereinbefore. Preferably the compound of the formula L-B(L¹)(L²) is, for example, boric acid or a tri-(1-4C)alkyl borate such as tri-isopropyl borate.
   In an alternative procedure, the aryl-boron reagent of the Formula III may be replaced with an organometallic compound of the formula aryl-M wherein M is a metal atom or a metallic group (that is a metal atom bearing suitable ligands). Suitable values for the metal atom include, for example, lithium and copper. Suitable values for the metallic group include, for example, groups which contain a tin, silicon, zirconium, aluminium, magnesium, mercury or zinc atom. Suitable ligands within such a metallic group include, for example, hydroxy groups, (1-6C)alkyl groups such as methyl, ethyl, propyl, isopropyl and butyl groups, halogeno groups such as chloro, bromo and iodo groups, and (1-6C)alkoxy groups such as methoxy, ethoxy, propoxy, isopropoxy and butoxy groups. A particular organometallic compound of the formula aryl-M is, for example, an organotin compound such as a compound of the formula aryl-SnBu₃, an organosilicon compound such as a compound of the formula aryl-Si(Me)F₂, an organozirconium compound such as a compound of the formula aryl-ZaCl₃, an organoaluminium compound such as a compound of the formula aryl-AlEt₂, an organomagnesium compound such as a compound of the formula aryl-MgBr, an organomercury compound such as a compound of the formula aryl-HgBr, or an organozinc compound such as a compound of the formula aryl-ZnBr.
   Protecting groups may in general be chosen from any of the groups described in the literature or known to the skilled chemist as appropriate for the protection of the group in question and may be introduced by conventional methods. Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.
   Specific examples of protecting groups are given below for the sake of convenience, in which "lower", as in, for example, lower alkyl, signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned are, of course, within the scope of the invention.
   A carboxy protecting group may be the residue of an ester-forming aliphatic or arylaliphatic alcohol or of an ester-forming silanol (the said alcohol or silanol preferably containing 1-20 carbon atoms). Examples of carboxy protecting groups include straight or branched chain (1-12C)alkyl groups (for example isopropyl, and *tert*-butyl); lower alkoxy- lower alkyl groups (for example methoxymethyl, ethoxymethyl and isobutoxymethyl); lower acyloxy-lower alkyl groups, (for example acetoxymethyl, propionyloxymethyl, butyryloxymethyl and pivaloyloxymethyl); lower alkoxycarbonyloxy-lower alkyl groups (for example 1-methoxycarbonyloxyethyl and 1-ethoxycarbonyloxyethyl); aryl-lower alkyl groups (for example benzyl, 4-methoxybenzyl, 2-nitrobenzyl, 4-nitrobenzyl, benzhydryl and phthalidyl); tri(lower alkyl)silyl groups (for example trimethylsilyl and *tert*-butyldimethylsilyl); tri(lower alkyl)silyl-lower alkyl groups (for example trimethylsilylethyl); and (2-6C)alkenyl groups (for example allyl). Methods particularly appropriate for the removal of carboxyl protecting groups include for example acid-, base-; metal- or enzymically-catalysed-cleavage.
   Examples of hydroxy protecting groups include lower alkyl groups (for example *tert*-butyl), lower alkenyl groups (for example allyl); lower alkanoyl groups (for example acetyl); lower alkoxycarbonyl groups (for example *tert*-butoxycarbonyl); lower alkenyloxycarbonyl groups (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); tri(lower alkyl)silyl (for example trimethylsilyl and *tert*-butyldimethylsilyl) and aryl-lower alkyl (for example benzyl) groups.
   Examples of amino protecting groups include formyl, aryl-lower alkyl groups (for example benzyl and substituted benzyl, 4-methoxybenzyl, 2-nitrobenzyl and 2,4-dimethoxybenzyl, and triphenylmethyl); di-4-anisylmethyl and furylmethyl groups; lower alkoxycarbonyl (for example *tert*-butoxycarbonyl); lower alkenyloxycarbonyl (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); trialkylsilyl (for example trimethylsilyl and *tert*-butyldimethylsilyl); alkylidene (for example methylidene) and benzylidene and substituted benzylidene groups.
   Methods appropriate for removal of hydroxy and amino protecting groups include, for example, acid-, base-, metal- or enzymically-catalysed hydrolysis for groups such as 2-nitrobenzyloxycarbonyl, hydrogenation for groups such as benzyl and photolytically for groups such as 2-nitrobenzyloxycarbonyl.
   The reader is referred to Advanced Organic Chemistry, 4th Edition, by J. March, published by John Wiley & Sons 1992, for general guidance on reaction conditions and reagents and to Protective Groups in Organic Synthesis, 2nd Edition, by T. Green et al., also published by John Wiley & Son, for general guidance on protecting groups.
   pyrimidine starting materials of the Formula II may be obtained by conventional procedures such as those disclosed in the Examples that are set out hereinafter. For example, for the production of those compounds of the Formula II wherein X¹ is N(R¹³)CO, an amine of the Formula X 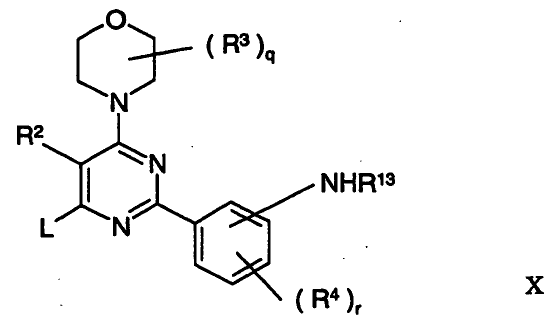 wherein L, R², q, R³, r, R⁴ and R¹³ have any of the meanings defined hereinbefore except that any functional group is protected if necessary, may be acylated, conveniently in the presence of a suitable base as defined hereinbefore, with a carboxylic acid of the Formula V

   HO₂C - Q¹ V

   or a reactive derivative thereof as defined hereinafter, wherein Q¹ has any of the meanings defined hereinbefore except that any functional group is protected if necessary, whereafter any protecting group that is present is removed by conventional means.
(b) For the production of those compounds of the Formula I wherein X¹ is N(R¹³)CO, the acylation, conveniently in the presence of a suitable base, of an amine of the Formula IV 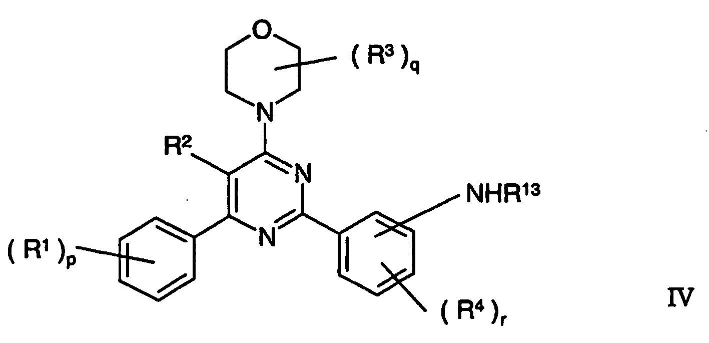 wherein p, R¹, R², q, R³, r, R⁴ and R¹³ have any of the meanings defined hereinbefore except that any functional group is protected if necessary, with a carboxylic acid of the Formula V

   HO₂C - Q¹ V

   or a reactive derivative thereof, wherein Q¹ has any of the meanings defined hereinbefore except that any functional group is protected if necessary, whereafter any protecting group that is present is removed by conventional means.
   A suitable base is, for example, an organic amine base such as, for example, pyridine, 2,6-lutidine, collidine, 4-dimethylaminopyridine, triethylamine, morpholine, *N*-methylmorpholine or diazabicyclo[5.4.0]undec-7-ene, or, for example, an alkali or alkaline earth metal carbonate or hydroxide, for example sodium carbonate, potassium carbonate, calcium carbonate, sodium hydroxide or potassium hydroxide, or, for example, an alkali metal amide, for example sodium hexamethyldisilazane, or, for example, an alkali metal hydride, for example sodium hydride.
   A suitable reactive derivative of a carboxylic acid of the Formula V is, for example, an acyl halide, for example an acyl chloride formed by the reaction of the acid with an inorganic acid chloride, for example thionyl chloride; a mixed anhydride, for example an anhydride formed by the reaction of the acid with a chloroformate such as isobutyl chloroformate; an active ester, for example an ester formed by the reaction of the acid with a phenol such as pentafluorophenol, with an ester such as pentafluorophenyl trifluoroacetate or with an alcohol such as methanol, ethanol, isopropanol, butanol or N-hydroxybenzotriazole; an acyl azide, for example an azide formed by the reaction of the acid with an azide such as diphenylphosphoryl azide; an acyl cyanide, for example a cyanide formed by the reaction of an acid with a cyanide such as diethylphosphoryl cyanide; or the product of the reaction of the acid with a carbodiimide such as dicyclohexylcarbodiimide or with a uronium compound such as 2-(7-azabenzotriazol-1-yl)-1,1,3,3-tetramethyluronium hexafluorophosphate(V).
   The reaction is conveniently carried out in the presence of a suitable inert solvent or diluent, for example an alcohol or ester such as methanol, ethanol, isopropanol or ethyl acetate, a halogenated solvent such as methylene chloride, chloroform or carbon tetrachloride, an ether such as tetrahydrofuran or 1,4-dioxan, an aromatic solvent such as toluene. Conveniently, the reaction is conveniently carried out in the presence of a dipolar aprotic solvent such as *N,N*-dimethylformamide, *N,N*-dimethylacetamide, *N*-methylpyrrolidin-2-one or dimethylsulphoxide. The reaction is conveniently carried out at a temperature in the range, for example, 0 to 120°C, preferably at or near ambient temperature.
   Pyrimidine starting materials of the Formula IV may be obtained by conventional procedures such as those disclosed in the Examples that are set out hereinafter.
   For example, a pyrimidine of the Formula XI 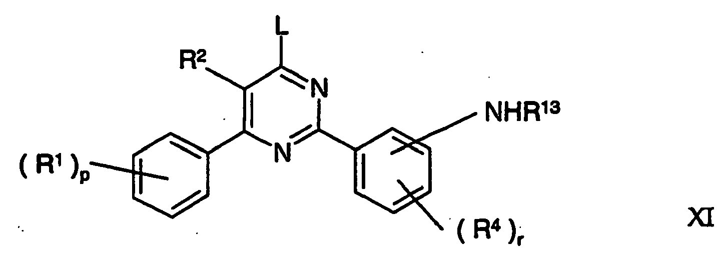 wherein L is a displaceable group as defined hereinbefore and p, R¹, R², r, R⁴ and R¹³ have any of the meanings defined hereinbefore except that any functional group is protected if necessary, may be reacted with a morpholine of the Formula VII 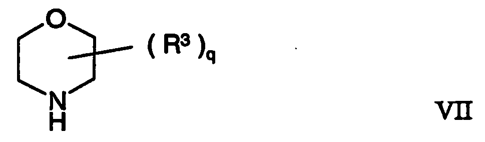 wherein q and R³ have any of the meanings defined hereinbefore except that any functional group is protected if necessary, whereafter any protecting group that is present is removed by conventional means.
   Alternatively, a pyrimidine of the Formula XII 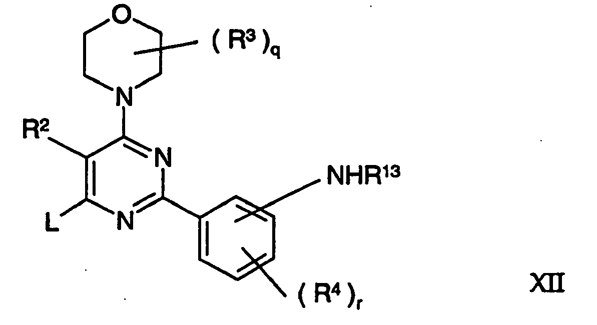 wherein L is a displaceable group as defined hereinbefore and R², q, R³, r, R⁴ and R¹³ have any of the meanings defined hereinbefore except that any functional group is protected if necessary, may be reacted, conveniently in the presence of a suitable catalyst as defined hereinbefore, with an organoboron reagent of the Formula III 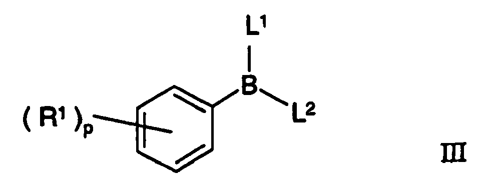 wherein each of L¹ and L², which may be the same or different, is a suitable ligand as defined hereinbefore and p and R¹ have any of the meanings defined hereinbefore except that any functional group is protected if necessary, whereafter any protecting group that is present is removed by conventional means.
(c) The reaction of a pyrimidine of the Formula VI 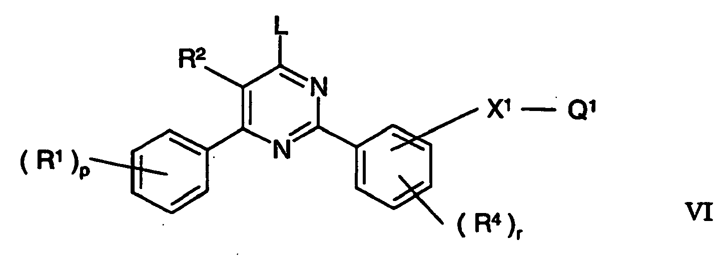 wherein L is a displaceable group as defined hereinbefore and p, R¹, R², r, R⁴, X¹ and Q¹ have any of the meanings defined hereinbefore except that any functional group is protected if necessary, with a morpholine of the Formula VII 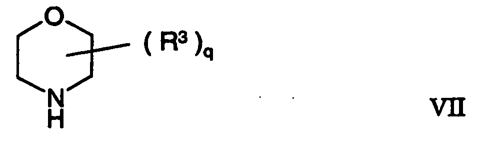 wherein q and R³ have any of the meanings defined hereinbefore except that any functional group is protected if necessary, whereafter any protecting group that is present is removed by conventional means.
   The reaction may conveniently be carried out in the presence of a suitable acid or in the presence of a suitable base. A suitable acid is, for example, an inorganic acid such as, for example, hydrogen chloride or hydrogen bromide. A suitable base is, for example, an organic amine base such as, for example, pyridine, 2,6-lutidine, collidine, 4-dimethylaminopyridine, triethylamine, morpholine, *N*-methylmorpholine or diazabicyclo[5.4.0]undec-7-ene, or, for example, an alkali or alkaline earth metal carbonate or hydroxide, for example sodium carbonate, potassium carbonate, calcium carbonate, sodium hydroxide or potassium hydroxide, or, for example, an alkali metal amide, for example sodium hexamethyldisilazane, or, for example, an alkali metal hydride, for example sodium hydride.
   The reaction is conveniently carried out in the presence of a suitable inert solvent or diluent, for example an alcohol or ester such as methanol, ethanol, isopropanol or ethyl acetate, a halogenated solvent such as methylene chloride, chloroform or carbon tetrachloride, an ether such as tetrahydrofuran or 1,4-dioxan, an aromatic solvent such as toluene, or a dipolar aprotic solvent such as *N,N-*dimethylformamide, *N,N*-dimethylacetamide, *N*-methylpyrrolidin-2-one or dimethylsulphoxide. The reaction is conveniently carried out at a temperature in the range, for example, 0 to 250°C, preferably in the range 25 to 150°C.
   Typically, the pyrimidine of the Formula VI may be reacted with a morpholine of the Formula VII in the presence of an aprotic solvent such as *N,N*-dimethylformamide or *N,N*-dimethylacetamide, conveniently in the presence of a suitable base, for example potassium carbonate or sodium hexamethyldisilazane, and at a temperature in the range, for example, 0 to 200°C, preferably in the range, for example, 25 to 150°C.
   Pyrimidine starting materials of the Formula VI may be obtained by conventional procedures such as those disclosed in the Examples that are set out hereinafter.
   For example, a pyrimidine of the Formula XIII 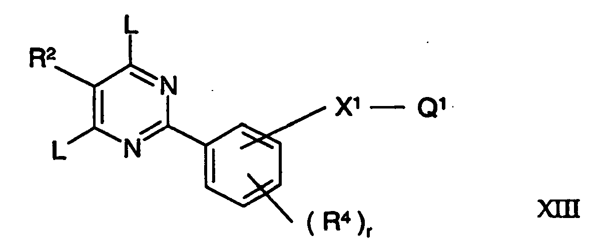 wherein L is a displaceable group as defined hereinbefore and R², r, R⁴, X¹ and Q¹ have any of the meanings defined hereinbefore except that any functional group is protected if necessary, may be reacted, conveniently in the presence of a suitable catalyst as defined hereinbefore, with an organoboron reagent of the Formula III 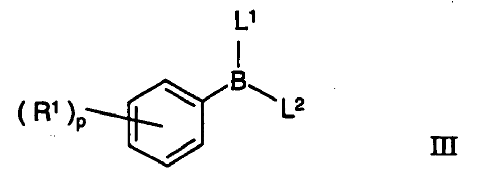 wherein each of L¹ and L², which may be the same or different, is a suitable ligand as defined hereinbefore and p and R¹ have any of the meanings defined hereinbefore except that any functional group is protected if necessary, whereafter any protecting group that is present is removed by conventional means.
(d) For the production of those compounds of the Formula I wherein X¹ is N(R¹³)CON(R¹³), the coupling, conveniently in the presence of a suitable base as defined hereinbefore, of phosgene, or a chemical equivalent thereof, with an amine of the Formula IV 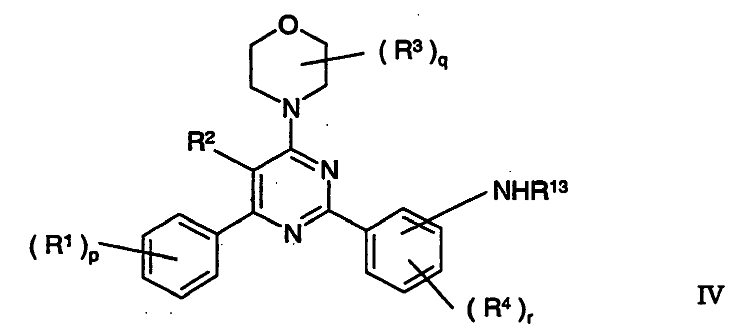 and an amine of the Formula VIII

   R¹³NH - Q¹ VIII

   wherein p, R¹, R², q, R³, r, R⁴, R¹³ and Q¹ have any of the meanings defined hereinbefore except that any functional group is protected if necessary, whereafter any protecting group that is present is removed by conventional means.
   A suitable chemical equivalent of phosgene is, for example, a compound of the Formula IX

   L-CO-L IX

   wherein L is a suitable displaceable group as defined hereinbefore. For example, a suitable displaceable group L is, for example, an alkoxy, aryloxy or sulphonyloxy group, for example a methoxy, phenoxy, methanesulphonyloxy or toluene-4-sulphonyloxy group. Alternatively, a suitable chemical equivalent of phosgene is a carbonate derivative such as disuccinimido carbonate.
   The reaction is conveniently carried out in the presence of a suitable inert solvent or diluent as defined hereinbefore and at a temperature in the range, for example, 0 to 120°C, preferably at or near ambient temperature.
(e) The reaction, conveniently in the presence of a suitable catalyst as defined hereinbefore, of a pyrimidine of the Formula XIV 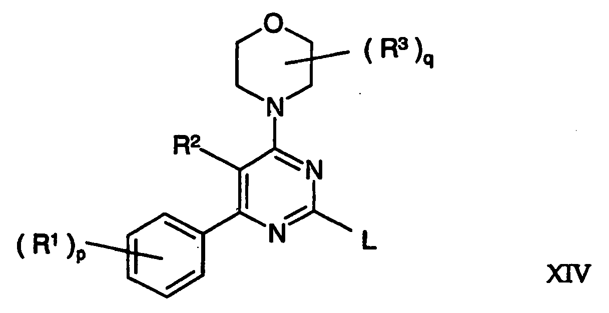 wherein L is a displaceable group as defined hereinbefore and p, R¹, R², q and R³ have any of the meanings defined hereinbefore except that any functional group is protected if necessary, with an organoboron reagent of the Formula XV 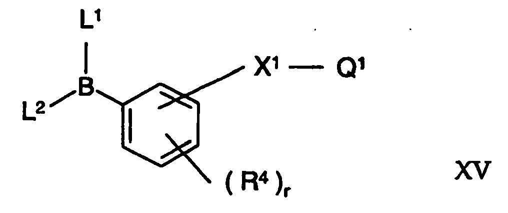 wherein each of L¹ and L², which may be the same or different, is a suitable ligand for the boron atom as defined hereinbefore and r, R⁴, X¹ and Q¹ have any of the meanings defined hereinbefore except that any functional group is protected if necessary, whereafter any protecting group that is present is removed by conventional means.
   Conveniently, the reaction may be carried out in the presence of a suitable base such as an alkali or alkaline earth metal carbonate or hydroxide, for example sodium bicarbonate, sodium carbonate, potassium bicarbonate, potassium carbonate, calcium carbonate, caesium carbonate, sodium hydroxide or potassium hydroxide, or, for example, an alkali metal alkoxide, for example sodium *tert*-butoxide, or, for example, an alkali metal amide, for example sodium hexamethyldisilazane, or, for example, an alkali metal hydride, for example sodium hydride.
   The reaction is conveniently carried out in the presence of a suitable inert solvent or diluent, for example an ether such as tetrahydrofuran, 1,4-dioxan or 1,2-dimethoxyethane, an aromatic solvent such as benzene, toluene or xylene, or an alcohol such as methanol or ethanol, and the reaction is conveniently carried out at a temperature in the range, for example 10 to 250°C, preferably in the range 40 to 150°C.
   Aryl-boron reagents of the Formula XV may be obtained by standard procedures of organic chemistry which are within the ordinary skill of an organic chemist, for example by the reaction of an aryl-metal reagent where the metal is, for example, lithium or the magnesium halide portion of a Grignard reagent, with an organoboron compound of the formula L-B(L¹)(L²) wherein L is a displaceable group as defined hereinbefore. Preferably the compound of the formula L-B(L¹)(L²) is, for example, boric acid or a tri-(1-4C)alkyl borate such as tri-isopropyl borate.
   In an alternative procedure, the aryl-boron reagent of the Formula XV may be replaced with an organometallic compound of the formula aryl-M wherein M is a metal atom or a metallic group (that is a metal atom bearing suitable ligands). Suitable values for the metal atom include, for example, lithium and copper. Suitable values for the metallic group include, for example, groups which contain a tin, silicon, zirconium, aluminium, magnesium, mercury or zinc atom. Suitable ligands within such a metallic group include, for example, hydroxy groups, (1-6C)alkyl groups such as methyl, ethyl, propyl, isopropyl and butyl groups, halogeno groups such as chloro, bromo and iodo groups, and (1-6C)alkoxy groups such as methoxy, ethoxy, propoxy, isopropoxy and butoxy groups. A particular organometallic compound of the formula aryl-M is, for example, an organotin compound such as a compound of the formula aryl-SnBu₃, an organosilicon compound such as a compound of the formula aryl-Si(Me)F₂, an organozirconium compound such as a compound of the formula aryl-ZrCl₃, an organoaluminium compound such as a compound of the formula aryl-AlEt₂, an organomagnesium compound such as a compound of the formula aryl-MgBr, an organomercury compound such as a compound of the formula aryl-HgBr, or an organozinc compound such as a compound of the formula aryl-ZnBr.
   Pyrimidine starting materials of the Formula XIV may be obtained by conventional procedures that are analogous to those disclosed in the Examples that are set out hereinafter.
(f) For the production of those compounds of the Formula I wherein X¹ is CON(R¹³), the acylation, conveniently in the presence of a suitable base as defined hereinbefore, of an amine of the Formula VIII

   R¹³NH -Q¹ VIII

   wherein R¹³ and Q¹ have any of the meanings defined hereinbefore except that any functional group is protected if necessary, with a carboxylic acid, or a reactive derivative thereof as defined hereinbefore, of the Formula XVI 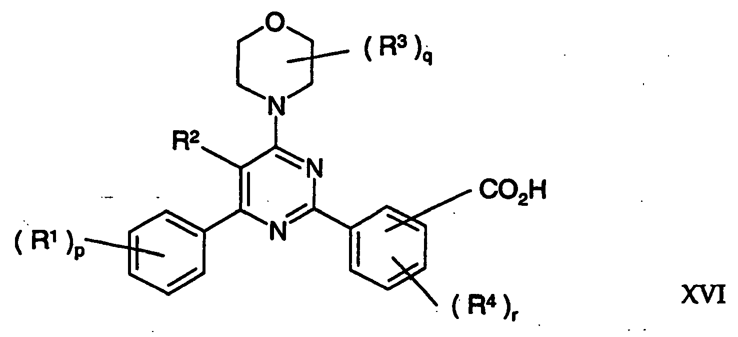 wherein p, R¹, R², q, R³, r and R⁴ have any of the meanings defined hereinbefore except that any functional group is protected if necessary, whereafter any protecting group that is present is removed by conventional means.
   The reaction is conveniently carried out in the presence of a suitable inert solvent or diluent, for example an alcohol or ester such as methanol, ethanol, isopropanol or ethyl acetate, a halogenated solvent such as methylene chloride, chloroform or carbon tetrachloride, an ether such as tetrahydrofuran or 1,4-dioxan, an aromatic solvent such as toluene. Conveniently, the reaction is conveniently carried out in the presence of a dipolar aprotic solvent such as *N,N*-dimethylformamide, *N*,*N*-dimethylacetamide, *N-*methylpyrrolidin-2-one or dimethylsulphoxide. The reaction is conveniently carried out at a temperature in the range, for example, 0 to 120°C, preferably at or near ambient temperature.
   Pyrimidine starting materials of the Formula XVI may be obtained by conventional procedures that are analogous to those disclosed in the Examples that are set out hereinafter.
(g) For the production of those compounds of the Formula I wherein X¹ is CO and Q¹ is a *N*-linked heterocyclyl group, the acylation, conveniently in the presence of a suitable base as defined hereinbefore, of a *N*-containing heterocyclic compound wherein any functional group is protected if necessary, with a carboxylic acid, or a reactive derivative thereof as defined hereinbefore, of the Formula XVI 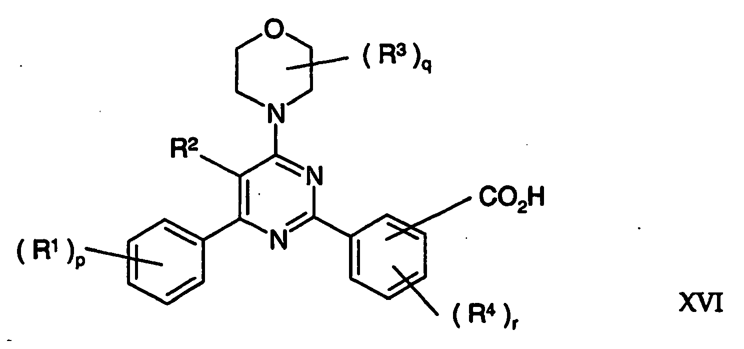
wherein p, R¹, R², q, R³, r and R⁴ have any of the meanings defined hereinbefore except that any functional group is protected if necessary, whereafter any protecting group that is present is removed by conventional means.

The reaction is conveniently carried out in the presence of a suitable inert solvent or diluent, for example an alcohol or ester such as methanol, ethanol, isopropanol or ethyl acetate, a halogenated solvent such as methylene chloride, chloroform or carbon tetrachloride, an ether such as tetrahydrofuran or 1,4-dioxan, an aromatic solvent such as toluene. Conveniently, the reaction is conveniently carried out in the presence of a dipolar aprotic solvent such as *N*,*N*-dimethylformamide, *N*,*N*-dimethylacetamide, *N*-methylpyrrolidin-2-one or dimethylsulphoxide. The reaction is conveniently carried out at a temperature in the range, for example, 0 to 120°C, preferably at or near ambient temperature.

The pyrimidine derivative of the Formula I may be obtained from the process variants described hereinbefore in the form of the free base or alternatively it may be obtained in the form of a salt with the acid of the formula H-L wherein L has the meaning defined hereinbefore. When it is desired to obtain the free base from the salt, the salt may be treated with a suitable base, for example, an organic amine base such as, for example, pyridine, 2,6-lutidine, collidine, 4-dimethylaminopyridine, triethylamine, morpholine, *N*-methylmorpholine or diazabicyclo[5.4.0]undec-7-ene, or, for example, an alkali or alkaline earth metal carbonate or hydroxide, for example sodium carbonate, potassium carbonate, calcium carbonate, sodium hydroxide or potassium hydroxide.

When a pharmaceutically-acceptable salt of a pyrimidine derivative of the Formula I is required, for example an acid-addition salt, it may be obtained by, for example, reaction of said pyrimidine derivative with a suitable acid using a conventional procedure.

When a pharmaceutically-acceptable pro-drug of a pyrimidine derivative of the Formula I is required, it may be obtained using a conventional procedure. For example, an *in vivo* cleavable ester of a pyrimidine derivative of the Formula I may be obtained by, for example, reaction of a compound of the Formula I containing a carboxy group with a pharmaceutically-acceptable alcohol or by reaction of a compound of the Formula I containing a hydroxy group with a pharmaceutically-acceptable carboxylic acid. For example, an *in vivo* cleavable amide of a pyrimidine derivative of the Formula I may be obtained by, for example, reaction of a compound of the Formula I containing a carboxy group with a pharmaceutically-acceptable amine or by reaction of a compound of the Formula I containing an amino group with a pharmaceutically-acceptable carboxylic acid.

Many of the intermediates defined herein are novel and these are provided as a further feature of the invention. For example, many compounds of the Formulae II, IV, VI, XIV and XVI are novel compounds.

### Biological Assays

The following assays can be used to measure the effects of the compounds of the present invention as PI3 kinase inhibitors, as inhibitors *in vitro* of the activation of PI3 kinase signalling pathways, as inhibitors *in vitro* of the proliferation of MDA-NO-468 human breast adenocarcinoma cells, and as inhibitors *in vivo* of the growth in nude mice of xenografts of MDA-MB-468 carcinoma tissue.

### (a) In Vitro Enzyme Assay

The assay used AlphaScreen technology (Gray et al., Analytical Biochemistry. 2003, 313: 234-245) to determine the ability of test compounds to inhibit phosphorylation by recombinant Type I PI3K enzymes of the lipid PI(4,5)P2.

DNA fragments encoding human PI3K catalytic and regulatory subunits were isolated from cDNA libraries using standard molecular biology and PCR cloning techniques. The selected DNA fragments were used to generate baculovirus expression vectors. In particular, full length DNA of each of the p110α, p110β and p110δ Type Ia human PI3K p110 isoforms (EMBL Accession Nos. HSU79143, S67334, Y10055 for p110α, p110β and p110δ respectively) were sub-cloned into a pDEST10 vector (Invitrogen Limited, Fountain drive, Paisley, UK). The vector is a Gateway-adapted version of Fastbac1 containing a 6-His epitope tag. A truncated form of Type Ib human PI3K p110γ isoform corresponding to amino acid residues 144-1102 (ENNIBL Accession No. X8336A) and the full length human p85α regulatory subunit (EMBL Accession No. HSP13KIN) were also sub-cloned into pFastBac1 vector containing a 6-His epitope tag. The Type Ia p110 constructs were co-expressed with the p85α regulatory subunit. Following expression in the baculovirus system using standard baculovirus expression techniques, expressed proteins were purified using the His epitope tag using standard purification techniques.

DNA corresponding to amino acids 263 to 380 of human Grp1 PH domain was isolated from a cDNA library using standard molecular biology and PCR cloning techniques. The resultant DNA fragment was sub-cloned into a pGEX 4T1 *E*. *coli* expression vector containing a GST epitope tag (Amersham Pharmacia Biotech, Rainham, Essex, UK) as described by Gray et al., Analytical Biochemistry, 2003, 313: 234-245). The GST-tagged Grp1 PH domain was expressed and purified using standard techniques.

Test compounds were prepared as 10 mM stock solutions in DMSO and diluted into water as required to give a range of final assay concentrations. Aliquots (2 µl) of each compound dilution were placed into a well of a Greiner 384-well low volume (LV) white polystyrene plate (Greiner Bio-one, Brunel Way, Stonehouse, Gloucestershire, UK Catalogue No. 784075). A mixture of each selected recombinant purified PI3K enzyme (15 ng), DiC8-PI(4,5)P2 substrate (40 µM; Cell Signals Inc., Kinnear Road, Columbus, USA, Catalogue No. 901), adenosine triphosphate (ATP; 4 µM) and a buffer solution [comprising Tris-HCl pH7.6 buffer (40 mM, 10 µl), 3-[(3-cholamidopropyl)dimethylammonio]-1-propanesulphonate (CHAPS; 0.04%), dithiothreitol (DTT; 2 mM) and magnesium chloride (10 mM)] was agitated at ambient temperature for 20 minutes.

Control wells that produced a minimum signal corresponding to maximum enzyme activity were created by using 5% DMSO instead of test compound. Control wells that produced a maximum signal corresponding to fully inhibited enzyme were created by adding wortmannin (6 µM; Calbiochem / Merck Bioscience, Padge Road, Beeston, Nottingham, UK, Catalogue No. 681675) instead of test compound. These assay solutions were also agitated for 20 minutes at ambient temperature.

Each reaction was stopped by the addition of 10 µl of a mixture of EDTA (100 mM), bovine serum albumin (BSA, 0.045 %) and Tris-HCl pH7.6 buffer (40 mM).

Biotinylated-DiC8-PI(3,4,5)P3 (50 nM; Cell Signals Inc., Catalogue No. 107), recombinant purified GST-Grp1 PH protein (2.5 nM) and AlphaScreen Anti-GST donor and acceptor beads (100 ng; Packard Bioscience Limited, Station Road, Pangbourne, Berkshire, UK, Catalogue No. 6760603M) were added and the assay plates were left for about 5 to 20 hours at ambient temperature in the dark. The resultant signals arising from laser light excitation at 680 nm were read using a Packard AlphaQuest instrument.

PI(3,4,5)P3 is formed *in situ* as a result of PI3K mediated phosphorylation of PI(4,5)P2. The GST-Grp1 PH domain protein that is associated with AlphaScreen Anti-GST donor beads forms a complex with the biotinylated PI(3,4,5)P3 that is associated with Alphascreen Streptavidn acceptor beads. The enymatically-produced PI(3,4,5)P3 competes with biotinylated PI(3,4,5)P3 for binding to the PH domain protein. Upon laser light excitation at 680 nm, the donor bead : acceptor bead complex produces a signal that can be measured. Accordingly, PI3K enzme activity to form PI(3,4,5)P3 and subsequent competition with biotinylated PI(3,4,5)P3 results in a reduced signal. In the presence of a PI3K enzyme inhibitor, signal strength is recovered.

PI3K enzyme inhibition for a given test compound was expressed as an IC₅₀ value.

### (b) In Vitro phospho-Ser473 Akt assay

This assay determines the ability of test compounds to inhibit phosphorylation of Serine 473 in Akt as assessed using Acumen Explorer technology (Acumen Bioscience Limited), a plate reader that can be used to rapidly quantitate features of images generated by laser-scanning.

A MDA-MB-468 human breast adenocarcinoma cell line (LGC Promochem, Teddington, Middlesex, UK, Catalogue No. HTB-132) was routinely maintained at 37°C with 5% CO₂ up to a confluency of 70-90% in Dulbecco's modified Eagle's growth medium (DMEM; Invitrogen Limited, Paisley, UK Catalogue No. 41966-029) containing 10% heat-inactivated foetal calf serum (FCS; Sigma, Poole, Dorset, UK, Catalogue No. F0392) and 1 % L-glutamine (Gibco, Catalogue No. 25030-024).

For the assay, the cells were detached from the culture flask using 'Accutase' (Innovative Cell Technologies Inc., San Diego, CA, USA; Catalogue No. AT104) using standard tissue culture methods and resuspended in media to give 1.7x10⁵ cells per ml. Aliquots (90 µl) were seeded into each of the inner 60 wells of a black Packard 96 well plate (PerkinElmer, Boston, MA, USA; Catalogue No. 6005182) to give a density of ~15000 cells per well. Aliquots (90 µl) of culture media were placed in the outer wells to prevent edge effects. The cells were incubated overnight at 37°C with 5% CO₂ to allow them to adhere.

On day 2, the cells were treated with test compounds and incubated for 2 hours at 37°C with 5% CO₂. Test compounds were prepared as 10 mM stock solutions in DMSO and serially diluted as required with growth media to give a range of concentrations that were 10-fold the required final test concentrations. Aliquots (10 µl) of each compound dilution were placed in a well (in triplicate) to give the final required concentrations. As a minimum reponse control, each plate contained wells having a final concentration of 100 µM LY294002 (Calbiochem, Beeston, UK, Catalogue No. 440202). As a maximum response control, wells contained 1% DMSO instead of test compound. Following incubation, the contents of the plates were fixed by treatment with a 1.6% aqueous formaldehyde solution (Sigma, Poole, Dorset, UK, Catalogue No. F1635) at ambient temperature for 1 hour.

All subsequent aspiration and wash steps were carried out using a Tecan 96 well plate washer (aspiration speed 10 mm/sec). The fixing solution was removed and the contents of the plates were washed with phosphate-buffered saline (PBS; 50 µl; Gibco, Catalogue No. 10010015). The contents of the plates were treated for 10 minutes at ambient temperature with an aliquot (50 µl) of a cell permeabilisation buffer consisting of a mixture of PBS and 0.5% Tween-20. The 'permeabilisation' buffer was removed and non-specific binding sites were blocked by treatment for 1 hour at ambient temperature of an aliquot (50 µl) of a blocking buffer consisting of 5% dried skimmed milk ['Marvel' (registered trade mark); Premier Beverages, Stafford, GB] in a mixture of PBS and 0.05% Tween-20. The 'blocking' buffer was removed and the cells were incubated for 1 hour at ambient temperature with rabbit anti phospho-Akt (Ser473) antibody solution (50 µl per well; Cell Signalling, Hitchin, Herts, U.K., Catalogue No 9277) that had been diluted 1:500 in 'blocking' buffer. Cells were washed three times in a mixture of PBS and 0.05% Tween-20. Subsequently, cells were incubated for 1 hour at ambient temperature with Alexafluor488 labelled goat anti-rabbit IgG (50 µl per well; Molecular Probes, Invitrogen Limited, Paisley, UK, Catalogue No. A11008) that had been diluted 1:500 in 'blocking' buffer. Cells were washed 3 times with a mixture of PBS and 0.05% Tween-20. An aliquot of PBS (50 µl) was added to each well and the plates were sealed with black plate sealers and the fluorescence signal was detected and analysed.

Fluorescence dose response data obtained with each compound were analysed and the degree of inhibition of Serine 473 in Akt was expressed as an IC₅₀ value.

### (c) In Vitro MDA-MB-468 human breast adenocarcinoma Proliferation Assay

This assay determines the ability of test compounds to inhibit cell proliferation as assessed using Cellomics Arrayscan technology. A MDA-MB-468 human breast adenocarcinoma cell line (LGC Promochem, Catalogue No. HTB-132) was routinely maintained as described in Biological Assay (b) hereinbefore.

For the proliferation assay, the cells were detached from the culture flask using Accutase and seeded into the inner 60 wells of a black Packard 96 well plate at a density of 8000 cells per well in 100 µl of complete growth media. The outer wells contained 100 µl of sterile PBS. The cells were incubated overnight at 37°C with 5% CO₂ to allow them to adhere.

On day 2, the cells were treated with test compounds and incubated for 48 hours at 37°C with 5% CO₂. Test compounds were prepared as 10 mM stock solutions in DMSO and serially diluted as required with growth media to give a range of test concentrations. Aliquots (50 µl) of each compound dilution were placed in a well and the cells were incubated for 2 days at 37°C with 5% CO₂. Each plate contained control wells without test compound.

On day 4, BrdU labelling reagent (Sigma, Catalogue No. B9285) at a final dilution of 1:1000 was added and the cells were incubated for 2 hours at 37°C. The medium was removed and the cells in each well were fixed by treatment with 100 µl of a mixture of ethanol and glacial acetic acid (90% ethanol, 5% glacial acetic acid and 5% water) for 30 minutes at ambient temperature. The cells in each well were washed twice with PBS (100 µl). Aqueous hydrochloric acid (2M, 100 µl) was added to each well. After 20 minutes at ambient temperature, the cells were washed twice with PBS. Hydrogen peroxide (3%, 50 µl; Sigma, Catalogue No. H1009) was added to each well. After 10 minutes at ambient temperature, the wells were washed again with PBS.

BrdU incorporation was detected by incubation for 1 hour at ambient temperature with mouse anti-BrdU antibody (50 µl; Caltag, Burlingame, CA, US; Catalogue No. MD5200) that was diluted 1:40 in PBS containing 1% BSA and 0.05% Tween-20. Unbound antibody was removed with two washes of PBS. For visualisation of incorporated BrdU, the cells were treated for 1 hour at ambient temperature with PBS (50 µl) and 0.05% Tween-20 buffer containing a 1:1000 dilution of Alexa fluor 488-labelled goat anti-mouse IgG. For visualisation of the cell nucleus, a 1:1000 dilution of Hoechst stain (Molecular Probes, Catalogue No. H3570) was added. Each plate was washed in turn with PBS. Subsequently, PBS (100 µl) was added to each well and the plates were analysed using a Cellomics array scan to assess total cell number and number of BrdU positive cells.

Fluorescence dose response data obtained with each compound were analysed and the degree of inhibition of MDA-MB-468 cell growth was expressed as an IC₅₀ value.

### (d) In Vivo MDA-MB-468 Xenograft Growth Assay

This test measures the ability of compounds to inhibit the growth of MDA-MB-468 human breast adenocarcinoma cells grown as a tumour in athymic nude mice (Alderley Park nu/nu strain). A total of about 5 x 10⁶ MDA-MB-468 cells in matrigel (Beckton Dickinson Catalogue No. 40234) are injected subcutaneously into the left flank of each test mouse and the resultant tumours are allowed to grow for about 14 days. Tumour size is measured twice weekly using callipers and a theoretical volume is calculated. Animals are selected to provide control and treatment groups of approximately equal average tumour volume. Test compounds are prepared as a ball-milled suspension in 1 % polysorbate vehicle and dosed orally once daily for a period of about 28 days. The effect on tumour growth is assessed.

Although the pharmacological properties of the compounds of the Formula I vary with structural change as expected, in general activity possessed by compounds of the Formula I, may be demonstrated at the following concentrations or doses in one or more of the above tests (a), (b), (c) and (d) :-
- Test (a):-: IC₅₀ versus p110α Type Ia human PI3K in the range, for example, 0.1- 50 µM;
- Test (b):-: IC₅₀ in the range, for example, 0.1- 50 *µ*M*;*
- Test (c):-: IC₅₀ in the range, for example, 0.1- 50 µM;
- Test (d):-: activity in the range, for example, 1-200 mg/kg/day.

In general, many of the particular compounds of the Formula I provided hereinafter as Examples possess activity at the following concentrations or doses in one or more of the above tests (a), (b), (c) and (d) :-
- Test (a):-: IC₅₀ versus p110α Type Ia human PI3K in the range, for example, 0.1- 20 µM;
- Test (b):-: IC₅₀ in the range, for example, 0.1- 20 µM;
- Test (c):-: IC₅₀ in the range, for example, 0.1- 20 µM;
- Test (d):-: activity in the range, for example, 1-200 mg/kg/day.

For example, the pyrimidine compound disclosed within Example 4(3) possesses activity in Test (a) with an IC₅₀ versus p110α Type Ia human PI3K of approximately 1 µM; the pyrimidine compound disclosed within Example 6(33) possesses the following activity :-
Test (a), IC₅₀ versus p110α Type Ia human PI3K of approximately 0.7 µM;
Test (b), IC₅₀ of approximately 5.1 *µ*M.; and Test (c), IC₅₀ of approximately 10 µM; and the pyrimidine compound disclosed within Example 6(48) possesses the following activity :-
   Test (a), IC₅₀ versus p110α Type Ia human PI3K of approximately 1.0 µM; and
   Test (b), IC₅₀ of approximately 1.8 µM; and Test (c), IC₅₀ of approximately 5 *µ*M.

No untoward toxicological effects are expected when a compound of Formula I, or a pharmaceutically-acceptable salt thereof, as defined hereinbefore is administered at the dosage ranges defined hereinafter.

According to a further aspect of the invention there is provided a pharmaceutical composition which comprises a pyrimidine derivative of the Formula I, or a pharmaceutically-acceptable salt, solvate or pro-drug thereof, as defined hereinbefore in association with a pharmaceutically-acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intraperitoneal or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

The amount of active ingredient that is combined with one or more excipients to produce a single dosage form will necessarily vary depending upon the host treated and the particular route of administration. For example, a formulation intended for oral administration to humans will generally contain, for example, from 1 mg to 1 g of active agent (more suitably from 1 to 250 mg, for example from 1 to 100 mg) compounded with an appropriate and convenient amount of excipients which may vary from about 5 to about 98 percent by weight of the total composition.

The size of the dose for therapeutic or prophylactic purposes of a compound, of the Formula I will naturally vary according to the nature and severity of the disease state, the age and sex of the animal or patient and the route of administration, according to well known principles of medicine.

In using a compound of the Formula I for therapeutic or prophylactic purposes it will generally be administered so that a daily dose in the range, for example, 1 mg/kg to 100 mg/kg body weight is received, given if required in divided doses. In general, lower doses will be administered when a parenteral route is employed. Thus, for example, for intravenous administration, a dose in the range, for example, 1 mg/kg to 25 mg/kg body weight will generally be used. Similarly, for administration by inhalation, a dose in the range, for example, 1 mg/kg to 25 mg/kg body weight will be used. Oral administration is however preferred, particularly in tablet form. Typically, unit dosage forms will contain about 10 mg to 0.5 g of a compound of this invention.

As stated above, it is known that PI3K enzymes contribute to tumourigenesis by one or more of the effects of mediating proliferation of cancer and other cells, mediating angiogenic events and mediating the motility, migration and invasiveness of cancer cells. We have found that the pyrimidine derivatives of the present invention possess potent anti-tumour activity which it is believed is obtained by way of inhibition of one or more of the Class I PI3K enzymes (such as the Class Ia PI3K enzymes and/or the Class Ib PI3K enzyme) that are involved in the signal transduction steps which lead to the proliferation and survival of tumour cells and the invasiveness and migratory ability of metastasising tumour cells.

Accordingly, the derivatives of the present invention are of value as anti-tumour agents, in particular as selective inhibitors of the proliferation, survival, motility, dissemination and invasiveness of mammalian cancer cells leading to inhibition of tumour growth and survival and to inhibition of metastatic tumour growth. Particularly, the pyrimidine derivatives of the present invention are of value as anti-proliferative and anti-invasive agents in the containment and/or treatment of solid tumour disease. Particularly, the compounds of the present invention are expected to be useful in the prevention or treatment of those tumours which are sensitive to inhibition of one or more of the multiple PI3K enzymes such as the Class Ia PI3K enzymes and the Class Ib PI3K enzyme that are involved in the signal transduction steps which lead to the proliferation and survival of tumour cells and the migratory ability and invasiveness of metastasising tumour cells. Further, the compounds of the present invention are expected to be useful in the prevention or treatment of those tumours which are mediated alone or in part by inhibition of PI3K enzymes such as the Class Ia PI3K enzymes and the Class Ib PI3K enzyme, *i.e*. the compounds may be used to produce a PI3K enzyme inhibitory effect in a warm-blooded animal in need of such treatment.

As stated hereinbefore, inhibitors of PI3K enzymes should be of therapeutic value for treatment of; for example; cancer of the breast, colorectum, lung (including small cell lung cancer, non-small cell lung cancer and bronchioalveolar cancer) and prostate, and of cancer of the bile duct, bone, bladder, head and neck, kidney, liver, gastrointestinal tissue, oesophagus, ovary, pancreas, skin, testes, thyroid, uterus, cervix and vulva, and of leukaemias [including acute lymphoctic leukaemia (ALL) and chronic myelogenous leukaemia (CML)], multiple myeloma and lymphomas.

According to a further aspect of the invention there is provided a pyrimidine derivative of the Formula I, or a pharmaceutically-acceptable salt, solvate or pro-drug thereof, as defined hereinbefore for use as a medicament in a warm-blooded animal such as man.

According to a further aspect of the invention, there is provided a pyrimidine derivative of the Formula I, or a pharmaceutically-acceptable salt, solvate or pro-drug thereof, as defined hereinbefore for use in the production of an anti-proliferative effect in a warm-blooded animal such as man.

According to a further feature of this aspect of the invention there is provided a pyrimidine derivative of the Formula I, or a pharmaceutically-acceptable salt, solvate or pro-drug thereof, as defined hereinbefore for use in a warm-blooded animal such as man as an anti-invasive agent in the containment and/or treatment of solid tumour disease.

According to a further aspect of the invention, there is provided the use of a pyrimidine derivative of the Formula I, or a pharmaceutically-acceptable salt, solvate or pro-drug thereof, as defined hereinbefore for the production of an anti-proliferative effect in a warm-blooded animal such as man.

According to a further feature of this aspect of the invention there is provided the use of a pyrimidine derivative of the Formula I, or a pharmaceutically-acceptable salt, solvate or pro-drug thereof, as defined hereinbefore in the manufacture of a medicament for use in the production of an anti-proliferative effect in a warm-blooded animal such as man.

According to a further feature of this aspect of the invention there is provided the use of a pyrimidine derivative of the Formula I, or a pharmaceutically-acceptable salt, solvate or pro-drug thereof, as defined hereinbefore in the manufacture of a medicament for use in a warm-blooded animal such as man as an anti-invasive agent in the containment and/or treatment of solid tumour disease.

According to a further aspect of the invention there is provided the use of a pyrimidine derivative of the Formula I, or a pharmaceutically-acceptable salt, solvate or pro-drug thereof, as defined hereinbefore in the manufacture of a medicament for use in the prevention or treatment of solid tumour disease in a warm-blooded animal such as man.

According to a further feature of this aspect of the invention there is provided a method for the prevention or treatment of solid tumour disease in a warm-blooded animal, such as man, in need of such treatment which comprises administering to said animal an effective amount of a pyrimidine derivative of the Formula I, or a pharmaceutically-acceptable salt, solvate or pro-drug thereof, as defined hereinbefore.

According to a further aspect of the invention there is provided a pyrimidine derivative of the Formula I, or a pharmaceutically-acceptable salt, solvate or pro-drug thereof, as defined hereinbefore for use in the prevention or treatment of those tumours which are sensitive to inhibition of PI3K enzymes (such as the Class Ia enzymes and/or the Class Ib PI3K enzyme) that are involved in the signal transduction steps which lead to the proliferation, survival, invasiveness and migratory ability of tumour cells.

According to a further feature of this aspect of the invention there is provided the use of a pyrimidine derivative of the Formula I, or a pharmaceutically-acceptable salt, solvate or pro-drug thereof, as defined hereinbefore in the manufacture of a medicament for use in the prevention or treatment of those tumours which are sensitive to inhibition of PI3K enzymes (such as the Class Ia enzymes and/or the Class Ib PI3K enzyme) that are involved in the signal transduction steps which lead to the proliferation, survival, invasiveness and migratory ability of tumour cells.

According to a further aspect of the invention there is provided a pyrimidine derivative of the Formula I, or a pharmaceutically-acceptable salt, solvate or pro-drug thereof, as defined hereinbefore for use in providing a PI3K enzyme inhibitory effect (such as a Class Ia PI3K enzyme or Class Ib PI3K enzyme inhibitory effect).

According to a further feature of this aspect of the invention there is provided the use of a pyrimidine derivative of the Formula I, or a pharmaceutically-acceptable salt, solvate or pro-drug thereof, as defined hereinbefore in the manufacture of a medicament for use in providing a PI3K enzyme inhibitory effect (such as a Class Ia PI3K enzyme or Class Ib PI3K enzyme inhibitory effect).

As stated hereinbefore, certain compounds of the present invention, possess substantially better potency against Class Ia PI3K enzymes than against the Class Ib PI3K enzyme or against EGF receptor tyrosine kinase, VEGF receptor tyrosine kinase or Src non-receptor tyrosine kinase enzymes. Such compounds possess sufficient potency against Class Ia PI3K enzymes that they may be used in an amount sufficient to inhibit Class Ia PI3K enzymes whilst demonstrating little activity against the Class Ib PI3K enzyme or against EGF receptor tyrosine kinase, VEGF receptor tyrosine kinase or Src non-receptor tyrosine kinase enzymes. Such compounds are likely to be useful for the selective inhibition of Class Ia PI3K enzymes and are likely to be useful for the effective treatment of, for example Class Ia PI3K enzyme driven tumours.

According to this aspect of the invention there is provided a pyrimidine derivative of the Formula I, or a pharmaceutically-acceptable salt, solvate or pro-drug thereof, as defined hereinbefore for use in providing a selective Class Ia PI3K enzyme inhibitory effect.

According to a further feature of this aspect of the invention there is provided the use of a pyrimidine derivative of the Formula I, or a pharmaceutically-acceptable salt, solvate or pro-drug thereof, as defined hereinbefore in the manufacture of a medicament for use in providing a selective Class Ia PI3K enzyme inhibitory effect.

By "a selective Class Ia PI3K enzyme inhibitory effect" is meant that the pyrimidine derivatives of the Formula I are more potent against Class Ia PI3K enzymes than against other kinase enzymes. In particular, some of the compounds according to the invention are more potent against Class Ia PI3K enzymes than against other kinases such as receptor or non-receptor tyrosine kinases or serine/threonine kinases. For example a selective Class Ia PI3K enzyme inhibitor according to the invention is at least 5 times more potent, preferably at least 10 times more potent, more preferably at least 100 times more potent, against Class Ia PI3K enzymes than against other kinases.

According to a further feature of the invention there is provided a pyrimidine derivative of the Formula I, or a pharmaceutically-acceptable salt, solvate or pro-drug, as defined hereinbefore for use in the treatment of cancer of the breast, colorectum, lung (including small cell lung cancer, non-small cell lung cancer and bronchioalveolar cancer) and prostate.

According to a further feature of this aspect of the invention there is provided a pyrimidine derivative of the Formula I, or a pharmaceutically-acceptable salt, solvate or pro-drug thereof, as defined hereinbefore for use in the treatment of cancer of the bile duct, bone, bladder, head and neck, kidney, liver, gastrointestinal tissue, oesophagus, ovary, pancreas, skin, testes, thyroid, uterus, cervix and vulva, and of leukaemias (including ALL and CML), multiple myeloma and lymphomas.

According to a further feature of this aspect of the invention there is provided the use of a pyrimidine derivative of the Formula I, or a pharmaceutically-acceptable salt, solvate or pro-drug thereof, as defined hereinbefore in the manufacture of a medicament for use in the treatment of cancer of the breast, colorectum, lung (including small cell lung cancer, non-small cell lung cancer and bronchioalveolar cancer) and prostate.

According to a further feature of this aspect of the invention there is provided the use of a pyrimidine derivative of the Formula I, or a pharmaceutically-acceptable salt, solvate or pro-drug thereof, as defined hereinbefore in the manufacture of a medicament for use in the treatment of cancer of the bile duct, bone, bladder, head and neck, kidney, liver, gastrointestinal tissue, oesophagus, ovary, pancreas, skin, testes, thyroid, uterus, cervix and vulva, and of leukaemias (including ALL and CML), multiple myeloma and lymphomas.

As stated hereinbefore, the *in vivo* effects of a compound of the Formula I may be exerted in part by one or more metabolites that are formed within the human or animal body after administration of a compound of the Formula I.

The anti-cancer treatment defined hereinbefore may be applied as a sole therapy or may involve, in addition to the pyrimidine derivative of the invention, conventional surgery or radiotherapy or chemotherapy. Such chemotherapy may include one or more of the following categories of anti-tumour agents :-
(i) other antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea; antitumour antibiotics (for example-anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(ii) cytostatic agents such as antioestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) anti-invasion agents (for example c-Src kinase family inhibitors like 4-(6-chloro-2,3-methylenedioxyanilino)-7-[2-(4-methylpiperazin-1-yl)ethoxy]-5-tetrahydropyran-4-yloxyqyuinazoline (AZD0530; International Patent Application WO 01/94341) and *N*-(2-chloro-6-methylphenyl)-2-{6-[4-(2-hydroxyethyl)piperazin-1-yl]-2-methylpyrimidin-4-ylamino}thiazole-5-carboxanude (dasatinib; BMS-354825; J. Med. Chem., 2004, 47, 6658-6661), and metalloproteinase inhibitors like marimastat and inhibitors of urokinase plasminogen activator receptor function);
(iv) inhibitors of growth factor function: for example such inhibitors include growth factor antibodies and growth factor receptor antibodies (for example the anti-erbB2 antibody trastuzumab [Herceptin™] and the anti-erbB 1 antibody cetuximab [C225]); such inhibitors also include, for example, tyrosine kinase inhibitors, for example inhibitors of the epidermal growth factor family (for example EGFR family tyrosine kinase inhibitors such as *N*-(3-chloro-4-fluorophenyl)-7-methoxy-6-(3-morpholinopropoxy)quinazolin-4-amine (gefitinib, ZD1839), *N*-(3-ethynylphenyl)-6,7-bis(2-methoxyethoxy)quinazolin-4-amine (erlotinib, OSI-774) and 6-acrylamido-*N*-(3-chloro-4-fluorophenyl)-7-(3-morpholinopropoxy)quinazolin-4-amine (CI 1033) and erbB2 tyrosine kinase inhibitors such as lapatinib), inhibitors of the hepatocyte growth factor family, inhibitors of the platelet-derived growth factor family such as imatinib, inhibitors of serine/threonine kinases (for example Ras/Raf signalling inhibitors such as farnesyl transferase inhibitors, for example sorafenib (BAY 43-9006)) and inhibitors of cell signalling through MEK, AKT and/or PI3K kinases;
(v) antiangiogenic agents such as those which inhibit the effects of vascular endothelial growth factor, [for example the anti-vascular endothelial cell growth factor antibody bevacizumab (Avastin™) and VEGF receptor tyrosine kinase inhibitors such as 4-(4-bromo-2-fluoroanilino)-6-methoxy-7-(1-methylpiperidin-4-ylmethoxy)quinazoline (ZD6474; Example 2 within WO 01/32651), 4-(4-fluoro-2-methylindol-5-yloxy)-6-methoxy-7-(3-pyrrolidin-1-ylpropoxy)quinazoline (AZD2171; Example 240 within WO 00/47212), vatalanib (PTK787; WO 98/35985) and SU11248 (sunitinib; WO 01/60814), and compounds that work by other mechanisms (for example linomide, inhibitors of integrin αvβ3 function and angiostatin)];
(vi) vascular damaging agents such as Combretastatin A4 and compounds disclosed in International Patent Applications WO 99/02166, WO 00/40529, WO 00/41669, WO 01/92224, WO 02/04434 and WO 02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

According to this aspect of the invention there is provided a pharmaceutical product comprising a pyrimidine derivative of the formula I as defined hereinbefore and an additional anti-tumour agent as defined hereinbefore for the conjoint treatment of cancer.

Although the compounds of the Formula I are primarily of value as therapeutic agents for use in warm-blooded animals (including man), they are also useful whenever it is required to inhibit the effects of PI3K enzymes. Thus, they are useful as pharmacological standards for use in the development of new biological tests and in the search for new pharmacological agents.

The invention will now be illustrated in the following Examples in which, generally :
(i) operations were carried out at ambient temperature, *i.e.* in the range 17 to 25°C and under an atmosphere of an inert gas such as nitrogen or argon unless otherwise stated;
(ii) in general, the course of reactions was followed by thin layer chromatography (TLC) and/or analytical high pressure liquid chromatography (HPLC); the reaction times that are given are not necessarily the minimum attainable;
(iii) when necessary, organic solutions were dried over anhydrous magnesium sulphate, work-up procedures were carried out after removal of residual solids by filtration, evaporations were carried out by rotary evaporation *in vacuo;*
(iv) yields, where present, are not necessarily the maximum attainable, and, when necessary, reactions were repeated if a larger amount of the reaction product was required;
(v) in general, the structures of the end-products of the Formula I were confirmed by nuclear magnetic resonance (NMR) and/or mass spectral techniques; electrospray mass spectral data were obtained using a Waters ZMD or Waters ZQ LC/mass spectrometer acquiring both positive and negative ion data, generally, only ions relating to the parent structure are reported; proton NMR chemical shift values were measured on the delta scale using a Bruker Spectrospin DPX300 spectrometer operating at a field strength of 300 MHz; the following abbreviations have been used: s, singlet; d, doublet; t, triplet; q, quartet; m, multiplet; br, broad;
(vi) unless stated otherwise compounds containing an asymmetric carbon and/or sulphur atom were not resolved;
(vii) intermediates were not necessarily fully purified but their structures and purity were assessed by TLC, analytical HPLC, infra-red (IR) and/or NMR analysis;
(viii) unless otherwise stated, column chromatography (by the flash procedure) and medium pressure liquid chromatography (MPLC) were performed on Merck Kieselgel silica (Art. 9385);
(ix) preparative HPLC was performed on C18 reversed-phase silica, for example on a Waters 'Xterra' preparative reversed-phase column (5 microns silica, 19 mm diameter, 100 mm length) using decreasingly polar mixtures as eluent, for example decreasingly polar mixtures of water (containing 1% acetic acid or 1% aqueous ammonium hydroxide (d=0.88) and acetonitrile;
(x) the following analytical HPLC methods were used; in general, reversed-phase silica was used with a flow rate of about 1ml per minute and detection was by Electrospray Mass Spectrometry and by UV absorbance at a wavelength of 254 nm; for each method Solvent A:was water and Solvent B was acetonitrile; the following columns and solvent mixtures were used :-
Method A1 : Phenomenex Synergi MAX-RP 80Å column (4 microns silica, 2.1 mm diameter, 50 mm length) using a Solvent C comprising 0.1% aqueous ammonium hydroxide (d=0.88) in deionised water and a solvent gradient over 4 minutes from a 90:5:5 mixture of Solvents A, B and C respectively to a 95:5 mixture of Solvents B and C;
Method A2 : Phenomenex Synergi MAX-RP 80Å column (4 microns silica, 2.1 mm diameter, 50 mm length) using a Solvent C comprising 0.1% aqueous ammonium hydroxide (d=0.88) in deionised water and a solvent gradient over 4 minutes from a 95:5 mixture of Solvents B and C respectively to a 58:37:5 mixture of Solvents A, B and C respectively;
Method A3 : Waters 'Xterra' reversed-phase column (5 microns silica, 2 mm diameter, 50 mm length) using a Solvent C comprising a 10 mM aqueous ammonium bicarbonate solution (adjusted to pH10 by the addition of ammonia) and a solvent gradient over 4 minutes from a 1:99 mixture of Solvents B and C to 100% Solvent B;
Method B1 : Phenomenex Synergi MAX-RP 80Å column (4 microns silica, 2.1 mm diameter, 50 mm length) using a Solvent C comprising a 1:1 mixture of water and acetonitrile (the mixture containing 1% formic acid) and a solvent gradient over 4 minutes from a 90:5:5 mixture of Solvents A, B and C respectively to a 95:5 mixture of Solvents B and C;
Method B2 : Phenomenex Synergi MAX-RP 80Å column (4 microns silica, 2.1 mm diameter, 50 mm length) using a Solvent C comprising a 1:1 mixture of water and acetonitrile (the mixture containing 1% formic acid) and a solvent gradient over 4 minutes from a 95:5 mixture of Solvents A and C to a 58:37:5 mixture of Solvents A, B and C respectively;
Method B3 : Phenomenex Synergi MAX-RP 80Å column (4 microns silica, 2.1 mm diameter, 50 mm length) using a Solvent C comprising a 1:1 mixture of water and acetonitrile (the mixture containing 1% formic acid) and a solvent gradient over 4 minutes from a 35:60:5 mixture of Solvents A,B and C respectively to a 95:5 mixture of Solvents B and C;

(xi) where certain compounds were obtained as an acid-addition salt, for example a mono-hydrochloride salt or a di-hydrochlorick salt, the stoichiometry of the salt was based on the number and nature of the basic groups in the compound, the exact stoichiometry of the salt was generally not determined, for example by means of elemental analysis data;
(xii) the following abbreviations have been used:-

| | |
|---|---|
| DMF | N,N-dimethylformamide |
| DMSO | dimethylsulphoxide |
| THF | tetrahydrofuran |
| DMA | N,N-dimethylacetamide |

### Example 1

### 2-(3-acetamidophenyl)-4-(3-hydroxyphenyl)-6-morpholinopyrimidine

A mixture of 2-(3-acetamidophenyl)-4-bromo-6-morpholinopyrimidine (0.037 g), 3-hydroxyphenylboronic acid (0.017 g), tetraltis(triphenylphosphine)palladium(0) (3 mg), a saturated aqueous sodium bicarbonate solution (0.2 ml) and 1,2-dimethoxyethane (2 ml) was stirred and heated to 60°C for 18 hours under an atmosphere of nitrogen. A second portion of each of 3-hydroxyphenylboronic acid (0.017 g), tetrakis(triphenylphosphine)palladium(0) -(2 mg), a saturated aqueous sodium bicarbonate solution (0.2 ml) and 1,2-dimethoxyethane (1 ml) was added and the resultant mixture was heated to 60°C for a further 18 hours. The resultant reaction mixture was evaporated and the residue was triturated under a 5:1 mixture (1 ml) of methylene chloride and methanol. The soluble material was purified by column chromatography on reversed-phase silica using an 'Isolute SCX' column (1 g; International Sorbent Technology Limited, Mid Glamorgan, UK) by initially washing the column with methanol (5 ml) followed by elution with a 5:3:2 mixture of methanol, methylene chloride and a 7M methanolic ammonia solution. The material so obtained was dried under vacuum. There was thus obtained the title compound as a solid (0.038 g); NMR Spectrum: (DMSOd₆) 2.09 (s, 3H), 3.78 (d, 8H), 6.92 (d, 1H), 7.2 (s, 1H), 7.32 (t, 1H), 7.41 (t, 1H), 7.68-7.7 (m, 2H), 7.88 (d, 1H), 8.17 (d, 1H), 8.52 (s, 1H), 9.54 (s, 1H), 10.06 (s, 1H); Mass Spectrum: M+H⁺ 391.

The 2-(3-acetamidophenyl)-4-bromo-6-morpholinopyrimidine used as a starting material was prepared as follows :-

3-Nitrobenzamidine hydrochloride (20 g) was added to a stirred solution of sodium methoxide (20.5 g) in methanol (200 ml) and the mixture was stirred under an atmosphere of nitrogen at ambient temperature for 30 minutes. Diethyl malonate (22.6 ml) was added dropwise over 3 minutes and the resultant mixture was stirred at ambient temperature for 18 hours. The reaction mixture was evaporated. The residue was treated with hot water (400 ml) and the insoluble material was removed by filtration. The filtrate was acidified to pH5 with acetic acid. The precipitated solid was collected and washed with water. The resultant solid was triturated under hot ethanol. The insoluble material was collected by filtration, washed with ethanol and dried under vacuum. There was thus obtained 6-hydroxy-2-(3-nitrophenyl)pyrimidin-4(3*H*)-one (9.4 g); NMR Spectrum: (DMSOd₆) 5.58 (s, 1H), 7.83 (t, 1H), 8.38-8.42 (m, 1H), 8.58 (d, 1H), 8.99 (s, 1H).

Phosphorus oxybromide (49.1 g) was added portionwise to a stirred mixture of 6-hydroxy-2-(3-nitrophenyl)pyrimidin-4(3*H*)-one (5 g), triethylamine (11.9 ml) and acetonitrile (200 ml) that had been cooled to 5°C under an atmosphere of nitrogen. The mixture was allowed to warm to ambient temperature and was then heated to 65°C for 2 hours. After cooling, the mixture was filtered and the filtrate was evaporated. Toluene was added and the filtrate residue was re-evaporated. The resultant residue was suspended in methylene chloride and washed with a 1:1 mixture of aqueous ammonium hydroxide solution (d=0.88) and water. The organic layer was separated, dried over magnesium sulphate and evaporated. The residue was purified by column chromatography on silica using methylene chloride as eluent. There was thus obtained 4,6-dibromo-2-(3-nitrophenyl)pyrimidine (1.45 g); NMR Spectrum: (CDCl₃) 7.58-7.66 (m, 2H), 8.32 (d, 1H), 8.70 (d, 1H), 9.22 (s, 1H).

Morpholine (2.0 g) was added dropwise over 5 minutes to a solution of 4,6-dibromo-2-(3-nitrophenyl)pyrimidine (7.5 g) in DMA (75 ml) that had been cooled to 5°C. The mixture was stirred at ambient temperature for 1 hour. Further portions of morpholine (0.6 ml, 0.5 ml and 0.3 ml) were added in turn until all of the starting material had been consumed. The reaction mixture was evaporated and the residue was triturated under a cold aqueous ammonium hydroxide solution. The resultant solid was isolated, washed with water and dried under vacuum. There was thus obtained 4-bromo-6-morpholino-2-(3-nitrophenyl)pyrimidine as a pale yellow solid (8.4 g); NMR Spectrum: (DMSOd₆) 3.66-3.81 (m, 8H), 7.15 (s, 1H), 7.79 (t, 1H), 8.37 (d, 1H), 8.69 (d, 1H), 8.99 (s, 1H).

A mixture of 4-bromo-6-morpholino-2-(3-nitrophenyl)pyrimidine (8.2 g), stannous chloride dihydrate (17.7 g) and ethyl acetate (500 ml) was stirred and heated to reflux for 8 hours under an atmosphere of nitrogen. After cooling to ambient temperature, aqueous ammonium hydroxide solution (d=0.88) was added over 5 minutes with vigorous stirring to produce a white slurry of tin residues. The ethyl acetate layer was decanted and the aqueous slurry was washed with ethyl acetate. The organic layers were combined, dried over magnesium sulphate and evaporated. The residue was purified by column chromatography on silica using an increasingly polar solvent gradient from 0% to 20% ethyl acetate in methylene chloride as eluent. There was thus obtained 2-(3-aminophenyl)-4-bromo-6-morpholinopyrimidine (5.49 g); NMR Spectrum: (DMSOd₆) 3.7 (s, 8H), 5.24 (s, 2H), 6.66-6.72 (m, 1H), 6.98 (s, 1H), 7.11 (t, 1H). 7.44 (d, 1H), 7.54 (s, 1H).

Acetic anhydride (30 ml) was added to a stirred solution of 2-(3-aminophenyl)-4-bromo-6-morpholinopyrimidine (2.5 g) in DMA (50 ml) and the resultant mixture was heated to 60°C under an atmosphere of nitrogen for 1 hour. After cooling, the mixture was evaporated and the residue was dried under vacuum. There was thus obtained 2-(3-acetamidophenyl)4-bromo-6-morphohnopyrimidine (2.54 g); NMR Spectrum: (DMSOd₆) 2.06 (s, 3H), 3.73 (s, 8H), 7.04 (s, 1H), 7.4 (t, 1H), 7.88 (d, 1H), 7.95 (d, 1H), 8.38 (s, 1H), 10.08 (s, 1H).

### Example 2

Using an analogous procedure to that described in Example 1, the appropriate 4-halo-6-morpholino-2-arylpyrimidine was reacted with the appropriate arylboronic acid to give the compounds described in Table I.

**Table I**

| | | |
|---|---|---|
| 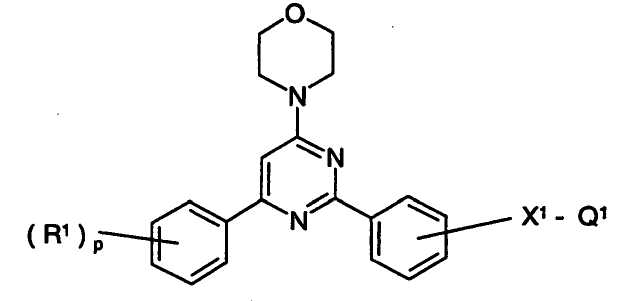 | | |

| No. & Note | (R¹)ₚ | X¹-Q¹ |
|---|---|---|
| [1] | 3-amino | 3-acetamido |
| [2] | 4-carbamoyl | 3-acetamido |
| [3] | 3-hydroxymethyl | 3-acetamido |
| [4] | 2-hydroxymethyl | 3-acetamido |

| | | |
|---|---|---|
| Notes The products gave the characterising data shown below. [1] Mass Spectrum: M+H⁺ 390; HPLC: method B1, Retention Time 1.15 minutes. [2] NMR Spectrum: (DMSOd₆) 2.14 (s, 3H), 3.81 (d, 8H), 7.38-7.5 (m, 3H), 7.89 (d, 1H), 8.03 (d, 2H), 8.09-8.16 (m, 2H), 8.38 (d, 2H), 8.57 (s, 1H), 10.0 (s, 1H); Mass Spectrum: M+H⁺ 418. [3] NMR Spectrum: (DMSOd₆) 2.14 (s, 3H), 3.81 (d, 8H), 4.63 (d, 2H), 5.27 (t,1H), 7.27 (s, 1H), 7.42 (t, 1H), 7.5 (d, 2H), 7.88 (d, 1H), 8.13-8.21 (m, 3H), 8.54 (s, 1H), 10.07 (s, 1H); Mass Spectrum: M+H⁺ 405. [4] Mass Spectrum: M+H⁺ 405; HPLC: method B1, Retention Time 1.21 minutes. | | |

### Example 3

### 4-(4-acetamidophenyl)-6-morpholino-2-(3-piperidin-4-ylcarbonylaminophenyl)pyrimidine

A mixture of 4-bromo-2-[3-(1-*tert*-butoxycarbonylpiperidin-4-ylcarbonylamino)phenyl]-6-morpholinopyrimidine (0.055 g), 4-acetamidophenylboronic acid (0.043 g), tetrakis(triphenylphosphine)palladium(0) (10 mg), a saturated aqueous sodium bicarbonate solution (1 ml) and 1,2-dimethoxyethane (3.5 ml) was stirred and heated to 60°C for 18 hours under an atmosphere of nitrogen. The resultant reaction mixture was evaporated and the residue was triturated under a 4:1 mixture (1 ml) of methylene chloride and methanol. The resultant mixture was filtered and the filtrate was evaporated. A mixture of the residue from the evaporation and a 4M solution of hydrogen chloride in 1,4-dioxane (1 ml) was stirred at ambient temperature for 4 hours. The mixture was evaporated and the residue was purified by column chromatography on reversed-phase silica using an 'Isolute SCX-3' column (1 g) by initially washing the column with methanol followed by elution with a 3M methanolic ammonia solution. The material so obtained was purified further by preparative HPLC on a Waters 'Xterra' preparative C18 reversed-phase column (5 microns silica, 19 mm diameter, 100 mm length) using decreasingly polar mixtures of water [containing 1 % aqueous ammonium hydroxide (d=0.88)] and acetonitrile as eluent. There was thus obtained the title compound as a solid (0.021 g); NMR Spectrum: (DMSOd₆) 1:52-1:63 (m, 2H), 1.74 (d, 2H), 2.09 (s, 3H), 3.79 (d, 8H), 7.23 (s, 1H), 7.4 (t, 1H), 7.75 (d, 2H), 7.96 (d, 1H), 8.13 (d, 1H), 8.26 (d, 2H), 8.54 (s, 1H), 9.96 (s, 1H), 10.15 (s, 1H); Mass Spectrum: M+H⁺ 501.

The 4-bromo-2-[3-(1-*tert*-butoxycarbonylpiperidin-4-ylcarbonylamino)phenyl]-6-morpholinopyrimidine used as a starting material was prepared as follows :-
2-(7-Azabenzotriazol-1-yl)-1,1,3,3-tetramethyluronium hexafluorophosphate(V) (0.625 g) was added to a mixture of *N*-(*tert*-butoxycarbonyl)piperidine-4-carboxylic acid (0.375 g), diisopropylethylamine (0.353 g) and DMA (20 ml) and the mixture was stirred at ambient temperature for 1 hour under an atmosphere of nitrogen. 2-(3-Aminophenyl)-4-bromo-6-morpholinopyrimidine (0.5 g) was added and the reaction mixture was stirred at ambient temperature for 18 hours. The reaction mixture was evaporated and the residue was purified by column chromatography on silica using an increasingly polar gradient of 0% to 30% ethyl acetate in methylene chloride as eluent. There was thus obtained 4-bromo-2-[3-(1-*tert*-butoxycarbonylpiperidin-4-ylcarbonylamino)phenyl]-6-morpholinopyrimidine (0.37 g); NMR Spectrum: (CDCl₃) 1.4 (s, 9H), 1.66-1.75 (m, 2H), 1.83-1.87 (m, 2H), 2.29-2.37 (m, 1H), 2.69-2.77 (m, 2H), 3.64-3.76 (m, 8H), 4.09 (d, 2H), 6.53 (s, 1H), 7.34 (t, 1H), 7.91 (d, 1H), 8.03 (d, 1H), 8.11 (s, 1H).

### Example 4

Using analogous procedures to those described in Example 3, the appropriate 4-halo-6-morpholino-2-arylpyrimidine was reacted with the appropriate arylboronic acid to give the compounds described in Table II.

**Table II**

| | | |
|---|---|---|
| 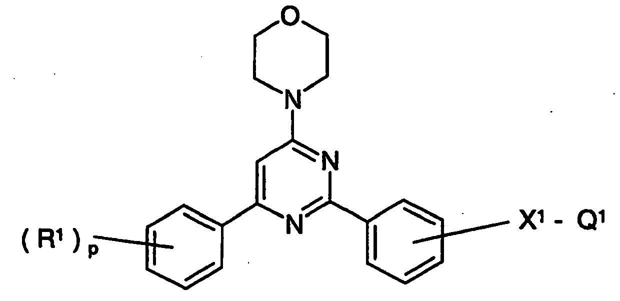 | | |

| No.& Note | (R¹)ₚ | X¹-Q¹ |
|---|---|---|
| [1] | 3-acetamido | 3-piperidin-4-ylcarbonylamino |
| [2] | 2-hydroxy | 3-piperidin-4-ylcarbonylamino |
| [3] | 3-hydroxy | 3-piperidin-4-ylcarbonylamino |
| [4] | 2-hydroxymethyl | 3-piperidin-4-ylcarbonylamino |
| [5] | 4-hydroxymethyl | 3-piperidin-4-ylcarbonylamino |

| | | |
|---|---|---|
| Notes The products gave the characterising data shown below. [1] Mass Spectrum: M+H⁺ 501; HPLC: method A3, Retention Time 2.55 minutes. [2] Mass Spectrum: M+H⁺ 460; HPLC: method A3, Retention Time 2.9 minutes. [3] Mass Spectrum: M+H⁺ 460; HPLC: method A3, Retention Time 2.64 minutes. [4] NMR Spectrum: (DMSOd₆) 1.48-1.59 (m, 2H), 1.68-1.72 (m, 2H), 3.0 (d, 2H), 3.87 (s, 8H), 4.67 (s, 2H), 6.95 (s, 1H), 7.36-7.41 (m, 2H), 7.49 (t, 1H), 7.57 (d, 1H), 7.65 (d, 1H), 7.92 (d, 1H), 8.02 (d, 1H), 8.47 (s, 1H), 9.54 (s, 1H); Mass Spectrum: M+H⁺ 474. [5] Mass Spectrum: M+H⁺ 474; HPLC: method A3, Retention Time 2.61 minutes. | | |

### Example 5

### 2-(3-glycylaminophenyl)-4-(3-hydroxymethylphenyl)-6-morpholinopyrimidine

Diisopropylethylamine (0.054 ml) was added to a stirred mixture of *N*-*tert*-butoxycarbonylglycine (0.028 g), 2-(7-azabenzotriazol-1-yl)-1,1,3,3-tetramethyluronium hexafluorophosphate(V) (0.053 g) and DMF (0.35 ml) and the resultant mixture was stirred for approximately 1 minute under an atmosphere of nitrogen. A solution of 2-(3-aminophenyl)4-(3-hydroxymethylphenyl)-6-morpholinopyrimidine (0.044 g) in DMF (0.3 ml) was added and the mixture was stirred for 5 hours. The DMF was evaporated. Trifluoroacetic acid (2 ml) was added to the residue and the mixture was stirred at ambient temperature for 2 hours. The mixture was evaporated and the residue was purified by HPLC using a Waters 'Xterra' preparative reversed-phase column (5 microns silica, 19 mm diameter, 100 mm length) using decreasingly polar mixtures of water and acetonitrile (containing 1% acetic acid) as eluent. There was thus obtained the title compound as a solid (0.025 g); NMR Spectrum: (DMSOd₆) 3.74-3.87 (m, 8H), 4.62 (s, 2H), 5.27 (s, 1H), 7.27 (s, 1H), 7.41-7.54 (m, 3H), 7.86-7.93 (m, 1H), 8.13-8.25 (m, 3H), 8.61 (t, 1H); Mass Spectrum: M+H⁺ 420; Analytical HPLC: method A1, Retention Time 1.77 minutes.

The 2-(3-aminophenyl)-4-(3-hydroxymethylphenyl)-6-morpholinopyrimidine used as a starting material was prepared as follows :-
A mixture of 6-hydroxy-2-(3-nitrophenyl)pyrimidin-4(3*H*)-one (5 g) and phosphorus oxychloride (100 ml) was stirred and heated to 105°C under an atmosphere of nitrogen for 3 hours. The resultant reaction mixture was evaporated, toluene (50 ml) was added and the mixture was evaporated again. The residue was dissolved in methylene chloride (200 ml), an excess of solid potassium carbonate was added portionwise until effervescence ceased and the mixture was stirred at ambient temperature for 30 minutes. The mixture was filtered and the filtrate was evaporated. The residue was purified by column chromatography on silica gel using a 1:1 mixture of isohexane and methylene chloride as eluent. There was thus obtained 4,6-dichloro-2-(3-nitrophenyl)pyrimidine (4.38 g) as a white solid; NMR Spectrum: (DMSOd₆) 7.89 (t, 1H), 8.1 (s, 1H), 8.49 (d, 1H), 8.71 (d, 1H), 9.0 (s, 1H).
A mixture of 4,6-dichloro-2-(3-nitrophenyl)pyrimidine (8 g), 3-hydroxymethylphenylboronic acid (5.85 g), caesium fluoride (11.2 g), [1,1'-bis(diphenylphosphino)ferrocene]dichloropalladium(II) 1:1 complex with methylene chloride (0.362 g) and methanol (160 ml) was stirred under an atmosphere of nitrogen at ambient temperature for 10 minutes and then heated to 44°C for 3 hours. The reaction mixture was cooled to ambient temperature and the precipitate was collected by filtration, washed in turn with methanol, water and methanol and dried under vacuum. There was this obtained 6-chloro-4-(3-hydroxymethylphenyl)-2-(3-nitrophenyl)pytimidine as a white solid (10.5 g); NMR Spectrum: (DMSOd₆) 4.66 (s, 2H), 5.35 (t, 1H), 7.55-7.6 (m, 2H), 7.91 (t, 1H), 8.25-8.33 (m, 3H), 8.46 (d, 1H), 8.89 (d, 1H), 9.13 (s, 1H).
A mixture of a portion (5.83 g) of the material so obtained, morpholine (5.94 ml) and DMA (50 ml) was stirred and heated to 80°C for 40 minutes. The reaction mixture was evaporated and the residue was triturated under water. The solid was collected by filtration and dried under vacuum. There was thus obtained 4-(3-hydroxymethylphenyl)-6-morpholino-2-(3-nitrophenyl)pyrimidine (8.12 g) which was used in the next stage without further purification.
A mixture of the material so obtained, stannous chloride dihydrate (15.34 g) and ethanol (100 ml) was stirred and heated to 80°C for 1 hour. The reaction mixture was allowed to cool to ambient temperature. DMF was added to dissolve the gummy precipitate. Aqueous ammonium hydroxide solution (d=0.88) was added over 5 minutes with vigorous stirring to produce a white slurry of tin residues. The mixture was filtered and the filtrate was evaporated. The residue was purified by column chromatography on silica using increasingly polar mixtures of isohexane and ethyl acetate as eluent. The material so obtained was triturated under diethyl ether. There was thus obtained 2-(3-aminophenyl)-4-(3-hydroxymethylphenyl)-6-morpholinopyrimidine as a solid (2.94 g); NMR Spectrum: (DMSOd₆) 3.81 (s, 8H), 4.67 (m, 3H), 5.16 (t, 1H), 6.74 (m, 1H), 7.01 (s, 1H), 7.15 (t, 1H), 7.41-7.49 (m, 2H), 7.75 (d, 1H), 7.8 (t, 1H), 8.03-8.11 (m, 1H), 8.16 (s, 1H).

### Example 6

Using analogous procedures to those described in Example 5, the appropriate 2-(3-aminophenyl)pyrimidine was reacted with the appropriate carboxylic acid to give the compounds described in Table III. Unless otherwise stated, primary and secondary amino groups in the carboxylic acid were protected by a *N*-*tert*-butoxycarbonyl group which was removed by treatment with trifluoroacetic acid as described within Example 5.

**Table III**

| | | |
|---|---|---|
| 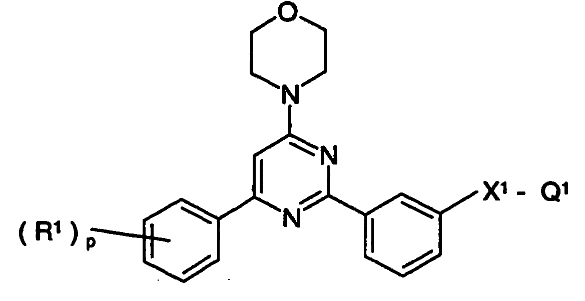 | | |

| No. & Note | (R¹)ₚ | X¹-Q¹ |
|---|---|---|
| [1] | 3-hydroxymethyl | L-alanylamino |
| [2] | 3-hydroxymethyl | (2S)-2-aminobutanoylamino |
| [3] | 3-hydroxymethyl | L-valylamino |
| [4] | 3-hydroxymethyl | 2-aminopent-4-ynoylamino |
| [5] | 3-hydroxymethyl | 2-aminopentanoylamino |
| [6] | 3-hydroxymethyl | L-isoleucylamino |
| [7] | 3-hydroxymethyl | L-leucylamino |
| [8] | 3-hydroxymethyl | 2-methylalanylamino |
| [9] | 3-hydroxymethyl | 2-methyl-L-leucylamino |
| [10] | 3-hydroxymethyl | (*N*-methylalanyl)amino |
| [11] | 3-hydroxymethyl | (*N*-methyl-L-valyl)amino |
| [12] | 3-hydroxymethyl | (*N*-methyl-L-leucyl)amino |
| [13] | 3-hydroxymethyl | D-glutaminylamino |
| [14] | 3-hydroxymethyl | serylamino |
| [15] | 3-hydroxymethyl | L-threonylamino |
| [16] | 3-hydroxymethyl | (*O*-methyl-L-seryl)amino |
| [17] | 3-hydroxymethyl | (*N*-methyl-L-seryl)amino |
| [18] | 3-hydroxymethyl | (*S*-methyl-L-cysteinyl)amino |
| [19] | 3-hydroxymethyl | L-methionylamino |
| [20] | 3-hydroxymethyl | 3-amino-3-methylbutanoylamino |
| [21] | 3-hydroxymethyl | (3S)-3-aminobutanoylamino |
| [22] | 3-hydroxymethyl | D-asparaginylamino |
| [23] | 3-hydroxymethyl | 5-aminopentanoylamino |
| [24] | 3-hydroxymethyl | 6-aminohexanoylamino |
| [25] | 3-hydroxymethyl | (*O*-methyl-L-homoseryl)amino |
| [26] | 3-hydroxymethyl | (4R)-4-amino-4-methoxycarbonylbutanoylamino |
| [27] | 3-hydroxymethyl | glycylglycylamino |
| [28] | 3-hydroxymethyl | (1R,2S)-2-aminocyclopent-1-ylcarbonylamino |
| [29] | 3-hydroxymethyl | (1S,2R)-2-aminocyclopent-1-ylcarbonylamino |
| [30] | 3-hydroxymethyl | 4-aminomethylcyclohex-1-ylcarbonylamino |
| [31] | 3-hydroxymethyl | *trans*-4-aminomethylcyclohex-1-ylcarbonylamino |
| [32] | 3-hydroxymethyl | 4-aminocyclohex-1-ylcarbonylamino |
| [33] | 3-hydroxymethyl | 2-[(1R,3S)-3-aminocyclohex-1-yl]acetamido |
| [34] | 3-hydroxymethyl | pyrrolidin-3-ylcarbonylamino |
| [35] | 3-hydroxymethyl | (2S)-3-pyrrolin-2-ylcarbonylamino |
| [36] | 3-hydroxymethyl | piperidin-2-ylcarbonylamino |
| [37] | 3-hydroxymethyl | piperidin-3-ylcarbonylamino |
| [38] | 3-hydroxymethyl | morpholin-2-ylcarbonylamino |
| [39] | 3-hydroxymethyl | 4-aminotetrahydropyran-4-ylcarbonylamino |
| [40] | 3-hydroxymethyl | 4-aminotetrahydrothiopyran-4-ylcarbonylamino |
| [41] | 3-hydroxymethyl | 2-piperidin-4-ylacetamido |
| [42] | 3-hydroxymethyl | 3-piperidin-4-ylpropionamido |
| [43] | 3-hydroxymethyl | 2-[(2S)-pyrrolidin-2-yl]acetamido |
| [44] | 3-hydroxymethyl | 2-piperidin-4-yloxyacetamido |
| [45] | 3-hydroxymethyl | isoindolin-1-ylcarbonylamino |
| [46] | 3-hydroxymethyl | 4-methylaminobenzamido |
| [47] | 3-hydroxymethyl | 2-aminobenzamido |
| [48] | 3-hydroxymethyl | 3-aminomethylbenzamido |
| [49] | 3-hydroxymethyl | 4-aminomethylbenzamido |
| [50] | 3-hydroxymethyl | 2-amino-4-methylthiazol-5-ylcarbonylamino |
| [51] | 3-hydroxymethyl | 2-(4-aminophenyl)acetamido |
| [52] | 3-hydroxymethyl | 2-amino-2-phenylacetamido |
| [53] | 3-hydroxymethyl | 3-(3-aminophenyl)propionamido |
| [54] | 3-hydroxymethyl | 3-amino-3-phenylpropionamido |
| [55] | 3-hydroxymethyl | (*N*-benzylglycyl)amino |
| [56] | 3-hydroxymethyl | 2-amino-2-(3-thienyl)acetamido |
| [57] | 3-hydroxymethyl | 2-(5-amino-1,2,4-thiadiazol-3-yl)acetamido |

Notes The products gave the characterising data shown below.
[1] Mass Spectrum: M+H⁺ 434; HPLC: method A1, Retention Time 2.58 minutes.
[2] Mass Spectrum: M+H⁺ 448; HPLC: method A1, Retention Time 2.68 minutes.
[3] Mass Spectrum: M+H⁺ 462; HPLC: method A1, Retention Time 2.15 minutes.
[4] Mass Spectrum: M+H⁺ 458; HPLC: method A1, Retention Time 2.68 minutes.
[5] Mass Spectrum: M+H⁺ 462; HPLC: method A1, Retention Time 2.83 minutes.
[6] Mass Spectrum: M+H⁺ 476; HPLC: method A1, Retention Time 2.31 minutes.
[7] Mass Spectrum: M+H⁺ 476; HPLC: method A1, Retention Time 2.95 minutes.
[8] Mass Spectrum: M+H⁺ 448; HPLC: method A1, Retention Time 2.73 minutes.
[9] Mass Spectrum: M+H⁺ 490; HPLC: method A1, Retention Time 3.09 minutes.
[10] Mass Spectrum: M+H⁺ 448; HPLC: method A1, Retention Time 2.66 minutes.
[11] Mass Spectrum: M+H⁺ 476; HPLC: method A1, Retention Time 2.92 minutes.
[12] Mass Spectrum: M+H⁺ 490; HPLC: method A1, Retention Time 2.43 minutes.
[13] Mass Spectrum: M+H⁺ 491; HPLC: method A1, Retention Time 2.41 minutes.
[14] Mass Spectrum: M+H⁺ 450; HPLC: method A1, Retention Time 2.42 minutes.
[15] Mass Spectrum: M+H⁺ 464; HPLC: method A1, Retention Time 2.49 minutes.
[16] Mass Spectrum: M+H⁺ 464; HPLC: method A1, Retention Time 2.61 minutes.
[17] Mass Spectrum: M+H⁺ 464; HPLC: method A1, Retention Time 2.53 minutes.
[18] Mass Spectrum: M+H⁺ 480; HPLC: method A1, Retention Time 2.74 minutes.
[19] Mass Spectrum: M+H⁺ 494; HPLC: method A1, Retention Time 2.81 minutes.
[20] Mass Spectrum: M+H⁺ 462; HPLC: method A1, Retention Time 2.83 minutes.
[21] Mass Spectrum: M+H⁺ 448; HPLC: method A1, Retention Time 2.73 minutes.
[22] Mass Spectrum: M+H⁺ 477; HPLC: method A1, Retention Time 2.4 minutes.
[23] Mass Spectrum: M+H⁺ 462; HPLC: method A1, Retention Time 2.6 minutes.
[24] Mass Spectrum: M+H⁺ 476; HPLC: method A1, Retention Time 1.94 minutes.
[25] Mass Spectrum: M+H⁺ 478; HPLC: method A1, Retention Time 2.63 minutes.
[26] Mass Spectrum: M+H⁺ 506; HPLC: method A1, Retention Time 2.62 minutes.
[27] NMR Spectrum: (DMSOd₆) 3.73-3.87 (m, 8H), 4.0 (s, 2H), 4.63 (s, 2H), 7.27 (s, 1H), 7.4-7.52 (m, 3H), 7.84-7.92 (m, 1H), 8:11-8.33 (m, 4H), 8.55 (t, 1H), 10.14 (s, 1H); Mass Spectrum: M+H⁺ 477.
[28] Mass Spectrum: M+H⁺ 474; HPLC: method A1, Retention Time 2.71 minutes.
[29] Mass Spectrum: M+H⁺ 474; HPLC: method A1, Retention Time 2.48 minutes.
[30] Mass Spectrum: M+H⁺ 502; HPLC: method A1, Retention Time 2.8 minutes.
[31] NMR Spectrum: (DMSOd₆) 0.83-1.0 (m, 2H), 1.16-1.29 (m, 1H), 1.37-1.53 (m, 2H), 1.88 (t, 4H), 2.26-2.43 (m, 3H), 3.72-3.88 (m, 8H), 4.62 (s, 2H), 5.28 (s, 1H), 7.26 (s, 1H), 7.41 (t, 1H), 7.47-7.51 (m, 2H), 7.92-7.98 (m, 1H), 8.11-8.23 (m, 3H), 8.54 (s, 1H), 9.96 (s, 1H); Mass Spectrum: M+H⁺ 502.
[32] Mass Spectrum: M+H⁺ 488; HPLC: method A1, Retention Time 3.09 minutes.
[33] The product gave the following characterising data :- NMR Spectrum: (DMSOd₆) 1.0-0.7 (m, 3H), 1.36-1.16 (m, 1H), 1.91-1.61 (m, 4H), 2.32-2.17 (m, 2H), 3.87-3.74 (m, 8H), 4.63 (s, 2H), 7.27 (s, 1H), 7.42 (t, 2H), 7.52-7.47 (m, 3H), 7.97-7.92 (m, 1H), 8.23-8.13 (m, 3H), 8.53 (t, 1H), 10.01 (s, 1H); Mass Spectrum: M+H⁺ 502.
[34] Mass Spectrum: M+H⁺ 460; HPLC: method A1, Retention Time 2.66 minutes.
[35] Mass Spectrum: M+H⁺ 458; HPLC: method A1, Retention Time 2.84 minutes.
[36] The product gave the following characterising data :- NMR Spectrum: (DMSOd₆) 1.32-1.58 (m, 4H), 1.77-1.9 (m, 2H), 2.54-2.65 (m, 1H), 2.96-3.05 (m, 1H), 3.72-3.88 (m, 8H), 4.62 (s, 2H), 5.27 (s, 1H), 7.27 (s, 1H), 7.39-7.54 (m, 3H), 7.88-7.94 (m, 1H), 8.14-8.2 (m, 2H), 8.21 (s, 1H), 8.62 (t, 1H), 9.79 (s, 1H); Mass Spectrum: M+H⁺ 474.
[37] The product gave the following characterising data :- NMR Spectrum: (DMSOd₆) 2.56-2.67 (m, 3H), 2.79-2.82 (m, 1H), 2.97 (d, 1H), 3.15 (d, 2H), 3.71-3.87 (m, 8H), 4.65 (m, 2H), 5.27 (t, 1H), 5.23-5.31 (m, 1H), 7.27 (s, 1H), 7.38-7.53 (m, 3H), 7.92 (d, 1H), 7.89-7.97 (m, 1H), 8.14-8.21 (m, 3H), 8.55 (s, 1H), 10.15 (s, 1H); Mass Spectrum: M+H⁺ 474.
[38] Mass Spectrum: M+H⁺476; HPLC: method A1, Retention Time 2.62 minutes.
[39] Mass Spectrum: M+H⁺ 490; HPLC: method A1, Retention Time 2.68 minutes.
[40] Mass Spectrum: M+H⁺ 506; HPLC: method A1, Retention Time 2.94 minutes.
[41] The product gave the following characterising data :- NMR Spectrum: (DMSOd₆) 1.04-1.18 (m, 2H), 1.57-1.65 (m, 2H), 2.22-2.27 (m, 2H), 2.88-2.95 (m, 2H), 3.73-3.84 (m, 8H), 4.61 (s, 2H), 7.25 (s, 1H), 7.4 (t, 1H), 7.46-7.5 (m, 2H), 7.9-7.95 (m, 1H), 8.11-8.17 (m, 3H), 8.19 (s, 1H), 8.51 (t, 1H), 10.0 (s, 1H); Mass Spectrum: M+H⁺ 488.
[42] Mass Spectrum: M+H⁺ 502; HPLC: method A1, Retention Time 2.81 minutes.
[43] Mass Spectrum: M+H⁺ 474; HPLC: method A1, Retention Time 2.69 minutes.
[44] The product gave the following characterising data :- NMR Spectrum: (DMSOd₆) 1.31-1.42 (m, 2H), 1.94-1.85 (m, 2H), 2.64-2.74 (m, 1H), 2.87-2.98 (m, 2H), 3.72-3.86 (m, 8H), 4.1 (s, 2H), 4.61 (s, 2H), 7.26 (s, 1H), 7.41-7.5 (m, 3H), 7.83-7.88 (m, 1H), 8.14-8.23 (m, 3H), 8.62 (t, 1H), 9.77 (s, 1H); Mass Spectrum: M+H⁺ 504.
[45] Mass Spectrum: M+H⁺ 508; HPLC: method A1, Retention Time 3.14 minutes.
[46] Mass Spectrum: M+H⁺ 496; HPLC: method A1, Retention Time 3.01 minutes.
[47] Mass Spectrum: M+H⁺ 482; HPLC: method A1, Retention Time 3.1 minutes.
[48] Mass Spectrum: M+H⁺ 496; HPLC: method A1, Retention Time 2.77 minutes.
[49] The product gave the following characterising data :- NMR Spectrum: (DMSOd₆) 3.41 (s, 1H), 3.73-3.93 (m, 10H), 4.63 (s, 2H), 4.63 (s, 2H), 7.28 (s, 1H), 7.46-7.56 (m, 5H), 7.94-8.08 (m, 3H), 8.16-8.26 (m, 3H), 8.77 (t, 1H), 10.37 (s, 1H); Mass Spectrum: M+H⁺ 496.
[50] Mass Spectrum: M+H⁺ 503; HPLC: method A1, Retention Time 2.71 minutes.
[51] Mass Spectrum: M+H⁺ 496; HPLC: method A1, Retention Time 2.81 minutes.
[52] Mass Spectrum: M+H⁺ 496; HPLC: method A1, Retention Time 2.24 minutes.
[53] Mass Spectrum: M+H⁺ 510; HPLC: method A1, Retention Time 2.9 minutes.
[54] Mass Spectrum: M+H⁺ 510; HPLC: method A1, Retention Time 2.02 minutes.
[55] Mass Spectrum: M+H⁺ 510; HPLC: method A1, Retention Time 3.1 minutes.
[56] Mass Spectrum: M+H⁺ 502; HPLC: method A1, Retention Time 2.85 minutes.
[57] The 2-(5-*tert*-butoxycarbonylamino-1,2,4-thiadiazol-3-yl)acetic acid used as a starting material is described in J. Antibiotics, 1983, 36, 1020-1033. The product gave the following characterising data:- Mass Spectrum: M+H⁺ 504; HPLC: method A1, Retention Time 2.59 minutes.

### Example 7

### 4-(3-hydroxymethylphenyl)-2-[3-(1-methylpiperidin-4-ylcarbonylamino)phenyl]-6-morpholinopyrimidine

Diisopropylethylamine (0.054 ml) was added to a stirred mixture of 1-methylpiperidin-4-ylcarboxylic acid (0.02 g), 2-(7-azabenzotriazol-1-yl)-1,1,3,3-tetramethyluronium hexafluorophosphate(V) (0.053 g) and DMF (0.35 ml) and the resultant mixture was stirred for approximately 1 minute under an atmosphere of nitrogen. A solution of 2-(3-aminophenyl)-4-(3-hydroxymethylphenyl)-6-morpholinopyrimidine (0.044 g) in DMF (0.3 ml) was added and the mixture was stirred for 5 hours. The DMF was evaporated and the residue was purified by HPLC using a Waters 'Xterra' preparative reversed-phase column (5 microns silica, 19 mm diameter, 100 mm length) using decreasingly polar mixtures of water and acetonitrile (containing 1% acetic acid) as eluent. There was thus obtained the title compound as a solid (0.03 g); NMR Spectrum: (DMSOd₆) 1.61-1.96 (m, 5H) 2.18 (s, 3H), 3.74-3.87 (m, 8H), 4.63 (d, 2H), 5.27 (t, 1H), 7.27 (s, 1H), 7.41 (t, 2H), 7.48-7.53 (m, 2H), 7.93-7.99 (m, 1H), 8.11-8.23 (m, 3H), 8.52 (t, 1H), 9.99 (s, 1H); Mass Spectrum: M+H⁺ 488; Analytical HPLC: method A1, Retention Time 2.87 minutes.

### Example 8

Using an analogous procedure to that described in Example 7, the appropriate 2-(3-aminophenyl)pyrimidine was reacted with the appropriate carboxylic acid to give the compounds described in Table IV.

**Table IV**

| | | |
|---|---|---|
| 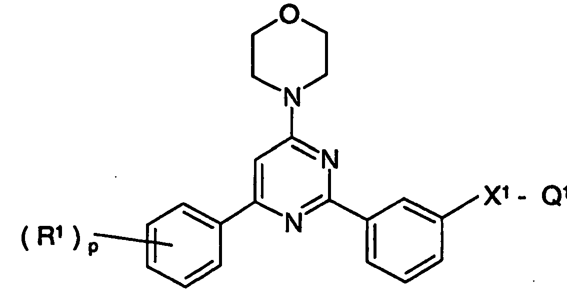 | | |

| No. & Note | (R¹)ₚ | X¹-Q¹ |
|---|---|---|
| [1] | 3-hydroxymethyl | (*N,N-*dimethylglycyl)amino |
| [2] | 3-hydroxymethyl | 6-dimethylaminohexanoylamino |
| [3] | 3-hydroxymethyl | (1-methylprolyl)amino |
| [4] | 3-hydroxymethyl | 2-piperidin-1-ylacetamido |
| [5] | 3-hydroxymethyl | 2-(4-methylpiperazin-1-yl)acetamido |
| [6] | 3-hydroxymethyl | 2-(4-oxo-1,4-dihydropyridin-1-yl)acetamido |
| [7] | 3-hydroxymethyl | 1-methylimidazol-2-ylcarbonylamino |
| [8] | 3-hydroxymethyl | 2-imidazol-1-ylacetamido |
| [9] | 3-hydroxymethyl | 2-(2-azabicyclo[2.2.1]hept-2-yl)acetamido |

| | | |
|---|---|---|
| Notes [1] Mass Spectrum: M+H⁺ 448; HPLC: method A1 Retention Time 2.85 minutes. [2] NMR Spectrum: (DMSOd₆) 1.30-1.49 (m, 4H), 1.64 (m, 2H), 2.12 (s, 6H), 2.21 (t, 2H), 2.35 (t, 2H), 3.73-3.87 (m, 8H), 4.62 (d, 2H), 5.27 (t, 1H), 7.27 (s, 1H), 7.42 (t, 1H), 7.47-7.55 (m, 2H), 7.89-7.96 (m, 1H), 8.12-8.23 (m, 3H), 8.53 (t, 1H), 10.01 (s, 1H); Mass Spectrum: M+H⁺ 504. [3] Mass Spectrum: M+H⁺ 474; HPLC: method A1, Retention Time 3.02 minutes. [4] Mass Spectrum: M+H⁺ 488; HPLC: method A1, Retention Time 3.29 minutes. [5] NMR Spectrum: (DMSOd₆) 2.17 (s, 3H), 2.3-2.41 (m, 4H), 3.14 (s, 2H), 3.73-3.84 (m, 8H), 4.61 (d, 2H), 5.25 (t, 1H), 7.26 (s, 1H), 7.43 (t, 1H), 7.46-7.52 (m, 2H), 7.82-7.86 (m, 1H), 8.14-8.18 (m, 2H), 8.2-8.22 (m, 1H), 8.6 (t, 1H), 9.85 (s, 1H); Mass Spectrum: M+H⁺ 503. [6] Mass Spectrum: M+H⁺ 498; HPLC: method A1, Retention Time 2.46 minutes. [7] Mass Spectrum: M+H⁺ 471; HPLC: method A1, Retention Time 2.99 minutes. [8] Mass Spectrum: M+H⁺ 471; HPLC: method A1, Retention Time 2.62 minutes. [9] The 2-(2-azabicyclo[2.2.1]hept-2-yl)acetic acid used as a starting material was prepared by the reaction of 2-azabicyclo[2.2.1]heptane and ethyl bromoacetate followed by basic hydrolysis of the ester group. The product of the amide coupling reaction gave the following characterising data; NMR Spectrum: (DMSOd₆) 1.23-1.38 (m, 2H), 1.39-1.6 (m, 2H), 1.65-1.81 (m, 2H), 2.3 (d, 1H), 2.35 (s, 1H), 2.83-2.9 (m, 1H), 3.23-3.28 (m, 2H), 3.73-3.87 (m, 8H), 4.63 (d, 2H), 5.26 (t, 1H), 7.27 (s, 1H), 7.4-7.53 (m, 3H), 7.83 (d, 1H), 7.96 (s, 1H), 8.14-8.25 (m, 3H), 8.67 (s, 1H), 9.76 (s, 1H); Mass Spectrum: M+H⁺ 500. | | |

### Example 9

### 2-{3-[3-(3-dimethylaminopropyl)ureido]phenyl}-4-(3-hydroxymethylphenyl)-6-morpholinopyrimidine

Disuccinimido carbonate (0.051 g) was added to a mixture of 2-(3-aminophenyl)-4-(3-*tert*-butoxymethylphenyl)-6-morpholinopyrimidine (0.084 g) and methylene chloride (2 ml) and the resultant mixture was stirred at ambient temperature for 10 minutes. 3-Dimethylaminopropylamine (25 ml) was added and stirring was continued for a further 20 minutes. Trifluoroacetic acid (2 ml) was added and the resultant mixture was allowed to stand at ambient temperature for 16 hours. The mixture was evaporated. The residue was dissolved in methanol and the mixture was neutralised by the addition of a few drops of aqueous ammonium hydroxide solution. The mixture was re-evaporated and the residue was purified by HPLC using a Waters 'Xterra' preparative reversed-phase column (5 microns silica, 19 mm diameter, 100 mm length) using decreasingly polar mixtures of water and acetonitrile (containing 1% acetic acid) as eluent. The product containing fractions were combined and evaporated to produce the title compound as a solid (0.023 g); NMR Spectrum: (DMSOd₆) 1.63-1.72 (m, 2H), 2.41 (s, 6H), 2.6 (t, 2H), 3.13-3.19 (m, 3H), 3.73-3.86 (m, 8H), 4.62 (s, 2H), 6.26 (t, 1H), 7.26 (s, 1H), 7.36 (t, 1H), 7.46-7.54 (m, 2H), 7.71-7.77 (m, 1H), 8.0-8.05 (m, 1H), 8.14-8.17 (m, 2H), 8.21 (s, 1H), 8.3 (t, 1H), 8.7 (s, 1H); Mass Spectrum: M+H⁺491; Analytical HPLC: method B1, Retention Time 0.87 minutes.

The 2-(3-aminophenyl)-4-(3*-tert*-butoxymethylphenyl)-6-morpholinopyrimidine used as a starting material was prepared as follows :-
A mixture of 4,6-dichloro-2-(3-nitrophenyl)pyrimidine (5.4 g), 3-*tert*-butoxymethylphenylboronic acid (7.28 g), caesium fluoride (7.59 g), [1,1'-bis(diphenylphosphino)ferrocene]dichloropalladium(II) 1:1 complex with methylene chloride (1.63 g) and DMA (20 ml) was stirred and heated to 80°C under an atmosphere of nitrogen for 1.5 hours. The reaction mixture was cooled to ambient temperature and evaporated. The residue was partitioned between methylene chloride and water. The organic phase was washed with a saturated brine solution, dried over anhydrous sodium sulphate and evaporated. The residue was purified by column chromatography on silica using an increasingly polar solvent gradient from 0% to 30% ethyl acetate in isohexane as eluent. The material so obtained was triturated under a mixture of isohexane and diethyl ether. There was thus obtained 4-(3-*tert*-butoxymethylphenyl)-6-chloro-2-(3-nitrophenyl)pyrimidine as a solid (3.97 g); Mass Spectrum: M+H⁺ 398; HPLC: method A1, Retention Time 3.91 minutes.
A mixture of the material so obtained, morpholine (3 ml) and DMA (30 ml) was stirred and heated to 80°C for 20 minutes. The reaction mixture was evaporated and the residue was triturated under water. The solid was collected by filtration and dried under vacuum. There was thus obtained to yield 4-(3-*tert*-butoxymethylphenyl)-6-morpholino-2-(3-nitrophenyl)pyrimidine (4.31 g) Mass Spectrum: M+H⁺449; HPLC: method B1, Retention Time 3.01 minutes:
A mixture of the material so obtained, stannous chloride dihydrate (8.6 g) and ethanol (80 ml) was stirred and heated to 80°C for 3 hours. The reaction mixture was allowed to cool to ambient temperature. DMF (30 ml) was added. Aqueous ammonium hydroxide solution (d=0.88) was added over 5 minutes with vigorous stirring to produce a white slurry of tin residues. The mixture was filtered and the filtrate was evaporated. The residue was purified by column chromatography on silica using increasingly polar mixtures of isohexane and ethyl acetate as eluent. The material so obtained was triturated under a mixture of isohexane and diethyl ether. There was thus obtained 2-(3-aminophenyl)-4-(3-*tert*-butoxymethylphenyl)-6-morpholinopyrimidine as a solid (2.96 g); NMR Spectrum: (DMSOd₆) 1.83 (s, 9H), 3.72-3.84 (m, 8H), 4.53 (s, 2H), 5.17 (s, 2H), 7.13 (t, 2H), 7.22 (s, 2H), 7.45-7.5 (m, 2H), 7.63-7.68 (m, 1H), 7.73 (t, 1H), 8.13-8.2 (m, 2H).

### Example 10

### 2-[3-(3-benzylureido)phenyl]-4-(3-hydroxymethylphenyl)-6-morpholinopyrimidine

Using an analogous procedure to that described in Example 9, 2-(3-aminophenyl)-4-(3-*tert*-butoxymethylphenyl)-6-morpholinopyrimidine was reacted with benzylamine and converted into the title compound in 22% yield; Mass Spectrum: M+H⁺496; HPLC: method A1, Retention Time 2.44 minutes.

### Example 11

### 4-(3-hydroxymethylphenyl)-6-morpholino-2-(3-piperidin-4-ylcarbonylaminophenyl)pyrimidine

A mixture of 4-bromo-2-[3-(1-*tert*-butoxycarbonylpiperidin-4-ylcarbonylamino)phenyl]-6-morpholinopyrimidine (0.15 g), 3-hydroxymethylphenylboronic acid (0.053 g), tetrakis(triphenylphosphine)palladium(0) (10 mg), a saturated aqueous sodium bicarbonate solution (1 ml) and 1,2-dimethoxyethane (10 ml) was stirred and heated to 75°C for 3 hours under an atmosphere of nitrogen. The resultant reaction mixture was evaporated and the residue was triturated under a 10:1 mixture of methylene chloride and methanol. The resultant mixture was filtered and the filtrate was evaporated. A mixture of the residue from the evaporation, trifluoroacetic acid (1.5 ml) and methylene chloride (1.5 ml) was stirred at ambient temperature for 30 minutes. The mixture was evaporated. Toluene was added and the mixture was re-evaporated. The residue was purified by column chromatography on reversed-phase silica using an 'Isolute SCX' column (2 g) by initially washing the column with methanol followed by elution with a 7M methanolic ammonia solution. The material so obtained was purified further by column chromatography on silica using a 17:3 mixture of methylene chloride and a 7M methanolic ammonia solution as eluent. The material so obtained was triturated under isohexane. There was thus obtained the title compound as a solid (0.071 g); NMR Spectrum: (DMSOd₆) 1.93-2.04 (m, 2H), 2.62-2.74 (m, 1H), 2.88-2.98 (m, 2H), 3.31-3.39 (m, 2H), 3.72-3.77 (d, 4H), 3.77-3.83 (d, 4H), 4.6 (s, 2H), 7.23 (s, 1H), 7.4 (t, 1H), 7.45-7.5 (m, 2H), 7.92 (d, 1H), 8.11-8.2 (m, 3H), 8.52 (s, 1H); Mass Spectrum: M+H⁺474.

### Example 12

### 4-(3-hydroxymethylphenyl)-6-(2-methylmorpholin-4-yl)-2-(3-piperidin-4-ylcarbonylaminophenyl)pyrimidine

A mixture of 2-[3-(1-*tert*-butoxycarbonylpiperidin-4-ylcarbonylamino)phenyl]-6-chloro-4-(3-hydroxymethylphenyl)pyrimidine (0.063 g), 2-methylmorpholine (Tetrahedron, 1980, 36, 409-415; 0.04 g) and DMA (1 ml) was stirred and heated to 130°C for 5 hours under an atmosphere of nitrogen. The reaction mixture was evaporated. Toluene was added and the mixture was re-evaporated. The residue was purified by column chromatography on silica using an increasingly polar solvent gradient from 0% to 10% methanol in methylene chloride as eluent. A mixture of the material so obtained and trifluoroacetic acid (1 ml) was stirred at ambient temperature for 30 minutes. The resultant mixture was evaporated and the residue was purified by column chromatography on an 'Isolute SCX' column (1 g) by initially washing the column with methylene chloride and then with methanol followed by elution with a 7M methanolic ammonia solution. There was thus obtained the title compound (0.038 g); NMR Spectrum: (DMSOd₆) 1.21 (d, 3H), 1.92-2.03 (m, 2H), 2.61-2.79 (m, 2H), 2.88-3.12 (m, 3H), 3.32=3.42 (m, 2H), 3.53-3.66 (m, 2H), 3.93-4.02 (m, 1H), 4.45-4.57 (m, 2 H), 4.62 (s, 2H), 7.23 (s, 1H), 7.42 (t, 1H), 7.48 (d, 2H), 7.91 (d, 1H), 8.11-8.2 (m, 3H), 8.56 (s, 1H); Mass Spectrum: M+H⁺488.

The 2-[3-(1-*tert*-butoxycarbonylpiperidin-4-ylcarbonylamino)phenyl]-6-chloro-4-(3-hydroxymethylphenyl)pyrimidine used as a starting material was prepared as follows :-
A mixture of 6-chloro-4-(3-hydroxymethylphenyl)-2-(3-nitrophenyl)pyrimidine (14.5-g), stannous chloride dihydrate (38 g) and DMA (100 ml) was stirred and heated to 50°C for 2 hours. The reaction mixture was allowed to cool to ambient temperature. Methylene chloride and an aqueous ammonium hydroxide solution (d=0.88) were added and the mixture was stirred for 5 minutes. The resultant mixture was filtered and the filtrate was evaporated. Toluene was added and the mixture was re-evaporated. Methylene chloride was added, the resultant mixture was filtered and the filtrate was evaporated. The resultant residue was purified by column chromatography on silica using an increasingly polar solvent gradient from 0% to 50% ethyl acetate in methylene chloride as eluent. There was thus obtained 2-(3-aminophenyl)-6-chloro-4-(3-hydroxymethylphenyl)pyrimidine as an oil (11.9 g); NMR Spectrum: (CDCl₃) 4.78 (s, 2H), 6.76-6.8 (m, 1H), 7.23 (t, 1H), 7.43-7.51 (m, 2H), 7.54 (s, 1H), 7.8-7.83 (m, 1H), 7.9 (d, 1H), 8.01-8.05 (m, 1H), 8.11 (s, 1H); Mass Spectrum: M+H⁺ 312.
2-(7-Azabenzotriazol-1-yl)-1,1,3,3-tetramethyluronium hexafluorophosphate(V) (8.55 g) was added to a mixture of *N*-(*tert*-butoxycarbonyl)piperidine-4-carboxylic acid (5.16 g), diisopropylethylamine (3.58 ml) and methylene chloride (100 ml) and the mixture was stirred at ambient temperature for 20 minutes under an atmosphere of nitrogen. The mixture was cooled to 5°C and a solution of 2-(3-aminophenyl)-6-chloro-4-(3-hydroxymethylphenyl)pyrimidine (7.8 g) in methylene chloride (30 ml) was added. The resultant mixture was allowed to warm and was stirred at ambient temperature for 2 hours. The reaction mixture was partitioned between methylene chloride and water. The organic phase was washed in turn with water and with an aqueous ammonium hydroxide solution (d=0.88). The organic solution was dried over magnesium sulphate and evaporated. The residue was purified by column chromatography on silica using an increasingly polar gradient of 0% to 30% ethyl acetate in methylene chloride as eluent. There was thus obtained 2-[3-(1-*tert*-butoxycarbonylpiperidin-4-ylcarbonylamino)phenyl]-6-chloro-4-(3-hydroxymethylphenyl)pyrimidine as a foam (8.04 g); NMR Spectrum: (CDCl₃) 1.48 (s, 9H), 1.73-1.85 (m, 2H), 1.89-1.98 (m, 2H), 2.22 (t, 1H), 2.37-2.49 (m, 1H), 2.73-2.88 (m, 2H), 4.12-4.29 (m, 2H), 4.84 (d, 2H), 7.44-7.57 (m, 4H), 7.62 (s, 1H), 8.01-8.09 (m, 2H), 8.21 (s, 1H), 8.3 (d, 1H). 8.37 (s, 1H).

### Example 13

### 4-(3-hydroxymethylphenyl)-5-methyl-6-morpholino-2-(3-piperidin-4-ylcarbonylaminophenyl)pyrimidine

Diisopropylethylamine (1 ml) was added to a mixture of 1-(*tert*-butoxycarbonyl)piperidine-4-carboxylic acid (0.177 g), 2-(7-azabenzotriazol-1-yl)-1,1,3,3-tetramethyluronium hexafluorophosphate(V) (0.293 g) and DMF (1 ml) and the mixture was stirred at ambient temperature for 2 minutes under an atmosphere of nitrogen. A solution of 2-(3-aminophenyl)-4-(3-hydroxymethylphenyl)-5-methyl-6-morpholinopyrimidine (0.264 g) in DMF (1 ml) was added and the resultant mixture was stirred at ambient temperature for 20 hours. The DMF was evaporated. Trifluoroacetic acid (2.85 ml) and water (0.15 ml) were added to the residue and the mixture was stirred at ambient temperature for 30 minutes. The mixture was evaporated and the residue was purified by HPLC using a Waters 'Xterra' preparative reversed-phase column (5 microns silica, 19 mm diameter, 100 mm length) using decreasingly polar mixtures of water and acetonitrile (containing 1% acetic acid) as eluent. There was thus obtained the title compound as a solid (0.088 g); NMR Spectrum: (DMSOd₆) 1.43-1.63 (m, 2H), 1.63-1.76 (m, 2H), 2.21 (s, 3H), 2.94-3.03 (m, 3H), 3.52 (t, 4H), 3.8 (t, 4H), 4.6 (d, 2H), 5.27 (t, 1H), 7.35-7.65 (m, 5H), 7.9-7.95 (m, 1H), 8.03-8.08 (m, 1H), 8.46 (t, 1H), 9.94 (s, 1H); Mass Spectrum: M+H⁺488; Analytical HPLC: method B1, Retention Time 0.93 minutes.

The 2-(3-aminophenyl)-4-(3-hydroxymethylphenyl)-5-methyl-6-morpholinopyrimidine used as a starting material was prepared as follows:-
Using an analogous procedure to that described in the portion of Example 1 that is concerned with the preparation of starting materials, 3-nitrobenzamidine hydrochloride was reacted with diethyl α-methymalonate to give 6-hydroxy-5-methyl-2-(3-nitmphenyl)pyrimidin-4(3*H*)-one in 20% yield which, using analogous procedures to those described in the portion of Example 5 that is concerned with the preparation of starting materials, was converted into 2-(3-aminophenyl)-4-(3-hydroxymethylphenyl)-5-methyl-6-morpholinopyrimidine in 5% yield; Mass Spectrum: M+H⁺ 377.

### Example 14

### 4-(4-chloro-3-hydroxymethylphenyl)-6-morpholino-2-(3-piperidin-4-ylcarbonylaminophenyl)pyrimidine

Diisopropylethylamine (0.096 ml) was added to a mixture of 1-(*tert*-butoxycarbonyl)piperidine-4-carboxylic acid (0.063 g), 2-(7-azabenzotriazol-1-yl)-1,1,3,3-tetramethyluronium hexafluorophosphate(V) (0.105 g) and DMF (1 ml) and the mixture was stirred at ambient temperature for 2 minutes under an atmosphere of nitrogen. A solution of 2-(3-aminophenyl)-4-(4-chloro-3-hydroxymethylphenyl)-6-morpholinopyrimidine (0.099 g) in DMF (1 ml) was added and the resultant mixture was stirred at ambient temperature for 20 hours. The DMF was evaporated. Trifluoroacetic acid (2.85 ml) and water (0.15 ml) were added to the residue and the mixture was stirred at ambient temperature for 30 minutes. The mixture was evaporated and the residue was purified by HPLC using a Waters. 'Xterra' preparative reversed-phase column (5 microns silica, 19 mm diameter, 100 mm length) using decreasingly polar mixtures of water and acetonitrile (containing 1 % acetic acid) as eluent. There was thus obtained the title compound as a solid (0.045 g); NMR Spectrum: (DMSOd₆) 1.54-1.71 (m, 2H), 1.74-1.84 (m, 2H), 2.57-2.68 (m, 2H), 3.1 (m, 2H), 3.71-3.91 (m 10H), 4.67 (s, 2H), 5.47 (s, 1H), 7.28 (s, 1H), 7.42 (t, 1H), 7.57 (d, 1H), 7.92-7.98 (m, 1H), 8.12-8.23 (m, 2H), 8.44 (d, 1H), 8.52 (t, 1H), 10.02 (s, 1H); Mass Spectrum: M+H⁺ 508 & 510; Analytical HPLC: method B2, Retention Time 2.51 minutes.

The 2-(3-aminophenyl)-4-(4-chloro-3-hydroxymethylphenyl)-6-morpholinopyrimidine used as a starting material was prepared as follows :-
A mixture of 5-bromo-2-chlorobenzyl alcohol (2.22 g), *tert*-butyldimethylsilyl chloride (2.26 g), imidazole (1.36 g) and DMF (25 ml) was stirred at ambient temperature for 1 hour. The mixture was evaporated and the residue was partitioned between ethyl acetate and water. The organic phase was washed in turn with water and with a saturated brine solution, dried over anhydrous sodium sulphate and evaporated The residue was purified by column chromatography on silica using an increasingly polar gradient of from 0% to 40% ethyl acetate in isohexane as eluent. There was thus obtained 3-*tert*-butyldimethylsilyloxymethyl-4-chlorobromobenzene as an oil (2.78 g) which was used without further purification.
Under a stream of dry nitrogen, n-butyl lithium (1.6M in hexane; 5.64 ml) was added to a stirred solution of 3-*tert*-butyldimethylsilyloxymethyl-4-chlorobromobenzene (2.78 g) in dry THF (25 ml) that had been cooled to -78°C. The resultant solution was stirred at -78°C for 90 minutes. A solution of dry zinc bromide (2.2 g) in THF (25 ml) was added and the resultant solution was stirred at -78°C for 45 minutes. The reaction mixture allowed to warm to ambient temperature and 4,6-dichloro-2-(3-nitrophenyl)pyrimidine (2.03 g) and tetrakis(triphenylphosphine)palladium(0) (0.435 g) were added in turn. The resultant reaction mixture was stirred and heated to reflux for 16 hours. The mixture was cooled to ambient temperature and partitioned between ethyl acetate and water. The ethyl acetate layer was washed in turn with water and with a saturated brine solution, dried over anhydrous sodium sulphate and evaporated. The material so obtained was triturated under ethanol. The resultant solid was collected by filtration and dried. There was thus obtained 4-(3-*tert*-butyldimethylsilyloxymethyl-4-chlorophenyl)-6-chloro-2-(3-nitrophenyl)pyrimidine (2.644 g); Analytical HPLC: method B3, Retention Time 3.97 minutes.
A mixture of a portion (0.491 g) of the material so obtained, morpholine (0.436 ml) and DMF (5 ml) was stirred and heated to 120°C for 20 minutes. The reaction mixture was evaporated and the residue was triturated under ethanol. The solid was collected by filtration and dried under vacuum. There was thus obtained 4-(3*-tert* butyldimethylsilyloxymethyl-4-chlorophenyl)-6-morpholino-2-(3-nitrophenyl)pyrimidine (0.357 g); Mass Spectrum: M+H⁺ 541 & 543; Analytical HPLC: method B3, Retention Time 3.72 minutes.

A mixture of 4-(3-*tert*-butyldimethylsilyloxymethyl-4-chlorophenyl)-6-morpholino-2-(3-nitrophenyl)pyrimidine (3.68 g), stannous chloride dihydrate (3.92 g) and ethanol (50 ml) was stirred and heated to reflux for 1 hour. The reaction mixture was allowed to cool to ambient temperature and aqueous ammonium hydroxide solution (d=0.88) was added with vigorous stirring until no further precipitation of tin residues was noted. The mixture was filtered and the solid was washed in turn with ethanol and with DMF. The filtrate and washings were combined and evaporated. The residue was triturated under ethanol. There was thus obtained 2-(3-aminophenyl)-4-(4-chloro-3-hydroxymethylphenyl)-6-morpholinopyrimidine as a solid (1.46 g); Mass Spectrum: M+H⁺ 397 & 399; Analytical HPLC: method B1, Retention Time 1.39 minutes.

### Example 15 4-(3-hydroxymethylphenyl)-6-morpholino-2-(3-piperazin-1-ylcarbonylaminophenyl)pyrimidine

Disuccinimido carbonate (0.051 g) was added to a mixture of 2-(3-aminophenyl)-4-(3-*tert*-butoxymethylphenyl)-6-morpholinopyrimidine (0.084 g) and methylene chloride (2 ml) and the resultant mixture was stirred at ambient temperature for 10 minutes. *tert-*Butyl piperazine-1-carboxylate (0.037 g) was added and stirring was continued for a further 20 minutes. Trifluoroacetic acid (20 ml) was added and the resultant mixture was stirred at ambient temperature for 2 hours. The mixture was evaporated. The residue was dissolved in methanol and the mixture was re-evaporated. The residue was purified by HPLC using a Waters 'Xterra' preparative reversed-phase column (5 microns silica, 19 mm diameter, 100 mm length) using decreasingly polar mixtures of water and acetonitrile (containing 1% acetic acid) as eluent. The product containing fractions were combined and evaporated to produce the title compound as a solid (0.027 g); Mass Spectrum: M+H⁺ 475; Analytical HPLC: method A1, Retention Time 1.9 minutes.

## Claims

1. A pyrimidine derivative of the Formula I 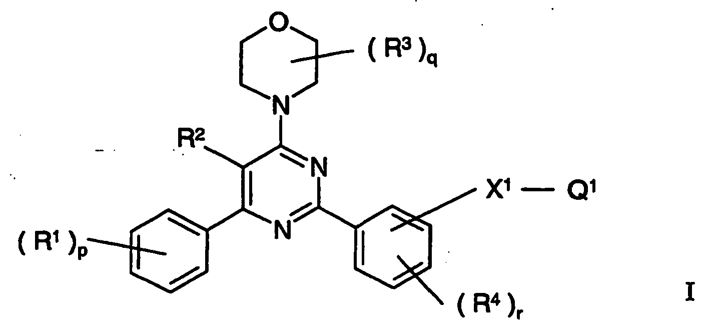 wherein **p** is 1, 2 or 3;
**each R¹ group,** which may be the same or different, is selected from halogeno, trifluoromethyl, cyano, isocyano, nitro, hydroxy, mercapto, amino, formyl, carboxy, carbamoyl, ureido, (1-8C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, *N*-(1-6C)alkylcarbamoyl, *N,N-*di-[(1-6C)alkyl]carbamoyl, (2-6C)alkanoyl, (2-6C)alkanoyloxy, (2-6C)alkanoylamino, *N*-(1-6C)alkyl-(2-6C)alkanoylamino, (3-6C)alkenoylamino, *N*-(1-6C)allcyl-(3-6C)alkenoylamino, (3-6C)alkynoylamino, *N*-(1-6C)alkyl-(3-6C)alkynoylamino, *N'*-(1-6C)alkylureido, *N',N'*-di-[(1-6C)alkyl]ureido, *N*-(1-6C)alkylureido, *N*,*N'*-di-[(1-6C)alkyl]ureido, *N,N',N'*-tri-[(1-6C)alkyl]ureido, *N*-(1-6C)alkylsulphamoyl, *N,N-*di-[(1-6C)allcyl]sulphamoyl, (1-6C)alkanesulphonylamino and *N*-(1-6C)alkyl-(1-6C)alkanesulphonylamino, or from a group of the formula:
Q²-X²-
wherein X² is a direct bond or is selected from O, S, SO, SO₂, N(R⁵), CO, CH(OR⁵), CON(R⁵), N(R⁵)CO, N(R⁵)CON(R⁵), SO₂N(R⁵), N(R⁵)SO₂, OC(R⁵)₂, SC(R⁵)₂ and N(R⁵)C(R⁵)₂, wherein R⁵ is hydrogen or (1-8C)alkyl, and Q² is aryl, aryl-(1-6C)alkyl, (3-8C)cycloalkyl, (3-8C)cycloalkyl-(1-6C)alkyl, (3-8C)cycloalkenyl, (3-8C)cycloalkenyl-(1-6C)alkyl, heteroaryl, heteroaryl-(1-6C)alkyl, heterocyclyl or heterocyclyl-(1-6C)alkyl, or (R¹)ₚ is (1-3C)alkylenedioxy,
and wherein any CH, CH₂ or CH₃ group within a R¹ substituent optionally bears on each said CH, CH₂ or CH₃ group one or more halogeno or (1-8C)alkyl substituents and/or a substituent selected from hydroxy, mercapto, amino, cyano, carboxy, carbamoyl, ureido, (1-6C)alkoxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, *N*-(1-6C)alkylcarbamoyl, *N*,*N*-di-[(1-6C)alkyl]carbamoyl, (2-6C)alkanoyl, (2-6C)alkanoyloxy, (2-6C)alkanoylamino, *N-*(1-6C)alkyl-(2-6C)alkanoylamino, *N*-(1-6C)alkylureido, *N'*-(1-6C)alkylureido, *N*',*N*'-di-[(1-6C)alkyl]ureido, *N,N'*-di-[(1-6C)alkyl]ureido, *N*,*N'*,*N'*-tri-[(1-6C)alkyl]ureido, *N-*(1-6C)alkylsulphamoyl, *N,N* di-[(1-6C)alkyl]sulphamoyl, (1-6C)alkanesulphonylamino and *N*-(1-6C)alkyl-(1-6C)alkanesulphonylamino, or from a group of the formula :
-X³-Q³
wherein X³ is a direct bond or is selected from O, S, SO, SO₂, N(R⁶), CO, CH(OR⁶), CON(R⁶), N(R⁶)CO, N(R⁶)CON(R⁶), SO₂N(R⁶), N(R⁶)SO₂, C(R⁶)₂O, C(R⁶)₂S and C(R⁶)₂N(R⁶), wherein R⁶ is hydrogen or (1-8C)alkyl, and Q³ is aryl, aryl-(1-6C)alkyl, (3-8C)cycloalkyl, (3-8C)cycloalkyl-(1-6C)alkyl, (3-8C)cycloalkenyl, (3-8C)cycloalkenyl-(1-6C)alkyl, heteroaryl, heteroaryl-(1-6C)alkyl, heterocyclyl or heterocyclyl-(1-6C)alkyl,
and wherein any aryl, (3-8C)cycloalkyl, (3-8C)cycloalkenyl, heteroaryl or heterocyclyl group within a substituent on R¹ optionally bears 1, 2 or 3 substituents, which may be the same or different, selected from halogeno, trifluoromethyl, cyano, nitro, hydroxy, amino, carboxy, carbamoyl, ureido, (1-8C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, (2-6C)alkanoyl, (2-6C)alkanoyloxy, *N*-(1-6C)alkylcarbamoyl, *N*,*N*-di-[(1-6C)alkyl]carbamoyl, (2-6C)alkanoylamino, *N-*(1-6C)alkyl-(2-6C)alkanoylamino, *N-*(1-6C)alkylureido, *N*'-(1-6C)alkylureido, *N*',*N*'-di-[(1-6C)alkyl]ureido, *N*,*N*'-di-[(1-6C)alkyl]ureido, *N*,*N*',*N*'-tri-[(1-6C)alkyl]ureido, *N*-(1-6C)alkylsulphamoyl, *N,N-*di-[(1-6C)alkyl]sulphamoyl, (1-6C)alkanesulphonylamino and *N*-(1-6C)alkyl-(1-6C)alkanesulphonylamino, or from a group of the formula:
-X⁴-R⁷
wherein X⁴ is a direct bond or is selected from O and N(R⁸), wherein R⁸ is hydrogen or (1-8C)alkyl, and R⁷ is halogeno-(1-6C)alkyl, hydroxy-(1-6C)alkyl, mercapto-(1-6C)alkyl, (1-6C)alkoxy-(1-6C)alkyl, (1-6C)alkylthio-(1-6C)alkyl, cyano-(1-6C)alkyl, amino-(1-6C)alkyl, (1-6C)alkylamino-(1-6C)alkyl, di-[(1-6C)alkyl]amino-(1-6C)alkyl, (2-6C)alkanoylamino-(1-6C)alkyl, (1-6C)alkoxycarbonylamino-(1-6C)alkyl, *N*-(1-6C)alkylureido-(1-6C)alkyl, *N*'-(1-6C)alkylureido-(1-6C)alkyl, *N',N*'-di-[(1-6C)alkyl]ureido-(1-6C)alkyl, *N,N*'-di-[(1-6C)alkyllureido-(1-6C)alkyl or *N,N',N*'-tri-[(1-6C)alkyl]ureido-(1-6C)alkyl, or from a group of the formula:
-X⁵-Q⁴
wherein X⁵ is a direct bond or is selected from O, CO and N(R⁹), wherein R⁹ is hydrogen or (1-8C)alkyl, and Q⁴ is aryl, aryl-(1-6C)alkyl, heteroaryl, heteroaryl-(1-6C)alkyl, heterocyclyl or heterocyclyl-(1-6C)alkyl which optionally bears 1 or 2 substituents, which may be the same or different, selected from halogeno, hydroxy, (1-8C)alkyl and (1-6C)alkoxy,
and wherein any heterocyclyl group within a substituent on R¹ optionally bears 1 or 2 oxo or thioxo substituents,
and wherein adjacent carbon atoms in any (2-6C)alkylene chain within a R¹ substituent are optionally separated by the insertion into the chain of a group selected from O, S, SO, SO₂, N(R¹⁰), CO, CH(OR¹⁰), CON(R¹⁰), N(R¹⁰)CO, N(R¹⁰)CON(R¹⁰), SO₂N(R¹⁰), N(R¹⁰)SO₂, CH=CH and C≡C wherein R¹⁰ is hydrogen or (1-8C)alkyl;
**R²** is hydrogen or (1-8C)alkyl;
**q** is 0, 1,2,3 or 4;
**each R³ group,** which may be the same or different, is (1-8C)alkyl or a group of the formula:
-X⁶-R¹¹
wherein X⁶ is a direct bond or is selected from O and N(R¹²), wherein R¹² is hydrogen or (1-8C)alkyl, and R¹¹ is halogeno-(1-6C)alkyl, hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl, di-[(1-6C)alkyl]amino-(1-6C)alkyl or (2-6C)alkanoylamino-(1-6C)alkyl;
**r** is 0, 1 or 2;
**each R⁴ group,** which may be the same or different, is selected from halogeno, trifluoromethyl, cyano, nitro, hydroxy, mercapto, amino, carboxy, carbamoyl, ureido, (1-8C)alkyl, (2-8C)alkenyl, (2-8C)alkynyl, (1-6C)alkoxy, (1-6C)alkylthio, (1-6C)alkylsulphinyl, (1-6C)alkylsulphonyl, (1-6C)alkylamino, di-[(1-6C)alkyl]amino, (1-6C)alkoxycarbonyl, *N*-(1-6C)alkylcarbamoyl, *N,N*-di-[(1-6C)alkyl]carbamoyl, (2-6C)alkanoyl, (2-6C)alkanoyloxy, (2-6C)alkanoylamino, *N*-(1-6C)alkyl-(2-6C)alkanoylamino, *N'*-(1-6C)alkylureido, *N'*,*N'*-di-[(1-6C)alkyl]ureido, *N*-(1-6C)alkylureido, *N*,*N'*-di-[(1-6C)alkyl]ureido, *N*,*N'*,*N*'-tri-[(1-6C)alkyl]ureido, *N*-(1-6C)alkylsulphamoyl, *N*,*N*-di-[(1-6C)alkyl]sulphamoyl, (1-6C)alkanesulphonylamino and *N*-(1-6C)alkyl-(1-6C)alkanesulphonylamino;
**X¹** is selected from CO, N(R¹³)CO, CON(R¹³), N(R¹³)CON(R¹³), N(R¹³)COC(R¹³)₂O, N(R¹³)COC(R¹³)₂S, N(R¹³)COC(R¹³)₂N(R¹³) and N(R¹³)COC(R¹³)₂N(R¹³)CO, wherein R¹³ is hydrogen or (1-8C)alkyl; and
**Q¹** is (1-8C)alkyl, (2-8C)alkenyl, (2-8C)alkynyl, halogeno-(1-6C)alkyl, hydroxy-(1-6C)alkyl, mercapto-(1-6C)alkyl, (1-6C)alkoxy-(1-6C)alkyl, cyano-(1-6C)alkyl, amino-(1-6C)alkyl, (1-6C)alkylamino-(1-6C)alkyl, di-[(1-6C)alkyl]amino-(1-6C)alkyl. (1-6C)alkylthio-(1-6C)alkyl, (1-6C)alkylsulphinyl-(1-6C)alkyl, (1-6C)alkylsulphonyl-(1-6C)alkyl, (2-6C)alkanoylamino-(1-6C)alkyl, *N*-(1-6C)alkyl-(2-6C)alkanoylamino-(1-6C)alkyl, (1-6C)alkoxycarbonylamino-(1-6C)alkyl, *N*-(1-6C)alkylureido-(1-6C)alkyl, *N*-(1-6C)alkylureido-(1-6C)alkyl, *N*',*N*'-di-[(1-6C)alkyl]ureido-(1-6C)alkyl,*N*,*N*'-di-[(1-6C)alkyl]ureido-(1-6C)alkyl, *N*,*N'*,*N'*-tri-[(1-6C)alkyl]ureido-(1-6C)alkyl, (1-6C)alkanesulphonylamino-(1-6C)alkyl or *N*-(1-6C)alkyl-(1-6C)alkanesulphonylamino-(1-6C)alkyl, or **Q¹** is aryl, aryl-(1-6C)alkyl, (3-8C)cycloalkyl, (3-8C)cycloalkyl-(1-6C)alkyl, (3-8C)cycloalkenyl, (3-8C)cycloalkenyl-(1-6C)alkyl, heteroaryl, heteroaryl-(16C)alkyl, heterocyclyl or heterocyclyl-(1-6C)alkyl,
and wherein any CH, CH₂ or CH₃ group within the Q¹ group optionally bears on each said CH, CH₂ or CH₃ group one or more halogeno or (1-8C)alkyl substituents and/or a substituent selected from hydroxy, mercapto, amino, cyano, carboxy, carbamoyl, ureido, (1-6C)alkoxy. (1-6C)alkylthio. (1-6C)alkylsulphinyl, (1-6C)alkylsulphonyl. (1-6C)alkylamino. di-[(1-6C)alkyl]amino, (1-6C)alkoxycarbonyl, *N*-(1-6C)alkylcarbamoyl, *N*,*N*-di-[(1-6C)alkyl]carbamoyl, (2-6C)alkanoyl, (2-6C)alkylanoyloxy, (2-6C)alkanoylamino, *N*-(1-6C)alkyl-(2-6C)alkanoylamino, *N*'-(1-6C)alkylureido, *N'*,*N*'-di-[(1-6C)alkyl]ureido, *N*-(1-6C)alkyluraido, *N*,*N'*-di-[(1-6C)alkyl]ureido, *N*,*N'*,*N'*-tri-[(1-6C)alkyl]ureido, *N*-(1-6C)alkylsulphamoyl, *N*,*N*-di-[(1-6C)alkyl]sulphamoyl, (1-6C)alkanesulphonylamino and *N*-(1-6C)alkyl-(1-6C)alkylcanesulphonylamino,
and wherein any aryl, (3-8C)cycloalkyl, (3-8C)cycloalkenyl, heteroaryl or heterocyclyl group within the Q¹ group optionally bears 1, 2 or 3 substituents, which may be the same or different, selected from halogeno, trifluoromethyl, cyano, nitro, hydroxy, amino, carboxy, carbamoyl, ureido, (1-8C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulphinyl, (1-6C)alkylsulphonyl, (1-6C)alkylamino, di-[(1-6C)allcyl]amino, (1-6C)alkoxycarbonyl, (2-6C)alkanoyl, (2-6C)alkanoyloxy, *N*-(1-6C)alkylcarbamoyl, *N,N*-di-[(1-6C)alkyl]carbamoyl, (2-6C)alkanoylamino, *N*-(1-6C)alkyl-(2-6C)alkanoylamino, *N*'-(1-6C)alkylureido, *N',N*'-di-[(1-6C)alkyl]ureido, *N*-(1-6C)alkylureido, *N,N'*-di-[(1-6C)alkyl]ureido, *N,N',N*'-tri-[(1-6C)alkyl]ureido, *N*-(1-6C)alkylsulphamoyl, *N,N*-di-[(1-6C)alkyl]sulphamoyl, (1-6C)alkanesulphonylamino and *N*-(1-6C)alkyl-(1-6C)alkanesulphonylamino, or from a group of the formula:
-X⁷ -R¹⁴
wherein X⁷ is a direct bond or is selected from O and N(R¹⁵), wherein R¹⁵ is hydrogen or (1-8C)alkyl, and R¹⁴ is halogeno-(1-6C)alkyl, hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl or di-[(1-6C)alkyl]amino-(1-6C)alkyl, or from a group of the formula:
-X⁸-Q⁵
wherein X⁸ is a direct bond or is selected from O, CO and N(R¹⁷), wherein R¹⁷ is hydrogen or (1-8C)alkyl, and Q⁵ is aryl, aryl-(1-6C)alkyl, heteroaryl, heteroaryl-(1-6C)alkyl, heterocyclyl or heterocyclyl-(1-6C)alkyl which optionally bears 1 or 2 substituents, which may be the same or different, selected from halogeno, hydroxy, (1-8C)alkyl and (1-6C)alkoxy,
and wherein any heterocyclyl group within the Q¹ group optionally bears 1 or 2 oxo or thioxo substituents,
and wherein adjacent carbon atoms in any (2-6C)alkylene chain within the Q¹ group are optionally separated by the insertion into the chain of a group selected from O, S, SO, SO₂ N(R¹⁶), N(R¹⁶)CO, CON(R¹⁶), N(R¹⁶)CON(R¹⁶), CO, CH(OR¹⁶), N(R¹⁶)SO₂, SO₂N(R¹⁶), CH=CH and C≡C wherein R¹⁶ is hydrogen or (1-8C)alkyl;
or a pharmaceutically-acceptable salt, solvate or pro-drug thereof.

2. A pyrimidine derivative of the Formula I according to claim 1 wherein :-
p is 1 or 2, and a first R¹ group is selected from hydroxy, carbamoyl, acetamido, propionamido, *N*-methylacetamido, *N*-methylpropionamido, hydroxymethyl, 1-hydroxyethyl and 1-hydroxy-1-methylethyl, and the optional second R¹ group is selected from fluoro, chloro, trifluoromethyl, cyano, hydroxy, methyl, ethyl, methoxy and ethoxy;
R² is hydrogen or methyl;
q is 0 or q is 1 and the R³ group is methyl;
r is 0 or r is 1 and the R⁴ group is selected from fluoro, chloro, trifluoromethyl, hydroxy, amino, methyl, methoxy, methylamino and dimethylamino;
the X¹-Q¹ group is located at the 3-position;
X¹ is selected from NHCO, NHCONH, NHCOCH₂O, NHCOCH₂NH and NHCOCH₂NHCO; and
Q¹ is methyl, ethyl, propyl, butyl, pentyl, aminomethyl, 2-aminoethyl, 2-amino-2-methylpropyl, 3-aminopropyl, 4-aminobutyl, 5-aminopentyl, methylaminomethyl, 2-methylaminoethyl, 3-methylaminopropyl, 4-methylaminobutyl, 5-methylaminopentyl, dimethylaminomethyl, 2-dimethylaminoethyl, 3-dimethylaminopropyl, 4-dimethylaminobutyl or 5-dimethylaminopentyl, or Q¹ is phenyl, benzyl, 2-phenylethyl, cyclopentyl, cyclohexyl, cyclopentylmethyl, cyclohexylmethyl, thienyl, imidazolyl, thiazolyl, thiadiazolyl, thienylmethyl, imidazolylmethyl, thiazolylmethyl, thiadiazolylmethyl, tetrahydrofuranyl, tetrahydropyranyl, tetrahydrothiopyranyl, pyrrolinyl, pyrrolidinyl, morpholinyl, tetrahydro-1,4-thiazinyl, piperidinyl, homopiperidinyl, piperazinyl, homopiperazinyl, indolinyl, isoindolinyl, pyrrolidinylmethyl, morpholinylmethyl, 2-(morpholinyl)ethyl, piperidinylmethyl, 2-(piperidinyl)ethyl, piperidinyloxymethyl, homopiperidinylmethyl, piperazinylmethyl, 2-(piperazinyl)ethyl, homopiperazinylmethyl or 2-azabicyclo[2.2.1]heptylmethyl,
and wherein any aryl, (3-8C)cycloalkyl, heteroaryl or heterocyclyl group within the Q¹ group optionally bears a substituent selected from fluoro, chloro, trifluoromethyl, hydroxy, amino, methyl, methoxy, methylamino and dimethylamine and any such aryl, (3-8C)cycloalkyl, heteroaryl or heterocyclyl group within the Q¹ group optionally bears a further substituent selected from aminomethyl, methylaminomethyl and dimethylaminomethyl; or a pharmaceutically-acceptable salt, solvate or pro-drug thereof.

3. A pyrimidine derivative of the Formula I according to claim 1 wherein :-
p is 1 and the R¹ group is located at the 3- or 4-position and is selected from hydroxy, carbamoyl, acetamido, hydroxymethyl, 1-hydroxyethyl and 1-hydroxy-1-methylethyl;
R² is hydrogen;
q is 0;
r is 0;
the X¹-Q¹ group is located at the 3-position;
X¹ is NHCO; and
Q¹ is methyl, aminomethyl, 2-aminopropyl, 2-amino-2-methylpropyl, 4-aminobutyl, 5-aminopentyl, methylaminomethyl, dimethylaminomethyl or 5-dimethylaminopentyl, or Q¹ is phenyl, benzyl, 2-phenylethyl, cyclopentyl; cyclohexyl, cyclohexylmethyl, thiazol-5-yl, thien-3-ylmethyl, imidazol-1-ylmethyl, 1,2,4-thiadiazol-3-ylmethyl, tetrahydropyran-4-yl, tetrahydrothiopyran-4-yl, 3-pyrrolin-2-yl, pyrrolidin-2-yl, pyrrolidin-3-yl, morpholin-2-yl, piperidin-2-yl, piperidin-3-yl, piperidin-4-yl, piperazin-1-yl, isoindolin-1-yl, pyrrolidin-2-ylmethyl, piperidin-4-ylmethyl, 2-(piperidin-4-yl)ethyl, piperidin-4-yloxymethyl, piperazin-1-ylmethyl or 2-azabicyclo[2.2.1]hept-2-ylmethyl,
and wherein any aryl, (3-8C)cycloalkyl, heteroaryl or heterocyclyl group within the Q¹ group optionally bears a substituent selected from amino, methyl, methylamino and aminomethyl;
or a pharmaceutically-acceptable salt, solvate or pro-drug thereof.

4. A pyrimidine derivative of the Formula I according to claim 1 wherein :-
p is 1 and the R¹ group is located at the 3- or 4-position and is selected from hydroxy, acetamido, hydroxymethyl, 1-hydroxyethyl and 1-hydroxy-1-methylethyl;
R² is hydrogen;
q is 0;
r is 0 or r is 1 and the R⁴ group is selected from fluoro, chloro and methyl;
the X¹-Q¹ group is located at the 3- or 4-position;
X¹ is NHCO, N(Me)CO, CONH or CON(Me); and
Q¹ is methyl, ethyl, propyl, isopropyl, 2-ethoxyethyl, 3-ethoxypropyl, cyanomethyl, 2-cyanoethyl, aminomethyl, 2-aminoethyl, methylaminomethyl, 2-methylaminoethyl, ethylaminomethyl, 2-ethylaminoethyl, dimethylaminomethyl, 2-dimethylaminoethyl, 4-dimethylaminobutyl, 2-methylsulphonylethyl or acetamidomethyl, or Q¹ is phenyl, benzyl, cyclopropyl, cyclobutyl, cyclopentyl, cyclohexyl, cyclopropylmethyl, cyclobutylmethyl, cyclopentylmethyl, cyclohexylmethyl, oxazol-5-yl, isoxazol-3-yl, isoxazol-4-yl, imidazol-2-yl, imidazol-4-yl, pyrazol-3-yl, thiazol-5-yl, 1,2,3-triazol-5-yl, tetrazol-5-yl, pyridin-2-yl, pyridin-3-yl, pyridin-4-yl, pyrazin-2-yl, pyridazin-4-yl, pyrimidin-2-yl, pyrimidin-4-yl, thien-3-ylmethyl, oxazol-4-ylmethyl, isoxazol-3-ylmethyl, isoxazol-4-ylmethyl, imidazol-1-ylmethyl, imidazol-2-ylmethyl, 2-imidazol-1-ylethyl, 2-imidazol-2-ylethyl, 2-imidazol-4-ylethyl, pyrazol-1-ylmethyl, pyrazol-3-ylmethyl, 1,2,3-triazol-1-ylmethyl, 1,2,3-triazol-4-ylmethyl, 1,2,4-oxadiazol-3-ylmethyl, 1,2,3-thiadiazol-3-ylmethyl, tetrazol-1-ylmethyl, tetrazol-5-ylmethyl, pyridin-2-ylmethyl, pyridin-3-ylmethyl, pyridin-4-ylmethyl, 2-pyridin-2-ylethyl-, 2-pyridin-3-ylethyl, 2-pyridin-4-ylethyl, pyrazin-2-ylmethyl, 2-pyrazin-2-ylethyl, pyridazin-4-ylmethyl, 2-pyridazin-4-ylethyl, pyrimidin-2-ylmethyl, pyrimidin-4-ylmethyl, 2-pyrimidin-2-ylethyl; 2-pytimidin-4-ylethyl, tetrahydrofuran-2-yl, tetrahydropyran-4-yl, tetrahydrothiopyran-4-yl, azetidin-2-yl, 3-pyrrolin-2-yl, pyrrolidin-1-yl, pyrrolidin-2-yl, pyaolidin-3-yl, morpholino, morpholin-2-yl, piperidino, piperidin-2-yl, piperidin-3-yl, piperidin-4-yl, piperazin-1-yl, isoindolin-1-yl, tetrahydrofuran-2-ylmethyl, tetrahydropyran-4-ylmethyl, 1,3-dioxolan-2-ylmethyl, . 1,4-dioxan-2-ylmethyl, pytrolidin-2-ylmethyl, piperidin-2-ylmethyl, pipendin-3-ylmethyl, piperidin-4-ylmethyl, 2-(piperidin-4-yl)ethyl, piperidin-4-yloxymethyl, piperazin-1-ylmethyl or 2-(piperazin-1-yl)ethyl,
and wherein any CH, CH₂ or CH₃ group within the Q¹ group optionally bears on each said CH, CH₂ or CH₃ group a substituent selected from hydroxy, carbamoyl, methoxycarbonyl, ethoxycarbonyl, *N*-methylcarbamoyl, *N*-ethylcarbamoyl, *N*-isopropylcarbamoyl, *N,N-*dimethylcarbamoyl, acetyl, propionyl, pivaloyl, acetamido and *N-*methylacetamido,
and wherein any aryl, (3-8C)cycloalkyl, heteroaryl or heterocyclyl group within the Q¹ group optionally bears 1 or 2 substituents, which may be the same or different, selected from fluoro, chloro, hydroxy, amino, carbamoyl, methyl, methylamino, dimethylamino, hydroxymethyl, methoxymethyl, cyanomethyl, aminomethyl, methylaminomethyl, dimethylaminomethyl and 1-methylpiperidin-4-ylmethyl;
or a pharmaceutically-acceptable salt, solvate or pro-drug thereof.

5. A pyrimidine derivative of the Formula I according to claim 1 wherein :-
p is 1 and R¹ is a hydroxy or hydroxymethyl group that is located at the 3-position;
R² is hydrogen;
q is 0;
r is 0 or r is 1 and the R⁴ group is selected from fluoro and methyl;
the X¹-Q¹ group is located at the 3- or 4-position;
X¹ is NHCO or N(Me)CO; and
Q¹ is aminomethyl, methylaminomethyl, ethylaminomethyl, dimethylaminomethyl, acetamidomethyl, 3-aminomethylphenyl, 4-aminomethylphenyl, 5-methylisoxazol-3-yl, 1-methylpyrazol-3-yl, 1*H*-1,2,3-triazol-5-yl, pyridin-4-yl, pyrazin-2-yl, 2-imidazol-1-ylethyl, 2-imidazol-2-ylethyl, 3,5-dimethyl-1*H*-pyrazol-1-ylmethyl, 1*H*-tetrazol-5-ylmethyl, 2-pyridin-3-ylethyl, 2-pyridazin-4-ylethyl, azetidin-2-yl, 3-pyrrolin-2-yl, *N*-methylpyrrolidin-2-yl, 4-hydroxypyrrolidin-2-yl, piperidin-3-yl, piperidin-4-yl, *N*-methylpiperidin-4-yl, piperazin-1-yl, piperidin-3-ylmethyl, piperidin-4-yloxymethyl or piperazin-1-ylmethyl;
or a pharmaceutically-acceptable salt, solvate or pro-drug thereof.

6. A pyrimidine derivative of the Formula I according to claim 1 wherein :-
p is 1 and R¹ is a hydroxy or hydroxymethyl group that is located at the 3-position;
R² is hydrogen;
q is 0;
r is 0 or r is 1 and the R⁴ group is selected from fluoro and methyl;
the X¹-Q¹ group is located at the 3- or 4-position;
X¹ is CONH or CON(Me); and
Q¹ is methyl, ethyl, propyl, isopropyl, 2-ethoxyethyl, 3-ethoxypropyl, cyanomethyl, 1-cyano-1-methylethyl, 2-cyanoethyl, 5-cyanopentyl, 2-aminoethyl, 2-methylaminoethyl, 2-dimethylaminoethyl, 4-dimethylaminobutyl, 2-methylsulphonylethyl, 3-methoxycarbonylpropyl, carbamoylmethyl, 1-carbamoylethyl, 2-carbamoylethyl, *N*-methylcarbamoylmethyl, *N*-isopropylcarbamoylmethyl, *N,N*-dimethylcarbamoylmethyl, pivaloylmethyl, 4-aminomethylphenyl, 4-aminobenzyl, cyclopropyl, cyclobutyl, cyclopentyl, cyclopropylmethyl, cyclobutylmethyl, cyclopentylmethyl, thien-3-ylmethyl, oxazol-4-ylmethyl, 5-methylisoxazol-3-ylmethyl, isoxazol-4-ylmethyl, 1*H* imidazol-1-ylmethyl, 1*H*-imidazol-2-ylmethyl, 2-(1*H*-imidazol-1-yl)ethyl, 2-(1*H*-imidazol-2-yl)ethyl, 2-(1*H*-imidazol-4-yl)ethyl, pyridin-2-ylmethyl, pyridin-3-ylmethyl, pyridin-4-ylmethyl, 2-pyridin-2-ylethyl, 2-pyridin-3-ylethyl, 2-pyridin-4-ylethyl, pyrazin-2-ylmethyl, 5-methylpyrazin-2-ylmethyl, tetrahydropyran-4-yl, tetrahydrothiopyran-4-yl, tetrahydrofuran-2-ylmethyl, tetrahydropyran-4-ylmethyl, 1,3-dioxolan-2-ylmethyl or 1,4-dioxan-2-ylmethyl;
or a pharmaceutically-acceptable salt, solvate or pro-drug thereof.

7. A pyrimidine derivative of the Formula I according to claim 1 wherein :-
p is 1 and R¹ is a hydroxy or hydroxymethyl group that is located at the 3-position;
R² is hydrogen;
q is 0;
r is 0;
the X¹-Q¹ group is located at the 3-position;
X¹ is NHCO; and
Q¹ is 2-amino-2-methylpropyl, 5-aminopentyl, 3-aminomethylphenyl, 4-aminomethylphenyl, 2-aminocyclopent-1-yl, 4-aminocyclohex-1-yl, 3-aminocyclohex-1-ylmethyl, piperidin-3-yl, piperidin-4-yl, piperidin-4-ylmethyl or piperidin-4-yloxymethyl;
or a pharmaceutically-acceptable salt, solvate or pro-drug thereof.

8. A pyrimidine derivative of the Formula I according to claim 1 selected from :-
4-(3-hydroxyphenyl)-6-morpholino-2-(3-piperidin-4-ylcarbonylaminophenyl)pyrimidine, 2-[3-(6-dimethylaminohexanoylamino)phenyl]-4-(3-hydroxymethylphenyl)-6-morpholinopyrimidine,
2-(3-{2-[(1R,3S)-3-aminocyclohex-1-yl]acetamido]phenyl}-4-(3-hydroxymethylphenyl)-6-morpholinopyimidine,
4-(3-hydroxymethylphenyl)-6-morpholino-2-[3-(2-piperidin-4-yloxyacetamido)phenyl]-pyrimidine,
2-[3-(3-aminomethylbenzamido)phenyl]-4-(3-hydroxymethylphenyl)-6-morpholinopyrimidine, 4-(3-hydroxymethylphenyl)-6-morpholino-2-(3-piperidin-4-ylcarbonylaminophenyl)pyrimidine and 4-(3-hydroxymethylphenyl)-6-morpholino-2-(3-piperazin-1-ylcarbonylaminophenyl)-pyrimidine;
or a pharmaceutically-acceptable salt, solvate or pro-drug thereof.

9. A process for the preparation of a pyrimidine derivative of the Formula I, or a pharmaceutically-acceptable salt, solvate or pro-drug thereof, according to claim 1 which comprises :-
(a) the reaction of a pyrimidine of the Formula II 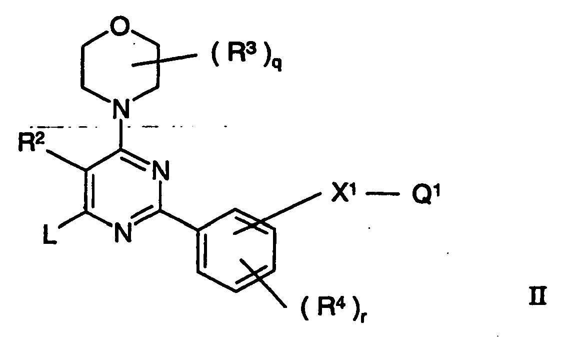 wherein L is a displaceable group and R², q, R³, r, R⁴, X¹ and Q¹ have any of the meanings defined in claim 1 except that any functional group is protected if necessary, with an organoboron reagent of the Formula III 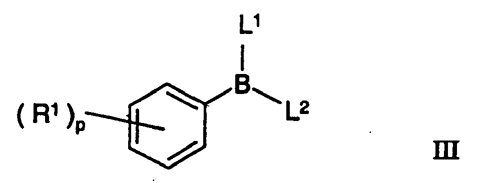 wherein each of L¹ and L², which may be the same or different, is a suitable ligand and p and R¹ have any of the meanings defined in claim 1 except that any functional group is protected if necessary, whereafter any protecting group that is present is removed;
(b) for the production of those compounds of the Formula I wherein X¹ is N(R¹³)CO, the acylation of an amine of the Formula IV 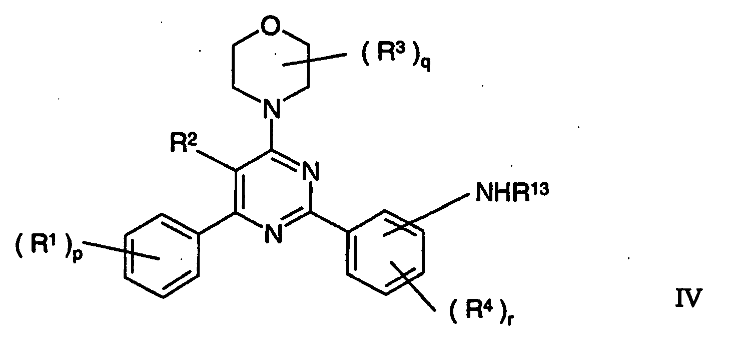 wherein p, R¹, R², q, R³, r, R⁴ and R¹³ have any of the meanings defined in claim 1 except that any functional group is protected if necessary, with a carboxylic acid of the Formula V
HO₂C -Q¹ V
or a reactive derivative thereof, wherein Q¹ has any of the meanings defined in claim 1 except that any functional group is protected if necessary, whereafter any protecting group that is present is removed;
(c) the reaction of a pyrimidine of the Formula VI 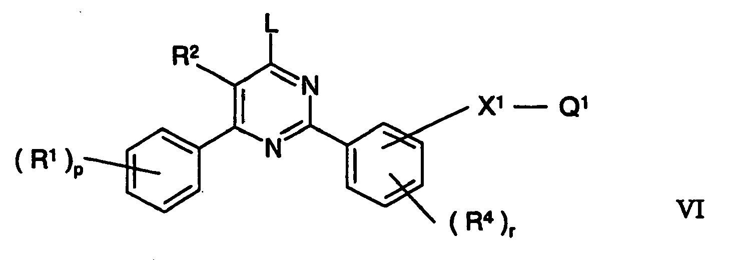 wherein L is a displaceable group and p, R¹, R², r, R⁴, X¹ and Q¹ have any of the meanings defined in claim 1 except that any functional group is protected if necessary, with a morpholine of the Formula VII 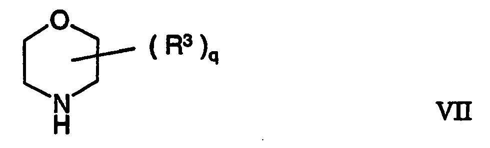 wherein q and R³ have any of the meanings defined in claim 1 except that any functional group is protected if necessary, whereafter any protecting group that is present is removed;
(d) for the production of those compounds of the Formula I wherein X¹ is N(R¹³)CON(R¹³), the coupling of phosgene, or a chemical equivalent thereof, with an amine of the Formula IV 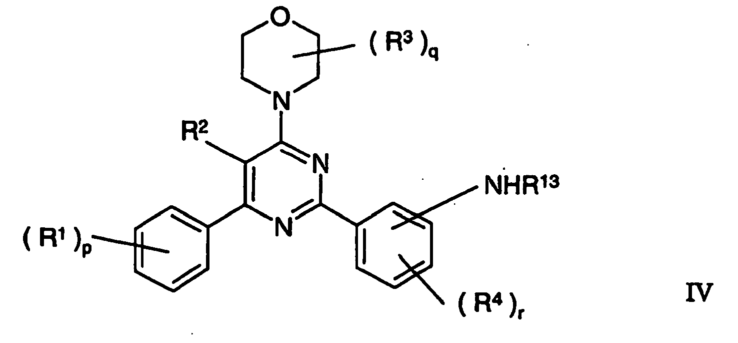 and an amine of the Formula VIII
R¹³NH - Q¹ VIII
wherein p, R¹, R², q, R³, r, R⁴, R¹³ and Q¹ have any of the meanings defined in claim 1 except that any functional group is protected if necessary, whereafter any protecting group that is present is removed;
(e) the reaction of a pyrimidine of the Formula XIV 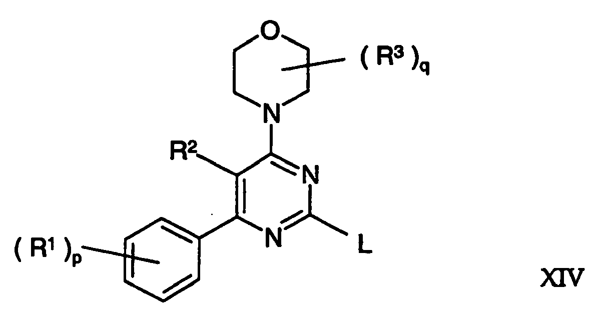 wherein L is a displaceable group and p, R¹, R², q and R³ have any of the meanings defined in claim 1 except that any functional group is protected if necessary, with an organoboron reagent of the Formula XV 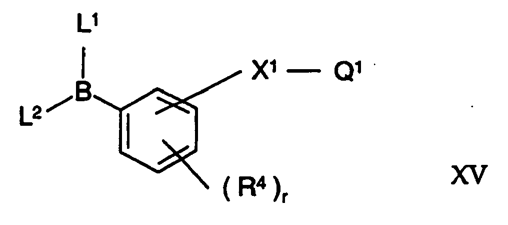 wherein each of L¹ and L², which may be the same or different, is a suitable ligand and r, R⁴, X¹ and Q¹ have any of the meanings defined in claim 1 except that any functional group is protected if necessary, whereafter any protecting group that is present is removed;
(f) For the production of those compounds of the Formula I wherein X¹ is CON(R¹³), the acylation of an amine of the Formula VIII
R¹³NH - Q¹ VIII
wherein R¹³ and Q¹ have any of the meanings defined in claim 1 except that any functional group is protected if necessary, with a carboxylic acid, or a reactive derivative thereof, of the Formula XVI 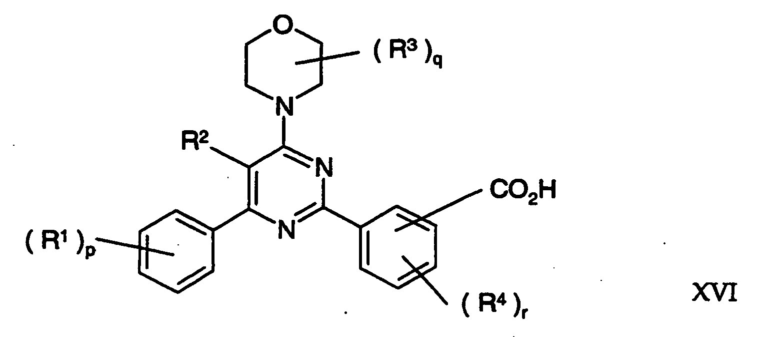 wherein p, R¹, R², q, R³, r and R⁴ have any of the meanings defined in claim 1 except that any functional group is protected if necessary, whereafter any protecting group that is present is removed; or
(g) for the production of those compounds of the Formula I wherein X¹ is CO and Q¹ is a *N*-linked heterocyclyl group, the acylation of a *N*-containing heterocyclic compound wherein any functional group is protected if necessary, with a carboxylic acid, or a reactive derivative thereof of the Formula XVI 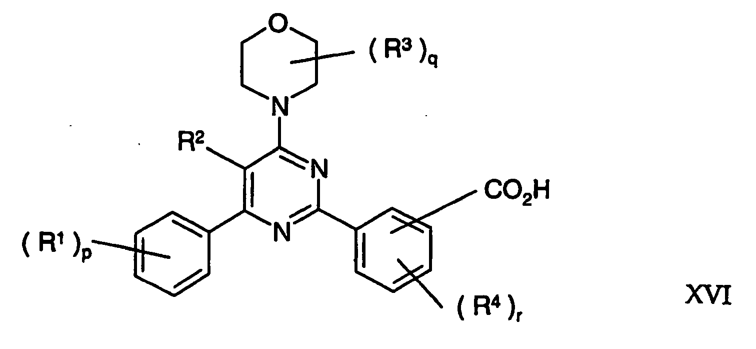
wherein p, R¹, R², q, R³, r and R⁴ have any of the meanings defined in claim 1 except that any functional group is protected if necessary, whereafter any protecting group that is present is removed;
and when a pharmaceutically-acceptable salt of a pyrimidine derivative of the Formula I is required it may be obtained by reaction of said pyrimidine derivative with a suitable acid;
and when a pharmaceutically acceptable pro-drug of a pyrimidine derivative of the Formula I is required, it may be obtained using a conventional procedure.

10. A pharmaceutical composition which comprises a pyrimidine derivative of the Formula I, or a pharmaceutically-acceptable salt, solvate or pro-drug thereof, according to claim 1 in association with a pharmaceutically-acceptable diluent or carrier.

11. The use of a pyrimidine derivative of the Formula I, or a pharmaceutically-acceptable salt, solvate or pro-drug thereof, according to claim 1 in the manufacture of a medicament for use in the production of an anti-proliferative effect in a warm-blooded animal such as man.

## Patentansprüche

1. Pyrimidinderivat der Formel I 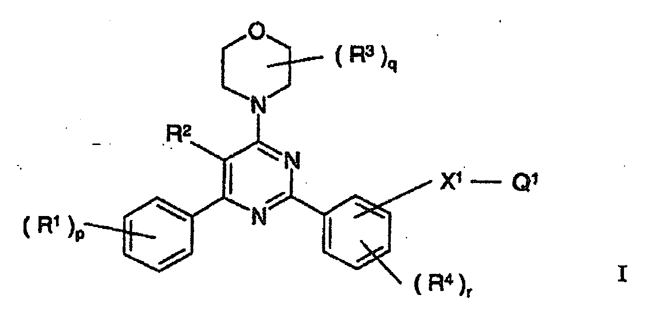 worin **p** für 1, 2 oder 3 steht;
**jede Gruppe R¹**, die gleich oder verschieden sein kann, unter Halogen, Trifluormethyl, Cyano, Isocyano, Nitro, Hydroxy, Mercapto, Amino, Formyl, Carboxy, Carbamoyl, Ureido, C₁₋₈-Alkyl, C₂₋₈-Alkenyl, C₂₋₈-Alkinyl, C₁₋₆-Alkoxy, C₂₋₆-Alkenyloxy, C₂₋₆-Alkinyloxy, C₁₋₆-Alkylthio, C₁₋₆-Alkylsulfinyl, C₁₋₆-Alkylsulfonyl, C₁₋₆-Alkylamino, Di-(C₁₋₆-alkyl)-amino, C₁₋₆-Alkoxycarbonyl, *N*-C₁₋₆-Alkylcarbamoyl, *N*,*N*-Di-(C₁₋₆-alkyl) carbamoyl, C₂₋₆-Alkanoyl, C₂₋₆-Alkanoyloxy, C₂₋₆-Alkanoylamino, *N*-C₁₋₆-Alkyl-C₂₋₆-alkanoylamino, C₃₋₆-Alkenoylamino, *N*-C₁₋₆-Alkyl-C₃-₆-alkenoylamino, C₃₋₆-Alkinoylamino, *N*-C₁₋₆-Alkyl-C₃₋₆-alkinoylamino, *N*'-C₁₋₆-Alkylureido, *N',N'*-Di-(C₁₋₆-alkyl)ureido, *N*-C₁₋₆-Alkylureido, *N,N'-Di-* (C₁₋₆-alkyl) ureido, *N,N',N'*-Tri-(C₁₋₆-alkyl) ureido, *N-*C₁₋₆-Alkylsulfamoyl, *N,N*-Di-(C₁₋₆-alkyl) sulfamoyl, C₁₋₆-Alkansulfonylamino und *N*-C₁₋₆-Alkyl-C₁₋₆-alkansulfonylamino oder einer Gruppe der Formel:
Q²-X²-
worin X² für eine direkte Bindung steht oder unter O, S, SO, SO₂, N(R⁵), CO, CH(OR⁵), CON(R⁵), N (R⁵) CO, N(R⁵)CON(R⁵), SO₂N(R⁵), N(R⁵)SO₂, OC(R⁵)₂, SC(R⁵)₂ und N (R⁵) C (R⁵)₂, worin R⁵ Wasserstoff oder C₁₋₈-Alkyl bedeutet, ausgewählt ist und Q² für Aryl, Aryl-C₁₋₆-alkyl, C₃₋₈-Cycloalkyl, C₃₋₈-Cycloalkyl-C₁₋₆-alkyl, C₃₋₈-Cycloalkenyl, C₃₋₈-Cycloalkenyl-C₁₋₆-alkyl, Heteroaryl, Heteroaryl-C₁₋₆-alkyl, Heterocyclyl oder Heterocyclyl-C₁₋₆-alkyl steht, ausgewählt ist oder (R¹)ₚ für C₁₋₃-Alkylendioxy steht, und worin jede CH-, CH₂- oder CH₃-Gruppe in einem Substituenten R¹ an jeder CH-, CH₂- oder CH₃-Gruppe gegebenenfalls einen oder mehrere Halogen- oder C₁₋₈-Alkylsubstituenten und/oder einen unter Hydroxy, Mercapto, Amino, Cyano, Carboxy, Carbamoyl, Ureido, C₁₋₆-Alkoxy, C₁₋₆-Alkylthio, C₁₋₆-Alkylsulfinyl, C₁₋₆-Alkylsulfonyl, C₁₋₆-Alkylamino, Di- (C₁₋₆-alkyl) amino, C₁₋₆-Alkoxycarbonyl, *N*-C₁₋₆-Alkylcarbamoyl, *N,N*-Di-(C₁₋₆-Alkyl)carbamoyl, C₂₋₆-Alkanoyl, C₂₋₆-Alkanoyloxy, C₂₋₆-Alkanoylamino, *N-*C₁₋₆-Alkyl-C₂₋₆-alkanoylamino, *N*-C₁₋₆-Alkylureido, *N*'-C₁₋₆-Alkylureido, *N',N*'-Di- (C₁₋₆-alkyl) ureido, *N*,*N'*-Di- (C₁₋₆-alkyl) ureido, *N,N',N'-Tri-* (C₁₋₆-alkyl) ureido, *N*-C₁₋₆-Alkylsulfamoyl, N,N-Di- (C₁₋₆-alkyl)sulfamoyl, C₁₋₆-Alkansulfonylamino und *N*-C₁₋₆-Alkyl-C₁₋₆-alkansulfonylamino oder einer Gruppe der Formel:
-X³-Q³
worin X³ für eine direkte Bindung steht oder unter O, S, SO, SO₂, N(R⁶), CO, CH(OR⁶), CON(R⁶), N(R⁶) CO, N(R⁶)CON(R⁶), SO₂N (R⁶), N(R⁶)SO₂, C(R⁶)₂O, C(R⁶)₂S und C(R⁶)₂N(R⁶), worin R⁶ Wasserstoff oder C₁₋₈-Alkyl bedeutet, ausgewählt ist und Q³ für Aryl, Aryl-C₁₋₆-alkyl, C₃₋₈-Cycloalkyl, C₃₋₈-Cycloalkyl-C₁₋₆-alkyl, C₃₋₈-Cycloalkenyl, C₃₋₈-Cycloalkenyl-C₁₋₆-alkyl, Heteroaryl, Heteroaryl-C₁₋₆-alkyl, Heterocyclyl oder Heterocyclyl-C₁₋₆-alkyl steht, ausgewählten Substituenten trägt,
und worin jede Aryl-, C₃₋₈-Cycloalkyl-, C₃₋₈-Cycloalkenyl-, Heteroaryl- oder Heterocyclylgruppe in einem Substituenten an R¹ gegebenenfalls 1, 2 oder 3 Substituenten trägt, die gleich oder verschieden sein können und unter Halogen, Trifluormethyl, Cyano, Nitro, Hydroxy, Amino, Carboxy, Carbamoyl, Ureido, C₁₋₈-Alkyl, C₂₋₈-Alkenyl, C₂₋₈-Alkinyl, C₁₋₆-Alkoxy, C₂₋₆-Alkenyloxy, C₂₋₆-Alkinyloxy, C₁₋₆-Alkylthio, C₁₋₆-Alkylsulfinyl, C₁₋₆-Alkylsulfonyl, C₁₋₆-Alkylamino, Di-(C₁₋₆-alkyl) amino, C₁₋₆-Alkoxycarbonyl, C₂₋₆-Alkanoyl, C₂₋₆-Alkanoyloxy, *N*-C₁₋₆-Alkylcarbamoyl, *N*,*N*-Di-(C₁₋₆-alkyl) carbamoyl, C₂₋₆-Alkanoylamino, *N*-C₁₋₆-Alkyl-C₂₋₆-alkanoylamino, N-C₁₋₆-Alkylureido, *N*'-C₁₋₆-Alkylureido, *N',N*'-Di-(C₁₋₆-alkyl) ureido, *N,N*'-Di-(C₁₋₆-alkyl) ureido, *N,N',N'*-Tri-(C₁₋₆-alkyl) ureido, *N*-C₁₋₆-Alkylsulfamoyl, *N,N-*Di-(C₁₋₆-alkyl)sulfamoyl, C₁₋₆-Alkansulfonylamino und *N*-C₁₋₆-Alkyl-C₁₋₆-alkansulfonylamino oder einer Gruppe der Formel:
-X⁴-R⁷
worin X⁴ für eine direkte Bindung steht oder unter O und N (R⁸), worin R⁸ Wasserstoff oder C₁₋₈-Alkyl bedeutet, ausgewählt ist und R⁷ für Halogen-C₁₋₆-alkyl, Hydroxy-C₁₋₆-alkyl, Mercapto-C₁₋₆-alkyl, C₁₋₆-Alkoxy-C₁₋₆-alkyl, C₁₋₆-Alkyl thio-C₁₋₆-alkyl, Cyano-C₁₋₆-alkyl, Amino-C₁₋₆-alkyl, C₁₋₆-Alkylamino-C₁₋₆-alkyl, Di- (C₁₋₆-alkyl) amino-C₁₋₆-alkyl, C₂₋₆-Alkanoylamino-C₁₋₆-alkyl, C₁₋₆-Alkoxycarbonylamino-C₁₋₆-alkyl, *N*-C₁₋₆-Alkylureido-C₁₋₆-alkyl, *N*'-C₁₋₆-Alkylureido-C₁₋₆-alkyl, *N',N'-*Di*-* (C₁₋₆-alkyl) ureido-C₁₋₆-alkyl, *N*,*N'*-Di-(C₁₋₆-alkyl) ureido-C₁₋₆-alkyl oder *N*,*N'*,*N'*-Tri-(C₁₋₆-alkyl) ureido-C₁₋₆-alkyl steht, oder einer Gruppe der Formel:
-X⁵-Q⁴
worin X⁵ für eine direkte Bindung steht oder unter O, CO und N(R⁹), worin R⁹ Wasserstoff oder C₁₋₈-Alkyl bedeutet, ausgewählt ist und Q⁴ für Aryl, Aryl-C₁₋₆-alkyl, Heteroaryl, Heteroaryl-C₁₋₆-alkyl, Heterocyclyl oder Heterocyclyl-C₁₋₆-alkyl, welches gegebenenfalls 1 oder 2 Substituenten, die gleich oder verschieden sein können und unter Halogen, Hydroxy, C₁₋₈-Alkyl und C₁₋₆-Alkoxy ausgewählt sind, trägt, steht, ausgewählt sind,
und worin jede Heterocyclylgruppe in einem Substituenten an R¹ gegebenenfalls 1 oder 2 Oxo-oder Thioxosubstituenten trägt;
und worin benachbarte Kohlenstoffatome in jeder C₂₋₆-Alkylenkette in einem Substituenten R¹ gegebenenfalls durch Einschub einer unter O, S, SO, SO₂, N(R¹⁰), CO, CH(OR¹⁰) , CON(R¹⁰) , N(R¹⁰)CO, N(R¹⁰) CON (R¹⁰), SO₂N (R¹⁰), N (R¹⁰) SO₂, CH=CH und C=C ausgewählten Gruppe in die Kette getrennt sind, wobei R¹⁰ für Wasserstoff oder C₁₋₈-Alkyl steht,
**R²** für Wasserstoff oder C₁₋₈-Alkyl steht;
**q** für 0, 1, 2, 3 oder 4 steht;
**jede Gruppe R³,** die gleich oder verschieden sein kann, für C₁₋₈-Alkyl oder eine Gruppe der Formel:
-X⁶-R¹¹
worin X⁶ für eine direkte Bindung steht oder unter O und N (R¹²), worin R¹² Wasserstoff oder C₁₋₈-Alkyl bedeutet, ausgewählt ist und R¹¹ für Halogen-C₁₋₆-alkyl, Hydroxy-C₁₋₆-alkyl, C₁₋₆-Alkoxy-C₁₋₆-alkyl, Cyano-C₁₋₆-alkyl, Amino-C₁₋₆-alkyl, C₁₋₆ -Alkylamino-C₁₋₆-alkyl, Di-(C₁₋₆-alkyl)amino-C₁₋₆-alkyl oder C₂₋₆-Alkanoylamino-C₁₋₆-alkyl steht, ausgewählt ist;
**r** für 0, 1 oder 2 steht;
**jede Gruppe R⁴**, die gleich oder verschieden sein kann, unter Halogen, Trifluormethyl, Cyano, Nitro, Hydroxy, Mercapto, Amino, Carboxy, Carbamoyl, Ureido, C₁₋₈-Alkyl, C₂₋₈-Alkenyl, C₂₋₈-Alkinyl, C₁₋₆-Alkoxy, C₁₋₆-Alkylthio, C₁₋₆-Alkylsulfinyl, C₁₋₆-Alkylsulfonyl, C₁₋₆-Alkylamino, Di- (C₁₋₆-alkyl)-amino, C₁₋₆-Alkoxycarbonyl, *N*-C₁₋₆-Alkylcarbamoyl, *N*,*N*-Di-(C₁₋₆-alkyl) carbamoyl, C₂₋₆-Alkanoyl, C₂₋₆-Alkanoyloxy, C₂₋₆-Alkanoylamino, N-C₁₋₆-Alkyl-C₂₋₆-alkanoylamino, *N*'-C₁₋₆-Alkylureido, *N',N'-*Di*-* (C₁₋₆-alkyl) ureido, *N*-C₁₋₆-Alkylureido, *N,N'*-Di- (C₁₋₆-alkyl) ureido, *N,N',N* '-Tri-(C₁₋₆-alkyl) ureido, *N-*C₁₋₆-Alkylsulfamoyl, *N*,*N*-Di-(C₁₋₆-alkyl) sulfamoyl, C₁₋₆-Alkansulfonylamino und *N*-C₁₋₆-Alkyl-C₁₋₆-alkansulfonylamino ausgewählt ist;
**X¹** unter CO, N(R¹³)CO, CON(R¹³), N(R¹³) CON(R¹³), N(R¹³) COC(R¹³)₂O, N(R¹³)COC(R¹³)₂S, N(R¹³)COC(R¹³)₂N(R¹³) und N(R¹³)COC(R¹³)₂N(R¹³)CO, worin R¹³ Wasserstoff oder C₁₋₈-Alkyl bedeutet, ausgewählt ist und
**Q¹** für C₁₋₈-Alkyl, C₂₋₈-Alkenyl, C₂₋₈-Alkinyl, Halogen-C₁₋₆-alkyl, Hydroxy-C₁₋₆-alkyl, Mercapto-C₁₋₆-alkyl, C₁₋₆-Alkoxy-C₁₋₆-alkyl, Cyano-C₁₋₆-alkyl, Amino-C₁₋₆-alkyl, C₁₋₆-Alkylamino-C₁₋₆-alkyl, Di-(C₁₋₆-alkyl) amino-C₁₋₆-alkyl, C₁₋₆-Alkylthio-C₁₋₆-alkyl, C₁₋₆-Alkylsulfinyl-C₁₋₆-alkyl, C₁₋₆-Alkylsulfonyl-C₁₋₆-alkyl, C₂₋₆-Alkanoylamino-C₁₋₆-alkyl, *N*-C₁₋₆-Alkyl-C₂₋₆-alkanoylamino-C₁₋₆-alkyl, C₁₋₆-Alkoxycarbonylamino-C₁₋₆-alkyl, *N*-C₁₋₆-Alkyl-ureido-C₁₋₆-alkyl, *N*'-C₁₋₆-Alkylureido-C₁₋₆-alkyl, *N'*,*N'*-Di- (C₁₋₆-alkyl) ureido-C₁₋₆-alkyl, N,N'-Di-(C₁₋₆-alkyl) ureido-C₁₋₆-alkyl, *N*,*N'*,*N*-Tri-(C₁₋₆-alkyl) ureido-C₁₋₆-alkyl, C₁₋₆-Alkansulfonylamino-C₁₋₆-alkyl oder *N*-C₁₋₆-Alkyl-C₁₋₆-alkansulfonylamino-C₁₋₆-alkyl steht,
oder **Q¹** für Aryl, Aryl-C₁₋₆-alkyl, C₃₋₈-Cycloalkyl, C₃₋₈-Cycloalkyl-C₁₋₆-alkyl, C₃₋₈-Cycloalkenyl, C₃₋₈-Cycloalkenyl-C₁₋₆-alkyl, Heteroaryl, Heteroaryl-C₁₋₆-alkyl, Heterocyclyl oder Heterocyclyl-C₁₋₆-alkyl steht,
und worin jede CH-, CH₂- oder CH₃-Gruppe in der Gruppe Q¹ an jeder CH-, CH₂- oder CH₃-Gruppe gegebenenfalls einen oder mehrere Halogen- oder C₁₋₈-Alkylsubstituenten und/oder einen unter Hydroxy, Mercapto, Amino , Cyano, Carboxy, Carbamoyl, Ureido, C₁₋₆-Alkoxy, C₁₋₆-Alkylthio, C₁₋₆-Alkylsulfinyl, C₁₋₆-Alkylsulfonyl, C₁₋₆-Alkylamino, Di- (C₁₋₆-alkyl) amino, C₁₋₆-Alkoxycarbonyl, *N*-C₁₋₆-Alkylcarbamoyl, *N,N*-Di-(C₁₋₆-Alkyl) carbamoyl, C₂₋₆-Alkanoyl, C₂₋₆-Alkanoyloxy, C₂₋₆-Alkanoylamino, *N-*C₁₋₆-Alkyl-C₂₋₆-alkanoylamino, *N'* -C₁₋₆-Alkylureido, *N*',*N*'-Di-(C₁₋₆-alkyl) ureido, *N*-C₁₋₆-Alkylureido, *N*,*N'*-Di-(C₁₋₆-alkyl) ureido, *N*,*N',N'*-Tri-(C₁₋₆-alkyl)ureido, *N*-C₁₋₆-Alkylsulfamoyl, *N,N-Di-*(C₁₋₆-alkyl)sulfamoyl, C₁₋₆-Alkansulfonylamino und *N*-C₁₋₆-Alkyl-C₁₋₆-alkansulfonylamino ausgewählten Substituenten trägt,
und worin jede Aryl-, C₃₋₈-Cycloalkyl-, C₃₋₈-Cycloalkenyl-, Heteroaryl- oder Heterocyclylgruppe in der Gruppe Q¹ gegebenenfalls 1, 2 oder 3 Substituenten trägt, die gleich oder verschieden sein können und unter Halogen, Trifluormethyl, Cyano, Nitro, Hydroxy, Amino, Carboxy, Carbamoyl, Ureido, C₁₋₈-Alkyl, C₂₋₈-Alkenyl, C₂₋₈-Alkinyl, C₁₋₆-Alkoxy, C₂₋₆-Alkenyloxy, C₂₋₆-Alkinyloxy, C₁₋₆-Alkylthio, C₁₋₆-Alkylsulfinyl, C₁₋₆-Alkylsulfonyl, C₁₋₆-Alkylamino, Di- (C₁₋₆-alkyl) amino, C₁₋₆-Alkoxycarbonyl, C₂₋₆-Alkanoyl, C₂₋₆-Alkanoyloxy, *N*-C₁₋₆-Alkylcarbamoyl, *N,N-*Di*-*(C₁₋₆-alkyl) carbamoyl, C₂₋₆-Alkanoylamino, *N*-C₁₋₆-Alkyl-C₂₋₆-alkanoylamino, *N*'-C₁₋₆-Alkylureido, *N*',*N*'-Di-(C₁₋₆-alkyl)ureido, *N-*C₁₋₆-Alkylureido, *N,N'*-Di- (C₁₋₆-alkyl) ureido, *N,N',N'-*Tri*-* (C₁₋₆-alkyl) ureido, *N*-C₁₋₆-Alkylsulfamoyl, *N,N-*Di*-* (C₁₋₆-alkyl) sulfamoyl, C₁₋₆-Alkansulfonylamino und *N*-C₁₋₆-Alkyl-C₁₋₆-alkansulfonylamino oder einer Gruppe der Formel:
-X⁷-R¹⁴
worin X⁷ für eine direkte Bindung steht oder unter O und N (R¹⁵), worin R¹⁵ Wasserstoff oder C₁₋₈-Alkyl bedeutet, ausgewählt ist und R¹⁴ für Halogen-C₁₋₆-alkyl, Hydroxy-C₁₋₆-alkyl, C₁₋₆-Alkoxy-C₁₋₆-alkyl, Cyano-C₁₋₆-alkyl, Amino-C₁₋₆-alkyl, C₁₋₆-Alkylamino-C₁₋₆-alkyl oder Di- (C₁₋₆-alkyl) amino-C₁₋₆-alkyl steht, oder einer Gruppe der Formel:
-X⁸-Q⁵
worin X⁸ für eine direkte Bindung steht oder unter O, CO und N (R¹⁷), worin R¹⁷ Wasserstoff oder C₁₋₈-Alkyl bedeutet, ausgewählt ist und Q⁵ für Aryl, Aryl-C₁₋₆-alkyl, Heteroaryl, Heteroaryl-C₁₋₆-alkyl, Heterocyclyl oder Heterocyclyl-C₁₋₆-alkyl, welches gegebenenfalls 1 oder 2 Substituenten, die gleich oder verschieden sein können und unter Halogen, Hydroxy, C₁₋₈-Alkyl und C₁₋₆-Alkoxy ausgewählt sind, trägt, steht, ausgewählt sind,
und worin jede Heterocyclylgruppe in der Gruppe Q¹ gegebenenfalls 1 oder 2 Oxo- oder Thioxosubstituenten trägt;
und worin benachbarte Kohlenstoffatome in jeder C₂₋₆-Alkylenkette in der Gruppe Q¹ gegebenenfalls durch Einschub einer unter O, S, SO, SO₂, N (R¹⁶), N(R¹⁶)CO, CON (R¹⁶), N (R¹⁶) CON (R¹⁶), CO, CH (OR¹⁶), N(R¹⁶)SO₂, SO₂N (R¹⁶), CH=CH und C=C ausgewählten Gruppe in die Kette getrennt sind, wobei R¹⁶ für Wasserstoff oder C₁₋₈-Alkyl steht;
oder ein pharmazeutisch annehmbares Salz, Solvat oder Prodrug davon.

2. Pyrimidinderivat der Formel I nach Anspruch 1,
worin:
p für 1 oder 2 steht und eine erste Gruppe R¹ unter Hydroxy, Carbamoyl, Acetamido, Propionamido, N-Methylacetamido, N-Methylpropionamido, Hydroxymethyl, 1-Hydroxyethyl und 1-Hydroxy-1-methylethyl ausgewählt ist und die fakultative zweite Gruppe R¹ unter Fluor, Chlor, Trifluormethyl, Cyano, Hydroxy, Methyl, Ethyl, Methoxy und Ethoxy ausgewählt ist;
R² für Wasserstoff oder Methyl steht;
q für 0 steht oder q für 1 steht und die Gruppe R³ für Methyl steht;
r für 0 steht oder r für 1 steht und die Gruppe R⁴ unter Fluor, Chlor, Trifluormethyl, Hydroxy, Amino, Methyl, Methoxy, Methylamino und Dimethylamino ausgewählt ist;
die Gruppe X¹-Q¹ in 3-Position steht;
X¹ unter NHCO, NHCONH, NHCOCH₂O, NHCOCH₂NH und NHCOCH₂NHCO ausgewählt ist und
Q¹ für Methyl, Ethyl, Propyl, Butyl, Pentyl, Aminomethyl, 2-Aminoethyl, 2-Amino-2-methylpropyl, 3-Aminopropyl, 4-Aminobutyl, 5-Aminopentyl, Methylaminomethyl, 2-Methylaminoethyl, 3-Methylaminopropyl, 4-Methylaminobutyl, 5-Methylaminopentyl, Dimethylaminomethyl, 2-Dimethylaminoethyl, 3-Dimethylaminopropyl, 4-Dimethylaminobutyl oder 5-Dimethylaminopentyl steht oder Q¹ für Phenyl, Benzyl, 2-Phenylethyl, Cyclopentyl, Cyclohexyl, Cyclopentylmethyl, Cyclohexylmethyl, Thienyl, Imidazolyl, Thiazolyl, Thiadiazolyl, Thienylmethyl, Imidazolylmethyl, Thiazolylmethyl, Thiadiazolylmethyl, Tetrahydrofuranyl, Tetrahydropyranyl, Tetrahydrothiopyranyl, Pyrrolinyl, Pyrrolidinyl, Morpholinyl, Tetrahydro-1,4-thiazinyl, Piperidinyl, Homopiperidinyl, Piperazinyl, Homopiperazinyl, Indolinyl, Isoindolinyl, Pyrrolidinylmethyl, Morpholinylmethyl, 2-(Morpholinyl)ethyl, Piperidinylmethyl, 2-(Piperidinyl)ethyl, Piperidinyloxymethyl, Homopiperidinylmethyl, Piperazinylmethyl, 2-(Piperazinyl)ethyl, Homopiperazinylmethyl oder 2-Azabicyclo[2.2.1]heptylmethyl steht,
und worin jede Aryl-, C₃₋₈-Cycloalkyl-, Heteroaryl-oder Heterocyclylgruppe in der Gruppe Q¹ gegebenenfalls einen Substituenten trägt, der unter Fluor, Chlor, Trifluormethyl, Hydroxy, Amino, Methyl, Methoxy, Methylamino und Dimethylamino ausgewählt ist und jede derartige Aryl-, C₃₋₈-Cycloalkyl-, Heteroaryl- oder Heterocyclylgruppe in der Gruppe Q¹ gegebenenfalls einen weiteren Substituenten trägt, der unter Aminomethyl, Methylaminomethyl und Dimethylaminomethyl ausgewählt ist;
oder ein pharmazeutisch annehmbares Salz, Solvat oder Prodrug davon.

3. Pyrimidinderivat der Formel I nach Anspruch 1, worin:
p für 1 steht und die Gruppe R¹ in 3- oder 4-Position steht und unter Hydroxy, Carbamoyl, Acetamido, Hydroxymethyl, 1-Hydroxyethyl und 1-Hydroxy-1-methylethyl ausgewählt ist;
R² für Wasserstoff steht;
q für 0 steht;
r für 0 steht;
die Gruppe X¹-Q¹ in 3-Position steht;
X¹ für NHCO steht und
Q¹ für Methyl, Aminomethyl, 2-Aminopropyl, 2-Amino-2-methylpropyl, 4-Aminobutyl, 5-Aminopentyl, Methylaminomethyl, Dimethylaminomethyl oder 5-Dimethylaminopentyl steht oder Q¹ für Phenyl, Benzyl, 2-Phenylethyl, Cyclopentyl, Cyclohexyl, Cyclohexylmethyl, Thiazol-5-yl, Thien-3-ylmethyl, Imidazol-1-ylmethyl, 1,2,4-Thiadiazolylmethyl, Tetrahydropyran-4-yl, Tetrahydrothiopyran-4-yl, 3-Pyrrolin-2-yl, Pyrrolidin-2-yl, Pyrrolidin-3-yl, Morpholin-2-yl, Piperidin-2-yl, Piperidin-3-yl, Piperidin-4-yl, Piperazin-1-yl, Isoindolin-1-yl, Pyrrolidin-2-ylmethyl, Piperidin-4-ylmethyl, 2-(Piperidin-4-yl)ethyl, Piperidin-4-yloxymethyl, Piperazin-1-ylmethyl oder 2-Azabicyclo[2.2.1]hept-2-ylmethyl steht,
und worin jede Aryl-, C₃₋₈-Cycloalkyl-, Heteroaryl-oder Heterocyclylgruppe in der Gruppe Q¹ gegebenenfalls einen Substituenten trägt, der unter Amino, Methyl, Methylamino und Aminomethyl ausgewählt ist;
oder ein pharmazeutisch annehmbares Salz, Solvat oder Prodrug davon.

4. Pyrimidinderivat der Formel I nach Anspruch 1, worin:
p für 1 steht und die Gruppe R¹ in 3- oder 4-Position steht und unter Hydroxy, Acetamido, Hydroxymethyl, 1-Hydroxyethyl und 1-Hydroxy-1-methylethyl ausgewählt ist;
R² für Wasserstoff steht;
q für 0 steht;
r für 0 steht oder r für 1 steht und die Gruppe R⁴ unter Fluor, Chlor und Methyl ausgewählt ist;
die Gruppe X¹-Q¹ in 3- oder 4-Position steht;
X¹ für NHCO, N (Me) CO, CONH oder CON (Me) steht und
Q¹ für Methyl, Ethyl, Propyl, Isopropyl, 2-Ethoxyethyl, 3-Ethoxypropyl, Cyanomethyl, 2-Cyanoethyl, Aminomethyl, 2-Aminoethyl, Methylaminomethyl, 2-Methylaminoethyl, Ethylaminomethyl, 2-Ethylaminoethyl, Dimethylaminomethyl, 2-Dimethylaminoethyl, 4-Dimethylaminobutyl, 2-Methylsulfonylethyl oder Acetamidomethyl steht oder Q¹ für Phenyl, Benzyl, Cyclopropyl, Cyclobutyl, Cyclopentyl, Cyclohexyl, Cyclopropylmethyl, Cyclobutylmethyl, Cyclopentylmethyl, Cyclohexylmethyl, Oxazol-5-yl, Isoxazol-3-yl, Isoxazol-4-yl, Imidazol-2-yl, Imidazol-4-yl, Pyrazol-3-yl, Thiazol-5-yl, 1,2,3-Triazol-5-yl, Tetrazol-5-yl, Pyridin-2-yl, Pyridin-3-yl, Pyridin-4-yl, Pyrazin-2-yl, Pyridazin-4-yl, Pyrimidin-2-yl, Pyrimidin-4-yl, Thien-3-ylmethyl, Oxazol-4-ylmethyl, Isoxazol-3-ylmethyl, Isoxazol-4-ylmethyl, Imidazol-1-yl-methyl, Imidazol-2-ylmethyl, 2-Imidazol-1-ylethyl, 2-Imidazol-2-ylethyl, 2-Imidazol-4-ylethyl, Pyrazol-1-ylmethyl, Pyrazol-3-ylmethyl, 1,2,3-Triazol-1-ylmethyl, 1,2,3-Triazol-4-ylmethyl, 1,2,4-Oxadiazol-3-ylmethyl, 1,2,4-Thiadiazol-3-yl-methyl, Tetrazol-1-ylmethyl, Tetrazol-5-ylmethyl, Pyridin-2-ylmethyl, Pyridin-3-ylmethyl, Pyridin-4-ylmethyl, 2-Pyridin-2-ylethyl, 2-Pyridin-3-yl-ethyl, 2-Pyridin-4-ylethyl, Pyrazin-2-ylmethyl, 2-Pyrazin-2-ylethyl, Pyridazin-4-ylmethyl, 2-Pyridazin-4-ylethyl, Pyrimidin-2-ylmethyl, Pyrimidin-4-ylmethyl, 2-Pyrimidin-2-ylethyl, 2-Pyrimidin-4-ylethyl, Tetrahydrofuran-2-yl, Tetrahydropyran-4-yl, Tetrahydrothiopyran-4-yl, Azetidin-2-yl, 3-Pyrrolin-2-yl; Pyrrolidin-1-yl, Pyrrolidin-2-yl, Pyrrolidin-3-yl, Morpholino, Morpholin-2-yl, Piperidino, Piperidin-2-yl, Piperidin-3-yl, Piperidin-4-yl, Piperazin-1-yl, Isoindolin-1-yl, Tetrahydrofuran-2-ylmethyl, Tetrahydropyran-4-ylmethyl, 1,3-Dioxolan-2-yl-methyl, 1,4-Dioxan-2-ylmethyl, Pyrrolidin-2-ylmethyl, Piperidin-2-ylmethyl, Piperidin-2-ylmethyl, Piperidin-3-ylmethyl, Piperidin-4-ylmethyl, 2-(Piperidin-4-yl)ethyl, Piperidin-4-yloxymethyl, Piperazin-1-ylmethyl oder 2-(Piperazin-1-yl)ethyl steht,
und worin jede CH-, CH₂- oder CH₃-Gruppe in der Gruppe Q¹ an jeder CH-, CH₂- oder CH₃-Gruppe gegebenenfalls einen unter Hydroxy, Carbamoyl, Methoxycarbonyl, Ethoxycarbonyl, *N*-Methylcarbamoyl, *N*-Ethylcarbamoyl, *N*-Isopropylcarbamoyl, *N*,*N*-Dimethylcarbamoyl, Acetyl, Propionyl, Pivaloyl, Acetamido und *N*-Methylacetamido ausgewählten Substituenten trägt,
und worin jede Aryl-, C₃₋₈-Cycloalkyl-, Heteroaryl-oder Heterocyclylgruppe in der Gruppe Q¹ gegebenenfalls 1 oder 2 Substituenten trägt, die gleich oder verschieden sein können und unter Fluor, Chlor, Hydroxy, Amino, Carbamoyl, Methyl, Methylamino, Dimethylamino, Hydroxymethyl, Methoxymethyl, Cyanomethyl, Aminomethyl, Methylaminomethyl, Dimethylaminomethyl und 1-Methylpiperidin-4-ylmethyl ausgewählt sind;
oder ein pharmazeutisch annehmbares Salz, Solvat oder Prodrug davon.

5. Pyrimidinderivat der Formel I nach Anspruch 1, worin:
p für 1 steht und R¹ für eine Hydroxy- oder Hydroxymethylgruppe in 3-Position steht;
R² für Wasserstoff steht;
q für 0 steht;
r für 0 steht oder r für 1 steht und die Gruppe R⁴ unter Fluor und Methyl ausgewählt ist;
die Gruppe X¹-Q¹ in 3- oder 4-Position steht;
X¹ für NHCO oder N(Me)CO steht und
Q¹ für Aminomethyl, Methylaminomethyl, Ethylaminomethyl, Dimethylaminomethyl, Acetamidomethyl, 3-Aminomethylphenyl, 4-Aminomethylphenyl, 5-Methylisoxazol-3-yl, 1-Methylpyrazol-3-yl, 1*H-*1,2,3-Triazol-5-yl, Pyridin-4-yl, Pyrazin-2-yl, 2-Imidazol-1-ylethyl, 2-Imidazol-2-ylethyl, 3,5-Dimethyl-1*H*-pyrazol-1-ylmethyl, 1*H*-Tetrazol-5-yl-methyl, 2-Pyridin-3-ylethyl, 2-Pyridazin-4-yl-ethyl, Azetidin-2-yl, 3-Pyrrolin-2-yl, *N-*Methylpyrrolidin-2-yl, 4-Hydroxypyrrolidin-2-yl, Piperidin-3-yl, Piperidin-4-yl, *N*-Methylpiperidin-4-yl, Piperazin-1-yl, Piperidin-3-ylmethyl, Piperidin-4-yloxymethyl oder Piperazin-1-ylmethyl steht;
oder ein pharmazeutisch annehmbares Salz, Solvat oder Prodrug davon.

6. Pyrimidinderivat der Formel I nach Anspruch 1, worin:
p für 1 steht und und R¹ für eine Hydroxy- oder Hydroxymethylgruppe in 3-Position steht;
R² für Wasserstoff steht;
q für 0 steht;
r für 0 steht oder r für 1 steht und die Gruppe R⁴ unter Fluor und Methyl ausgewählt ist;
die Gruppe X¹-Q¹ in 3- oder 4-Position steht;
X¹ für CONH oder CON(Me) steht und
Q¹ für Methyl, Ethyl, Propyl, Isopropyl, 2-Ethoxyethyl, 3-Ethoxypropyl, Cyanomethyl, 1-Cyano-1-methylethyl, 2-Cyanoethyl, 5-Cyanopentyl, 2-Aminoethyl, 2-Methylaminoethyl, 2-Dimethylaminoethyl, 4-Dimethylaminobutyl, 2-Methylsulfonylethyl, 3-Methoxycarbonylpropyl, Carbamoylmethyl, 1-Carbamoylethyl, 2-Carbamoylethyl, *N*-Methylcarbamoylmethyl, *N*-Isopropylcarbamoylmethyl, *N,N-*Dimethylcarbamoylmethyl, Pivaloylmethyl, 4-Aminomethylphenyl, 4-Aminobenzyl, Cyclopropyl, Cyclobutyl, Cyclopentyl, Cyclopropylmethyl, Cyclobutylmethyl, Cyclopentylmethyl, Thien-3-ylmethyl, Oxazol-4-ylmethyl, 5-Methylisoxazol-3-ylmethyl, Isoxazol-4-ylmethyl, 1*H*-Imidazol-1-ylmethyl, 1*H-*Imidazol-2-ylmethyl, 2-(1*H*-Imidazol-1-yl)ethyl, 2-(1*H*-Imidazol-2-yl)ethyl, 2-(1*H*-Imidazol-4-yl)-ethyl, Pyridin-2-ylmethyl, Pyridin-3-ylmethyl, Pyridin-4-ylmethyl, 2-Pyridin-2-ylethyl, 2-Pyridin-3-ylethyl, 2-Pyridin-4-ylethyl, Pyrazin-2-ylmethyl, 5-Methylpyrazin-2-ylmethyl, Tetrahydropyran-4-yl, Tetrahydrothiopyran-4-yl, Tetrahydrofuran-2-ylmethyl, Tetrahydropyran-4-ylmethyl, 1,3-Dioxolan-2-ylmethyl oder 1,4-Dioxan-2-ylmethyl steht;
oder ein pharmazeutisch annehmbares Salz, Solvat oder Prodrug davon.

7. Pyrimidinderivat der Formel I nach Anspruch 1, worin:
p für 1 steht und R¹ für eine Hydroxy- oder Hydroxymethylgruppe in 3-Position steht;
R² für Wasserstoff steht;
q für 0 steht;
r für 0 steht;
die Gruppe X¹-Q¹ in 3-Position steht;
X¹ für NHCO steht und
Q¹ für 2-Amino-2-methylpropyl, 5-Aminopentyl, 3-Aminomethylphenyl, 4-Aminomethylphenyl, 2-Aminocyclopent-1-yl, 4-Aminocyclohex-1-yl, 3-Aminocyclohex-1-ylmethyl, Piperidin-3-yl, Piperidin-4-yl, Piperidin-4-ylmethyl oder Piperidin-4-yloxymethyl steht;
oder ein pharmazeutisch annehmbares Salz, Solvat oder Prodrug davon.

8. Pyrimidinderivat der Formel I nach Anspruch 1, ausgewählt unter:
4-(3-Hydroxyphenyl)-6-morpholino-2-(3-piperidin-4-ylcarbonylaminophenyl)pyrimidin,
2-[3-(6-Dimethylaminohexanoylamino)phenyl]-4-(3-hydroxymethylphenyl)-6-morpholinopyrimidin,
2-(3-{2-[(1R,3S)-3-Aminocyclohex-1-yl]acetamido]-phenyl}-4-(3-hydroxymethylphenyl)-6-morpholinopyrimidin,
4-(3-Hydroxymethylphenyl)-6-morpholino-2-[3-(2-piperidin-4-yloxyacetamido)phenyl]pyrimidin,
2-[3-(3-Aminomethylbenzamido)phenyl]-4-(3-hydroxymethylphenyl)-6-morpholinopyrimidin,
4-(3-Hydroxymethylphenyl)-6-morpholino-2-(3-piperidin-4-ylcarbonylaminophenyl)pyrimidin und
4-(3-Hydroxymethylphenyl)-6-morpholino-2-(3-piperazin-1-ylcarbonylaminophenyl)pyrimidin;
oder ein pharmazeutisch annehmbares Salz, Solvat oder Prodrug davon.

9. Verfahren zur Herstellung eines Pyrimidinderivats der Formel I oder eines pharmazeutisch annehmbaren Salzes, Solvats oder Prodrugs davon nach Anspruch 1, bei dem man:
(a) ein Pyrimidin der Formel II 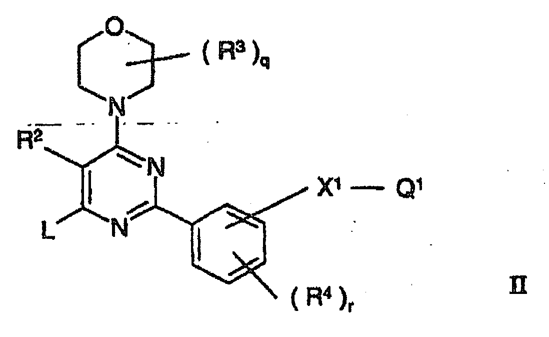 worin L für eine austauschbare Gruppe steht und R², q, R³, r, R⁴, X¹ und Q¹ eine der in Anspruch 1 definierten Bedeutungen besitzen, wobei jedoch jede funktionelle Gruppe gegebenenfalls geschützt ist, mit einem bororganischen Reagenz der Formel III 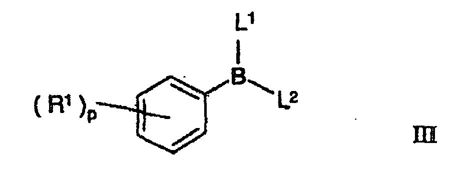 worin L¹ und L² gleich oder verschieden sein können und jeweils für einen geeigneten Liganden stehen und p und R¹ eine der in Anspruch 1 definierten Bedeutungen besitzen, wobei jedoch jede funktionelle Gruppe gegebenenfalls geschützt ist, umsetzt und danach jede vorhandene Schutzgruppe abspaltet;
(b) zur Herstellung derjenigen Verbindungen der Formel I, worin X¹ für N(R¹³)CO steht, ein Amin der Formel IV 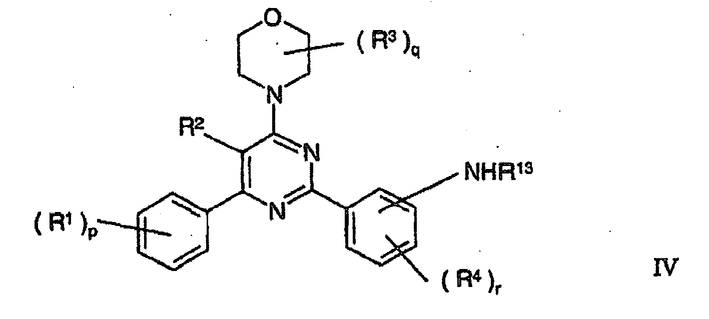 worin p, R¹, R², q, R³ r, R⁴ und R¹³ eine der in Anspruch 1 definierten Bedeutungen besitzen, wobei jedoch jede funktionelle Gruppe gegebenenfalls geschützt ist, mit einer Carbonsäure der Formel V
HO₂C-Q¹ V
oder einem reaktiven Derivat davon, worin Q¹ eine der in Anspruch 1 definierten Bedeutungen besitzt, wobei jedoch jede funktionelle Gruppe gegebenenfalls geschützt ist, acyliert und danach jede vorhandene Schutzgruppe abspaltet;
(c) ein Pyrimidin der Formel VI 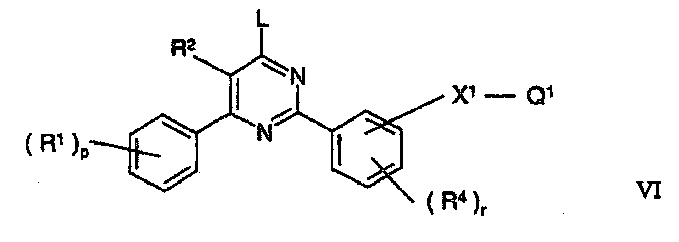 worin L für eine austauschbare Gruppe steht und p, R¹ , R² r, R⁴, X¹ und Q¹ eine der in Anspruch 1 definierten Bedeutungen besitzen, wobei jedoch jede funktionelle Gruppe gegebenenfalls geschützt ist, mit einem Morpholin der Formel VII 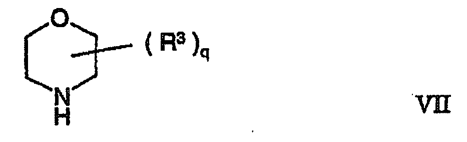 worin q und R³ eine der in Anspruch 1 definierten Bedeutungen besitzen, wobei jedoch jede funktionelle Gruppe gegebenenfalls geschützt ist, umsetzt und danach jede vorhandene Schutzgruppe abspaltet;
(d) zur Herstellung derjenigen Verbindungen der Formel I, worin X¹ für N (R¹³) CON (R¹³) steht, Phosgen oder ein chemisches Äquivalent davon mit einem Amin der Formel IV 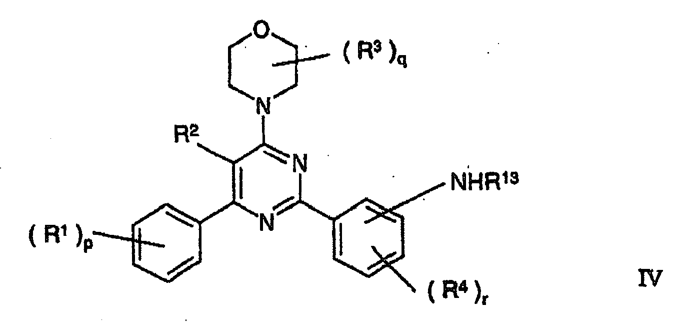 und einem Amin der Formel VIII
R¹³NH-Q¹ VIII
worin p, R¹, R², q, R³, r, R⁴, R¹³ und Q¹ eine der in Anspruch 1 definierten Bedeutungen besitzen, wobei jedoch jede funktionelle Gruppe gegebenenfalls geschützt ist, kuppelt und danach jede vorhandene Schutzgruppe abspaltet;
(e) ein Pyrimidin der Formel XIV 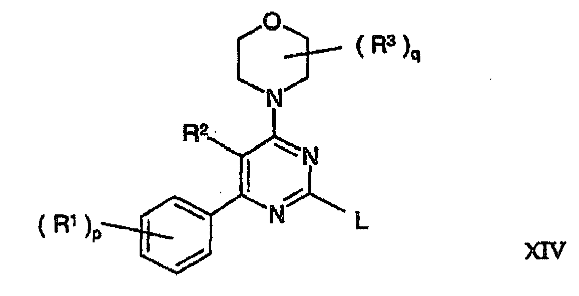 worin L für eine austauschbare Gruppe steht und p, R¹, R², q und R³ eine der in Anspruch 1 definierten Bedeutungen besitzen, wobei jedoch jede funktionelle Gruppe gegebenenfalls geschützt ist, mit einem bororganischen Reagenz der Formel XV 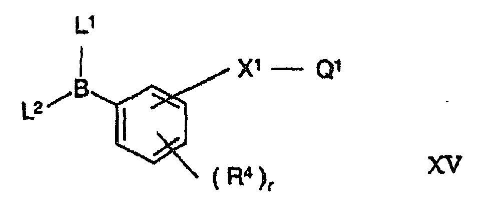 worin L¹ und L² gleich oder verschieden sein können und jeweils für einen geeigneten Liganden stehen und r, R⁴, X¹ und Q¹ eine der in Anspruch 1 definierten Bedeutungen besitzen, wobei jedoch jede funktionelle Gruppe gegebenenfalls geschützt ist, umsetzt und danach jede vorhandene Schutzgruppe abspaltet;
(f) zur Herstellung derjenigen Verbindungen der Formel I, worin X¹ für CON (R¹³) steht, ein Amin der Formel VIII
R¹³NH-Q¹ VIII
worin R¹³ und Q¹ eine der in Anspruch 1 definierten Bedeutungen besitzen, wobei jedoch jede funktionelle Gruppe gegebenenfalls geschützt ist, mit einer Carbonsäure der Formel XVI 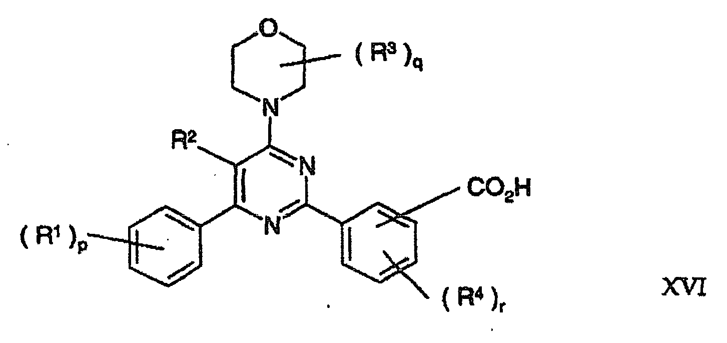 oder einem reaktiven Derivat davon, worin p, R¹, R², q, R³, r und R⁴ eine der in Anspruch 1 definierten Bedeutungen besitzen, wobei jedoch jede funktionelle Gruppe geschützt ist, acyliert und danach jede vorhandene Schutzgruppe abspaltet; oder
(g) zur Herstellung derjenigen Verbindungen der Formel I, worin X¹ für CO steht und Q¹ für eine N-verknüpfte Heterocyclylgruppe steht, eine N-haltige heterocyclsiche Verbindung, worin jede funktionelle Gruppe gegebenenfalls geschützt ist, mit einer Carbonsäure der Formel XVI 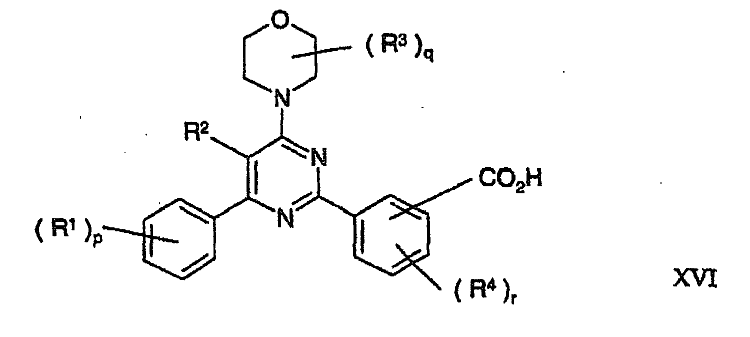 oder einem reaktiven Derivat davon, worin p, R¹, R², q, R³, r und R⁴ eine der in Anspruch 1 definierten Bedeutungen besitzen, wobei jedoch jede funktionelle Gruppe gegebenenfalls geschützt ist, acyliert und danach jede vorhandene Schutzgruppe abspaltet;
und ein gegebenenfalls gewünschtes pharmazeutisch annehmbares Salz eines Pyrimidinderivats der Formel I durch Umsetzung des Pyrimidinderivats mit einer geeigneten Säure erhältlich ist;
und ein gegebenenfalls gewünschtes pharmazeutisch annehmbares Prodrug eines Pyrimidinderivats der Formel I nach einer herkömmlichen Vorgehensweise erhältlich ist.

10. Pharmazeutische Zusammensetzung, die ein Pyrimidinderivat der Formel I oder ein pharmazeutisch annehmbares Salz, Solvat oder Prodrug davon nach Anspruch 1 zusammen mit einem pharmazeutisch annehmbaren Verdünnungsmittel oder Träger enthält.

11. Verwendung eines Pyrimidinderivats der Formel I oder eines pharmazeutisch annehmbaren Salzes, Solvats oder Prodrugs davon nach Anspruch 1 bei der Herstellung eines Arzneimittels zur Verwendung bei der Erzielung einer antiproliferativen Wirkung bei einem Warmblüter wie dem Menschen.

## Revendications

1. Dérivé de pyrimidine de formule I 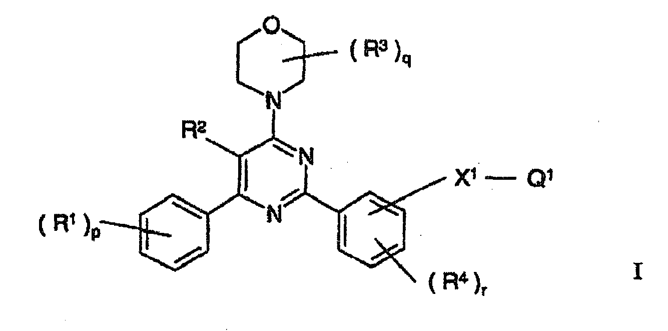 dans laquelle **p** est 1, 2 ou 3 ;
**chaque groupement R¹**, qui peut être identique ou différent, est choisi parmi halogéno, trifluorométhyle, cyano, isocyano, nitro, hydroxy, mercapto, amino, formyle, carboxy, carbamoyle, uréido, (1-8C)alkyle, (2-8C)alcényle, (2-8C)-alcynyle, (1-6C)alcoxy, (2-6C)alcényloxy, (2-6C)-alcynyloxy, (1-6C)alkylthio, (1-6C)alkylsulfinyle, (1-6C)alkylsulfonyle, (1-6C)alkylamino, di-[(1-6C)alkyl]amino, (1-6C)alcoxycarbonyle, *N*-(1-6C)alkylcarbamoyle, *N,N*-di-[(1-6C)alkyl]-carbamoyle, (2-6C)alcanoyle, (2-6C)alcanoyloxy, (2-6C) alcanoylamino, *N*-(1-6C)alkyl-(2-6C)alcanoylamino, (3-6C)alcénoylamino, *N*-(1-6C)alkyl-(3-6C)-alcénoylamino, (3-6C)alcynoylamino, *N*-(1-6C)alkyl-(3-6C)alcynoylamino, *N*'-(1-6C)alkyluréido, *N',N'-*di-[(1-6C)alkyl]uréido, *N*-(1-6C)alkyluréido, *N,N'-*di-[(1-6C)alkyl]uréido, *N*,*N*',*N*'-tri-[(1-6C)alkyl]-uréido, *N*-(1-6C)alkylsulfamoyle, *N*,*N*-di-[(1-6C)-alkyl]sulfamoyle, (1-6C)alcanesulfonylamino et *N*-(1-6C)alkyl-(1-6C)alcanesulfonylamino ou parmi un groupement de formule :
Q²-X²-
dans laquelle X² est une liaison directe ou est choisi parmi O, S, SO, SO₂, N(R⁵), CO, CH(OR⁵) , CON(R⁵), N(R⁵)CO, N(R⁵)CON(R⁵), SO₂N(R⁵), N(R⁵)SO₂, OC(R⁵) ₂, SC(R⁵)₂ et N(R⁵)C(R⁵)₂, où R⁵ est hydrogène ou (1-8C)alkyle, et Q² est aryle, aryl-(1-6C)alkyle, (3-8C)cycloalkyle, (3-8C)cycloalkyl-(1-6C)alkyle, (3-8C)cycloalcényle, (3-8C)cycloalcényl-(1-6C)alkyle, hétéroaryle, hétéroaryl-(1-6C)alkyle, hétérocyclyle ou hétérocyclyl-(1-6C)alkyle, ou (R¹)ₚ est (1-3C) alkylènedioxy ;
et où tout groupement CH, CH₂ ou CH₃ dans un substituant R¹ porte éventuellement, sur chacun desdits groupements CH, CH₂ ou CH₃, un ou plusieurs substituants halogéno ou (1-8C)alkyle et/ou un substituant choisi parmi hydroxy, mercapto, amino, cyano, carboxy, carbamoyle, uréido, (1-6C)alcoxy, (1-6C)alkylthio, (1-6C)alkylsulfinyle, (1-6C)-alkylsulfonyle, (1-6C)alkylamino, di-[(1-6C)-alkyl]amino, (1-6C)alcoxycarbonyle, *N*-(1-6C)alkylcarbamoyle, *N*,*N*-di-[(1-6C)alkyl]carbamoyle, (2-6C)alcanoyle, (2-6C)alcanoyloxy, (2-6C)-alcanoylamino, *N*-(1-6C)alkyl-(2-6C)alcanoylamino, *N*-(1-6C)alkyluréido, *N*'-(1-6C)alkyluréido, *N',N'-*di-[(1-6C)alkyl]uréido, *N,N*'-di-[(1-6C)alkyl]-uréido, *N*,*N*',*N*'-tri-[(1-6C)alkyl]uréido, N-(1-6C)-alkylsulfamoyle, *N*,*N*-di-[(1-6C)alkyl]sulfamoyle, (1-6C)alcanesulfonylamino et *N*-(1-6C)alkyl-(1-6C)-alcanesulfonylamino, ou parmi un groupement de formule :
**-X³-Q³**
dans laquelle X³ est une liaison directe ou est choisi parmi O, S, SO, SO₂, N(R⁶), CO, CH(OR⁶), CON(R⁶), N(R⁶)CO, N(R⁶)CON(R⁶), SO₂N(R⁶), N(R⁶)SO₂, C(R⁶)₂O, C(R⁶)₂S et C(R⁶)₂N(R⁶), où R⁶ est hydrogène ou (1-8C)alkyle, et Q³ est aryle, aryl-(1-6C)alkyle, (3-8C)cycloalkyle, (3-8C)cycloalkyl-(1-6C)alkyle, (3-8C)cycloalcényle, (3-8C)cycloalcényl-(1-6C)-alkyle, hétéroaryle, hétéroaryl-(1-6C)alkyle, hétérocyclyle ou hétérocyclyl-(1-6C)alkyle ;
et où tout groupement aryle, (3-8C)cycloalkyle, (3-8C)cycloalcényle, hétéroaryle ou hétérocyclyle dans un substituant sur R¹ porte éventuellement 1, 2 ou 3 substituants, qui peuvent être identiques ou différents, choisis parmi halogéno, trifluorométhyle, cyano, nitro, hydroxy, amino, carboxy, carbamoyle, uréido, (1-8C)alkyle, (2-8C)alcényle, (2-8C)alcynyle, (1-6C)alcoxy, (2-6C)alcényloxy, (2-6C)alcynyloxy, (1-6C)-alkylthio, (1-6C)alkylsulfinyle, (1-6C)-alkylsulfonyle, (1-6C)alkylamino, di-[(1-6C)-alkyl]amino, (1-6C)alcoxycarbonyle, (2-6C)-alcanoyle, (2-6C)alcanoyloxy, *N*-(1-6C)alkylcarbamoyle, *N,N-*di-[(1-6C)alkyl]carbamoyle, (2-6C)alcanoylamino, *N*-(1-6C)alkyl-(2-6C)alcanoylamino, *N*-(1-6C)alkyluréido, *N*'-(1-6C)alkyluréido, *N',N*'-di-[(1-6C)alkyl]uréido, *N,N'*-di-[(1-6C)-alkyl]uréido, *N,N',N'*-tri-[(1-6C)alkyl]uréido, *N*-(1-6C)alkylsulfamoyle, *N*,*N*-di-[(1-6C)alkyl]-sulfamoyle, (1-6C)alcanesulfonylamino et *N*-(1-6C)-alkyl-(1-6C)alcanesulfonylamino, ou parmi un groupement de formule :
**-X⁴-R⁷**
dans laquelle X⁴ est une liaison directe ou est choisi parmi O et N(R⁸), où R⁸ est hydrogène ou (1-8C)alkyle, et R⁷ est halogéno-(1-6C)alkyle, hydroxy-(1-6C)alkyle, mercapto-(1-6C)alkyle, (1-6C)alcoxy-(1-6C)alkyle, (1-6C)alkylthio-(1-6C)-alkyle, cyano-(1-6C)alkyle, amino-(1-6C)alkyle, (1-6C)alkylamino-(1-6C)alkyle, di-[(1-6C)alkyl]-amino-(1-6C)alkyle, (2-6C)alcanoylamino-(1-6C)-alkyle, (1-6C)alcoxycarbonylamino-(1-6C)alkyle, *N*-(1-6C)alkyluréido-(1-6C)alkyle, *N*'-(1-6C)alkyluréido-(1-6C)alkyle, *N',N*'-di-[(1-6C)alkyl]uréido-(1-6C)alkyle, *N,N'*-di-[(1-6C)alkyl]uréido-(1-6C)-alkyle ou *N,N',N*'-tri-[(1-6C)alkyl]uréido-(1-6C)-alkyle, ou parmi un groupement de formule :
**-X⁵-Q⁴**
dans laquelle X⁵ est une liaison directe ou est choisi parmi O, CO et N(R⁹), où R⁹ est hydrogène ou (1-8C)alkyle, et Q⁴ est aryle, aryl-(1-6C)alkyle, hétéroaryle, hétéroaryl-(1-6C)alkyle, hétérocyclyle ou hétérocyclyl-(1-6C)alkyle qui porte éventuellement 1 ou 2 substituants, qui peuvent être identiques ou différents, choisis parmi halogéno, hydroxy, (1-8C)alkyle et (1-6C)alcoxy ;
et où tout groupement hétérocyclyle dans un substituant sur R¹ porte éventuellement 1 ou 2 substituants oxo ou thioxo ;
et où des atomes de carbone adjacents dans toute chaîne (2-6C)alkylène dans un substituant R¹ sont éventuellement séparés par l'insertion dans la chaîne d'un groupement choisi parmi O, S, SO, SO₂, N(R¹⁰), CO, CH(OR¹⁰), CON(R¹⁰), N(R¹⁰)CO, N(R¹⁰)CON(R¹⁰), SO₂N(R¹⁰), N(R¹⁰)SO₂, CH=CH et C≡C où R¹⁰ est hydrogène ou (1-8C) alkyle ;
**R²** est hydrogène ou (1-8C)alkyle ;
**q** est 0, 1, 2, 3 ou 4 ;
**chaque groupement R³,** qui peut être identique ou différent, est (1-8C)alkyle ou un groupement de formule :
-X⁶-R¹¹
dans laquelle X⁶ est une liaison directe ou est choisi parmi O et N(R¹²), où R¹² est hydrogène ou (1-8C)alkyle, et R¹¹ est halogéno-(1-6C)alkyle, hydroxy-(1-6C)alkyle, (1-6C)alcoxy-(1-6C)alkyle, cyano-(1-6C)alkyle, amino-(1-6C)alkyle, (1-6C)-alkylamino-(1-6C)alkyle, di-[(1-6C)alkyl]amino-(1-6C)alkyle ou (2-6C)alcanoylamino-(1-6C)alkyle ;
**r** est 0, 1 ou 2 ;
**chaque groupement R⁴,** qui peut être identique ou différent, est choisi parmi halogéno, trifluorométhyle, cyano, nitro, hydroxy, mercapto, amino, carboxy, carbamoyle, uréido, (1-8C)alkyle, (2-8C)alcényle, (2-8C)alcynyle, (1-6C)alcoxy, (1-6C)alkylthio, (1-6C)alkylsulfinyle, (1-6C)-alkylsulfonyle, (1-6C)alkylamino, di-[(1-6C)-alkyl]amino, (1-6C)alcoxycarbonyle, *N*-(1-6C)alkylcarbamoyle, *N,N*-di-[(1-6C)alkyl]carbamoyle, (2-6C)alcanoyle, (2-6C)alcanoyloxy, (2-6C)-alcanoylamino, *N*-(1-6C)alkyl-(2-6C)alcanoylamino, *N*'-(1-6C)alkyluréido, *N*',*N*'-di-[(1-6C)alkyl]-uréido, *N*-(1-6C)alkyluréido, *N*,*N'*-di-[(1-6C)-alkyl]uréido, *N*,*N'*,*N'*-tri-[(1-6C)alkyl]uréido, *N*-(1-6C)alkylsulfamoyle, *N*,*N*-di-[(1-6C)alkyl]-sulfamoyle, (1-6C)alcanesulfonylamino et N-(1-6C)-alkyl-(1-6C)alcanesulfonylamino ;
**X¹** est choisi parmi CO, N (R¹³) CO, CON (R¹³), N(R¹³)CON(R¹³), N(R¹³)COC(R¹³)₂O, N(R¹³)COC(R¹³)₂S, N(R¹³)COC(R¹³)₂N(R¹³) et N(R¹³)COC(R¹³)₂N(R¹³)CO, où R¹³ est hydrogène ou (1-8C)alkyle ; et
**Q¹** est (1-8C)alkyle, (2-8C)alcényle, (2-8C)-alcynyle, halogéno-(1-6C)alkyle, hydroxy-(1-6C)-alkyle, mercapto-(1-6C)alkyle, (1-6C)alcoxy-(1-6C)alkyle, cyano-(1-6C)alkyle, amino-(1-6C)-alkyle, (1-6C)alkylamino-(1-6C)alkyle, di-[(1-6C)-alkyl]amino-(1-6C)alkyle, (1-6C)alkylthio-(1-6C)-alkyle, (1-6C)alkylsulfinyl-(1-6C)alkyle, (1-6C)-alkylsulfonyl-(1-6C)alkyle, (2-6C)alcanoylamino-(1-6C)alkyle, *N*-(1-6C)alkyl-(2-6C)alcanoylamino-(1-6C)alkyle, (1-6C)alcoxycarbonylamino-(1-6C)-alkyle, *N*-(1-6C)alkyluréido-(1-6C)alkyle, *N'-*(1-6C)alkyluréido-(1-6C)alkyle, *N'*,*N'*-di-[(1-6C)-alkyl]uréido-(1-6C)alkyle, *N*,*N'*-di-[(1-6C)alkyl]-uréido-(1-6C)alkyle, *N*,*N'*,*N'*-tri-[(1-6C)alkyl]-uréido-(1-6C)alkyle, (1-6C)alcanesulfonylamino-(1-6C)alkyle ou *N*-(1-6C)alkyl-(1-6C)alcanesulfonylamino-(1-6C)alkyle,
ou **Q¹** est aryle, aryl-(1-6C)alkyle, (3-8C)-cycloalkyle, (3-8C)cycloalkyl-(1-6C)alkyle, (3-8C)cycloalcényle, (3-8C)cycloalcényl-(1-6C)-alkyle, hétéroaryle, hétéroaryl-(1-6C)alkyle, hétérocyclyle ou hétérocyclyl-(1-6C)alkyle ;
et où tout groupement CH, CH₂ ou CH₃ dans le groupement Q¹ porte éventuellement, sur chacun desdits groupements CH, CH₂ ou CH₃, un ou plusieurs substituants halogéno ou (1-8C)alkyle et/ou un substituant choisi parmi hydroxy, mercapto, amino, cyano, carboxy, carbamoyle, uréido, (1-6C)alcoxy, (1-6C)alkylthio, (1-6C)alkylsulfinyle, (1-6C)-alkylsulfonyle, (1-6C)alkylamino, di-[(1-6C)-alkyl]amino, (1-6C)alcoxycarbonyle, *N*-(1-6C)-alkylcarbamoyle, *N*,*N*-di-[(1-6C)alkyl]carbamoyle, (2-6C)alcanoyle, (2-6C)alcanoyloxy, (2-6C)-alcanoylamino, *N*-(1-6C)alkyl-(2-6C)alcanoylamino, *N*'-(1-6C)alkyluréido, *N*',*N*'-di-[(1-6C)alkyl]-uréido, *N*-(1-6C)alkyluréido, *N*,*N*'-di-[(1-6C)-alkyl]uréido, *N*,*N'*,*N'*-tri-[(1-6C)alkyl]uréido, *N*-(1-6C)alkylsulfamoyle, *N*,*N*-di-[(1-6C)alkyl]-sulfamoyle, (1-6C)alcanesulfonylamino et *N-*(1-6C)alkyl-(1-6C)alcanesulfonylamino ;
et où tout groupement aryle, (3-8C)cycloalkyle, (3-8C)cycloalcényle, hétéroaryle ou hétérocyclyle dans le groupement Q¹ porte éventuellement 1, 2 ou 3 substituants, qui peuvent être identiques ou différents, choisis parmi halogéno, trifluorométhyle, cyano, nitro, hydroxy, amino, carboxy, carbamoyle, uréido, (1-8C)alkyle, (2-8C)alcényle, (2-8C)alcynyle, (1-6C)alcoxy, (2-6C)alcényloxy, (2-6C)alcynyloxy, (1-6C)-alkylthio, (1-6C)alkylsulfinyle, (1-6C)alkylsulfonyle, (1-6C)alkylamino, di-[(1-6C)alkyl]-amino, (1-6C)alcoxycarbonyle, (2-6C)alcanoyle, (2-6C)alcanoyloxy, *N*-(1-6C)alkylcarbamoyle, *N,N-*di-[(1-6C)alkyl]carbamoyle, (2-6C)alcanoylamino, *N*-(1-6C)alkyl-(2-6C)alcanoylamino, *N*'-(1-6C)alkyluréido, *N*',*N*'-di-[(1-6C)alkyl]uréido, *N*-(1-6C)-alkyluréido, *N*,*N*'-di-[(1-6C)alkyl]uréido, *N,N',N'-*tri-[(1-6C)alkyl]uréido, *N*-(1-6C)alkylsulfamoyle, *N*,*N*-di-[(1-6C)alkyl]sulfamoyle, (1-6C)alcanesulfonylamino et *N*-(1-6C)alkyl-(1-6C)alcanesulfonylamino, ou parmi un groupement de formule :
-X⁷-R¹⁴
dans laquelle X⁷ est une liaison directe ou est choisi parmi O et N (R¹⁵) , où R¹⁵ est hydrogène ou (1-8C)alkyle, et R¹⁴ est halogéno-(1-6C)alkyle, hydroxy-(1-6C)alkyle, (1-6C)alcoxy-(1-6C)alkyle, cyano-(1-6C)alkyle, amino-(1-6C)alkyle, (1-6C)-alkylamino-(1-6C)alkyle ou di-[(1-6C)alkyl]amino-(1-6C)alkyle, ou parmi un groupement de formule :
-X⁸-Q⁵
dans laquelle X⁸ est une liaison directe ou est choisi parmi O, CO et N (R¹⁷), où R¹⁷ est hydrogène
ou (1-8C)alkyle, et Q⁵ est aryle, aryl-(1-6C)-alkyle, hétéroaryle, hétéroaryl-(1-6C)alkyle, hétérocyclyle ou hétérocyclyl-(1-6C)alkyle qui porte éventuellement 1 ou 2 substituants, qui peuvent être identiques ou différents, choisis parmi halogéno, hydroxy, (1-8C)alkyle et (1-6C)-alcoxy ;
et où tout groupement hétérocyclyle dans le groupement Q¹ porte éventuellement 1 ou 2 substituants oxo ou thioxo ;
et où des atomes de carbone adjacents dans toute chaîne (2-6C)alkylène dans le groupement Q¹ sont éventuellement séparés par l'insertion dans la chaîne d' un groupement choisi parmi O, S, SO, SO₂, N(R¹⁶), N(R¹⁶)CO, CON(R¹⁶), N(R¹⁶)CON(R¹⁶), CO, CH(OR¹⁶), N(R¹⁶)SO₂, SO₂N(R¹⁶), CH=CH et C≡C où R¹⁶ est hydrogène ou (1-8C)alkyle ;
ou sel, solvate ou prodrogue pharmaceutiquement acceptable de celui-ci.

2. Dérivé de pyrimidine de formule I selon la revendication 1, **caractérisé en ce que** :
p est 1 ou 2, et un premier groupement R¹ est choisi parmi hydroxy, carbamoyle, acétamido, propionamido, *N*-méthylacétamido, *N*-méthylpropionamido, hydroxyméthyle, 1-hydroxyéthyle et 1-hydroxy-1-méthyléthyle, et le deuxième groupement R¹ éventuel est choisi parmi fluoro, chloro, trifluorométhyle, cyano, hydroxy, méthyle, éthyle, méthoxy et éthoxy ;
R² est hydrogène ou méthyle ;
q est 0 ou q est 1, et le groupement R³ est méthyle ;
r est 0 ou r est 1, et le groupement R⁴ est choisi parmi fluoro, chloro, trifluorométhyle, hydroxy, amino, méthyle, méthoxy, méthylamino et diméthylamino ;
le groupement X¹-Q¹ est situé en position 3 ;
X¹ est choisi parmi NHCO, NHCONH, NHCOCH₂O, NHCOCH₂NH et NHCOCH₂NHCO ; et
Q¹ est méthyle, éthyle, propyle, butyle, pentyle, aminométhyle, 2-aminoéthyle, 2-amino-2-méthylpropyle, 3-aminopropyle, 4-aminobutyle, 5-aminopentyle, méthylaminométhyle, 2-méthylaminoéthyle, 3-méthylaminopropyle, 4-méthylaminobutyle, 5-méthylaminopentyle, diméthylaminométhyle, 2-diméthylaminoéthyle, 3-diméthylaminopropyle, 4-diméthylaminobutyle ou 5-diméthylaminopentyle, ou Q¹ est phényle, benzyle, 2-phényléthyle, cyclopentyle, cyclohexyle, cyclopentylméthyle, cyclohexylméthyle, thiényle, imidazolyle, thiazolyle, thiadiazolyle, thiénylméthyle, imidazolylméthyle, thiazolylméthyle, thiadiazolylméthyle, tétrahydrofuranyle, tétrahydropyranyle, tétrahydrothiopyranyle, pyrrolinyle, pyrrolidinyle, morpholinyle, tétrahydro-1,4-thiazinyle, pipéridinyle, homopipéridinyle, pipérazinyle, homopipérazinyle, indolinyle, isoindolinyle, pyrrolidinylméthyle, morpholinylméthyle, 2-(morpholinyl)éthyle, pipéridinylméthyle, 2-(pipéridinyl)éthyle, pipéridinyloxyméthyle, homopipéridinylméthyle, pipérazinylméthyle, 2-(pipérazinyl)éthyle, homopipérazinylméthyle ou 2-azabicyclo[2,2,1]heptylméthyle ;
et où tout groupement aryle, (3-8C)cycloalkyle, hétéroaryle ou hétérocyclyle dans le groupement Q¹ porte éventuellement un substituant choisi parmi fluoro, chloro, trifluorométhyle, hydroxy, amino, méthyle, méthoxy, méthylamino et diméthylamino, et l'un quelconque de ces groupements aryle, (3-8C)cycloalkyle, hétéroaryle ou hétérocyclyle dans le groupement Q¹ porte éventuellement un autre substituant choisi parmi aminométhyle, méthylaminométhyle et diméthylaminométhyle ;
ou sel, solvate ou prodrogue pharmaceutiquement acceptable de celui-ci.

3. Dérivé de pyrimidine de formule I selon la revendication 1, **caractérisé en ce que** :
p est 1 et le groupement R¹ est situé en position 3 ou 4 et est choisi parmi hydroxy, carbamoyle, acétamido, hydroxyméthyle, 1-hydroxyéthyle et 1-hydroxy-1-méthyléthyle ;
R² est hydrogène;
q est 0 ;
r est 0 ;
le groupement X¹-Q¹ est situé en position 3 ;
X¹ est NHCO ; et
Q¹ est méthyle, aminométhyle, 2-aminopropyle, 2-amino-2-méthylpropyle, 4-aminobutyle, 5-aminopentyle, méthylaminométhyle, diméthylaminométhyle ou 5-diméthylaminopentyle, ou Q¹ est phényle, benzyle, 2-phényléthyle, cyclopentyle, cyclohexyle, cyclohexylméthyle, thiazol-5-yle, thién-3-ylméthyle, imidazol-1-ylméthyle, 1,2,4-thiadiazol-3-ylméthyle, tétrahydropyran-4-yle, tétrahydrothiopyran-4-yle, 3-pyrrolin-2-yle, pyrrolidin-2-yle, pyrrolidin-3-yle, morpholin-2-yle, pipéridin-2-yle, pipéridin-3-yle, pipéridin-4-yle, pipérazin-1-yle, isoindolin-1-yle, pyrrolidin-2-ylméthyle, pipéridin-4-ylméthyle, 2-(pipéridin-4-yl)éthyle, pipéridin-4-yloxyméthyle, pipérazin-1-ylméthyle ou 2-azabicyclo[2,2,1]hept-2-ylméthyle ;
et où tout groupement aryle, (3-8C)cycloalkyle, hétéroaryle ou hétérocyclyle dans le groupement Q¹ porte éventuellement un substituant choisi parmi amino, méthyle, méthylamino et aminométhyle ;
ou sel, solvate ou prodrogue pharmaceutiquement acceptable de celui-ci.

4. Dérivé de pyrimidine de formule I selon la revendication 1, **caractérisé en ce que** :
p est 1 et le groupement R¹ est situé en position 3 ou 4 et est choisi parmi hydroxy, acétamido, hydroxyméthyle, 1-hydroxyéthyle et 1-hydroxy-1-méthyléthyle ;
R² est hydrogène ;
q est 0 ;
r est 0 ou r est 1, et le groupement R⁴ est choisi parmi fluoro, chloro et méthyle ;
le groupement X¹-Q¹ est situé en position 3 ou 4 ;
X¹ est NHCO, N (Me) CO, CONH ou CON (Me) ; et
Q¹ est méthyle, éthyle, propyle, isopropyle, 2-éthoxyéthyle, 3-éthoxypropyle, cyanométhyle, 2-cyanoéthyle, aminométhyle, 2-aminoéthyle, méthylaminométhyle, 2-méthylaminoéthyle, éthyl-aminométhyle, 2-éthylaminoéthyle, diméthylaminométhyle, 2-diméthylaminoéthyle, 4-diméthylaminobutyle, 2-méthylsulfonyléthyle ou acétamido-méthyle, ou Q¹ est phényle, benzyle, cyclopropyle, cyclobutyle, cyclopentyle, cyclohexyle, cyclopropylméthyle, cyclobutylméthyle, cyclopentylméthyle, cyclohexylméthyle, oxazol-5-yle, isoxazol-3-yle, isoxazol-4-yle, imidazol-2-yle, imidazol-4-yle, pyrazol-3-yle, thiazol-5-yle, 1,2,3-triazol-5-yle, tétrazol-5-yle, pyridin-2-yle, pyridin-3-yle, pyridin-4-yle, pyrazin-2-yle, pyridazin-4-yle, pyrimidin-2-yle, pyrimidin-4-yle, thién-3-ylméthyle, oxazol-4-ylméthyle, isoxazol-3-ylméthyle, isoxazol-4-ylméthyle, imidazol-1-yl-méthyle, imidazol-2-ylméthyle, 2-imidazol-1-yléthyle, 2-imidazol-2-yléthyle, 2-imidazol-4-yléthyle, pyrazol-1-ylméthyle, pyrazol-3-ylméthyle, 1,2,3-triazol-1-ylméthyle, 1,2,3-triazol-4-ylméthyle, 1,2,4-oxadiazol-3-ylméthyle, 1,2,3-thiadiazol-3-ylméthyle, tétrazol-1-yl-méthyle, tétrazol-5-ylméthyle, pyridin-2-yl-méthyle, pyridin-3-ylméthyle, pyridin-4-ylméthyle, 2-pyridin-2-yléthyle, 2-pyridin-3-yléthyle, 2-pyridin-4-yléthyle, pyrazin-2-ylméthyle, 2-pyrazin-2-yléthyle, pyridazin-4-ylméthyle, 2-pyridazin-4-yléthyle, pyrimidin-2-ylméthyle, pyrimidin-4-ylméthyle, 2-pyrimidin-2-yléthyle, 2-pyrimidin-4-yléthyle, tétrahydrofuran-2-yle, tétrahydropyran-4-yle, tétrahydrothiopyran-4-yle, azétidin-2-yle, 3-pyrrolin-2-yle, pyrrolidin-1-yle, pyrrolidin-2-yle, pyrrolidin-3-yle, morpholino, morpholin-2-yle, pipéridino, pipéridin-2-yle, pipéridin-3-yle, pipéridin-4-yle, pipérazin-1-yle, isoindolin-1-yle, tétrahydro-furan-2-ylméthyle, tétrahydropyran-4-ylméthyle, 1,3-dioxolan-2-ylméthyle, 1,4-dioxan-2-ylméthyle, pyrrolidin-2-ylméthyle, pipéridin-2-ylméthyle, pipéridin-3-ylméthyle, pipéridin-4-ylméthyle, 2-(pipéridin-4-yl)éthyle, pipéridin-4-yloxy-méthyle, pipérazin-1-ylméthyle ou 2-(pipérazin-1-yl)éthyle ;
et où tout groupement CH, CH₂ ou CH₃ dans le groupement Q¹ porte éventuellement, sur chacun desdits groupements CH, CH₂ ou CH₃, un substituant choisi parmi hydroxy, carbamoyle, méthoxycarbonyle, éthoxycarbonyle, *N*-méthylcarbamoyle, *N*-éthylcarbamoyle, *N*-isopropylcarbamoyle, *N,N-*diméthylcarbamoyle, acétyle, propionyle, pivaloyle, acétamido et *N*-méthylacétamido ;
et où tout groupement aryle, (3-8C)cycloalkyle, hétéroaryle ou hétérocyclyle dans le groupement Q¹ porte éventuellement 1 ou 2 substituants, qui peuvent être identiques ou différents, choisis parmi fluoro, chloro, hydroxy, amino, carbamoyle, méthyle, méthylamino, diméthylamino, hydroxyméthyle, méthoxyméthyle, cyanométhyle, aminométhyle, méthylaminométhyle, diméthylaminométhyle et 1-méthylpipéridin-4-ylméthyle ;
ou sel, solvate ou prodrogue pharmaceutiquement acceptable de celui-ci.

5. Dérivé de pyrimidine de formule I selon la revendication 1, **caractérisé en ce que** :
p est 1 et R¹ est un groupement hydroxy ou hydroxyméthyle qui est situé en position 3 ;
R² est hydrogène ;
q est 0 ;
r est 0 ou r est 1, et le groupement R⁴ est choisi parmi fluoro et méthyle ;
le groupement X¹-Q¹ est situé en position 3 ou 4 ;
X¹ est NHCO ou N (Me) CO ; et
Q¹ est aminométhyle, méthylaminométhyle, éthylaminométhyle, diméthylaminométhyle, acétamidométhyle, 3-aminométhylphényle, 4-aminométhylphényle, 5-méthylisoxazol-3-yle, 1-méthylpyrazol-3-yle, 1*H*-1,2,3-triazol-5-yle, pyridin-4-yle, pyrazin-2-yle, 2-imidazol-1-yléthyle, 2-imidazol-2-yléthyle, 3,5-diméthyl-1*H*-pyrazol-1-ylméthyle, 1*H*-tétrazol-5-ylméthyle, 2-pyridin-3-yléthyle, 2-pyridazin-4-yléthyle, azétidin-2-yle, 3-pyrrolin-2-yle, *N*-méthylpyrrolidin-2-yle, 4-hydroxypyrrolidin-2-yle, pipéridin-3-yle, pipéridin-4-yle, *N*-méthylpipéridin-4-yle, pipérazin-1-yle, pipéridin-3-ylméthyle, pipéridin-4-yloxyméthyle ou pipérazin-1-ylméthyle ;
ou sel, solvate ou prodrogue pharmaceutiquement acceptable de celui-ci.

6. Dérivé de pyrimidine de formule I selon la revendication 1, **caractérisé en ce que**:
p est 1 et R¹ est un groupement hydroxy ou hydroxyméthyle qui est situé en position 3 ;
R² est hydrogène ;
q est 0 ;
r est 0 ou r est 1, et le groupement R⁴ est choisi parmi fluoro et méthyle ;
le groupement X¹-Q¹ est situé en position 3 ou 4 ;
X¹ est CONH ou CON(Me) ; et
Q¹ est méthyle, éthyle, propyle, isopropyle, 2-éthoxyéthyle, 3-éthoxypropyle, cyanométhyle, 1-cyano-1-méthyléthyle, 2-cyanoéthyle, 5-cyanopentyle, 2-aminoéthyle, 2-méthylaminoéthyle, 2-diméthylaminoéthyle, 4-diméthylaminobutyle, 2-méthylsulfonyléthyle, 3-méthoxycarbonylpropyle, carbamoylméthyle, 1-carbamoyléthyle, 2-carbamoyléthyle, *N*-méthylcarbamoylméthyle, *N*-isopropylcarbamoylméthyle, *N*,*N*-diméthylcarbamoylméthyle, pivaloylméthyle, 4-aminométhylphényle, 4-aminobenzyle, cyclopropyle, cyclobutyle, cyclopentyle, cyclopropylméthyle, cyclobutylméthyle, cyclopentylméthyle, thién-3-ylméthyle, oxazol-4-yl-méthyle, 5-méthylisoxazol-3-ylméthyle, isoxazol-4-ylméthyle, 1*H*-imidazol-1-ylméthyle, 1*H*-imidazol-2-ylméthyle, 2-(1*H*-imidazol-1-yl)éthyle, 2-(1*H-*imidazol-2-yl)éthyle, 2-(1*H*-imidazol-4-yl)éthyle, pyridin-2-ylméthyle, pyridin-3-ylméthyle, pyridin-4-ylméthyle, 2-pyridin-2-yléthyle, 2-pyridin-3-yl-éthyle, 2-pyridin-4-yléthyle, pyrazin-2-ylméthyle, 5-méthylpyrazin-2-ylméthyle, tétrahydropyran-4-yle, tétrahydrothiopyran-4-yle, tétrahydrofuran-2-ylméthyle, tétrahydropyran-4-ylméthyle, 1,3-di-oxolan-2-ylméthyle ou 1,4-dioxan-2-ylméthyle ;
ou sel, solvate ou prodrogue pharmaceutiquement acceptable de celui-ci.

7. Dérivé de pyrimidine de formule I selon la revendication 1, **caractérisé en ce que** :
p est 1 et R¹ est un groupement hydroxy ou hydroxyméthyle qui est situé en position 3 ;
R² est hydrogène ;
q est 0 ;
r est 0 ;
le groupement X¹-Q¹ est situé en position 3 ;
X¹ est NHCO ; et
Q¹ est 2-amino-2-méthylpropyle, 5-aminopentyle, 3-aminométhylphényle, 4-aminométhylphényle, 2-aminocyclopent-1-yle, 4-aminocyclohex-1-yle, 3-aminocyclohex-1-ylméthyle, pipéridin-3-yle, pipéridin-4-yle, pipéridin-4-ylméthyle ou pipéridin-4-yloxyméthyle ;
ou sel, solvate ou prodrogue pharmaceutiquement acceptable de celui-ci.

8. Dérivé de pyrimidine de formule I selon la revendication 1, choisi parmi :
la 4-(3-hydroxyphényl)-6-morpholino-2-(3-pipéridin-4-ylcarbonylaminophényl)pyrimidine,
la 2-[3-(6-diméthylaminohexanoylamino)phényl]-4-(3-hydroxyméthylphényl)-6-morpholinopyrimidine,
la 2-(3-{2-[(1R,3S)-3-aminocyclohex-1-yl]-acétamido]phényl}-4-(3-hydroxyméthylphényl)-6-morpholinopyrimidine,
la 4-(3-hydroxyméthylphényl)-6-morpholino-2-[3-(2-pipéridin-4-yloxyacétamido)phényl]pyrimidine,
la 2-[3-(3-aminométhylbenzamido)phényl]-4-(3-hydroxyméthylphényl)-6-morpholinopyrimidine,
la 4-(3-hydroxyméthylphényl)-6-morpholino-2-(3-pipéridin-4-ylcarbonylaminophényl)pyrimidine et
la 4-(3-hydroxyméthylphényl)-6-morpholino-2-(3-pipérazin-1-ylcarbonylaminophényl)pyrimidine ;
ou sel, solvate ou prodrogue pharmaceutiquement acceptable de celui-ci.

9. Procédé de préparation d'un dérivé de pyrimidine de formule I, ou d'un sel, d'un solvate ou d'une prodrogue pharmaceutiquement acceptable de celui-ci, selon la revendication 1, qui comprend :
(a) la réaction d'une pyrimidine de formule II 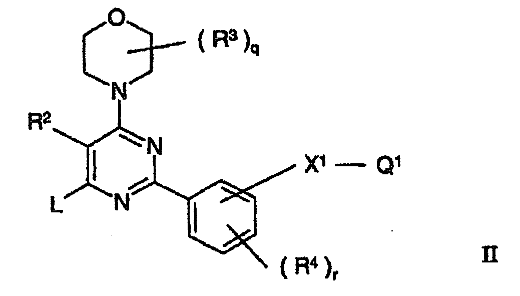 dans laquelle L est un groupement déplaçable et R², q, R³, r, R⁴, X¹ et Q¹ ont l'une quelconque des significations précisées dans la revendication 1, sauf que toute fonction est protégée si nécessaire, avec un réactif organoboré de formule III 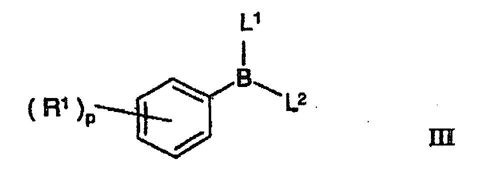 dans laquelle chacun des L¹ et L² , qui peuvent être identiques ou différents, est un ligand approprié, et p et R¹ ont l'une quelconque des significations précisées dans la revendication 1, sauf que toute fonction est protégée si nécessaire, puis tout groupement protecteur qui est présent est éliminé ;
(b) pour la production de ces composés de formule I dans laquelle X¹ est N (R¹³) CO, l'acylation d'une amine de formule IV 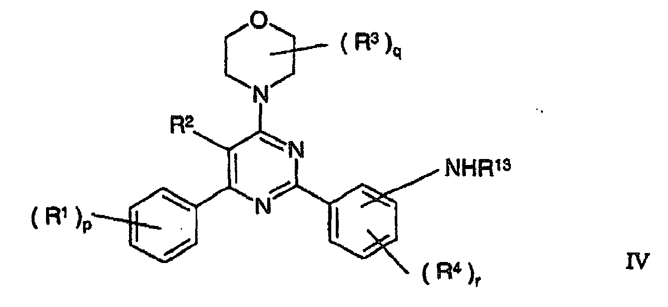 dans laquelle p, R¹, R², q, R³, r, R⁴ et R¹³ ont l'une quelconque des significations précisées dans la revendication 1, sauf que toute fonction est protégée si nécessaire, avec un acide carboxylique de formule V
**HO₂C-Q**¹ V
ou un dérivé réactif de celui-ci, dans laquelle Q¹ a l'une quelconque des significations précisées dans la revendication 1, sauf que toute fonction est protégée si nécessaire, puis tout groupement protecteur qui est présent est éliminé ;
(c) la réaction d'une pyrimidine de formule VI 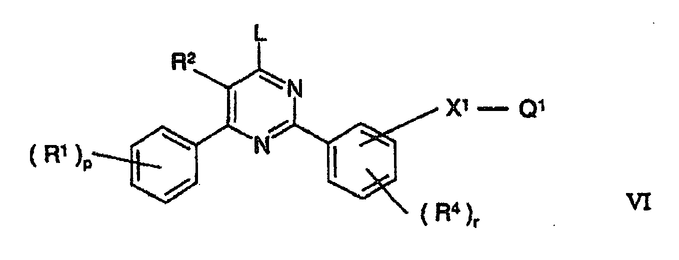 dans laquelle L est un groupement déplaçable et p, R¹, R², r, R⁴, X¹ et Q¹ ont l'une quelconque des significations précisées dans la revendication 1, sauf que toute fonction est protégée si nécessaire, avec une morpholine de formule VII 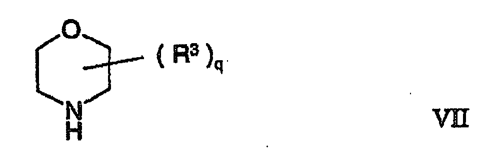 dans laquelle q et R³ ont l'une quelconque des significations précisées dans la revendication 1, sauf que toute fonction est protégée si nécessaire, puis tout groupement protecteur qui est présent est éliminé;
(d) pour la production de ces composés de formule I dans laquelle X¹ est N (R¹³) CON (R¹³), le couplage de phosgène, ou d'un équivalent chimique de celui-ci, avec une amine de formule IV 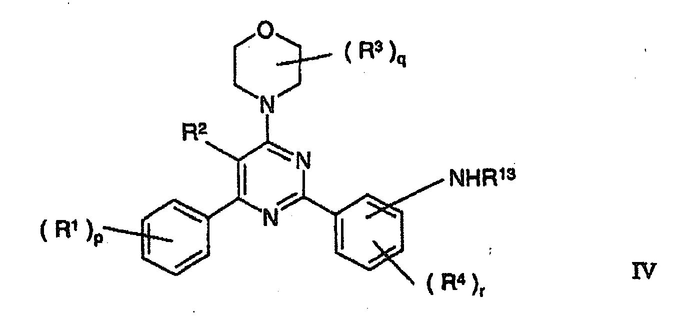 et une amine de formule VIII
**R¹³NH^{_}Q¹** VIII
dans lesquelles p, R¹, R², q, R³, r, R⁴ R¹³ et Q¹ ont l'une quelconque des significations précisées dans la revendication 1, sauf que toute fonction est protégée si nécessaire, puis tout groupement protecteur qui est présent est éliminé;
(e) la réaction d'une pyrimidine de formule XIV 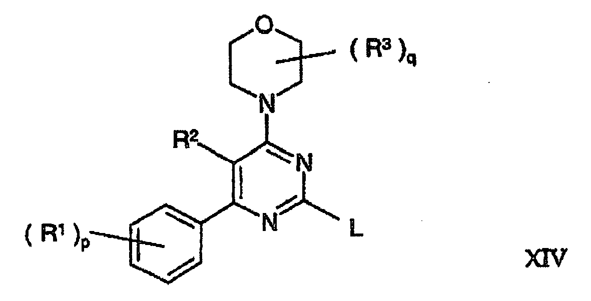 dans laquelle L est un groupement déplaçable et p, R¹, R², q et R³ ont l'une quelconque des significations précisées dans la revendication 1, sauf que toute fonction est protégée si nécessaire, avec un réactif organoboré de formule XV 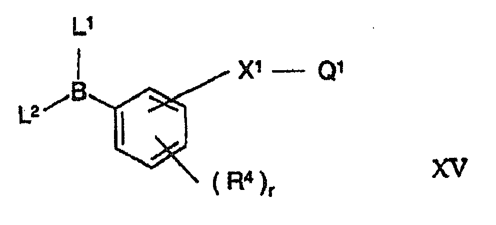 dans laquelle chacun des L¹ et L², qui peuvent être identiques ou différents, est un ligand approprié, et r, R⁴, X¹ et Q¹ ont l'une quelconque des significations précisées dans la revendication 1, sauf que toute fonction est protégée si nécessaire, puis tout groupement protecteur qui est présent est éliminé ;
(f) pour la production de ces composés de formule I dans laquelle X¹ est CON (R¹³), l'acylation d'une amine de formule VIII
**R¹³NH-Q¹** VIII
dans laquelle R¹³ et Q¹ ont l'une quelconque des significations précisées dans la revendication 1, sauf que toute fonction est protégée si nécessaire, avec un acide carboxylique, ou un dérivé réactif de celui-ci, de formule XVI 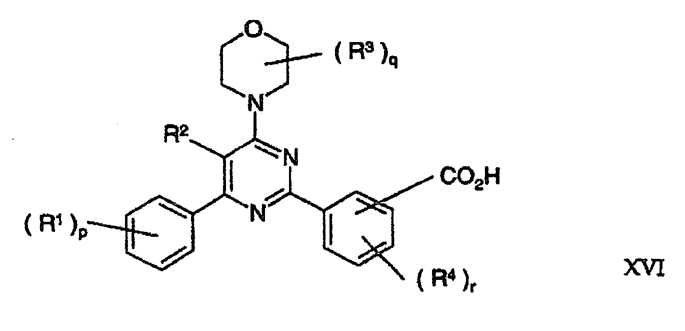 dans laquelle p, R¹, R², q, R³, r et R⁴ ont l'une quelconque des significations précisées dans la revendication 1, sauf que toute fonction est protégée si nécessaire, puis tout groupement protecteur qui est présent est éliminé ; ou
(g) pour la production de ces composés de formule I dans laquelle X¹ est CO et Q¹ est un groupement hétérocyclyle lié par *N*, l'acylation d'un composé hétérocyclique contenant *N* dans lequel toute fonction est protégée si nécessaire, avec un acide carboxylique, ou un dérivé réactif de celui-ci de formule XVI 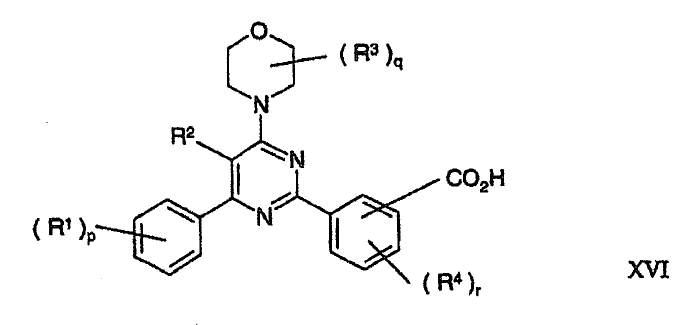
dans laquelle p, R¹, R², q, R³, r et R⁴ ont l' une quelconque des significations précisées dans la revendication 1, sauf que toute fonction est protégée si nécessaire, puis tout groupement protecteur qui est présent est éliminé ;
et lorsqu'un sel pharmaceutiquement acceptable d'un dérivé de pyrimidine de formule I est requis, il peut être obtenu par réaction dudit dérivé de pyrimidine avec un acide approprié ;
et lorsqu'une prodrogue pharmaceutiquement acceptable d'un dérivé de pyrimidine de formule I est requise, elle peut être obtenue en utilisant un mode opératoire classique.

10. Composition pharmaceutique qui comprend un dérivé de pyrimidine de formule I, ou un sel, un solvate ou une prodrogue pharmaceutiquement acceptable de celui-ci, selon la revendication 1, en association avec un diluant ou un support pharmaceutiquement acceptable.

11. Utilisation d'un dérivé de pyrimidine de formule I, ou d'un sel, d'un solvate ou d'une prodrogue pharmaceutiquement acceptable de celui-ci, selon la revendication 1, dans la fabrication d'un médicament destiné à être utilisé dans la production d'un effet antiprolifératif chez un animal à sang chaud, tel que l'homme.
